(19) Europäisches Patentamt
European Patent Office
Office européen des brevets

(11) EP 2 406 728 B1

(12) **EUROPEAN PATENT SPECIFICATION**

(45) Date of publication and mention of the grant of the patent:
**16.08.2017 Bulletin 2017/33**

(21) Application number: **10751101.6**

(22) Date of filing: **10.03.2010**

(51) Int Cl.:
***G01N 33/574*** *(2006.01)* ***G06F 19/20*** *(2011.01)*
***G06F 19/24*** *(2011.01)*

(86) International application number:
**PCT/SG2010/000078**

(87) International publication number:
**WO 2010/104472 (16.09.2010 Gazette 2010/37)**

(54) **A METHOD FOR IDENTIFICATION, PREDICTION AND PROGNOSIS OF CANCER AGGRESSIVENESS**

VERFAHREN ZUR IDENTIFIZIERUNG, VORHERSAGE UND PROGNOSE VON TUMORAGGRESSIVITÄT

PROCÉDÉ D'IDENTIFICATION, DE PRÉDICTION ET DE PRONOSTIC DE L'AGRESSIVITÉ D'UN CANCER

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.03.2009 SG 200901682**

(43) Date of publication of application:
**18.01.2012 Bulletin 2012/03**

(73) Proprietor: **Agency for Science, Technology and Research**
**Singapore 138632 (SG)**

(72) Inventors:
- **KUZNETSOV, Vladimir, A.**
  **Singapore 138671 (SG)**
- **MOTAKIS, Efthimios**
  **Singapore 138671 (SG)**

(74) Representative: **Bailey, Jennifer Ann et al**
**Marks & Clerk LLP**
**Alpha Tower**
**Suffolk Street Queensway**
**Birmingham B1 1TT (GB)**

(56) References cited:
- **P. BROET ET AL: "Identifying gene expression changes in breast cancer that distinguish early and late relapse among uncured patients", BIOINFORMATICS, vol. 22, no. 12, 15 June 2006 (2006-06-15) , pages 1477-1485, XP055064274, ISSN: 1367-4803, DOI: 10.1093/bioinformatics/btl110**
- **MOTAKIS E ET AL: "Identification of essential genes and gene pairs associated with survival time of cancer patients", PROCEEDINGS OF THE 2007 INTERNATIONAL CONFERENCE ON BIOINFORMATICS & COMPUTATIONAL BIOLOGY, BIOCOMP 2007 : [AT] WORLDCOMP'07, JUNE 25 - 28, 2007, LAS VEGAS, NEVADA, USA, CSREA PRESS, vol. 2, 1 January 2007 (2007-01-01), pages 753-759, XP009169912, ISBN: 1-60132-040-X**
- **MOTAKIS E ET AL: "Data-driven approach to predict survival of cancer patients", IEEE ENGINEERING IN MEDICINE AND BIOLOGY MAGAZINE, IEEE SERVICE CENTER, PISACATAWAY, NJ, US, vol. 28, no. 4, 1 July 2009 (2009-07-01), pages 58-66, XP011294616, ISSN: 0739-5175**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KUZNETSOV V A ET AL: "Low and high-agressive genetic breast cancer subtypes and significant survival gene signatures", NEURAL NETWORKS, 2008. IJCNN 2008. (IEEE WORLD CONGRESS ON COMPUTATIONAL INTELLIGENCE). IEEE INTERNATIONAL JOINT CONFERENCE ON, IEEE, PISCATAWAY, NJ, USA, 1 June 2008 (2008-06-01), pages 4151-4156, XP031328132, ISBN: 978-1-4244-1820-6
- GOETZ ET AL.: 'A Two-Gene Expression Ratio of Homeobox 13 and Interleukin-17B Receptor for Prediction of Recurrence and Survival in Women Receiving Adjuvant Tamoxifen' CLINICAL CANCER RESEARCH, [Online] vol. 12, no. 7, 01 April 2006, pages 2080 - 2087, XP002572497 Retrieved from the Internet: <URL:http://clincancerres.aacrjournals.org/content/12/7/2080.full.pdf> [retrieved on 2010-05-26]
- BHATTACHARJEE: 'Estimation in Hazard Regression Models under Ordered Departures from Proportionality', [Online] July 2003, XP004594362 Retrieved from the Internet: <URL:http://www.dspace.cam.ac.uk/bitstream/1810/369/1/cwpe0337.pdf> [retrieved on 2010-05-27]
- LI ET AL.: 'Failure event prediction using the Cox proportional hazard model driven by frequent failure signatures' IIE TRANSACTIONS, [Online] vol. 39, 2007, pages 303 - 315, XP008154634 Retrieved from the Internet: <URL:http://citeseerx.ist.psu.edu/viewdoc/download?doi=10.1.1.97.8549&rep=rep1&ctype=p d f> [retrieved on 2010-05-27]
- VAN DE VIJVER, M. J. ET AL.: 'A Gene-Expression Signature as a Predictor of Survival in Breast Cancer' THE NEW ENGLAND JOURNAL OF MEDICINE, [Online] vol. 347, no. 25, 19 December 2002, pages 1999 - 2009, XP008032093 Retrieved from the Internet: <URL:http://www.endo.gr/cgi/reprint/347/25/1999.pdf> [retrieved on 2010-04-12]

## Description

Related applications

The present application is related to US application 61/158,948 which was filed on 10 March 2009, and to Singapore patent application 200901682-5 from which it claims priority.

Field of the Invention

The present invention relates to identification of pairs of genes for which the respective gene expression values in a subject are statistically significant in relation to a medical condition, for example cancer, or more particularly breast cancer. The gene expression values may for example be indicative of the susceptibility of the subject to the medical condition, or the prognosis of a subject who exhibits the medical condition. The invention further relates to methods employing the identified gene pairs.

Background of the invention

Global gene expression profiles of subjects are often used to obtain information about those subjects, such as their susceptibility to a certain medical condition, or, in the case of subjects exhibiting medical conditions, their prognosis. For example, having determined that a particular gene is important, the level in which that gene is expressed in a subject can be used to classify the individual into one of a plurality of classes, each class being associated with a different susceptibility or prognosis. The class comparison analysis leads to a better understanding of the disease process by identifying gene expression in primary tumours associated with subject survival outcomes (Kuznetsov et al., 2006).

Currently available clinical prediction and prognostic scores, such as the follicular lymphoma international prognostic index (LeBrun et al, 2008), or breast cancer aggressiveness scores (the St. Gallen consensus criterion or the Nottingham grading score (Ivshina et al, 2006)), are not able to optimally predict cancer aggressive dynamics or poor prognosis. On the other hand, genome-wide sequencing and expression technologies provide an enormous amount of information about genome and gene expression patterns associated with ethnology and pathogenesis of diseases, including cancers and other dangerous pathological process. However, these datasets provided by high-throughput technologies are still very noisy and biased so that selection of biologically meaningful and clinically essential genes and their products is imperative in further progress of diagnosis, prediction, prognosis and assignment of individual optimal therapy.

Furthermore, a given disease may exhibit many known and unknown genotype variations, which must be reliably defined on structural and functional levels. To this extent the task is twofold: first, the identification of reproducible, clinically-essential genetic (and phenotypic) sub-types of pathological cell types, and second the simultaneous optimal selection of the biologically meaningful genes. It is desirable to select genes (and or other markers) that correlate with survival time of the patients and provide in their combinations the best partition of the patients of relatively homogeneous distinct sub-groups of biological importance.

A gene and its products (mRNA transcripts, proteins) could be co-regulated and involved in many regulatory circuits, pathways and cellular programs which are very complex and poorly understood. However, several studies involving group analysis of expression profiles suggest that expression ratios of some gene pairs might be relatively reproducible indicators of the pathological process and, at the optimal thresholds for patient partition on to sub-groups, could be reliable prognostic indicators (Cui and Churchill, 2003; Motakis et al, 2008).

In some works, the combinations of individual gene pairs have been successfully used for diagnostics and prognosis (van't Veer, Sorlle et al, 2006; Ivshina et al, 2006). For example, In the work (LeBrun et al, 2008) the authors carried out so-called predictive interaction analysis (PIA) to examine whether any of the gene pairs generated from the very limited number of pres-selected genes (300 single genes) of follicular lymphoma were able to discriminate the 5-year outcomes more reliably than either single gene of the pair. Only gene pairs with both stringent and principal p-value gains of 10 times that of their respective single genes models were considered for further analysis. Of the 303 gene pairs that passed both criteria, the authors observed only 15 repeated gene pairs due to redundant features or genes represented by multiple probes on the array. The survival model in this and many other studies has been used only for validation of selected gene pairs. It was found that a high HOXB13/IL-17BR expression ratio is associated with increased relapse and death in patients with resected node-negative, ER-positive breast cancer treated with tamoxifen (Goetz et al, 2006). This case study showed that appropriate gene pairs may identify patients in whom alternative therapies should be studied.

However, several key questions remained outstanding:

(i) how to use survival analysis method(s) to select clinically synergistic gene pairs associated with a "hidden" subgroup of patients within a cohort, that is a subgroup of patients such that the pathological process indicates particular aggressiveness;
(ii) how to select the most appropriate statistical model of multivariate survival analysis which allows us to select the "survival significant" genes and synergistic gene pairs;
(iii) how, given that statistical model, to select the most "survival significant" genes (or other available variables) and biologically meaningful gene pairs among thousand of genes, and millions of gene pair combinations; and
(iv) how to define the noise background threshold(s) of the gene expression level ratios, which reliably reflect clinical heterogeneity even if the cohorts of individuals in our dataset include statistical imbalances of the patient groups.

We now present the background theory of survival analysis in more mathematical terms:

1. Analytical Method

First we will describe briefly the background theory of survival analysis. We denote by T the patient's *survival time. T* is a continuous non-negative random variable which can take values *t*, $t \in [0, \infty)$. T has density function *f(t)* and cumulative distribution function $F(t) = P(T \leq t)$. $F(t) = \int_0^t f(t')dt'$. We are primarily interested in estimating two quantities:

The survival function:

$$S(t) = P(T > t) = 1 - F(t)$$

The hazard function:

$$h(t) = \frac{f(t)}{S(t)} = \lim_{\Delta t \to 0} \frac{P(t \leq T < (t + \Delta t) | T \geq t)}{\Delta t}$$

The survival function expresses the probability of a patient to be alive at time *t*. It is often presented in the form $S(t) = \exp(-H(t))$, where $H(t) = \int_0^t h(u)du$ denotes the cumulative hazard. The hazard function assesses the instantaneous risk of death at time t, conditional on survival up to that time.
Notice that the hazard function is expressed in terms of the survival function. To this extent, survival distributions and hazard functions can be generated for any distribution defined for $t \in [0, \infty)$. By considering a random variable W, distributed in $(\infty,-\infty)$, we can generate a family of survival distributions by introducing location ($\alpha$) and scale ($\sigma$) changes of the form $\log T = \alpha + \sigma W$.
Alternatively, we can express the relationship of the survival distribution to covariates by means of a parametric model. The parametric model employs a "regressor" variable x. Take for example a model based on the exponential distribution and write: $\log(h(t)) = \alpha + \beta x$, or equivalently, $h(t) = \exp(\alpha + \beta x)$.
This is a linear model for the log-hazard, or, equivalently, a multiplicative model for the hazard. The constant $\alpha$ represents the log-baseline hazard (the hazard when the regressor $x = 0$) and the slope parameter $\beta$ gives the change in hazard rate as *x* varies. This is an easy example of how survival models can be obtained from simple distributional assumptions. In the next paragraphs we will see more specific examples.

2. The Cox Proportional hazards model

One of the most popular survival models is the Cox proportional hazards model (Cox, 1972):

$$\log \ h(t) = \alpha(t) + \beta x$$

where, as before, *t* is the survival time, *h*(*t*) represents the hazard function, $\alpha(t)$ is the baseline hazard, $\beta$ is the slope

parameter of the model and $x$ is the regressor. The popularity of this model lies in the fact that it leaves the baseline hazard function $\alpha(t)$ (which we may alternatively designate as log $h_0(t)$) unspecified (no distribution assumed). It can be estimated iteratively by the method of partial likelihood of Cox (1972). The Cox proportional hazards model is semi-parametric because while the baseline hazard can take any form, the covariates enter the model linearly.

Cox (1972) showed that the $\beta$ coefficient can be estimated efficiently by the Cox partial likelihood function. Suppose that for each of a plurality of $K$ subjects (labelled by $k$=1,...$k$), we observe at corresponding time $t_k$ a certain nominal (i.e. yes/no) clinical event has occurred (e.g. whether there has been metastasis). This knowledge is denoted $e_k$. For example $e_k$ may be 0 if the event has not occurred by time $t_k$ (e.g. no tumour metastasis at time $t_k$) and 1 if the event has occurred (e.g. tumour metastasis at time $t_k$). Cox (1972) showed that the $\beta$ coefficient can be estimated efficiently by the Cox partial likelihood function, estimated as:

$$L(\beta_i)=\prod_{k=1}^{K}\left\{\frac{\exp(\beta\ x_k)}{\sum_{j\in R(t_k)}\exp(\beta\ x_j)}\right\}^{e_k}$$

where $R(t_k) = \{j: t_j \geq t_k\}$ is the risk set at time $t_k$.

3. Application of Cox Proportional Hazards model to expression level data

Suppose that, for a microarray experiment with $i$ = 1, 2, ..., $N$ genes, the intensities are measured for $k$ = 1, 2, ...., $K$ subjects. The measure of the intensity of gene $i$ (i.e. the expression value of gene $i$) in subject $k$ may be denoted as $y_{i,k}$. This may be a direct expression value measurement, or some transformed value derived from it such as a logarithm of an expression value measurement. Denote the chance of survival of a given individual as $h_k(t)$, then the Cox proportional-hazards model is that:

$$\log\ h_k=\alpha(t)+\sum_{i=1}^{N}\beta_i y_{i,k}$$

where $\alpha(t)$ is unspecified and $\beta_i$, $i$=1,...$N$, is a set of $N$ parameters. Each $\beta_i$ is a measure of the significance of the corresponding gene $i$. The set of $N$ values $\beta_i$ can be considered as a 1 × $N$ vector of regression parameters denoted by $\beta$.

It is known to divide the group of patients into "high-risk" and "low risk" groups using a set of parameters $\{c^i\}$ according to whether:

$$x_k^i=\begin{cases}1\ (high\ \ risk),\ if\ \ y_{i,k}>c^i\\ 0\ \ (low\ \ risk), if\ \ y_{i,k}\geq c^i\end{cases}$$

The values $\{c^i\}$ may be referred to as "cut-off expression values".

In this case, the Cox proportional hazard regression model becomes (Cox, 1984):

$$\log h^i{}_k(t_k\,|\,x_k^i,\beta_i)=\alpha_i(t_k)+\beta_i\cdot x_k^i\ ,$$

where $h^j_k$ is the hazard function and $\alpha_i(t_k)$ represents the unspecified log-baseline hazard function; $\beta$ is the 1 × $N$ regression parameters vector with components $\beta_i$; and $t_k$ is the subjects' survival time.

A Wald statistic, corresponding to a partial likelihood for each of the $\beta_i$, may estimated for each gene $i$ as:

$$L(\beta_i)=\prod_{k=1}^{K}\left\{\frac{\exp(\beta_i^T x_k^i)}{\sum_{j\in R(t_k)}\exp(\beta_i^T x_j^i)}\right\}^{e_k}$$

where $R(t_k) = \{j: t_j \geq t_k\}$ is the risk set at time $t_k$.

4. Mean-Based Grouping (MBg)

One specific example of this procedure is Mean-based grouping (MBg). The following procedure is performed successively for $i$ = 1, 2, ..., $N$.

1. For $i$=1, estimate the mean expression signal $\mu i$ and then group the $k$ = 1, 2, ..., $K$ patients according to

$$x_k^i = \begin{cases} 1 \ (high \ \ risk), \ \ if \ \ y_{i,k} > c^i \\ 0 \ \ (low \ \ risk), if \ \ y_{i,k} \geq c^i \end{cases}$$

with $c^i = \mu_i$.

2. Evaluate the prognostic significance of gene $i$ by reporting the p-value of the estimated $\beta_i$ from the model

$$\log h^i{}_k(t_k \mid x_k^i, \beta_i) = \alpha_i(t_k) + \beta_i \cdot x_k^i$$

3. Perform steps 1 and 2 successively for all genes $i$ = 2, ..., $N$.

4. Select as prognostic significant the genes with significantly small p-values ($p < \alpha = 1$ %). Here $\alpha$ is the significance level of the test for each gene $i$: gene $i$ is regarded as significant if $p$ is below $\alpha$.

5. Alternatives to a Cox proportional Hazards model

Table 1 lists a number of known parametric survival models which are alternatives to the Cox proportional Hazards model. The survival function $S(t) = P(T > t)$ defines the probability of an individual to survive after time $t$.

Each of the survival models employ one or more parameters $\lambda, \gamma, \mu, \sigma$ which are fixed, in the sense that these values are selected so as to fit the survival model to a given data-set, such that the survival function then varies only with the regressor.

The first four survival models in Table 1 make a proportional hazards assumption. The last two do not. Note that the Hazard functions for the six survival models are what we will call here "structurally different". In other words, they differ not only in the values which certain fixed parameters take, but in the form of the mathematical function. In other words, the survival models cannot be transformed from one into another by a revaluing of any of the parameters.

Table 1

| Survival Model (distribution of $t$) | Survival Function $S(t)$ | Hazard function $h(t)$ | Proportional hazards assumption |
|---|---|---|---|
| Exponential | $S(t) = e^{-\lambda t}$ | $h(t) = \lambda$ | YES |
| Weibull | $S(t) = e^{(-\lambda t)^{\gamma}}$ | $h(t) = \lambda y(\lambda t)^{\gamma}$ | YES |
| Gaussian | $S(t) = 1 - \Phi\left(\frac{t-\mu}{\sigma}\right)$ | $h(t) = \frac{\varphi(t-\mu)}{\Phi(-t)}$ | YES |
| Logistic | $S(t) = e^{-\frac{\lambda e^{\gamma t}}{\gamma}}$ | $h(t) = \lambda e^{\gamma t}$ | YES |
| Loglogistic | $S(t) = \frac{1}{1+(\lambda t)^{\gamma}}$ | $h(t) = \frac{\lambda\gamma(\lambda t)^{\gamma-1}}{1+(\lambda t)^{\gamma}}$ | NO |

(continued)

| Survival Model (distribution of *t*) | Survival Function *S(t)* | Hazard function *h(t)* | Proportional hazards assumption |
|---|---|---|---|
| Lognormal | $S(t) = 1 - \Phi\left(\frac{\log t - \mu}{\sigma}\right)$ | $h(t,\sigma) = \frac{\left(\frac{1}{t\sigma}\right)\varphi\left(\frac{\log t - \mu}{\sigma}\right)}{\Phi\left(\frac{-\log t}{\sigma}\right)}$ | NO |
| $\Phi$ is the cumulative distribution function of the standard normal distribution and $\varphi$ is the probability density function of the standard normal distribution. | | | |

Yet another known model is a "stratified Cox model" as proposed by Kalbfleisch and Prentice, 1980. In this model,

$$\log\ h^i_{k,g}(t_k, x^i_k) = \alpha_{i,g}(t_k) + \beta_{i,g} x^i_k, \qquad g=1,2,\ldots,G$$

where $g$ labels $G$ "strata".

This modification of the Cox regression model includes the stratification of a covariate that does not satisfy the proportional hazards assumption. It assumes that there is one variable that does not satisfy the proportional hazards. The modification creates strata for this one variable, and within these strata estimates the effect of grouping on survival times. Covariates that are assumed to satisfy the proportional hazards assumption are included in the model, whereas the predictor being stratified is not included.

Summary of the invention

The present invention aims to provide new and useful methods for identifying genes which are statistically significant in relation to a medical condition. It further aims to provide new and useful methods for identifying statistical models employing data about the expression levels of the identified genes. It further suggests gene pairs which are statistically significant for certain medical conditions.

The invention proposes in general terms that, for a given set of patient data, there is a process of selecting from a plurality of survival models, which survival model best models a set of clinical data. In each of the multiple survival models, the hazard function is a structurally-different mathematical function of the time variable or other exogenous variables, i.e. the functions differ not only in that they include respective parameter(s) which take different respective values, but in that the hazard functions of the functions have a different functional dependency on the regressor variables (e.g. time). For example, one of the models is typically a Cox proportional hazards model, while another may be one of the other models specified in Table 1. For example, one or more of the survival models may be proportional survival models, and one or more of the survival models may be non-proportional survival models.

In one form, there may first be a step of determining whether the data set fits a single survival model (e.g. such that a numerical measure of the quality of the fit is above a predetermined level), and if it is found not to fit, one or more other survival models are tried out. In one example, the single survival model may be one involving proportional hazards, and the step of determining whether the data set fits the survival model may include determining whether the proportional hazards assumption holds.

The criterion for selecting which of the survival models best fits the data set may employ the Bayesian Information Criterion (BIC).

According to a first aspect of the invention there is provided a computerized method for identifying one or more genes, or pairs of genes, selected from a set of N genes, which are statistically associated with prognosis of a potentially fatal medical condition, the method employing test data which, for each subject $k$ of a set *of* $K$ subjects suffering from the medical condition, indicates (i) a survival time of subject $k$, and (ii) for each gene $i$, a corresponding gene expression value $y_{i,k}$ of subject $k$;
the method comprising:

(A) for each of a plurality of genes or pairs of genes, performing the sub-steps of:

(a) partitioning the $K$ subjects into two subsets using cut-off values, (b) computationally fitting a Cox proportional hazard regression model to the corresponding test data of at least one of the subsets of subjects;
(c) determining whether a proportionality assumption of the Cox proportional hazard regression model is satisfied using the Bayesian Information Criterion;

(d) if the proportionality assumption is satisfied, fitting one or more additional proportional hazard regression models to the data-set;

(e) if the proportionality assumption is not satisfied, fitting at least one hazard regression model which does not satisfy a proportionality assumption to the data-set;

(f) selecting, from the fitted hazard regression models, a hazard regression model having best quality of fit based on the Bayesian Information Criterion; and

(g) obtaining a significance value indicative of prognostic significance of the gene or gene pair using the selected hazard regression model;

wherein when operation (A) is performed for each of the plurality of pairs of genes (*i, j* with *i* # *j*) of the set of N genes, operation (A) comprises: generating a respective plurality of trial cut-off values of $c_{i,j}$, and for each of the trial cut-off values:

partitioning the *K* subjects into two subsets according to whether $log(y_{i,k}) - log(y_{j,k})$ is respectively above or below the trial cut-off value $c_{i,j}$;

performing sub-operations (b)-(f); and

obtaining a significance value indicative of prognostic significance of the gene pair *i,j* using the selected hazard regression model; and

for each of the pairs of genes, identifying the trial cut-off value $c_{i,j}$ for which the corresponding significance value indicates the highest prognostic significance for the gene pair *i,j*; and

(B) identifying one or more of the genes or pairs of genes for which the corresponding significance values have the highest prognostic significance.

Also described is a computerized method for identifying one or more genes, or pairs of genes, selected from a set of *N* genes, which are statistically associated with prognosis of a potentially fatal medical condition, the method employing test data which, for each subject *k* of a set *of K* subjects suffering from the medical condition, indicates (i) a survival time of subject *k*, and (ii) for each gene *i*, a corresponding gene expression value $y_{i,k}$ of subject *k*;
the method comprising:

(i) for each of a plurality of genes or pairs of genes, performing the sub-steps of:

(a) computationally fitting the test data to a pluralty of regression models, each model partitioning the *K* subjects into two subsets using cut-off values;

(b) identifying which of the regression models best fits the test-data according to a data fit measure, such as the Bayesian Information Criterion; and

(c) using the identified regression model to obtain a significance value indicative of prognostic significance of the gene or gene pair; and

(ii) identifying one or more of the genes or pairs of genes for which the corresponding significance values have the highest prognostic significance.

Further disclosed is a survival model which, for each of one or more pairs of genes, is a function of a measure of the ratio of expression levels of the pair of genes. For each pair of genes, there is a corresponding a cut--off value, such that patients are classified according to whether the corresponding measure is above or below the cut-off value. It is proposed that the cut-off value should be selected so as to maximize the separation of the respective survival curves of the two groups of patients. From another point of view, this means that the cut-off value is selected such that the partition of subjects which it implies is of maximal statistical significance. One embodiment relating to the first aspect of the invention is referred to below as "DDgR".

One possible expression of this model is a computerized method for identifying one or more pairs of genes, selected from a set of N genes, which are statistically associated with prognosis of a potentially fatal medical condition, the method employing test data which, for each subject *k* of a set *of K* subjects suffering from the medical condition, indicates (i) a survival time of subject *k*, and (ii) for each gene *i*, a corresponding gene expression value $y_{i,k}$ of subject *k*;
the method comprising:

(i) for each of a plurality of pairs of genes (*i, j* with *i* # *j*), generating a respective plurality of a trial cut-off values of $c_{i,j}$, and for each of the trial cut-off values:

(a) partitioning the $K$ subjects into two subsets according to whether $log(y_{i,k}) - log(y_{j,k})$ is respectively above or below the trial cut-off value $c_{i,j}$;
(b) computationally fitting the corresponding survival times of at least one of the subset of subjects to a Cox proportional hazard regression model; and
(c) obtaining a significance value indicative of prognostic significance of the gene pair $i,j$;

(ii) for each of the pairs of genes, identifying the trial cut-off value for which the corresponding significance value indicates the highest prognostic significance for the gene pair $i,j$; and

(iii) identifying one or more of the pairs of genes $i,j$ for which the corresponding significance values have the highest prognostic significance.

A certain embodiment of the invention relates to the usage of gene pairs identified by a method according to the first aspect of the invention. Further embodiments of the invention are defined by claims 1 to 11.
The present invention further relates to a computer system arranged to perform the method of the invention (any general purpose computer may be used for this purpose), and to data-carriers (such as tangible media, such as CDs) carrying software instructions for performing the method.
Information about a first subject in relation to breast cancer may be obtained by measuring in the first subject the expression levels of a pair of genes in one of the rows of Table 2 below, or one of the pairs of genes in Table 10 later in this document, and obtaining the information using a statistical model which employs the expression levels of those genes.
A microarray (or other kit) may be used for obtaining data for performing prognosis of breast cancer. Since the microarray is dedicated to this purpose it does not require sensing of large numbers of genes which are not of signficance. Instead, its cost can be reduced by designing it only to sense the presence of a limited number of genes (e.g. at most 100 genes, at most 50 genes, or at most 30 genes, or at most 20 genes) of which some or all have been determined to be a specific relevance to breast cancer.
One specific expression of this method is a microarray being for measuring the expression value of a set of no more than 20 genes (or not more than 100, or no more than 50, or no more than 30), including at least one of the pairs of genes which are the rows of Table 2.

Table 2

| | |
|---|---|
| TUBB3 | KIAA0999 |
| SPG20 | PGAM1 |
| H2AFZ | RBM35B |

Another specific expression of this method is a microarray (or other kit) for measuring the expression value of a set of no more than 20 genes (or not more than 100, no more than 50, or no more than 30) including at least one of the 16 pairs of genes which are respectively given in the upper 17 rows of Table 10, and preferably more than one of these pairs.

Detailed description of the embodiments

Embodiments relating to the invention will now be explained, with reference to the following figures in which:

Figure 1 is flow diagram of a method ("DDgR") which is related to an embodiment of the invention.

Figure 2 is a diagram used to explain the technique of "2-Dimensional" patient grouping.

Figure 3 is a flow diagram of a method which is related to an embodiment of the invention.

Figure 4 shows the distribution of survival p-values for Stockholm cohort (left panel) and Uppsala cohort (right panel) of 44928 probesets. The vertical dashed line at p=0.01 denotes the 1% significance level below which we declare a probe as survival significant.

Figure 5 shows the distribution of survival significant p-values for Stockholm cohort (left panel) and Uppsala cohort (right panel) of common 3375 probesets of the two cohorts.

Figure 6 shows the distribution of survival p-values of 20665 reproducible across cohort gene pairs in Stockholm (left top panel) and Uppsala (right top panel). Distribution of survival p-values of Bonferroni corrected and BCa-significant gene pairs in Stockholm (left bottom panel) and Uppsala (right bottom panel).

Figure 7 shows Kaplan-Meier survival curves of in Stockholm cohort for PGMA1 and SPG20 genes. Top-left panel: p-value of PGMA1 grouping = 6.2E-04; top-right panel: p-value of SPG20 grouping = 1.1E-04; bottom-left panel: 2D synergy p-value of PGMA1-SPG20 grouping = 1.5E-05 and ratio PGMA1/SPG20 grouping p-value = 9.7E-08.

Figure 8 shows Kaplan-Meier survival curves of in Uppsala cohort for PGMA1 and SPG20 genes. Top-left panel: pvalue of PGMA1 grouping = 9.4E-04; top-right panel: p-value of SPG20 grouping = 6.8E-07; bottom-left panel: 2D synergy p-value of PGMA1-SPG20 grouping = 9.5E-08 and ratio PGMA1/SPG20 grouping pvalue = 5.3E-09.

Figure 9 shows Kaplan-Meier survival curves of in Stockholm cohort for TUBB3 and KIAA0999 genes. Top-left panel: p-value of TUBB3 grouping = 2.1E-08; top-right panel: p-value of KIAA0999 grouping = 2.2E-07; bottom-left panel: 2D synergy p-value of TUBB3-KIAA0999 grouping = 1.3E-D8 and ratio TUBB3/KIAA0999 grouping p-value = 8.2E-10.

Figure 10 shows Kaplan-Meier survival curves of in Uppsala cohort for TUBB3 and KIAA0999 genes. Top-left panel: p-value of TUBB3 grouping = 2.7E-05; top-right panel: p-value of KIAA0999 grouping = 4.0E-05; bottom-left panel: 2D synergy p-value of TUBB3-KIAA0999 grouping = 1.4E-07 and ratio TUBB3/KIAA0999 grouping p-value = 3.3E-08.

Figure 11 shows Kaplan-Meier survival curves of in Stockholm cohort for H2AFZ and RBM35B genes. Top-left panel: p-value of H2AFZ grouping = 3.3E-07; top-right panel: p-value of RBM35B grouping = 2.9E-05; bottom-left panel: 2D synergy p-value of H2AFZ - RBM35B grouping = 2.1E-07 and ratio H2AFZ / RBM35B grouping p-value = 2.5e-01.

Figure 12 shows Kaplan-Meier survival curves of in Uppsala cohort for H2AFZ and RBM35B genes. Top-left panel: p-value of H2AFZ grouping = 3.6E-04; top-right panel: p-value of RBM35B grouping = 1.3E-04; bottom-left panel: 2D synergy p-value of H2AFZ - RBM35B grouping = 3.5E-11 and ratio H2AFZ / RBM35B grouping p-value = 1.9e-01.

## Definitions

"Array" or "microarray," as used herein, comprises a surface with an array, preferably an ordered array, of putative binding (e.g., by hybridization) sites for a sample which often has undetermined characteristics. An array can provide a medium for matching known and unknown nucleic acid molecules based on base-pairing rules and automating the process of identifying the unknowns. An array experiment can make use of common assay systems such as microplates or standard blotting membranes, and can be worked manually, or make use of robotics to deposit the sample. The array can be a macro-array (containing nucleic acid spots of about 250 microns or larger) or a micro-array (typically containing nucleic acid spots of less than about 250 microns).

The term "cut-off expression value" represented by $c_i$ refers to a value of the expression level of a particular nucleic acid molecule (or gene or probe) *i* in a subject. The cut-off expression value is used to partition the subjects into classes, according to whether the expression level of the corresponding gene is below or above the cut-off expression value. Note that it makes no difference whether all subjects for which the expression value is actually equal to the cut-off value are classified as being in one class or alternatively in the other. The term "cut-off value" is used for variables such as $c_{i,j}$ where the cut-off is used to classify based on a value other than the expression value of one nucleic acid molecule (or gene or probe), such as the ratio of the expression values two nucleic acid molecules *i*, *j*.

The term "gene" refers to a nucleic acid molecule that encodes, and/or expresses in a detectable manner, a discrete product, whether RNA or protein. It is appreciated that more than one nucleic acid molecule may be capable of encoding a discrete product. The term includes alleles and polymorphisms of a gene that encodes the same product or an analog thereof.

There are various methods for detecting gene expression levels. Examples include Reverse-Transcription PCR, RNAse protection, Northern hybridisation, Western hybridisation, Real-Time PCR and microarray analysis. The gene expression level may be determined at the transcript level, or at the protein level, or both. The nucleic acid molecule or probe may be immobilized on a support, for example, on an array or a microarray. The detection may be manual or automated. Standard molecular biology techniques known in the art and not specifically described may be employed as described in Sambrook and Russel, Molecular Cloning: A Laboratory Manual, Cold Springs Harbor Laboratory, New York (2001).

Gene expression may be determined from sample(s) isolated from the subject(s).

Algorithms referred to as R-algorithms, or routines "in the R library", refer to algorithms in the package described at http://cran.r-project.org/, or written in the language of the environment described there.

Detailed description of the embodiments

Embodiments related to the invention will now be explained. Before doing so, we discuss a technique ("DDg") which was not in the public domain at the priority date of the present application, and which is not independently claimed in the present application (it is described in detail in one of the related patent applications referenced above), but which is employed in certain embodiments of the present invention.

1. Data-driven grouping (DDg)

DDg is an alternative to the mean-based grouping (MBg) explained in the background section of the present application. The explanation of it given below uses terms with the same definition as in the explanation of MBg above.

For each gene *i*, compute the 10th quantile $(q_{10}^{i})$ and the 90th quantile $(q_{90}^{i})$ of the distribution of the K signal intensity values (i.e. the expression levels of the *K* patients). Within $(q_{10}^{i}, q_{90}^{i})$, we search for the value that most successfully discriminates the two unknown genetic classes. This can be done by the following steps:

1. Form the candidate cut-offs vector of dimension 1 x Q, $\underline{w}^{i} = y_{i}^{*}$, where $y_{i}^{*}$ is the log-transformed intensities within $(q_{10}^{i}, q_{90}^{i})$ and Q is the number of elements in $\underline{w}^{t}$. Each element of the $\underline{w}^{t}$ is a trial cut-off value. For *i* = 1 and $c^{i} = \underline{w}_{z}^{i}$, z = 1, ..., *Q* use

$$x_{k}^{i} = \begin{cases} 1\ (high-risk) & if \quad y_{i,k} > c^{i} \\ 0\ (low-risk) & if \quad y_{i,k} \le c^{i} \end{cases}$$

to separate the *K* subjects with $c^{i}$.

2. For z=1 (the first element in $\underline{w}^{i}$) evaluate the prognostic significance of gene *i* given cut-off $\underline{w}_{z}^{i}$ by estimating the $\beta_{i}^{z}$ from $log\ h_{k}^{i}(t_{k}|x_{k}^{i},\beta_{i}^{z}) = \alpha_{i}(t_{k}) + \beta_{i}^{z}x_{k}^{i}$ and

$$L(\beta_{i}^{z}) = \prod_{k=1}^{K}\left\{\frac{\exp\left(\beta_{i}^{z}x_{k}^{i}\right)}{\sum_{j\in R(t_{k})}\exp\left(\beta_{i}^{z}x_{k}^{i}\right)}\right\}^{\delta_{k}}.$$

3. Iterate for z = 2, ..., *Q* to estimate the *Q* p-values (corresponding to *Q* distinct cut-off expression value levels) for each *i*. The "optimal" cut-off expression value $c^{i}$ four each *i* is the taken as the one with the minimum $\beta_{i}^{z}$ p-value, provided that the sample size of each group is sufficiently large (formally above 30) and Cox proportional hazards model is plausible.

4. Iterate steps 1 to 3 for *i* = 2, ..., *N.*

2. Data-Driven Grouping for Ratios (DDgR)

We now turn to an embodiment related to the invention, illustrated by the flow diagram of Fig. 1. Ratio-based approaches have previously been used for the analysis of microarray data (e.g. normalization and differential expression analyses; see Cui and Churchill, 2003). Here, we develop a grouping method based on ratios and compare it against our data driven method (DDg) for individual genes. We claim that the ratio approach can significantly improve the biological classification and thus provide useful predictions of the clinical assignment of breast cancer patients.

Data-driven grouping for ratios is an extension of our original Data-driven method that takes into account ratios of intensities. Instead of considering each gene independently and attempting to identify the respective patients' grouping, this new approach takes into account ratios of raw gene intensities (or equivalently the difference of gene log-transformed intensities), which are then processed in the same way as in the DDg method. To this extent, the ratio-based approach utilizes pairs of genes (instead of single genes) and aims to identify significantly different groups of cancer patients. The steps of the algorithm are as follows:

1. Compute the difference of log-transformed intensities of two selected genes *i* and *j* (step 1 of Fig. 1).
2. Compute the quantile $(q_{10}^{i,j})$ and the 90th quantile $(q_{90}^{i,j})$ of the distribution of the *K* log-difference values $y_{i,k} - y_{j,k}$ (step 2 in Fig. 1).
3. Within $(q_{10}^{i,j}, q_{90}^{i,j})$ search for the cut-off value that most successfully discriminates the two unknown genetic classes, by forming the candidate cut-offs vector of dimension 1 x Q, $\underline{w}^{ij} = y_{ij}^{*}$, where $y_{ij}^{*}$ is the log-differenced intensities $y_{t,k} - y_{j,k}$ within $(q_{10}^{ij}, q_{90}^{ij})$ and *Q* is the number of elements in $\underline{w}^{ij}$. Each element of the $\underline{w}^{ij}$ is a trial cut-off value. For $i$ = 1 and $c^{ij} = \underline{w}_z^{ij}$, z = 1, ..., *Q* use

$$x_k^{ij} = \begin{cases} 1\ (high-risk) & if \quad y_{i,k} - y_{j,k} > c^{ij} \\ 0\ (low-risk) & if \quad y_{i,k} - y_{j,k} \le c^{ij} \end{cases}$$

to separate the *K* subjects with $c^{ij}$. This is step 3 of Fig. 1.
4. For z=1 (the first element in $\underline{w}^{i}$) evaluate the prognostic significance of gene pair *i,j* given the cut-off $c^{ij}$ by $\beta_{ij}^{z}$ estimating the form

$$log\ h_k^{ij}(t_k | x_k^{ij}, \beta_{ij}^{z}) = \alpha_{ij}(t_k) + \beta_{ij}^{z} x_k^{ij}$$

and

$$L(\beta_{ij}^{z}) = \prod_{k=1}^{K} \left\{ \frac{\exp\left(\beta_{ij}^{z} x_k^{ij}\right)}{\sum_{j \in R(t_k)} \exp\left(\beta_{ij}^{z} x_k^{ij}\right)} \right\}^{\delta_k}$$

5. Iterate steps 1 to 4 for z = 2, ..., *Q* to estimate the *Q p*-values (corresponding to *Q* distinct cut-off levels) for each *i* and *j*. The "optimal" cut-off value for each pair is the one with the minimum $\beta_{ij}^{z}$ p-value, provided that the sample size of each group is sufficiently large (formally above 30) and the Cox proportional hazards model is plausible. This is step 4 of Fig. 1.
6. Iterate steps 1-5 for the selected *i* and *j* pairs.

Statistically significant gene pairs are identified as those having low $\beta_{i,j}^{z}$ p-values.

3. 2-Dimensional ("2-D") Patient grouping

Before discussing further embodiments related to the invention we now discuss the concept of 2-dimensional patient grouping. Again, this idea is not independently claimed in the present application (it is described in detail in one of the related patent applications referenced above), but it is employed in certain embodiments of the present invention.

The idea of 2-D grouping, in general terms is that that pairs of genes are selected. For each gene pair, we use a respective cut-off value for each gene to generate a plurality of models, which represent ways of partitioning a set of subjects based on the expression values of the pair of genes in relation to the respective cut-off values. We then identify those gene pairs for which one of the models has a high prognostic significance. Specifically, the models are defined using the scheme illustrated in Fig. 2, where they are referred to respectively as "designs 1-7".

Specifically, for a given gene pair *i, j, i* ≠ *j,* and respective individual cut-off expression values $c^i$ and $c^j$, we define seven "models", each of which is a possible way in which the expression levels of the two genes might be significant. Then we test the data to see if any of the seven models are in fact statistically significant. The models are defined using Figure 2 which shows how a 2-D area having $y_{i,k}$, $y_{j,k}$ as axes. In Figure 2 gene *i* is referred to as "gene 1" and gene *j* as "gene 2". The 2-D area is divided into four regions A, B, C and D, defined as follows:

A: $y_{i,k} < c^j$ and $y_{j,k} < c^j$
B: $Y_{i,k} \geq c^i$ and $y_{j,k} < c^j$
C: $y_{i,k} < c^j$ and $y_{j,k} \geq c^j$
D: $y_{i,k} \geq c^i$ and $y_{j,k} \geq c^j$

Each of the seven models is then defined as a respective selection from among the four regions:

Model 1 is that a subject's prognosis is correlated with whether the subject's patient's expression levels are within regions A or D, rather than B or C.

Model 2 is that a subject's prognosis is correlated with whether the subject's patient's expression levels are within regions A, B or C, rather than D.

Model 3 is that a subject's prognosis is correlated with whether the subject's patient's expression levels are within regions A, C or D, rather than B.

Model 4 is that a subject's prognosis is correlated with whether the subject's patient's expression levels are within regions B, C or D, rather than A.

Model 5 is that a subject's prognosis is correlated with whether the subject's patient's expression levels are within regions A, B or D, rather than C.

Model 6 is that a subject's prognosis is correlated with whether the subject's patient's expression levels are within regions A or C, rather than B or D.

Model 7 is that a subject's prognosis is correlated with whether the subject's patient's expression levels are within regions A or B, rather than C or D.

Note that model 6 is equivalent to asking only whether the subject's expression level of gene 1 is below of above $c^1$ (i.e. it assumes that the expression value of gene 2 is not important). Model 7 is equivalent to asking only whether the subject's expression for gene 2 is above or below $c^2$ (it assumes that the expression value of gene 1 is not important). Thus, models 1-5 are referred to as "synergetic" (1 - 5), and the models 6 and 7 as "independent".

The 2-D patient grouping evaluates the significance of all possible gene pairs *i, j* as follows:

1. Set *i*=1 and *j*=2.

2. For each of the 7 models, obtain the respective sub-set of the subjects whose expression values for genes *i* and *j* obey the respective set of the conditions shown in Fig. 1. For example, for model 1, we obtain the subset of the *K* subjects whose expression values obey conditions A, B or C. This is the subset of the subjects for which $y_{i,k} < c^i$ and/or $y_{j,k} < c^j$ (i.e. the set of subjects which for which condition D is not obeyed). Let us define a parameter $x^m_{i,j,k}$, where $x^m_{i,j,k} = 1$ if and only if, for genes *i* and *j*, and model *m* (*m*=1,...7), the expression levels $y_{i,k}$ and $y_{j,k}$ meet the conditions of model *m.*

3. Fit the survival values to:

$$\log h^{i,j}{}_k(t_k \mid x^m_{i,j,k}, \beta^m_{i,j}) = \alpha_{i,j}(t_k) + \beta^m_{i,j} \cdot x^m_{i,j,k},$$

4. Estimate the seven Wald p-values $\beta^m_{i,j}$. Provided that the respective groups sample sizes are sufficiently large and the assumptions of the Cox regression model are satisfied, the model is the one with the smallest $\beta^m_{i,j}$ p-value.

5. Iterate steps 1 to 4 for all pairs of genes.

"2-D patient grouping" is grouping of *K* patients based on a pair of genes exhibiting potential interaction in context of survival time. "1-D grouping" is grouping of *K* patients based on individual genes. In view of the term "2-D patient grouping", DDg can be considered as more general and biologically meaningful apparoch than "1-D grouping", in which each gene is considered individually. Strictly speaking, MBg is a kind of 1-D grouping but it does not estimate the optimal cut-off. The optimal cut-off is estimated only by the DDg method.

4. Residuals Bootstrap for the Cox Proportional Hazards Model for 1D and 2D patients grouping

To validate the significance of our findings (in terms of the estimated Wald p-values) we bootstrap our samples (from the two cohorts) and estimate 99% confidence intervals for the $\beta_i$ coefficients of the Cox proportional hazards model. Note that the following discussion considers only the case of 1-D grouping. In the case of the 2-D grouping, we would substitute $\beta_i$ with $\beta_{i,j}^{m}$ in the following discussion. The bootstrap method though is the same in both cases (1-D and 2-D). Extending this technique for validating the ratio method DDgR is also straightforward, so will not be explained here.

We use the non-parametric residuals bootstrap for the proportional hazards model of Loughin (1995) using the boot package in R.

Specifically, the algorithm works as follows sequentially for each gene i:

1. Estimate $\beta_i$ of model:

$$\log h^i{}_k(t_k \mid x_k^i, \beta_i) = \alpha_i(t_k) + \beta_i \cdot x_k^i$$

by maximizing the likelihood:

$$L(\beta_i) = \prod_{k=1}^{K} \left\{ \frac{\exp(\beta_i^T x_k^i)}{\sum_{j \in R(t_k)} \exp(\beta_i^T x_j^i)} \right\}^{e_k}.$$

2. Calculate the independent and identically (Uniform [0,1]) distributed generalized residuals, calculated in Cox and Snell (1968) and Loughin (1995) by the "probability scale data"

$$u_k = [1 - F_o(t)]^{\exp(\beta_i^T x_k^i)}, \text{ for } k=1,2,...K$$

where $F_0(t) = P(T \leq t \mid \exp(\beta_i^T x^i) = 1)$ denotes the baseline failure time distribution. Typically, $\hat{F}_0(t)$ (the estimated value of $F_0(t)$) is a step function with jumps at the observed failure times (estimated automatically in the *survival* package of the R library), which does not affect the Uniformity of the generalized residuals (Loughin, 1995)

3. Consider the pairs $\{(u_1,e_1),...,(u_K,e_K)\}$ and resample with replacement *B* pairs of observations (*B* bootstrap samples) $\{(u_1^{(b)},e_1^{(b)}),..., (u_K^{(b)},e_K^{(b)})\}$, for b = 1, ..., *B* (a typical bootstrap step)

4. Calculate the probability scale survival times

$$t_k^{(b)} = 1 - \left[u_k^{(b)}\right]^{1/\exp(\beta_i^T x_k^i)}$$

and estimate the bootstrap coefficients $\beta_i^{(1)}, \beta_i^{(2)}, ..., \beta_i^{(B)}$ $\beta_i^{(1)},\beta_i^{(2)},...,\beta_i^{(B)}$ by numerically maximizing the partial likelihood:

$$L^{(b)}(\beta_i) = \prod_{k=1}^{K} \left\{ \frac{\exp(\beta_i^T x_k^i)}{\sum_{j \in R^{(b)}(t_k)} \exp(\beta_i^T x_j^i)} \right\}^{e_k^{(b)}} , \; b = 1, \ldots, B$$

Based on these coefficients, we estimate and report the Bias-Corrected accelerated (BCa) bootstrap confidence intervals for each $\beta_i$ coefficient that correct the simple quantile intervals of $\beta_i$ for bias and skewness in their distribution (Efron and Tibshirani, 1994). A detailed discussion (theory and applications) on BCa intervals is given in Efron and Tibshirani (1994). Here the 99% BCa were estimated by the *boot* package in the R library. Bootstrap test p-values based on quantile method gave similar results using the Wald test.

5. Incorporation of other survival models in our 1D, 2D and Ratio methods and a procedure for model selection

The most crucial assumption of the Cox proportional hazards survival model is the one concerning the effect of the parameters to the hazard ratio. Specifically, consider two patients $k_1$ and $k_2$ modelled by the Cox proportional hazards model

$$\log h^i{}_k(t_k \mid x_k^i, \beta_i) = \alpha_i(t_k) + \beta_i \cdot x_k^i$$

The hazard log-difference of these two groups must be:

$$\log h^i{}_{k_1}(t_{k_1} \mid x_{k_1}^i, \beta_i) - \log h_{k_2}^i(t_{k_2} \mid x_{k_2}^i, \beta_i) = \alpha_i(t_{k_1}) + \beta_i \cdot x_{k_1}^i - (\alpha_i(t_{k_2}) + \beta_i x_{k_2}^i) = \beta_i x_{k_1}^i - \beta_i x_{k_2}^i$$

which is independent of time *t.* We call this assumption the *proportional hazards assumption* of the Cox model. Under these circumstances it is desirable to determine whether the Cox regression model adequately fits the data or, in other words, whether the proportional hazards assumption holds. Tests and graphical diagnostics for proportional hazards may be based on the estimation of the scaled Schoenfeld residuals ($r_k$) (Schoenfeld, 1982), which for non-censored patients are estimated as:

$$r_i = x_k^i - \sum_{k=1}^{j \in R(t_k)} \beta_i x_k^i$$

Grambsch and Therneau (1994) developed the respective test statistic that has the form:

$$Test_i = \frac{\{\sum (g_i - \bar{g}) r_i\}^2}{t \times I_i \times \sum (g_i - \bar{g})^2}$$

where $r_i$ are the scaled Schoenfeld residuals for probe *i,* $g_i$ is the time scale, $\bar{g}$ is the average time scale, and $I_i$ is the information matrix elements for probe *i. t* is the observed vector of survival times and multiplying it by the $I_i$ matrix gives us a single number (i.e. a 1 x 1 matrix). This test follows a $\chi^2$ distribution with one degree of freedom and can be interpreted as a measure of significance for the correlation between the covariate specific residuals and the survival times (if significant correlation exists the proportionality assumption is rejected; see Thompson et al., 2003). Large values of the test (or small p-values) are an indication of non-proportionality in the hazards.

In the *survival* package in the R library, the proportional hazards test can be done more conveniently with the cox.zph function, which checks the proportional-hazards assumption by correlating the corresponding set of scaled Schoenfeld residuals with a suitable transformation of time for each covariate $x^i$ (for more information refer to the *survival* package in the R library).

The embodiments described below incorporate a test for the proportional hazards assumption applied to the results of the 1 D-, 2D- and ratio- patient grouping methods. When the assumption holds, we can rely on the Cox model and make inferences based on the results of our 1D/2D/ratio methods. However, when the assumption is not satisfied, we need to think of a proper approach to analyze the data. Note however, that preliminary analysis of our data showed that non-proportionality holds for approximately 1-1.5% of the probes/probe pairs.

*Survival Model Misspecification*

At this paragraph we wish to discuss a possible reason of the proportionality failure before we describe possible remedies of the problem. Schemper (1992) and Therneau and Grambsch (2000) suggest that certain model specification errors can lead to false positive results of the test. Such specification errors can be simply the omission of an important covariate of the model (either a single covariate or an interaction) or an incorrect functional form of the model. The latter implies that if a parametric model is more appropriate than Cox proportional model for the data, using Cox can lead to a misleading significant test result. In addition, we should take into account the case where proportional hazards assumption does not hold due to the data properties which requires the use of a model that does not make this assumption.

6. A second embodiment related to the invention

To solve the problem with respect to model misspecification, a second embodiment related to the invention uses the 4 different parametric survival models in the top lines of Table 1, along with any one of the 1D-, 2D- and ratio-methods. Note that these 4 models assume proportional hazards. Parametric survival models are constructed by choosing a specific probability distribution for the survival function. The choice of survival distribution expresses some particular information about the relation of time and any exogenous variables to survival. It is natural to choose a statistical distribution which has non-negative support since survival times are non-negative.

Here we describe the second embodiment in detail with reference to Figure 3:

1. First we partition the patients of a cohort into groups using any one of the 1D-, 2D- or ratio-methods (step 11 of Fig. 3), and obtain the corresponding Cox proportional hazards model. We estimate the p-value of the $\beta$ coefficient of the Cox proportional hazards model as before.

2. Subsequently, we use the cox. zph function of the R library to evaluate whether the assumption of proportional hazards holds. The function produces a p-value, which when it is below a significance level (typically 5%) indicates that the proportionality assumption is not satisfied (step 12 of Fig. 3). If the results indicate that the assumption is met then the method can optionally terminate there, or alternatively (and particularly if we suspect that one of the other parametric models should be more appropriate) we can ignore the results of the Cox model and proceed to step 3. If the result of the text indicates the proportionality assumption is not satisfied, jump to step 15 of Fig. 3 which is explained in the next section of this document.

3. Next (in step 13 of Fig. 3) successively fit each of the four parametric models listed in Table 1 to the same dataset, using the survreg function in the R-library, using the same one of the 1D-, or 2D- or ratio- methods, to estimate the p-value of the $\beta$ coefficient in the parametric-type survival models of the form:

$$t_k = \alpha_i(t_k) + \beta_i x_k^i$$

where t is distributed each time as indicated in the corresponding row of Table 1. The linear model we use to find significant genes is always of the form $\log h^i{}_k(t_k \mid x_k^i, \beta_i) = \alpha_i(t_k) + \beta_i \cdot x_k^i$, estimated with methods depending on the distribution of *t*.

For the estimation of $\beta$ one needs to maximize the appropriate likelihood $f(t|\theta)$ of each model, where $\theta$ denotes a vector of parameters. Notice that this step is similar to what we described in step 1, but requires the use of a parametric model instead of Cox's.

4. Estimate the Bayesian Information Criterion (BIC) to check which model best fits the data (step 14 of Fig. 3). BIC is estimated by:

$$BIC = -2 * \log L + p \times \log(K)$$

where *L* denotes the maximized value of the likelihood function of the estimated model, *p* is the number of parameters of the model and $K$ is the number of observations (patients). Alternatively BIC can be estimated by the step function in the R library. Note that BIC can compare only the parametric models with each other because all of them use as dependent variable the survival times. On the other hand, the Cox model uses ranked survival times and thus it is not straightforwardly comparable to the parametric alternatives.

*The non-proportional hazards case*

We will now discuss how the second embodiment deals with the case where proportionality fails due to omission of model parameters or simply because of the data properties (the hazards are not proportional).

Two possible solutions exist: first, these two cases may be handled simultaneously by considering the *stratified Cox proportional hazards model* of Kalbfleisch and Prentice (1980); second, the embodiment may use a parametric model that does not assume proportional hazards (e.g. the lower two models in Table 1) and fit it as described above. The second embodiment *first* uses the stratified Cox proportional hazards model, checks whether proportionality is obeyed, and then only if not, moves on to considering the parametric methods which do not assume proportional hazards.

As discussed above, the stratified Cox regression model is a modification of the Cox regression model by the stratification of a covariate that does not satisfy the proportional hazards assumption. Covariates that are assumed to satisfy the proportional hazards assumption are included in the model, whereas the predictor being stratified is not included. As discussed above, the embodiment initially assumes that the patients' grouping (as estimated by the 1D-, 2D- or ratio methods) satisfies the proportional hazards assumption. However, in the cases that this hypothesis is rejected, we assume that there is at least one additional variable that may cause the problem. Our aim is to create strata based on this additional variable and within this strata estimate the effect of our grouping on survival times. As an additional we consider a series of clinical variables such as the histological grade (obtained from SWS analysis of Kuznetsov et al., 2006), the patients' age, the patients' intrinsic molecular signature, the metastasis status and the endocrine status. For a detailed description of these variables see Kuznetsov et al. (2006) and Kuznetsov et al. (2008).

More mathematically, our procedure to solve non-proportionality problem by the stratified no-interaction Cox proportional hazards model (Kalbfleisch and Prentice, 1980; Ata and Sozer, 2007) or/and with a non-proportional hazards parametric approach can be described as follows:

1. Use first the stratified Cox model (step 15 of Fig. 3). Define the model as:

$$\log\ h^{i}_{k,g}(t_k, x^{i}_k) = \alpha_{i,g}(t_k) + \beta_{i,g} x^{i}_k, \qquad g=1,2,\ldots,G$$

where $g$ labels $G$ "strata". The strata are the different categorizations of the stratum variable, which is not implicitly used in the model (only the grouping variable x is used). To this extent, the baseline hazard $\alpha_{i,g}$ $(t_k)$ is different in each stratum but the hazards ratios are the same and thus the proportionality assumption holds. In this way we obtain estimates for the coefficients $\beta_{i,g}$ (which indicates the slope in each stratum).

2. To obtain the overall $\beta_i$ $\beta_{i,g}$ coefficients and consequently the p-value of interest, we need to multiply the likelihood functions of the one or more strata and maximize our results with respect to $\beta_i$ as before.

3. Repeat steps 1-2 for each of the additional variables used to define the strata (for example, we can take $g$ = 2 (user defined) and create strata with variable "tumor grade" or another variable, e.g. "tumor subtype"), and run the Cox proportional hazards test each time to check whether the respective proportionality assumption is satisfied (step 16 of Fig. 3).

4. If the assumption is not satisfied, fit each of the non-proportional parametric models listed in Table 1 by survreg function in the R library and, using our 1 D or 2D or ratio method, estimate the p-value of the $\beta$ coefficient in the parametric-type survival models of the form

$$t_k = a_i(t_k) + \beta_i x^{i}_k$$

where $t$ is distributed each time as indicated in Table 1 (step 17 of Fig. 3). Then, estimate the Bayesian Information Criterion (BIC) to check which model best fits the data (step 18 of Fig. 3).

7. Four detailed examples relating to the invention

We now list three detailed examples of algorithms which relate to the present invention. All three of these algorithms have the general structure illustrated in Figure 3.

7.1 Using 1-D data-driven patient grouping

1. For each gene *i*, compute the 10th quantile $(q_{10}^{i})$ and the 90th quantile $(q_{90}^{i})$ of the distribution of the *K* signal intensity values (i.e. the expression levels of the *K* patients). Within $(q_{10}^{i}, q_{90}^{i})$, we search for the value that most successfully discriminates the two unknown genetic classes.
2. Form the candidate cut-offs vector of dimension 1 x Q, $\underline{w}^{i} = y_{i}^{*}$, where $y_{i}^{*}$ is the log-transformed intensities within $(q_{10}^{i}, q_{90}^{i})$ and Q is the number of elements in $\underline{w}^{i}$. Each element of the $\underline{w}^{i}$ is a trial cut-off value. For *i* = 1 and $c^{i} = \underline{w}_{z}^{i}$, z = 1, ..., Q use

$$x_{k}^{i} = \begin{cases} 1\ (high-risk) & if \quad y_{i,k} > c^{i} \\ 0\ (low-risk) & if \quad y_{i,k} \le c^{i} \end{cases}$$

to separate the *K* subjects with $c^i$.
3. Evaluate the prognostic significance of gene *i* by reporting the p-value of the estimated $\beta_i$ from Cox proportional hazards model and test the proportional hazards assumption using Grambsch and Therneau (1994) test that uses the scaled Schoenfeld residuals (Schoenfeld, 1982):

$$r_{i} = x_{k}^{i} - \sum_{k=1}^{j \in R(t_k)} \beta_{i} x_{k}^{i}$$

4. Fit the parametric proportional hazards models of the form:

$$t_{k} = \alpha_{i}(t_{k}) + \beta_{i} x_{k}^{i}$$

and report the p-value of the estimated $\beta_i$ estimated coefficient. Then, estimate the Bayesian Information Criterion (BIC) to check which parametric model best fits the data. BIC is estimated by:

$$BIC = -2 * \log L + p \times \log(K)$$

where *L* denotes the maximized value of the likelihood function of the estimated model, *p* is the number of parameters of the model and *K* is the number of observations (patients).
5. If the proportional hazards assumption is satisfied one can keep either the Cox proportional hazards model or the best parametric model (in terms of BIC). Else, proceed to the next step.
6. Use the stratified Cox model using a variable to create the strata from a set of clinical variables available in Kuznetsov et al. (2006). Check whether the proportionality assumption is satisfied. If it is not satisfied proceed to the next step.
7. Fit the parametric survival models that do not assume proportional hazards and find the best one in terms of BIC
8. Iterate for all genes *i* = 2, ..., *N*
9. Select as prognostic significant the genes with significantly small p-values ($p < \alpha = 1\%$).

7.2 Using 2-D Data-driven grouping

1. For a given pair *i*, *j* with individual cut-offs $c^i$ and $c^j$, $i \neq j$, we may classify the patients by seven possible two-group designs.
2. For $i$ = 1 and $j$ = 2, group the patients by each of the seven designs of Figure 2 (using individual gene cut-offs) and for each design estimate the seven Wald p-values for $\beta_{i,j}$ using the Cox proportional hazards model. The best grouping scheme among the five "synergetic" (1-5) and the two "independent" (6-7) models is the one with the smallest p-value $\beta_{i,j}$.
3. Test the proportional hazards assumption using Grambsch and Therneau (1994) test that uses the scaled Schoenfeld residuals (Schoenfeld, 1982):

$$r_{ij} = x_k^{ij} - \sum_{k=1}^{j,\in R(t_k)} \beta_{ij} x_k^{ij}$$

4. Fit the parametric proportional hazards models of the form:

$$t_k = \alpha_{ij}(t_k) + \beta_{ij} x_k^{ij}$$

and report the p-value of the estimated $\beta_{i,j}$ estimated coefficient (*m* denotes the best model as estimated from step 2). Then, estimate the Bayesian Information Criterion (BIC) to check which parametric model best fits the data. BIC is estimated by:

$$BIC = -2 * \log L + p \times \log(K)$$

where *L* denotes the maximized value of the likelihood function of the estimated model, *p* is the number of parameters of the model and *K* is the number of observations (patients).
5. If the proportional hazards assumption is satisfied one can keep either the Cox proportional hazards model or the best parametric model (in terms of BIC). Else, proceed to the next step.
6. Use the stratified Cox model using a variable to create the strata from a set of clinical variables available in Kuznetsov et al. (2006). Check whether the proportionality assumption is satisfied. If it is not satisfied proceed to the next step.
7. Fit the parametric survival models that do not assume proportional hazards and find the best one in terms of BIC
8. Iterate for all genes $i$ = 2, ..., $N$ - 1 and $j$ = $i$ + 1, ..., $N$

Select as prognostic significant the genes with significantly small p-values ($p < \alpha = 1\%$).

7.3 Using Ratio data driven Grouping (the ratio-method)

1. Compute the difference of the log-transformed intensities of two selected genes *i* and *j*.
2. Compute the 10th quantile $(q_{10}^i)$ and the 90th quantile $(q_{90}^i)$ of the distribution of the *K* log-difference signal intensity values. Within $(q_{10}^i, q_{90}^i)$, we search for the value that most successfully discriminates the two unknown genetic classes.
3. Form the candidate cut-offs vector of dimension 1 x *Q*, $\underline{w}^i = y_{ij}^*$, where $y_{ij}^*$ is the log-differenced intensities $y_{i,k} - y_{j,k}$ within $(q_{10}^i, q_{90}^i)$ and *Q* is the number of elements in $\underline{w}^i$. Each element of the $\underline{w}^i$ is a trial cut-off value. For $i$ = 1 and $c^i = \underline{w}_z^i$, z = 1, ..., *Q* use

$$x_k^{ij} = \begin{cases} 1\ (high-risk) & if \quad y_{i,k} - y_{j,k} > c^{ij} \\ 0\ (low-risk) & if \quad y_{i,k} - y_{j,k} \le c^{ij} \end{cases}$$

to separate the *K* subjects with $c^{ij}$.

4. Evaluate the prognostic significance of gene *i* by reporting the p-value of the estimated $\beta_{i,j}$ from Cox proportional hazards model and test the proportional hazards assumption using Grambsch and Therneau (1994) test that uses the scaled Schoenfeld residuals (Schoenfeld, 1982):

$$r_{ij} = x_k^{ij} - \sum_{k=1}^{j_* \in R(t_k)} \beta_{ij} x_k^{ij}$$

1. Fit the parametric proportional hazards models of the form:

$$t_k = \alpha_{ij}(t_k) + \beta_{ij} x_k^{ij}$$

and report the p-value of the estimated $\beta_{i,j}$ estimated coefficient. Then, estimate the Bayesian Information Criterion (BIC) to check which parametric model best fits the data. BIC is estimated by:

$$BIC = -2 * \log L + p \times \log(K)$$

where *L* denotes the maximized value of the likelihood function of the estimated model, *p* is the number of parameters of the model and *K* is the number of observations (patients).

2. If the proportional hazards assumption is satisfied one can keep either the Cox proportional hazards model or the best parametric model (in terms of BIC). Else, proceed to the next step.

3. Use the stratified Cox model using a variable to create the strata from a set of clinical variables available in Kuznetsov et al. (2006). Check whether the proportionality assumption is satisfied. If it is not satisfied proceed to the next step.

4. Fit the parametric survival models that do not assume proportional hazards and find the best one in terms of BIC

5. Iterate for all genes *i* = 2, ..., *N*

6. Select as prognostic significant the genes with significantly small p-values ($p < \alpha = 1\%$).

8. Application and experimental results

Breast cancer is most common malignancy among women (van't Veer et al., 2002; Sotiriou et al.,2001). We compare our data-driven grouping for individual genes (DDg) and the data-driven grouping for ratios (DDgR) methods by considering the expression patterns of approximately 30,000 gene transcripts (representing by 44,928 probesets (p.s.) on Affymetrix U133A and U133B arrays) in 315 primary breast tumors (NCBI Gene Expression Omnibus (GEO) data sets GSE4922 and GSE1456). The tumor samples were derived from two independent population-based cohorts: Uppsala (249 samples) and Stockholm (159 samples). For details on patients, clinical information, tumor samples and microarrays see (Ivshina et al., 2006).

Information on patients' disease free survival (DFS) times/events and the expression patterns of gene transcripts on Affymetrix U133A and U133b arrays in all primary breast tumors were obtained from NCBI Gene Expression Omnibus (GEO) (Stockholm data set label is GSE4922; Uppsala data set label is GSE1456). The microarray intensities were normalized (by RMA) and the probe set signal intensities were log-transformed and scaled by adjusting the mean signal to a target value of log500 (Liu et al., 2006).

1 D *analysis*

The first step of our analysis involves estimating the $\beta_j$ of the Cox Proportional hazards survival model:

$$\log h^i{}_k(t_k \mid x^i_k, \beta_i) = \alpha_i(t_k) + \beta_i \cdot x^i_k$$

for each cohort, where $i$ = 1, ..., 44928 denotes the number of probesets. To do this, we apply our data-driven grouping for individual genes (DDg) using the *survival* package in the R library. As described above we are particularly interested in storing the p-value of each $\beta_i$ coefficient, which we consider as a measure of group discrimination. At significance level $\alpha$ = 1%, we identified 9930 prognostic significant probesets (6559 prognostic gene signatures) in Stockholm and 9737 in Uppsala (6353 prognostic gene signatures).

Figure 4 presents the distribution of p-values for the 44928 probesets and the 1% cut-off (red line) below which we declare a probe (and the respective gene) as survival significant. The two lists of significant genes obtained from each cohort were compared and 3375 common probesets identified. These common probesets, whose p-values distribution is presented at Figure 5, form a more reliable prognostic list since they are observed as significant in two independent cohorts.

To further validate the significance of our findings (in terms of the estimated p-values) we bootstrap the Stockholm and Uppsala samples and estimate 99% Bias Correction acceleration (BCa) confidence intervals for the $\beta_i$ coefficients of the Cox proportional hazards model. We use the non-parametric residuals bootstrap approach described above. By applying this second, independent test we intend to produce a more refined and reliable list of prognostic significant genes. Indeed, the residuals bootstrap method resulted in 1844 common Stockholm-Uppsala probesets, verified by two independent statistical procedures and two independent cohorts. Tables 3-4 present the 10 top-list probesets identified by our 1D algorithm under Cox and the best (in terms of BIC) parametric models in two cohorts.

Table 3. Top-level probesets identified by ID grouping in Stockholm. The p-value (and the cutoff) of the Cox and the best parametric model is reported along with the p-value of the test for proportional hazards (PHtest; p-values lower than 5% is an indication to reject the assumption of proportional hazards).

| AffyID | Symbol | Cox pvalue (cut) | PHtest | Parametric pvalue(cut) | Model(BIC) |
|---|---|---|---|---|---|
| A.201903_at | UQCRC1 | 4.2E-06(8.44) | 0.92 | 1.9E-06(8.44) | Exponential(323.58) |
| A.206163_at | MAB21L1 | 5.2E-05(5.38) | 0.71 | 5.0E-05(5.55) | Expooential(324.75) |
| A.202154_x_at | TUBB3 | 2.1E-08(8.62) | 0.78 | 2.8E-09(8.62) | Exponential(316.16) |
| A.213726_x_at | TUBB2C | 5.3E-07(9.63) | 0.31 | 1.3E-07(9.63) | Exponential(321.91) |
| A.213034_at | KIAA0999 | 2.2E-07(7.02) | 0.96 | 4.2E-08(7.02) | Exponential(318.13) |
| B.222989_s_at | UBQLN1 | 2.9E-09(7.66) | 0.75 | 5.2E-10(7.66) | Exponential(307.60) |
| A.203612 at | BYSL | 2.3E-07(6.95) | 0.06 | 3.4E-08(6.95) | Exponential(319.19) |
| B.200075_s_at | GUK1 | 2.7E-06(9.42) | 0.75 | 1.2E-06(9.42) | Exponential(323.93) |
| A.212188_at | KCTD12 | 5.2E-07(7.06) | 0.55 | 1.2E-07(7.06) | Exponential(324.65) |
| B.200065_s_at | ARF1 | 94E-07(10.1) | 0,67 | 2.2E-07(10.9) | Exponential(322.14) |

Table 4. Top-level probesets identified by ID grouping in Uppsala. The p-value (and the cutoff) of the Cox and the best parametric model is reported along with the p-value of the test for proportional hazards (PHtest; p-values lower than 5% is an indication to reject the assumption of proportional hazards).

| AffyID | Symbol | Cox pvalue (cut) | PHtest | Parametric pvalue(cut) | Model(BIC) |
|---|---|---|---|---|---|
| A.201903_at | UQCRC1 | 1.4E-08(8.61) | 0.96 | 8.2E-07(8.61) | Gaussian(808.96) |
| A.206163_at | MAB21L1 | 3.9E-09(4.58) | 0.98 | 1.7E-06(4.58) | Gaussian(811.10) |
| A.202154_x_at | TUBB3 | 2.7E-05(8.51) | 0.43 | 1.9E-04(8.51) | Gaussian(820.30) |
| A.213726_x_at | TUBB2C | 3.3E-06(9.63) | 0.26 | 1.5E-04(9.63) | Gaussian(820.09) |
| A.213034_at | KIAA0999 | 4.0E-05(7.09) | 0.62 | 4.4E-04(7.18) | Gaussian(821.80) |
| B.222989_s_at | UBQLN1 | 5.7E-04(7.22) | 0.50 | 5.5E-03(7.06) | Gaussian(825.70) |
| A.203612_at | BYSL | 9.3E-05(6.79) | 0.33 | 2.3E-04(6.79) | Gaussian(820.51) |

(continued)

| AffyID | Symbol | Cox pvalue (cut) | PHtest | Parametric pvalue(cut) | Model(BIC) |
|---|---|---|---|---|---|
| B.200075_s_at | GUK1 | 9.9E-06(9.79) | 0.85 | 6.2E-05(9.70) | Gaussian(818.27) |
| A.212188_at | KCTD12 | 7.0E-05(7.86) | 0.22 | 6.4E-05(7.88) | Gaussian(817.90) |
| B.200065_s_at | ARF1 | 4.6E-05(10.9) | 0.95 | 3.9E-04(11.1) | Gaussian(821.92) |

Notice that in Tables 3-4 none of the probesets rejects the assumptions of the proportionality of the hazards. Particularly, this is true for approximately 99% of the individual cases, where the stratified Cox proportional hazards model and the parametric non-proportional hazards alternatives needed to be evaluated.

*2D analysis*

We proceed further by using this reliable list of 1844 probes. By sorting the survival p-values in increasing order we identified the top-level 400 probes (corresponding to 310 unique genes) with which we form all possible 79800 gene pairs. Using these pairs we apply our 2D grouping method in Stockholm and Uppsala cohorts. The 2D grouping has been developed by Motakis et al (2008). The 2D grouping algorithm seems to improve significantly the patients' grouping obtained by individual genes. At 1% significance level, our analysis revealed 50021 significant probesets in Stockholm and 49986 in Uppsala. To validate mathematically our results and be able to select more reliable gene pairs, we refine our lists, based on three criteria;

*Criterion 1*: the gene synergy (as indicated by the p-values) is highly significant (we require the synergetic p-value to be significantly lower than the p-values of the individual genes of each pair);
*Criterion 2:* criterion 1 is satisfied in both cohorts;
*Criterion 3:* the patients' design (Figure 2) is the same in the two cohorts.

In several cases criterion 3 fails to be satisfied resulting in exclusion of several highly significant and biologically meaningful pairs. To solve this problem we propose here an extension of our 2D algorithm:

1. Estimate the $\beta_i^z$ $(z = 1, \ldots, Q)$ Wald p-values (the model coefficients for all possible cut-offs included in vector $\underline{w}_i$) for the two cohorts and sort them in increasing order. Store the design by which each p-value in the sorted list is obtained.
2. In each cohort, keep the best solution (triplet: minimum possible Wald p-value, design and patients grouping) which satisfy criteria 1-3.

This procedure results in 20665 highly significant, reproducible synergetic genes by the extended, design-corrected 2D method (i.e. the method of section 7.2 above) at significance level a = 1%. Figure 6 shows the distribution of these 20665 p-values. To further refine our list we apply the residuals bootstrap method to these 20665 significant probesets. In addition, we use Bonferroni correction to reduce the number of false positives. Bonferroni procedure is known to be a conservative test against false positives/negatives but it is still a reliable measure when the data are characterized by unknown but significant dependence. At such cases False Discovery Rate (FDR; Benjamini and Hochberg, 1995) has been reported to produce unreliable results (Benjamini and Yekutieli, 2001).

In the Stockholm cohort we identified 6562 and in Uppsala 8303 significant gene pairs (by applying both BCa and Bonferroni to the original 20665 reproducible pairs). The distribution of the p-values is plotted at the bottom panel of Figure 6. Finally, we have found 2414 common, highly significant, BCa and Bonferroni validated prognostic pairs. Tables 5-6 present the 10 top-list probeset pairs identified by our 2D algorithm under Cox and the best (in terms of BIC) parametric models in two cohorts.

Notice that in Tables 5-6 none of the probesets rejects the assumptions of the proportionality of the hazards. Particularly, this is true for approximately 98.5% of the paired cases, where the stratified Cox model and the loglogistic/lognormal models needed to be evaluated.

Table 5. Top-level probeset pairs identified by 2D grouping in Stockholm. The p-value (and the design) of the Cox and the best parametric model is reported along with the p-value of the test for proportional hazards (PHtest; p-values lower than 5% is an indication to reject the assumption of proportional hazards).

| AffyID1 (Symbol) | AffyID2 (Symbol) | Cox pval(des) | PHtest | Parametric pval(cut) | Model(BIC) |
|---|---|---|---|---|---|
| A.201903_at(UQCRC1) | A.204825_at(MELK) | 2.2E-08(2) | 0.63 | 6.1E-09(2) | Exponential(314.90) |
| A.208074_s_at(AP2S1) | A.209642_at(BUB1) | 5.8E-11(2) | 0.39 | 5.3E-12(2) | Exponential(295.84) |
| A.213476_x_at(TUBB3) | A.218883_s_at(MLF1IP) | 1.2E-08(2) | 0.15 | 3.0E-09(2) | Exponential(309.69) |
| A.213034_at(KIAA0999) | A.213726_x_at(TUBB2C) | 2.8E-08(3) | 0.42 | 1.4E-08(3) | Exponential(307.32) |
| A.204962_s_at(CENPA) | B.232652_x_at(SCAND1) | 9.9E-10(2) | 0.30 | 1.5E-10(2) | Exponential(309.89) |
| A.203799_at(CD302) | A.209832_s_at(CDT1) | 2.9E-07(5) | 0.07 | 2.5E-07(5) | Exponential(322.91) |
| A.200860_s_at(CNOT1) | A.208074_s_at(AP2S1) | 9.5E-10(2) | 0.77 | 3.8E-10(2) | Exponential(311.37) |
| A.202338_at(TK1) | A.212070_at(GPR56) | 8.5E-08(2) | 0.09 | 2.2E-08(2) | Exponential(308.25) |
| A.202095_s_at(BIRC5) | A.218354_at(TRAPPC2L) | 1.5E-08(2) | 0.52 | 8.9E-09(2) | Exponential(317.10) |
| A.212189_s_at(COG4) | B.225541_at(RLP22L1) | 1.3E-07(4) | 0.53 | 2.6E-08(4) | Exponential(313.86) |

Table 6. Top-level probeset pairs identified by 2D grouping in Stockholm. The p-value (and the design) of the Cox and the best parametric model is reported along with the p-value of the test for proportional hazards (PHtest; p-values lower than 5% is an indication to reject the assumption of proportional hazards).

| AffyID1 (Symbol) | AffyID2 (Symbol) | Cox pval(des) | PHtest | Parametric pval(cut) | Model(BIC) |
|---|---|---|---|---|---|
| A.201903_at(UQCRC1) | A.204825_at(MELK) | 2.2E-08(2) | 0.79 | 1.8E-07(2) | Gaussian(806.44) |
| A.208074_s_at(AP2S1) | A.209642_at(BUB1) | 5.8E-11(2) | 0.87 | 2.3E-05(2) | Gaussian(816.46) |
| A.213476_x_at(TUBB3) | A.21883_s_at(MLF1IP) | 1.2E-08(2) | 0.96 | 1.9E-06(2) | Gaussian(810.91) |
| A.213034_at(KIAA0999) | A.213726_x_at(TUBB2C) | 2.8E-08(3) | 0.90 | 8.0E-07(3) | Gaussian(809.91) |
| A.204962_s_at(CENPA) | B.232652_x_at(SCAND1) | 9.9E-10(2) | 0.58 | 2.5E-05(2) | Gaussian(815.97) |
| A.203799_at(CD302) | A.209832_s_at(CDT1) | 2.9E-07(5) | 0.29 | 1.3E-06(5) | Gaussian(810.85) |
| A.200860_s_at(CNOT1) | A.208074_s_at(AP2S1) | 9.5E-10(2) | 0.98 | 8.6E-06(2) | Gaussian(814.46) |
| A.202338_at(TK1) | A.212070_at(GPR56) | 8.5E-08(2) | 0.49 | 3.3E-06(2) | Gaussian(812.13) |
| A.202095_s_at(BIRC5) | A.218354_at(TRAPPC2L) | 1.5E-08(2) | 0.44 | 9.9E-07(2) | Gaussian(810.69) |
| A.212189_s_at(COG4) | B.225541_at(RLP22L1) | 1.3E-07(4) | 0.99 | 2.8E-08(4) | Gaussian(801.54) |

*Gene ratio analysis*

At the final step of our analysis we attempt to compare the results obtained so far by 1 D and 2D grouping approaches against our new ratio data-driven grouping method (DDgR). At this point we consider all 20665 significant probe pairs of the 2D DDg step. Notice that each individual gene, included in this list, is highly significant by our 1D data-driven method.

Each pair is transformed to a log ratio (or log-difference) of intensities; i.e. if we denote by $I_1$ as the vector of raw intensities of Affy1 and $I_2$ as the vector of raw intensities of Affy2 for two probesets Affy1 and Affy2 that form a pair, then the log-ratio (or log-difference):

$$\log\frac{I_1}{I_2} = \log I_1 - \log I_2$$

forms the ratio of interest. In this way we form a large sample of 20665 ratios of intensities composed of well-characterized, survival significant genes and gene pairs. By using the DDgR approach, we estimate the survival p-values of $\beta_1$ coefficients, which we compare to our previous results. Tables 7-8 give the 10 top-level probes (and gene symbols) identified by the DDgR and their respective survival p-values in Stockholm and Uppsala cohorts.

Table 7. Affy Ids (gene names; symbols) and survival p-values of 10 top-level genes by DDgR method in Stockholm. The p-value (and the cutoff) of the Cox and the best parametric model is reporter along with the p-value of the test for proportional hazards (PHtest; p-values lower than 5% is an indication to reject the assumption of proportional hazards).

| Affy ID1 (Symbol) | Affy ID2 (Symbol) | Cox pvalue (cut) | PHtest | Parametric pvalue(cut) | Model(BIC) |
|---|---|---|---|---|---|
| A.212189_s_at(COG4) | A.213034_at(KIAA0999) | 1.0E-10(-0.02) | 0.72 | 9.2E-12(-0.02) | Exponential(307.97) |
| A.206163_at(MAB2IL1) | A.2(38074_s_at(AP2S1) | 1.1E-08(-3.22) | 0.79 | 3.4E-09(-3.21) | Exponential(311.01) |
| A.202154_x_at(TUBB3) | A.213034_at(KIAA0999) | 8.2E-10(1.49) | 0.99 | 2.6E-07(1.07) | Lognormal(307.22) |
| A.208074_s_at(AP2S1) | A.214894_x_at(MACF1) | 6.6E-08(0.60) | 0.24 | 1.3E-08(0.59) | Exponential(319.12) |
| A.202763_at(CASP3) | A.218514_at(C12orf35) | 6.1E-06(-0.09) | 0.50 | 6.8E-06(-0.20) | Lognormal(321.44) |
| A.200075_s_at(GUK1) | A.206163_at(MAB2IL1) | 5.6E-08(3.16) | 0.85 | 4.5E-07(3.09) | Loglogistic(304.73) |
| A.200886_s_at(PGAM1) | A.212526_at(SPG20) | 9.7E-08(2.81) | 0.79 | 1.8E-08(2.80) | Exponential(318.86) |
| A.213892_s_at(APRT) | A.219563_at(C14orf139) | 6.5E-08(1.36) | 0.19 | 1.7E-08(1.35) | Exponential(311.92) |
| A.214077_x_at(MEIS3P1) | A.219395_at(RBM35B) | 9.2E-09(-0.40) | 0.61 | 1.6E-09(-0.40) | Exponential(315.29) |
| A.208968_s_at(CIAPIN1) | A.214077_x_at(MEIS3P1) | 1,1E-07(1,20) | 0.58 | 4.9E-06(0.65) | Lognormal(322.04) |

Table 8. Affy Ids (gene names; symbols) and survival p-values of 10 top-level genes by DDgR method in Uppsala. The p-value (and the cutoff) of the Cox and the best parametric model is reported along with the p-value of the test for proportional hazards (PHtest; p-values lower than 5% is an indication to reject the assumption of proportional hazards).

| AffyIDI (Symbol) | Affy ID2 (Symbol) | Cox pvalue (cut) | PHtest | Parametric pvalue(cut) | Model(BIC) |
|---|---|---|---|---|---|
| A.212189_s_at(COG4) | A.213034_at(KIAA0999) | 6.4E-08(-0.10) | 0.89 | 8.8E-07(-0.10) | Gaussian(809.01) |
| A.206163_at(MAB2IL1) | A.208074_s_at(AP2S1) | 1.6E-09(-3.90) | 0.99 | 2,8E-07(-3,34) | Gaussian(807.08) |
| A.202154_x_at(TUBB3) | A.213034_at(KIAA0999) | 3.3E-08(1.16) | 0.44 | 1.3E-06(1.16) | Gaussian(810.80) |
| A.208074_s_at(AP251) | A.214894_x_at(MACF1) | 1.6E-08(0.41) | 0.18 | 1.9E-07(0.41) | Gaussian(806.67) |
| A.202763_at(CASP3) | A.218614_at(C12orF35) | 2.9E-11(0.03) | 0.84 | 2.4E-08(-0.01) | Gaussian(803.13) |
| A.200075_s_at(GUK1) | A.206163_at(MAB2IL1) | 1.1E-08(3.75) | 0.49 | 2.4E-07(3.65) | Gaussian(806.56) |
| A.200886_s_at(PGAM1) | A.212526_at(SPG20) | 8.1E-09(3.44) | 0.45 | 5.2E-06(3.44) | Gaussian(813.63) |
| A.213892_s_at(APRT) | A.219563_at(C14orf139) | 3.2E-08(1.60) | 0.17 | 4.1E-06(1.60) | Gaussian(813.49) |
| A.214077_x_at(MEIS3P1) | A.219395_at(RBM35B) | 4.0E-07(-1.63) | 0.35 | 1.4E-05(-1.63) | Gaussian(815.89) |

Notice that in Tables 7-8 none of the probesets rejects the assumptions of the proportionality of the hazards. Particularly, this is true for approximately 99% of the paired cases, where the stratified Cox model and the loglogistic/lognormal models needed to be evaluated.

Our new ratio approach improved the classification of 603 out of 20665 (approximately 3%) probe pairs. Each of these survival p-values was lower than both the 2D data-driven and the two 1 D data-driven p-values of the respective pair. Note also that the ratio approach improved the patient grouping for 383 out of 400 individual probes (approximately 96%) that formed our gene pairs and consequently our gene ratios in our analysis. Figures 7-12 show 3 indicative examples of our comparative analysis across the 2 cohorts. Figures 7-10 show two successful implementations of our new DDgR approach while the last two figures indicate the superiority of 1D and 2D method in some cases.

**Biological Results and Conclusion**

The above preliminary results show that DDgR method and its combination with DDg can be considered as a novel fully automatic and robust methodology for a dual biomedical and patient grouping searching: (1) selection of survival /clinical significant genes (or other available tumor data) providing synergistic effect on patient survival and (2) optimal grouping of the patients onto poorly and good disease outcome groups. Such tasks are appeared in several biomedical studies.

The embodiments can provide practical interest for identification key genetic genes and their interactive gene pairs related directly to patho-biological mechanisms, prediction of disease outcome and screening novel therapeutic targets.

The embodiments were able to find many hundreds robust and statistically strong confidence genes and gene pairs. Several top-level robust gene markers present on the figures 7-12 and Tables 3-8.

Among identified survival significant genes, some of the genes have been considered as clinically significant genes For example, ARF1 (Aurka, STK6), and MELK are survival significant genes which have been included in the genomic grading classification of breast cancers (Ivshina et al, 2006, Kuznetsov et al, 2008). Some of the survival significant genes of Tables 3-8 are in clinical trials (e.g. Survivin, TYMS, MELK). Importantly, combination of mRNA markers in blood including Survivin and TK1(Thymidine kinase 1) has been used in the diagnosis of patients with breast cancer (Chen et al, 2006). It was also shown that patients' clinic-pathological characteristics tumor size ($p = 0.006$), histologic grade ($p = 0.012$), lymph node metastasis ($p = 0.001$) and TNM stage ($p = 0.006$) significantly correlated with the positive detection rate of these cancer markers. We suggest that not only Survivin and TK1 but other cancer-prognostic multi-markers of Tables 3-8 and could be also used in their combination to detect circulating tumor cells (CTCs) in the circulation of breast cancer patients with considerably high sensitivity and specificity.

Thus, our method can provide practical interest for identification key genetic genes and their interactive gene pairs and composed survival significant genes (SSGs) and their products (mRNAs, proteins, peptides) related directly to pathobiological mechanisms, prediction of disease outcome and screening novel therapeutic targets.

In particular, spartin (SPG20) gene (which is involved in the intracellular trafficking of EGFR and that impaired endocytosis may underlie the pathogenesis of Troyer syndrome) and gene PGAM1 (Phosphoglycerate mutase 1, involved in glycolise pathway) are strongly predicted by the DDgR as the new breast cancer survival synergetic genes pairs. In the literature, the both genes have not been associated with breast cancer and cancer survival. However, our additional analysis of expression PGAM1 based on longSAGE tag (cgap.nci.nih.gov/SAGE) DB demonstrates that this gene is strongly over-expressed in embryonic stem cells, and several cancers, including breast cancer cell line.

DDg and DDgR the both predict that synergic TUBB3--KIAA0999 gene pair. TUBB3 (Tubulin 3) is the major constituent of microtubules. It binds two moles of GTP, one at an exchangeable site on the beta chain and one at a non-exchangeable site on the alpha-chain. This gene is predicted to be survival significant pairing with unknown gene KIAA0999. We found that ChIP-PET experiments show over-expression TUBB3 in ER-positive breast cancer cell line MCF7 cells and in the human ESC hES3 cells. Recently, KIAA099 was included in the list of "candidate of breast cancer genes", which have been subjected to mutational selection during tumorgenesis (Sjoblom et al, 2006). However, survival significant of this hypothetical gene was not discussed.

We conclude that the embodiments provide a novel approach to identify small gene signatures providing statistically reliable, biological important and clinical significant molecular markers.

Table.9. PGAM1 gene top-level expression in ESC and many cancers. SAGE Tag: GGTCCAGTGT
http://cgap.nci.nih.gov/SAGE/FreqsOfTag?ORG=Hs&METHOD=SS10,LS10&FORMAT=html&TAG=GGTCCAGTGT

| Library | Total Tags in Library | Tag Counts | Tags per 200,000 |
|---|---|---|---|
| SAGE_Retina_macula_normal_B_HMAC2 | 102417 | 200 | 390 |
| SAGE Ovary normal CS HOSE 4 | 47728 | 90 | 377 |
| SAGE_Embryonic_stem_cell_H13_normal_p22_CL_SHE15 | 221101 | 381 | 344 |
| SAGE Colon carcinoma CL Hct116 p53 knockout Anoxia | 79053 | 124 | 313 |
| SAGE_Embryonic_stem_cell_HES3_normal_p16_CL_SHE10 | 205353 | 321 | 312 |
| SAGE_Retina_Peripheral_normal_B_2 | 105312 | 160 | 303 |
| SAGE_Vein_normal_B_hs0237 | 6135688 | 8698 | 280 |
| SAGE_Embryonic_stem_cell_HES4_normal_p36_CL_SHE11 | 209232 | 292 | 279 |
| SAGE_Testis_Embryonal_Carcinoma_CL_has0213 | 5935490 | 8307 | 279 |
| SAGE_Embryonic_stem_cell_H14_normal_p22_CL_SHE14 | 212170 | 273 | 257 |
| SAGE_Embryonic_stem_cell_H9_normal_p38_CL_SHES1 | 151735 | 191 | 251 |
| SAGE_Retina_Macula_normal_B_4Mac | 101417 | 126 | 248 |
| SAGE_Ovary_andometriosis_CL_E12 | 76097 | 93 | 244 |
| SAGE_Breast_carcinoma_CL_MDA435C | 47270 | 56 | 236 |
| SAGE_Colon_carcinoma_CL_Hct116_wildttype_p53_Anoxia | 79015 | 93 | 235 |
| SAGE_Colon_carcinoma_CL_Hct116_p53_knockout_Normal Oxygen | 79284 | 91 | 229 |
| SAGE_Brain_glioblastoma_control_CL_H247 | 60428 | 68 | 225 |
| SAGE_Ovary_normal_CL_IOSE29EC-11 | 47159 | 53 | 224 |
| SAGE_Testis_Embryonal_Carcinoma_CL_hs0212 | 6370161 | 7124 | 223 |
| SAGE_Embryonic_stem_cell_H1_normal_p31_CL_SHE17 | 276203 | 293 | 212 |
| SAGE_Brain_astroctytoma_grade_II_B_H501 | 128309 | 133 | 207 |
| SAGE_Kidney embryonic_CL_293+beta-catenin | 23519 | 24 | 204 |
| SAGE_Uterus endometrium_normal_CS_Dlt | 21991 | 22 | 200 |
| SAGE_Embryonic_stem_cell_H7_normal_p33_CL_SHE13 | 272465 | 271 | 198 |

(continued)

| Library | Total Tags in Library | Tag Counts | Tags per 200,000 |
|---|---|---|---|
| SAGE_Retina_Peripheral_normal_B 1 | 59661 | 59 | 197 |
| SAG_ Ovary_carcinoma_CL_A2780 | 21369 | 21 | 196 |

The DDg method (but not DDgR) predicts strong clinical significance of genes H2AFZ and RBM35B and its pair H2AFZ-RBM35B. Expression H2AFZ in breast cancer cells was increased and the prognostic power of these biomarkers demonstrated in clinical data (Hua at al., 2008). Gene RBM35B is involved in RNA binding; however, biological function and clinical significance of this gene has been not unknown.

RBM35B forms also the survival significant gene pair with gene MEIS3P1. MEIS3P1 is the homo sapiens Meis homeobox 3 pseudogene 1 which also might be classified as non-coding RNA gene. This gene appears to be a retrotransposed pseudogene based on its lack of exons compared to other family members, one of which is found on chromosome 19 (MEIS3). The MESI3P1 pseudogene lacks three exons compared to MESI3. However, both genes encode proteins of similar size and sequence and contain the same homeodomain, which is a one-of-a-kind DNA binding domain involved in the transcriptional regulation of key eukaryotic developmental processes. MESI3P1 could activate the cAMP-response element (CRE) pathway (Linjie Tian et al, 2007). MEI3P1 has DDgR partner CIAPIN1. CIAPIN1 is a cytokine-induced inhibitor of apoptosis with no relation to apoptosis regulatory molecules of the BCL2 (MIM 151430) or CASP (see MIM 147678) families. Expression of CIAPIN1 is dependent on growth factor stimulation (Shibayama et al., 2004). CIAPIN1, a newly identified apoptosis-related molecule, was found to be down-regulated in several human cancers as compared to the matched adjacent non-neoplastic tissues. It was found that CIAPIN1 is a suppressor of gastric cancer cell proliferation. Due to strong survival significance, we suggest that CIAPIN1might play an important role in the development of human breast cancer.

Thus, the list of selected genes demonstrates that our survival significance analysis via microarrays can be a powerful technique in elucidating the functions of many novel genes in normal and cancerous tissues. However, many of survival significant and synergetic genes pairs are poorly studied in context of prediction of biological functions and clinical prognostic significance. Our GO network analysis shows that more than 60% gene of top SSSG (Tables 3-8) are not included in the known gene interaction networks (data not presented). These findings strongly suggest that our methodology able to discovery many new clinically important markers and promising molecular targets for drug development and validation.

Among identified survival significant genes, some of the genes have been considered as a clinically significant genes, some of these genes are in clinical trials(e.g. survivin, TYMS, MELK), but the most of identified genes and synergetic genes pairs are novel in context of prediction of biological essentiality and clinical prognostic significance for specific (cancer) diseases.

Our final step involves determining the patients' composite group based on the final list of significant genes and gene pairs identified by our 1D, 2D and DDgR grouping algorithms. In other words, we combine the information from all significant synergetic genes and gene pairs of Tables 3-8 into a single final grouping scheme and then we test whether our algorithm was able to identify two biologically distinct patients' groups.

Our final list of significant findings includes multiple entries of the same genes (e.g. AP2S1 appears at least once in all grouping methods). We assumed that redundancy implies highly correlated groups, which we would like to avoid (e.g. all groupings based on AP2S1 tend to be correlated). To deal with this problem, for each probe set *i* we sort in ascending order (from the lowest to the highest) the 2D and DDgR Wald P values $p_{ij}$ it generates with other probe sets *j, j* = 1, ..., *J* and keep the *i, j* that produces the lowest $p_{ij}$. Subsequently, we do not reconsider *i* and *j* either as members of a different pair or as individual entities. Next, we enrich our list with all 1 D significant probe sets that do not form any significant pair. We resulted in 17 top-level, representative significant pairs and 4 significant probe sets. These are shown in Table 10 below.

Table 10. Integrated list of survival significant genes

| AffyID1 | AffyID2 | Grouping method |
|---|---|---|
| A.213476_x_at(TUBB3) | A.218883_s_at(MLF1IP) | 2D |
| A.213034_at(KIAA0999) | A.213726_x_at(TUBB2C) | 2D |
| A.209903_at(UQCRC1) | A.20482_at(MELK) | 2D |
| A.204962_s_at(CENPA) | B.23252_x_at(SCAND1) | 2D |
| A.203799_at(CD302) | A.209832_s_at(CDT1) | 2D |
| A.200860_s_at(CNOT1) | A.208074_s_at(AP2S1) | 2D |
| A.202338_at(TK1) | A.212070_at(GPR56) | 2D |
| A.202095_s_at(BIRC5) | A.218354_at(TRAPPC2L) | 2D |
| A.212189_s_at(COG4) | B.225541_at(RPL22L1) | 2D |
| | | |

(continued)

| AffyID1 | AffyID2 | Grouping method |
|---|---|---|
| A.200853_at(H2AFZ) | A.219395_at(RBM35B) | 2D |
| A.214077_x_at(MEIS3P1) | A.219395_at(RBM35B) | DDgR |
| A.202763_at(CASP3) | A.218614_at(C12orF35) | DDgR |
| A.208074_s_at(AP2S1) | A.214894_x_at(MACF1) | DDgR |
| A.200886_s_at(PGAM1) | A.212526_at(SPG20) | DDgR |
| A.200f375_s_at(GUK1) | A.206163_at(MAB21L1) | DDgR |
| A.208968_s_at(CIAPIN1) | A.214077_x_at(MEIS3P1) | DDgR |
| A.213892_s_at(APRT) | A.219563_at(C14orf139) | DDgR |
| B.222989_s_at(UBQLN1) | --- | 1D |
| A.203612_at(BYSL) | --- | 1D |
| A.2121188_at(KCTD12) | --- | 1D |
| B.200065_s_at(ARF1) | --- | 1D |

Table 11 indicates the SEQ ID NO. and the corresponding gene and GenBank reference in the sequence listing.

Where more than one transcript variant exists, the invention may be practiced with any one of the transcript variants, any of several of the transcript variants or all of the transcript variants.

Table 11. SEQ ID NOs and corresponding gene

| SEQ ID NO: | Gene | GenBank Reterence |
|---|---|---|
| 1 | TUBB3 | NM_006086.2 |
| 2 | KIA0999 (SIK3) | NM_025164.3 |
| 3 | SPG20 transcript variant 1 | NM_015087.4 |
| 4 | SPG20 transcript variant 2 | NM_001142296.1 |
| 5 | SPG20 transcript variant 3 | NM_01142295.1 |
| 6 | SPG20 var 4 | NM_001142294.1 |
| 7 | PGAM1 | NM_002629.2 |
| 8 | H2AFZ | NM_002106.3 |
| 9 | RBM35B (ESRP2) | NM_024939.2 |
| 10 | UQCRC1 | NM_003365.2 |
| 11 | TUBB2C | NM_006088.5 |
| 12 | MAB21L1 | NM_005584.3 |
| 13 | UBQLN1 var 1 | NM_013438.4 |
| 14 | UBQLN1 var 2 | NM_053067.2 |
| 15 | BYSL | NM_004053.3 |
| 16 | GUK1 var 1 | NM_001159390.1 |
| 17 | GUK1 var 2 | NM_000858.5 |
| 18 | GUK1 var 3 | NM_001159391.1 |
| 19 | KCTD12 | NM_138444.3 |
| 20 | ARF1 transcript variant 1 | NM_001024227.1 |

(continued)

| SEQ ID NO: | Gene | GenBank Reterence |
|---|---|---|
| 21 | ARF1 transcript variant 2 | NM_001024228.1 |
| 22 | ARF1 transcript variant 3 | NM_001024226.1 |
| 23 | ARF1 transcript variant 4 | NM_001658.3 |
| 24 | BUB1 | NM_004336.3 |
| 25 | MELK | NM_014791.2 |
| 26 | AP2S1 transcript variant AP17 | NM_004069.3 |
| 27 | AP2S1 transcript variant AP17delta | NM_021575.2 |
| 28 | MLF1IP | NM_024629.3 |
| 29 | CENPA transcript variant 1 | NM_001809.3 |
| 30 | CENPA transcript variant 2 | NM_001042426.1 |
| 31 | SCAND1 transcript variant 1 | NM_016558.2 |
| 32 | SCAND1 transcript var 2 | NM_033630.1 |
| 33 | CD302 | NM_014880.4 |
| 34 | CDT1 | NM_030928.3 |
| 35 | CNOT1 transcript var 1 | NM_016284.3 |
| 36 | CNOT1 transcript var 2 | NM_206999.1 |
| 37 | TK1 | NM_003258.4 |
| 38 | GPR56 transcript variant 1 | NM_005682.5 |
| 39 | GPR56 transcript variant 2 | NM_201524.2 |
| 40 | GPR56 transcript variant 3 | NM_201525.2 |
| 41 | GPR56 transcript variant 4 | NM_001145771.1 |
| 42 | GPR56 transcript variant 5 | NM_00145770.1 |
| 43 | GPR56 transcript variant 6 | NM_001145772.1 |
| 44 | GPR56 transcript variant 7 | NM_001145774.1 |
| 45 | GPR56 transcript variant 8 | NM_001145773.1 |
| 46 | BIRC5 transcript variant 1 | NM_001168.2 |
| 47 | BIRC transcript variant 2 | NM_001012270.1 |
| 48 | BIRC5 transcript variant 3 | NM_001012271.1 |
| 49 | TRAPPC2L | NM_016209.3 |
| 50 | COG4 | NM_015386.2 |
| 51 | RPL22L1 | NM_001099645.1 |
| 52 | MACF1 transcript variant 1 | NM_012090.3 |
| 53 | MACF1 transcript variant 2 | NM_033044.2 |
| 54 | CASP3 transcript variant alpha | NM_004346.3 |
| 55 | CASP3 transcript variant beta | NM_032991.2 |
| 56 | C12orf35 | NM_018169.3 |
| 57 | APRT transcript variant 1 | NM_000485.2 |

(continued)

| SEQ ID NO: | Gene | GenBank Reterence |
|---|---|---|
| 58 | APRT transcript variant 2 | NM_001030018.1 |
| 59 | C14orf139 | NR_026779.1 |
| 60 | MEIS3P1 | NR_002211.1 |
| 61 | CIAPIN1 | NM_020313.2 |

**References**

Ata, N. and Sozer, M.T. (2007). Cox regression models with non-proportional hazards applied to lung cancer survival data, Hacettepe Journal of Mathematics and Statistics, 36, 157-167.

Benjamini, Y. and Hochberg, Y. (1995). Controlling the false discovery rate: a practical and powerful approach to multiple testing, Journal of the Royal Statistical Society, Series B (Methodological) 57 (1): 289-300.

Benjamini, Y. and Yekutieli, D. (2001). The control of the false discovery rate in multiple testing under dependency, Annals of Statistics 29 (4): 1165-1188

Breslow, N.E., Covariance analysis of censored survival data, Biometrics, vol. 30, pp 89-99, 1974.

Broet P., Kuznetsov V.A., J. Bergh J., Liu E. T-B., Miller L.D. (2006) Identifying gene expression changes in breast cancer that distinguish early and late relapse among uncured patients. Bioinformatics, 22, 1477-1485.

Chen CC, Chang TW, Chen FM, Hou MF, Hung SY, Chong IW, Lee SC, Zhou TH, Lin SR. Combination of multiple mRNA markers (PTTG1, Survivin, UbcH10 and TK1) in the diagnosis of Taiwanese patients with breast cancer by membrane array. Oncology. 2006;70(6):438-46. Epub 2007 Jan 12.

Cox, D. R. (1972). Regression Models and Life Tables (with Discussion). Journal of the Royal Statistical Society, Series B 34:187-220.

Cox R.D. and Oakes, D.(1984). Analysis of Survival Data. London: Chapman and Hall.

Cox, D.R. and Snell, E.J. (1968). A general definition of residuals (with discussion)", Journal of the Royal Statistical Society, Series B, vol. 30, pp 248-265.

Cui, X. And Churchill, G.A. (2003). Statistical tests for differential expression in cDNA microarray experiments, Genome Biology, 4(4):210

Efron, B. and Tibshirani, R.J. (1994). An Introduction to the Bootstrap. New York: Chapman and Hall.

Goetz MP, Suman VJ, Ingle JN, Nibbe AM, Visscher DW, Reynolds CA, Lingle WL, Erlander M, Ma XJ, Sgroi DC, Perez EA, Couch FJ.(2006) A two-gene expression ratio of homeobox 13 and interleukin-17B receptor for prediction of recurrence and survival in women receiving adjuvant tamoxifen.Clin Cancer Res.;12(7 Pt 1):2080-7.

Grambsch, M.P. and Therneau, M.T. (1994). Proportional Hazards Tests and Diagnostics Based on Weighted Residuals, Biometrika, 81, 515-526.

Hua S, Kallen CB, Dhar R, Baquero MT, Mason CE, Russell BA, Shah PK, Liu J, Khramtsov A, Tretiakova MS, Krausz TN, Olopade OI, Rimm DL, White KP (2008). Genomic analysis of estrogen cascade reveals histone variant H2A.Z associated with breast cancer progression. Mol Syst Biol., 4:188. Epub 2008 Apr 15.

Ivshina AV, George J, Senko OV., Mow B, Putti TC, Smeds J, Nordgren H, Bergh J, Liu E T-B, Kuznetsov VA, Miller LD (2006). Genetic reclassification of histologic grade delineates new clinical subtypes of breast cancer. Cancer Res. 66, 10292-10301.

Kalbfleisch, J.D. and Prentice, R.L. (1980). The Statistical Analysis of Failure Time Data, New York: John Wiley & Sons, Inc.

Kuznetsov V. A., Senko O. V., Miller L. D. and Ivshina Anna V. (2006). Statistically Weighted Voting Analysis of Microarrays for Molecular Pattern Selection and Discovery Cancer Genotypes. Intern J of Computer Sciences and Network Security, 6, 73-83.

Kuznetsov, V.A., Motakis E. and Ivshina, A.V. (2008). Low- and High- Agressive Genetic Breast Cancer Subtypes and Significant Survival Gene Signatures, IEEE Congress on Computational Intelligence, Hong-Kong, June 1-6.

LeBrun D, Baetz T, Foster C, Farmer P, Sidhu R, Guo H, Harrison K, Somogyi R, Greller LD, Feilotter H. (2008) Predicting outcome in follicular lymphoma by using interactive gene pairs. Clin Cancer Res. 14(2):478-87.

Linjie Tian, Pingzhang Wang, 1, Jinh Guo, Xinyu Wang, Weiwei Deng, Chenying Zhang, Dongxu Fu, Xi Gao, Taiping Shi, Dalong Ma. Screening for novel human genes associated with CRE pathway activation with cell microarray' Genomics, 90, Issue 1, July 2007,28-34.

Loughin, T.M. (1995). A residual bootstrap for regression parameters in proportional hazards model", J. of Statistical and Computational Simulations, vol. 52, pp 367-384.
Motakis, E., Ivshina, A.V. and Kuznetsov, V.A. (2008). A data-driven method of identification of essential genes and gene pairs associated with survival time of cancer patients, IEEE Engineering in Medicine and Biology (to appear)
Ruth M. R., Adrian L. Harris, Lionel Tarassenko (2004). Non-linear survival analysis using neural networks, Statistics in Medicine, 23, 825-842
Schemper, M. (1992). Cox analysis of survival data with non-proportional hazard functions, Stafistician, 41, 455-465.
Schoenfeld D. (1982). Residuals for the proportional hazards regresssion model. Biometrika, 69(1):239-241.
Shibayama H, Takai E, Matsumura I, Kouno M, Morii E, Kitamura Y, Takeda J, Kanakura Y. Identification of a cytokine-induced antiapoptotic molecule anamorsin essential for definitive hematopoiesis.J Exp Med. 2004 Feb 16;199(4):581-92.
Sjoblom T, Jones S, Wood LD, Parsons DW, Lin J, Barber TD, Mandelker D, Leary RJ, Ptak J, Silliman N, Szabo S, Buckhaults P, Farrell C, Meeh P, Markowitz SD, Willis J, Dawson D, Willson JK, Gazdar AF, Hartigan J, Wu L, Liu C, Parmigiani G, Park BH, Bachman KE, Papadopoulos N, Vogelstein B, Kinzler KW, Velculescu VE.Science. 2006 Oct 13;314(5797):268-74. Epub 2006 Sep 7
Sotiriou, T., Perou, C.M., Tibshirani, R. et al. (2001). Gene expression patterns of breast carcinomas distinguish tumor subclasses with clinical implications. Proc Natl Acad Sci USA 98:10869-10874.
Therneau, M.T. and Grambsch, M.P. (2000). Modeling Survival Data: Extending The Cox Model. New York: Springer-Verlag.
Thompson, A.L., Chhikara, S.R. and Conkin, J. (2003). Cox proportional hazards models for modeling the time to onset of decompression sickness in hypobaric environments, NASA/TP-2003-210791, Manuscript.
Tibshirani, R., Hastie, T., Narasimhan, B. and Chu, G. (2002). Diagnosis of multiple cancer types by shrunken centroids of gene expression. PNAS, 99, 6567-6572. van't Veer, L.J., Dai, H., van de Vijver et al. (2002). Gene expression profiling predicts clinical outcome of breast cancer. Nature 415:530-536.

## Claims

**1.** A computerized method for identifying one or more genes, or pairs of genes, selected from a set of N genes, which are statistically associated with prognosis of a potentially fatal medical condition, the method employing test data which, for each subject k of a set *of K* subjects suffering from the medical condition, indicates (i) a survival time of subject k, and (ii) for each gene *i*, a corresponding gene expression value $y_{i,k}$ of subject *k*;
the method comprising:

(A) for each of a plurality of genes or pairs of genes, performing the sub-steps of:

(a) partitioning the K subjects into two subsets using cut-off values, (b) computationally fitting a Cox proportional hazard regression model to the corresponding test data of at least one of the subsets of subjects;
(c) determining whether a proportionality assumption of the Cox proportional hazard regression model is satisfied using the Bayesian Information Criterion;
(d) if the proportionality assumption is satisfied, fitting one or more additional proportional hazard regression models to the data-set;
(e) if the proportionality assumption is not satisfied, fitting at least one hazard regression model which does not satisfy a proportionality assumption to the data-set;
(f) selecting, from the fitted hazard regression models, a hazard regression model having best quality of fit based on the Bayesian Information Criterion; and
(g) obtaining a significance value indicative of prognostic significance of the gene or gene pair using the selected hazard regression model;

wherein when operation (A) is performed for each of the plurality of pairs of genes *(i, j* with $i \neq j$*)* of the set of N genes, operation (A) comprises:generating a respective plurality of trial cut-off values of $c_{i,j}$, and for each of the trial cut-off values:

partitioning the K subjects into two subsets according to whether $log(y_{i,k}) - log(y_{j,k})$ is respectively above or below the trial cut-off value $c_{i,j}$;
performing sub-operations (b)-(f); and
obtaining a significance value indicative of prognostic significance of the gene pair *i,j* using the selected hazard
regression model; and

for each of the pairs of genes, identifying the trial cut-off value $c_{i,j}$ for which the corresponding significance value indicates the highest prognostic significance for the gene pair *i,j*; and
(B) identifying one or more of the genes or pairs of genes for which the corresponding significance values have the highest prognostic significance.

**2.** A method according to claim 1 in which, in sub-step (e) the method includes fitting a stratified Cox model to the data-set.

**3.** A computerized method according to any preceding claim wherein the medical condition is cancer, and the expression levels are from samples of respective tumours in the K subjects.

**4.** A method according to claim 3 wherein the medical condition is breast cancer.

**5.** A method according to any preceding claim in which the survival time is survival time until mortality.

**6.** A method according to any preceding claim in which the survival time is a survival time without metastasis of a cancer.

**7.** A method according to any preceding claim further comprising:

(i) for each of the one or more identified genes, obtaining corresponding gene expression values of a first subject; and
(ii) obtaining information about the first subject in relation to the potentially-fatal medical condition using the obtained gene expression values, the selected hazard regression model and the cut-off values.

**8.** A method according to any preceding claim further comprising:

(i) for each of the one or more identified pairs genes, obtaining corresponding gene expression values of a first subject; and
(ii) obtaining information about the first subject in relation to the potentially-fatal medical condition using the obtained gene expression values, the selected hazard regression model and the identified trial cut-off value.

**9.** A method according to claim 7 or claim 8 in which said information is a prognosis for the first subject who is suffering from the medical condition, a susceptibility of the first subject to the medical condition, a prediction of the recurrence of the medical condition, or a recommended treatment for the medical condition.

**10.** A method according to any preceding claim, wherein sub-step (e) further includes fitting an additional non-proportional hazard regression model to the data-set.

**11.** A method according to any preceding claim further including performing step (A) for each of a plurality of genes and pairs of genes of the set of N genes.

**12.** A computer system arranged to perform a method according to any preceding claim.

**13.** A data carrier, such as a tangible data carrier, carrying software readable by a computer to cause the computer to perform a method according to any of claims 1 to 11.

**Patentansprüche**

**1.** Computergestütztes Verfahren zur Identifizierung eines oder mehrerer Gene oder Paare von Genen, die aus einem Satz von N Genen ausgewählt sind, die statistisch mit einer Prognose eines potenziell tödlichen medizinischen Leidens assoziiert sind, wobei das Verfahren Testdaten verwendet, die für jeden Patienten *k* aus einem Satz von K Patienten mit dem medizinischen Leiden (i) eine Überlebenszeit des Patienten *k* und (ii) für jedes Gen *i* einen entsprechenden Genexpressionswert $y_{i,k}$ des Patienten *k* indizieren:

wobei das Verfahren umfasst:

(A) für jedes einer Mehrzahl von Genen oder Paaren von Genen erfolgendes Durchführen der Teilschritte:

(a) Aufteilen der K Patienten unter Verwendung von Cutoff-Werten in zwei Teilsätze;
(b) rechnergestütztes Anpassen eines Proportional-Hazard-Regressionsmodells nach Cox auf die entsprechenden Testdaten zumindest eines der Teilsätze von Patienten;
(c) Ermitteln, ob eine Proportionalitätsannahme des Proportional-Hazard-Regressionsmodells nach Cox erfüllt ist, unter Verwendung des Bayesschen Informationskriteriums;
(d) wenn die Proportionalitätsannahme erfüllt ist, Anpassen einer oder mehrerer zusätzlicher Proportional-Hazard-Regressionsmodelle auf den Datensatz;
(e) wenn die Proportionalitätsannahme nicht erfüllt ist, Anpassen zumindest eines Hazard-Regressionsmodells, das eine Proportionalitätsannahme nicht erfüllt, an den Datensatz;
(f) Auswählen eines Hazard-Regressionsmodells mit der besten Anpassungsqualität auf Basis des Bayesschen Informationskriteriums aus den angepassten Hazard-Regressionsmodellen; und
(g) Erhalten eines Signifikanzwerts, der für eine Prognosesignifikanz des Gens oder Genpaars indikativ ist, unter Verwendung des ausgewählten Hazard-Regressionsmodells;

wobei, wenn Vorgang (A) für jedes der Mehrzahl von Paaren von Genen $(i, j,$ wobei $i \neq j)$ des Satzes von N Genen durchgeführt wird, Vorgang (A) umfasst: Generieren einer jeweiligen Mehrzahl von Test-Cutoff-Werten von $c_{i,j}$ und für jeden der Test-Cutoff-Werte:

Aufteilen der K Patienten in zwei Teilsätze auf Basis dessen, ob $log(y_{i,k}) - log(y_{j,k})$ jeweils über oder unter dem Test-Cutoff-Wert $c_{i,j}$ liegt;
Durchführen der Teilvorgänge (b)-(f); und
Erhalten eines Signifikanzwerts, der für eine Prognosesignifikanz des Genpaars *i*, *j* indikativ ist, unter Verwendung des ausgewählten Hazard-Regressionsmodells; und
für jedes der Paare von Genen erfolgendes Identifizieren der Test-Cutoff-Werte $c_{i,j}$, in Bezug auf welche der entsprechende Signifikanzwert die höchste Prognosesignifikanz für das Genpaar *i, j* indiziert; und

(B) Identifizieren eines oder mehrerer der Gene oder Paare von Genen, in Bezug auf welche die entsprechenden Signifikanzwerte die höchste Prognosesignifikanz haben.

**2.** Verfahren nach Anspruch 1, wobei das Verfahren im Teilschritt (e) das Anpassen eines stratifizierten Cox-Modells auf den Datensatz umfasst.

**3.** Computergestütztes Verfahren nach einem der vorstehenden Ansprüche, wobei das medizinische Leiden Krebs ist und die Expressionsniveaus von Proben von jeweiligen Tumoren der K Patienten abgeleitet sind.

**4.** Verfahren nach Anspruch 3, wobei das medizinische Leiden Brustkrebs ist.

**5.** Verfahren nach einem der vorstehenden Ansprüche, wobei die Überlebenszeit die Überlebenszeit bis zur Mortalität ist.

**6.** Verfahren nach einem der vorstehenden Ansprüche, wobei die Überlebenszeit eine Überlebenszeit ohne Krebsmetastasenbildung ist.

**7.** Verfahren nach einem der vorstehenden Ansprüche, das ferner umfasst:

(i) für jedes des einen oder der mehreren identifizierten Gene erfolgendes Erhalten entsprechender Genexpressionswerte eines ersten Patienten; und
(ii) Erhalten von Informationen über den ersten Patienten in Bezug auf das potenziell tödliche medizinische Leiden unter Verwendung der erhaltenen Genexpressionswerte, des ausgewählten Hazard-Regressionsmodells und der Cutoff-Werte.

**8.** Verfahren nach einem der vorstehenden Ansprüche, das ferner umfasst:

(i) für jedes der einen oder mehreren identifizierten Paare Gene erfolgendes Erhalten entsprechender Genexpressionswerte eines ersten Patienten; und
(ii) Erhalten von Informationen über den ersten Patienten in Bezug auf das potenziell tödliche medizinische Leiden unter Verwendung der erhaltenen Genexpressionswerte, des ausgewählten Hazard-Regressionsmodells und der identifizierten Test-Cutoff-Werte.

9. Verfahren nach Anspruch 7 oder 8, wobei die Informationen eine Prognose für den ersten Patienten mit dem medizinischen Leiden, eine Anfälligkeit des ersten Patienten für das medizinische Leiden, eine Prognose des Wiederauftretens des medizinischen Leidens oder eine empfohlene Behandlung für das medizinische Leiden ist.

10. Verfahren nach einem der vorstehenden Ansprüche, wobei der Teilschritt (e) ferner das Anpassen eines zusätzlichen Non-Proportional-Hazard-Regressionsmodells auf den Datensatz umfasst.

11. Verfahren nach einem der vorstehenden Ansprüche, das ferner das Durchführen von Schritt (A) für jedes der Mehrzahl von Genen und Paaren von Genen des Satzes von N Genen umfasst.

12. Computersystem, das so ausgelegt ist, dass es ein Verfahren nach einem der vorstehenden Ansprüche durchführt.

13. Datenträger wie z. B. ein materieller Datenträger, der Software trägt, die von einem Computer lesbar ist, um den Computer zu veranlassen, ein Verfahren nach einem der Ansprüche 1 bis 11 durchzuführen.

**Revendications**

1. Procédé informatisé d'identification d'un ou de plusieurs gènes, ou de paires de gènes, sélectionnés parmi un ensemble de N gènes, qui sont statistiquement associés au pronostic d'une condition médicale potentiellement mortelle, le procédé utilisant des données de test qui, pour chaque sujet *k* d'un ensemble de K sujets souffrant de la condition médicale, indiquent (i) un temps de survie du sujet *k,* et (ii) pour chaque gène *i,* une valeur d'expression génique correspondante $y_{i,k}$ du sujet *k;*
le procédé comprenant:

(A) pour chacun d'une pluralité de gènes ou de paires de gènes, effectuer les sous-étapes consistant à:

(a) partager les sujets K en deux sous-ensembles en utilisant des valeurs de coupure,
(b) ajuster computationnellement un modèle de Cox de régression à risques proportionnels aux données de test correspondantes d'au moins l'un des sous-ensembles de sujets;
(c) déterminer si une supposition de proportionnalité du modèle de Cox de régression à risques proportionnels est satisfaite en utilisant le critère d'information bayésien;
(d) si la supposition de proportionnalité est satisfaite, ajuster un ou plusieurs modèles de régression à risques proportionnels supplémentaires au jeu de données;
(e) si la supposition de proportionnalité n'est pas satisfaite, ajuster au moins un modèle de régression à risques qui ne satisfait pas à une supposition de proportionnalité au jeu de données;
(f) sélectionner, parmi les modèles de régression à risques ajustés, un modèle de régression à risques ayant la meilleure qualité d'ajustement sur la base du critère d'information bayésien; et
(g) obtenir une valeur de signification indicative de signification pronostique du gène ou de la paire de gènes en utilisant le modèle de régression à risques sélectionné;

où lorsque l'opération (A) est effectuée pour chacune de la pluralité de paires de gènes *(i, j avec* $i \neq j$*)* de l'ensemble de N gènes, l'opération (A) comprend le fait de: générer une pluralité respective de valeurs de coupure d'essai de $c_{i,j}$ et pour chacune des valeurs de coupure d'essai:

partager les *K* sujets en deux sous-ensembles selon si $log(y_{i,k})$ - $log(_{j,k})$ est respectivement au-dessus ou au-dessous de la valeur de coupure d'essai $c_{i,j}$;
effectuer les sous-opérations (b)-(f); et
obtenir une valeur de signification indicative de signification pronostique de la paire de gènes *i,j* en utilisant le modèle de régression à risques sélectionné; et
pour chacune des paires de gènes, identifier la valeur de coupure d'essai $c_{i,j}$ pour laquelle la valeur de signification correspondante indique la signification pronostique la plus haute pour la paire de gènes *i,j;* et

(B) identifier un ou plusieurs des gènes ou des paires de gènes pour lesquels les valeurs de signification correspondantes ont la signification pronostique la plus haute.

2. Procédé selon la revendication 1, dans lequel, à la sous-étape (e) le procédé comprend ajuster un modèle de Cox stratifié au jeu de données.

**3.** Procédé informatisé selon l'une quelconque des revendications précédentes, dans lequel la condition médicale est un cancer, et les niveaux d'expression proviennent d'échantillons de tumeurs respectives chez les K sujets.

**4.** Procédé selon la revendication 3, dans lequel la condition médicale est le cancer du sein.

**5.** Procédé selon l'une quelconque des revendications précédentes dans lequel le temps de survie est le temps de survie jusqu'à la mortalité.

**6.** Procédé selon l'une quelconque des revendications précédentes, dans lequel le temps de survie est un temps de survie sans métastase d'un cancer.

**7.** Procédé selon l'une quelconque des revendications précédentes, comprenant en outre:

(i) pour chacun de l'un ou des plusieurs gènes identifiés, obtenir des valeurs d'expression génique correspondante d'un premier sujet; et
(ii) obtenir des informations sur le premier sujet se rapportant à la condition médicale potentiellement mortelle en utilisant les valeurs d'expression génique obtenues, le modèle de régression à risques sélectionné et les valeurs de coupure.

**8.** Procédé selon l'une quelconque des revendications précédentes, comprenant en outre:

(i) pour chacune de l'une ou des plusieurs paires de gènes identifiées, obtenir des valeurs d'expression génique correspondante d'un premier sujet; et
(ii) obtenir des informations sur le premier sujet se rapportant à la condition médicale potentiellement mortelle en utilisant les valeurs d'expression génique obtenues, le modèle de régression à risques sélectionné et la valeur de coupure d'essai identifiée.

**9.** Procédé selon la revendication 7 ou la revendication 8, dans lequel lesdites informations sont un pronostic pour le premier sujet qui souffre de la condition médicale, une sensibilité du premier sujet à la condition médicale, une prédiction de la récurrence de la condition médicale, ou un traitement recommandé pour la condition médicale.

**10.** Procédé selon l'une quelconque des revendications précédentes, dans lequel la sous-étape (e) comprend en outre ajuster un modèle de régression à risques non proportionnels supplémentaire au jeu de données.

**11.** Procédé selon l'une quelconque des revendications précédentes, comprenant en outre le fait d'effectuer l'étape (A) pour chacun de la pluralité de gènes et de paires de gènes de l'ensemble de N gènes.

**12.** Système informatique arrangé pour effectuer un procédé selon l'une quelconque des revendications précédentes.

**13.** Porteuse de données, telle qu'une porteuse de données tangible, portant du logiciel lisible par un ordinateur pour faire que l'ordinateur effectue un procédé selon l'une quelconque des revendications 1 à 11.

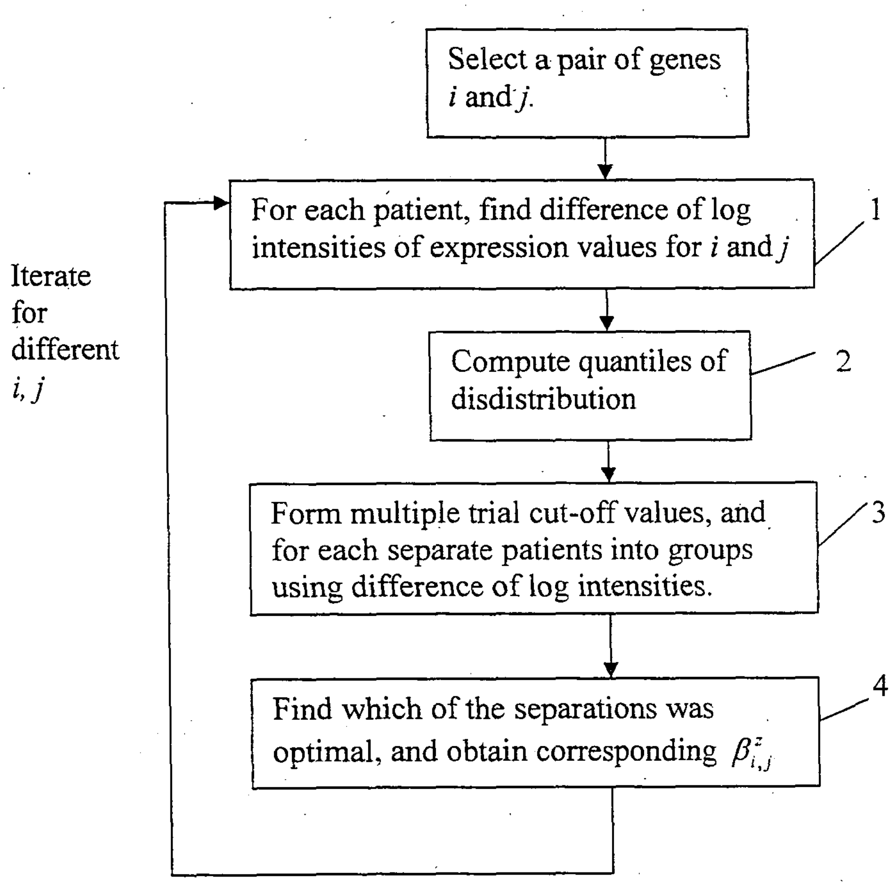
Select a pair of genes
i and j.
For each patient, find difference of log intensities of expression values for i and j
1
Compute quantiles of disdistribution
2
Form multiple trial cut-off values, and for each separate patients into groups using difference of log intensities.
3
Find which of the separations was optimal, and obtain corresponding $\beta^z_{i,j}$
4
Iterate for different i, j

Fig. 1

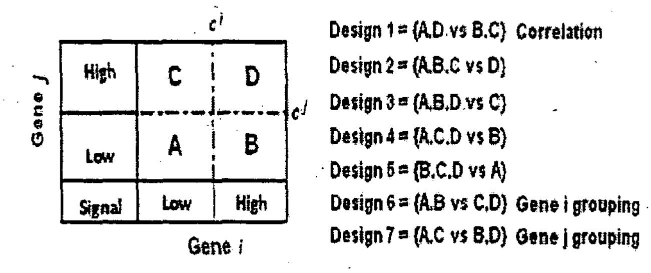

$c^i$
High
C
D
$c^j$
Low
A
B
Signal
Low
High
Gene $i$
Gene $j$
Design 1 = {A,D vs B,C} Correlation
Design 2 = {A,B,C vs D}
Design 3 = {A,B,D vs C}
Design 4 = {A,C,D vs B}
Design 5 = {B,C,D vs A}
Design 6 = {A,B vs C,D} Gene i grouping
Design 7 = {A,C vs B,D} Gene j grouping


Fig. 2

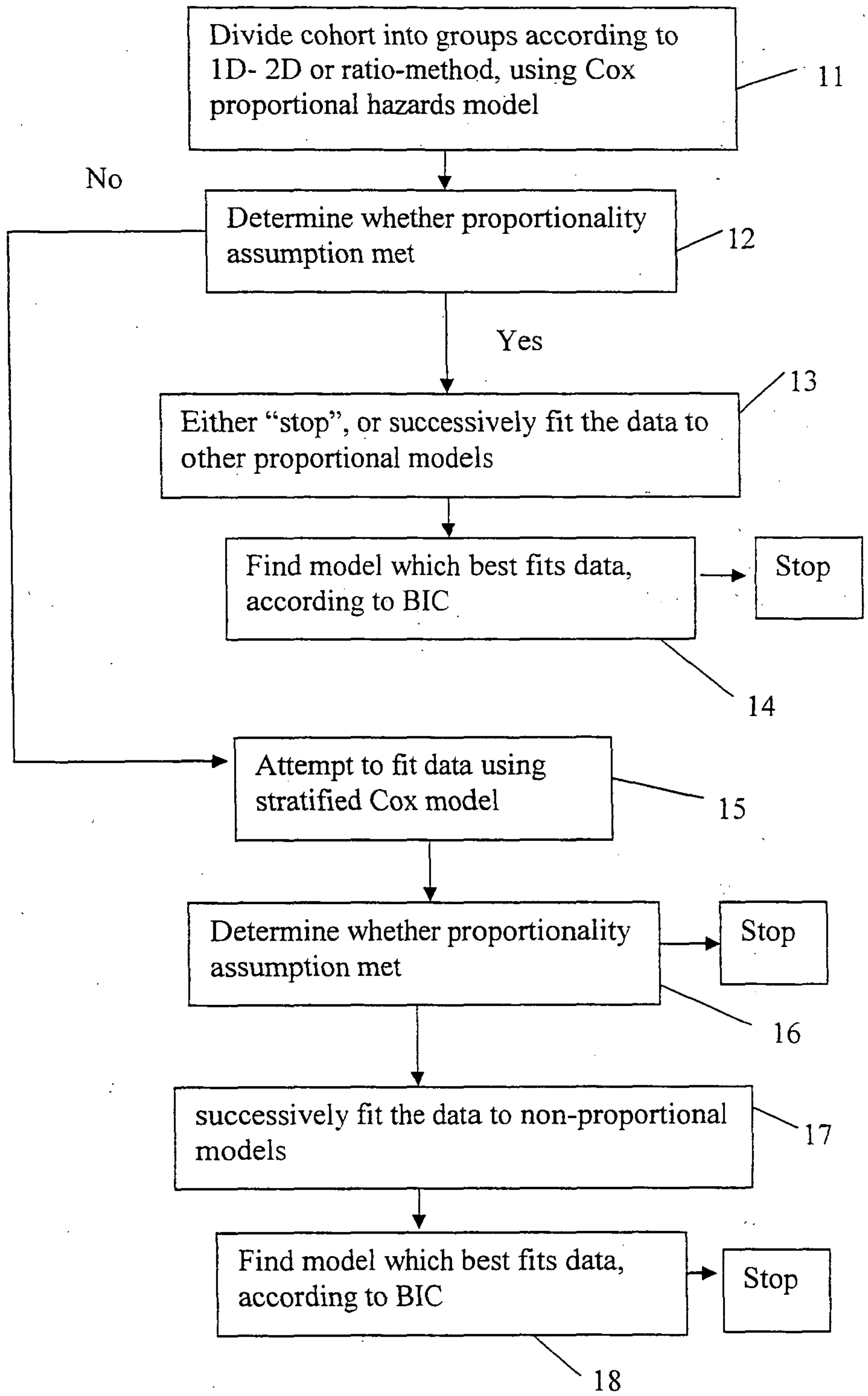
Divide cohort into groups according to 1D- 2D or ratio-method, using Cox proportional hazards model
11
No
Determine whether proportionality assumption met
12
Yes
Either "stop", or successively fit the data to other proportional models
13
Find model which best fits data, according to BIC
Stop
14
Attempt to fit data using stratified Cox model
15
Determine whether proportionality assumption met
Stop
16
successively fit the data to non-proportional models
17
Find model which best fits data, according to BIC
Stop
18

Fig. 3

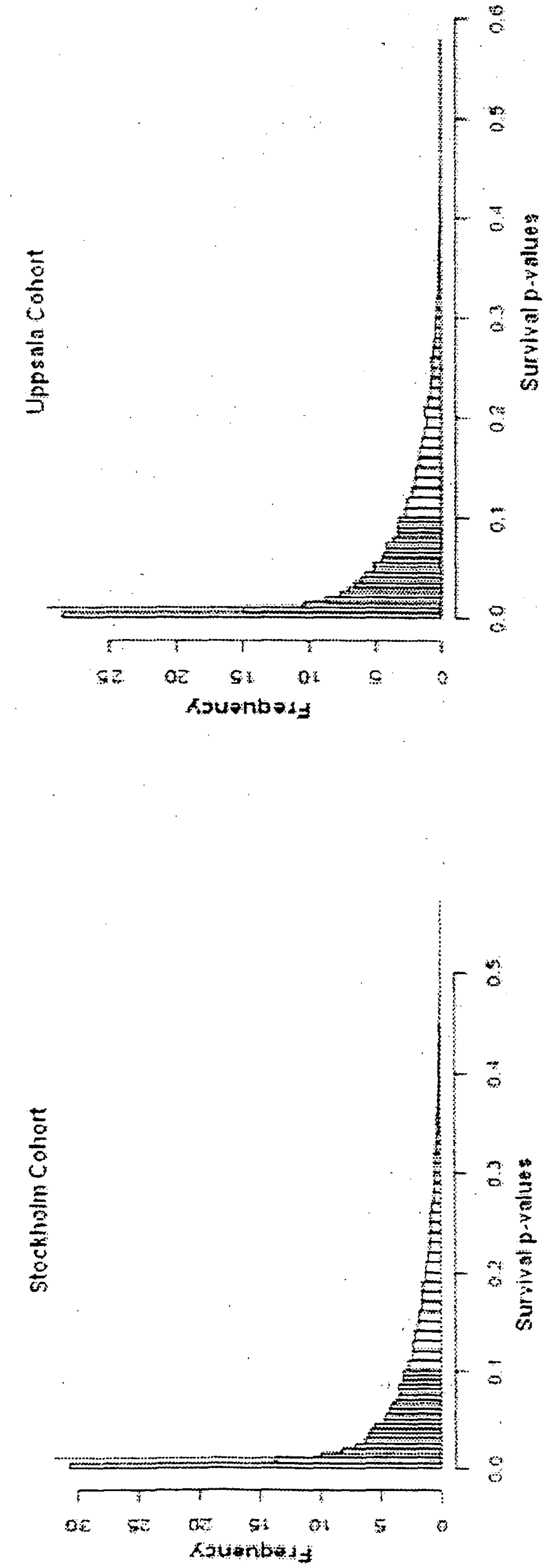
Uppsala Cohort
Frequency
Survival p-values
Stockholm Cohort
Frequency
Survival p-values

Fig. 4

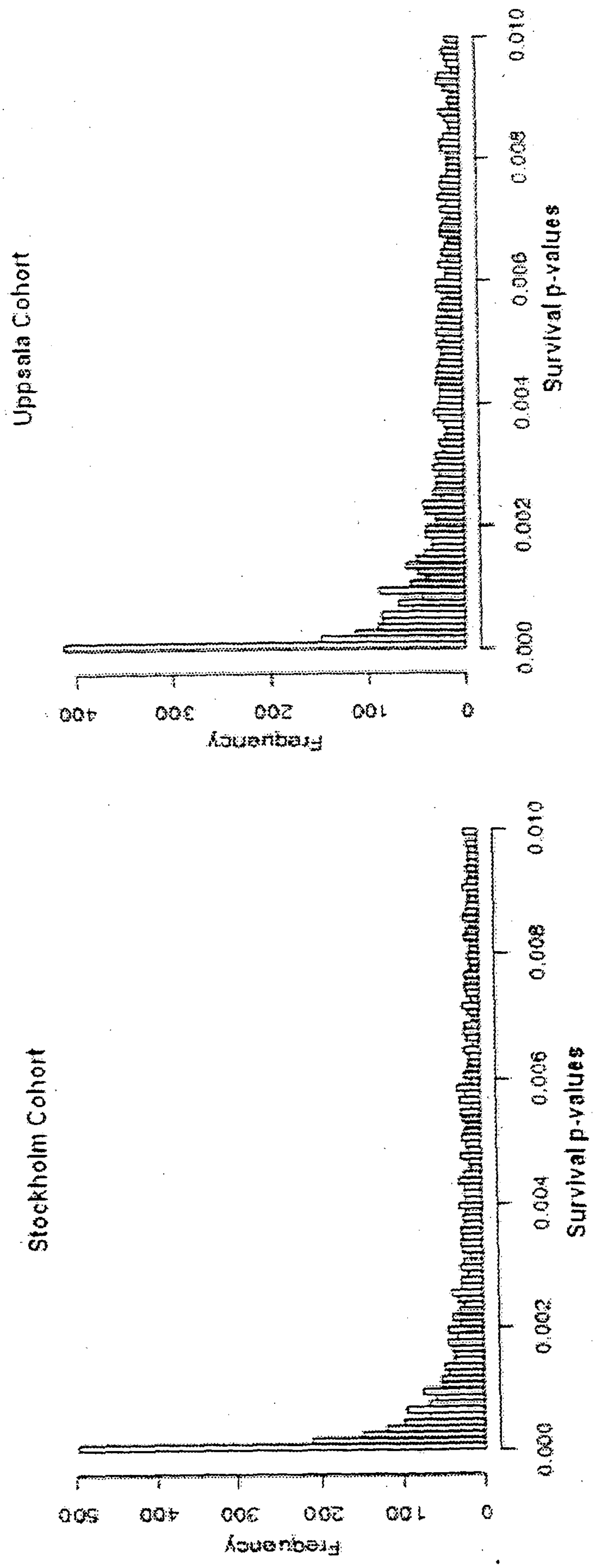
Uppsala Cohort
Frequency
0
100
200
300
400
Survival p-values
0.000
0.002
0.004
0.006
0.008
0.010
Stockholm Cohort
Frequency
0
100
200
300
400
500
Survival p-values
0.000
0.002
0.004
0.006
0.008
0.010

Fig. 5

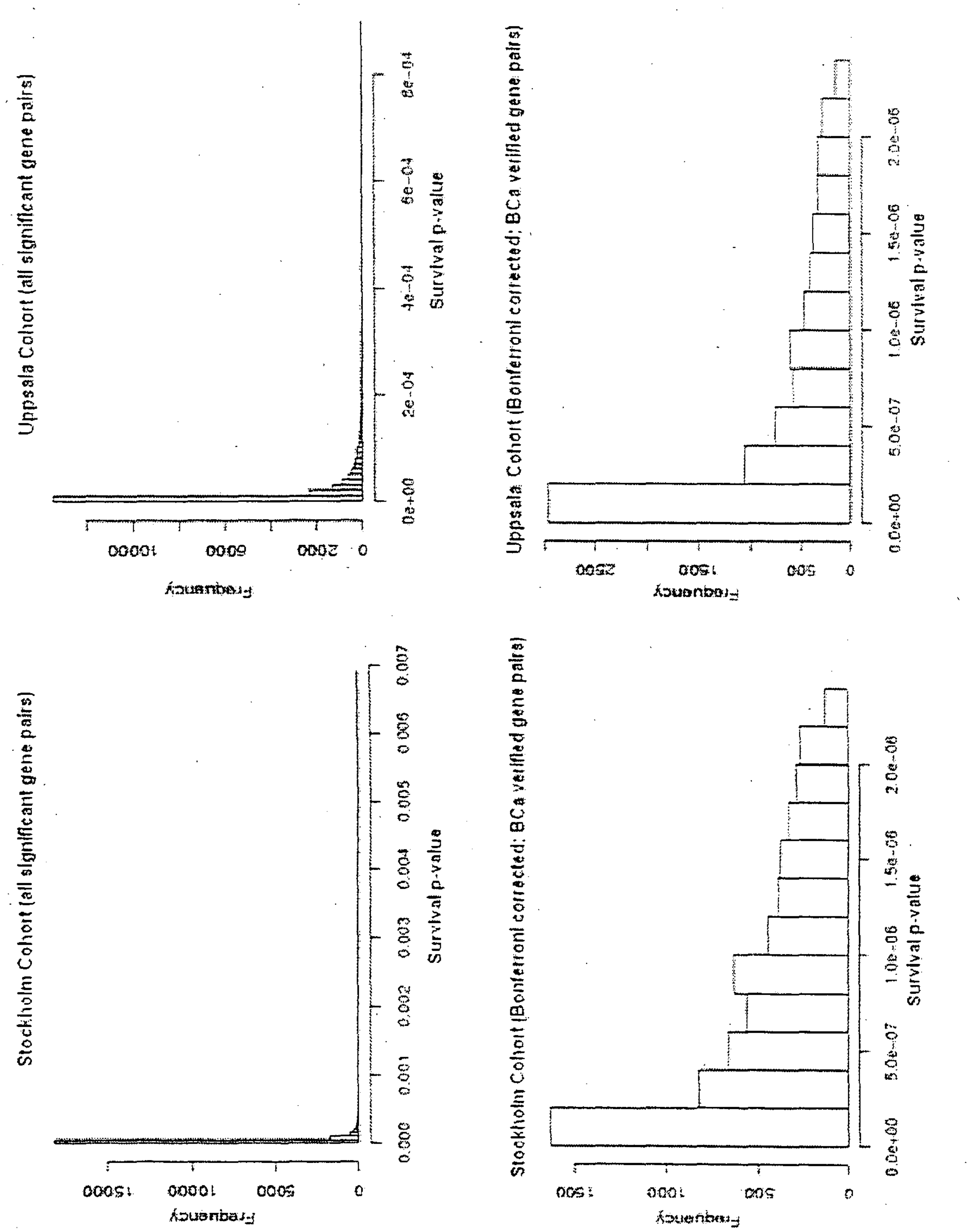
Uppsala Cohort (all significant gene pairs)
Frequency
Survival p-value
Stockholm Cohort (all significant gene pairs)
Frequency
Survival p-value
Uppsala Cohort (Bonferroni corrected; BCa verified gene pairs)
Frequency
Survival p-value
Stockholm Cohort (Bonferroni corrected; BCa verified gene pairs)
Frequency
Survival p-value

Fig. 6

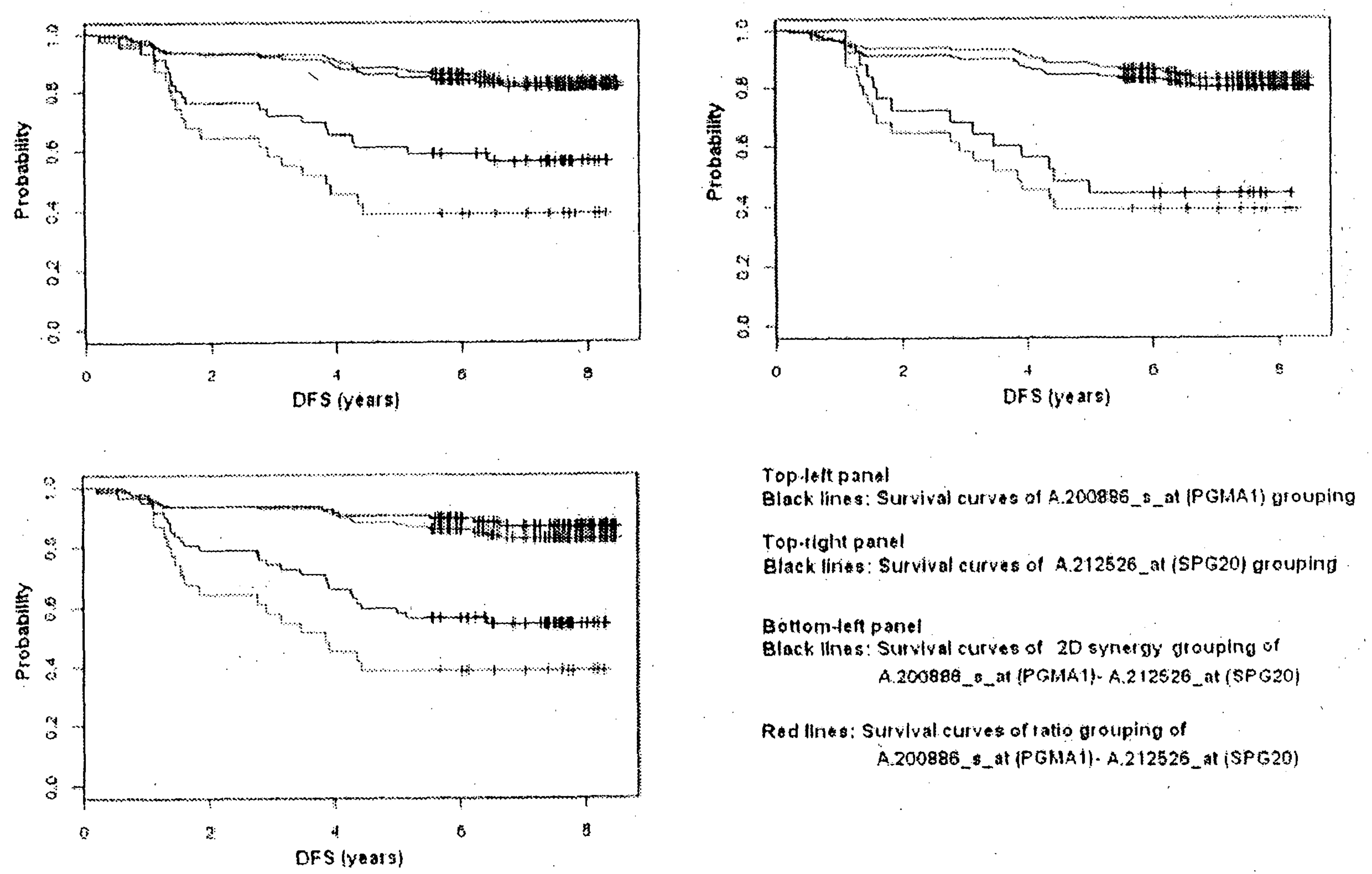


Top-left panel
Black lines: Survival curves of A.200886_s_at (PGMA1) grouping

Top-right panel
Black lines: Survival curves of A.212526_at (SPG20) grouping

Bottom-left panel
Black lines: Survival curves of 2D synergy grouping of
A.200886_s_at (PGMA1)- A.212526_at (SPG20)

Red lines: Survival curves of ratio grouping of
A.200886_s_at (PGMA1)- A.212526_at (SPG20)

Fig. 7

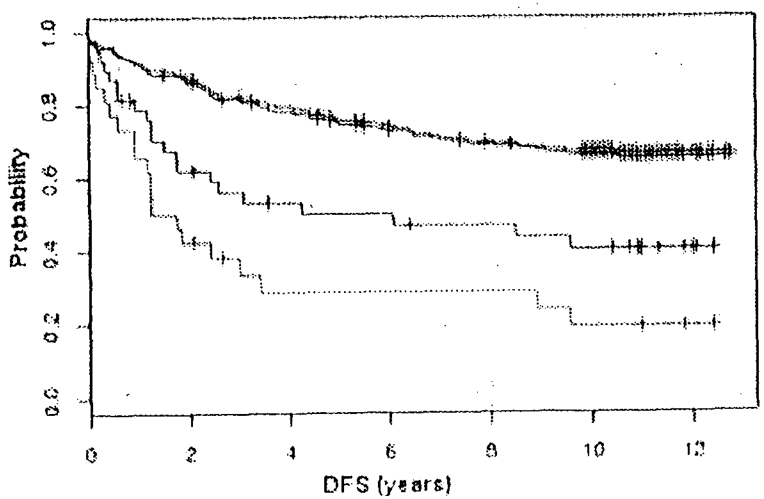


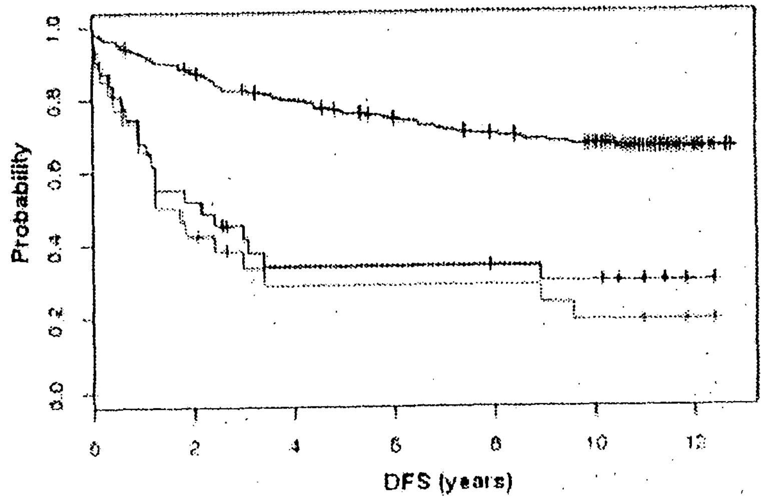


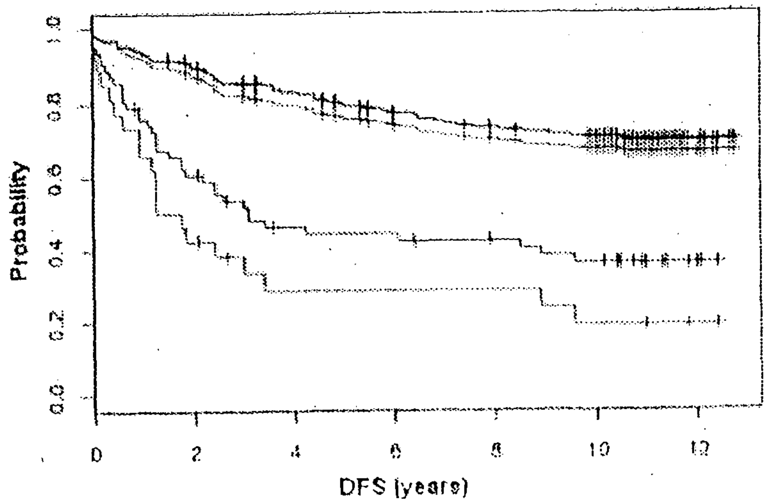


Top-left panel
Black lines: Survival curves of A.200886_s_at (PGMA1) grouping

Top-right panel
Black lines: Survival curves of A.212526_at (SPG20) grouping

Bottom-left panel
Black lines: Survival curves of 2D synergy grouping of A.200886_s_at (PGMA1)- A.212526_at (SPG20)

Red lines: Survival curves of ratio grouping of A.200886_s_at (PGMA1)- A.212526_at (SPG20)

Fig. 8

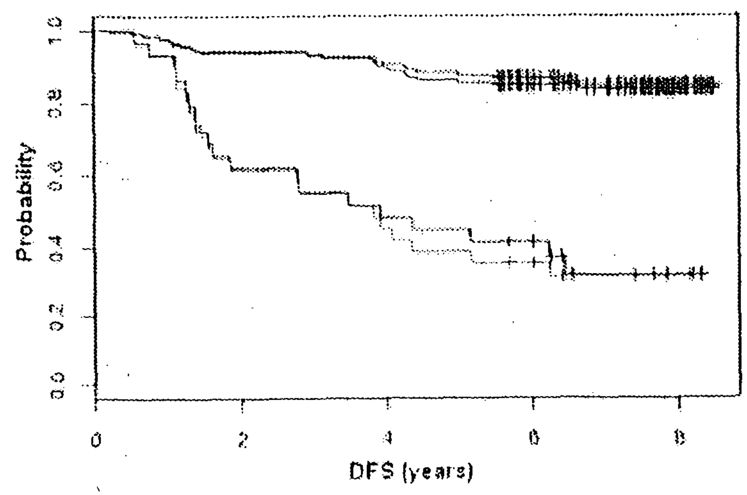


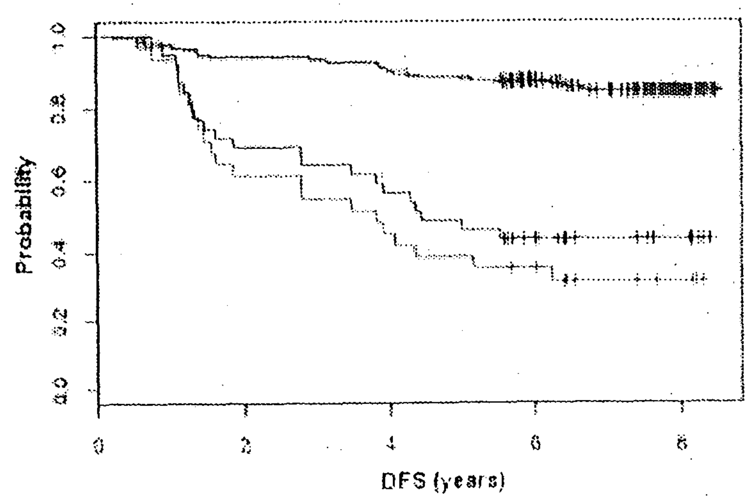


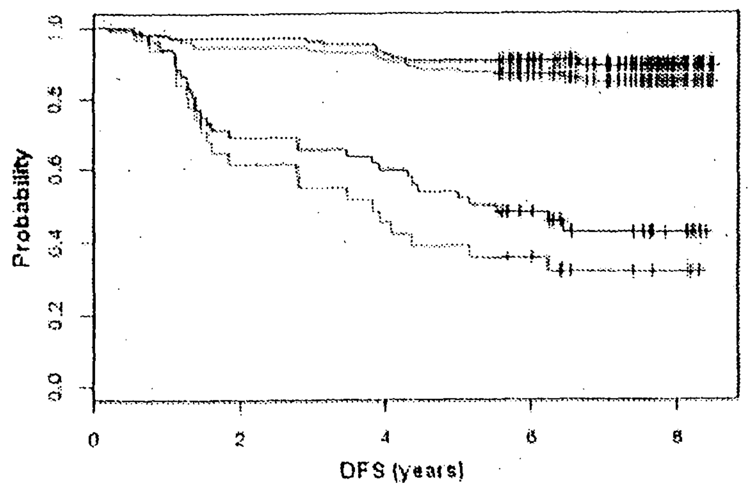


Top-left panel
Black lines: Survival curves of A.202154_x_at (TUBB3) grouping

Top-right panel
Black lines: Survival curves of A.213034_at (KIAA0999) grouping

Bottom-left panel
Black lines: Survival curves of 2D synergy grouping of
A.202154_x_at (TUBB3) - A.213034_at (KIAA0999)

Red lines: Survival curves of ratio grouping of
A.202154_x_at (TUBB3) - A.213034_at (KIAA0999)

Fig. 9

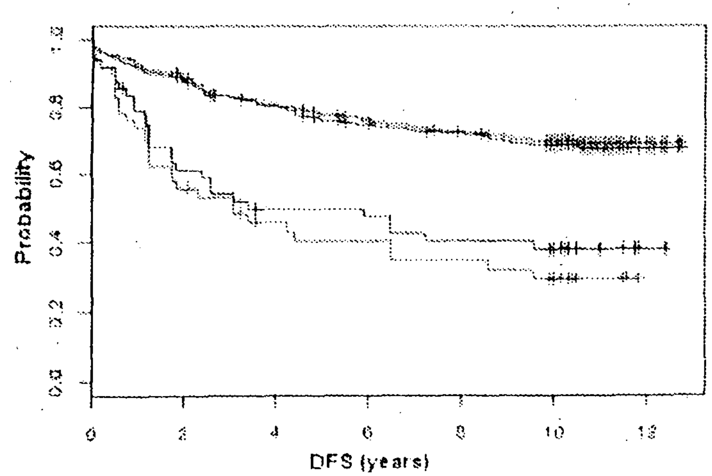


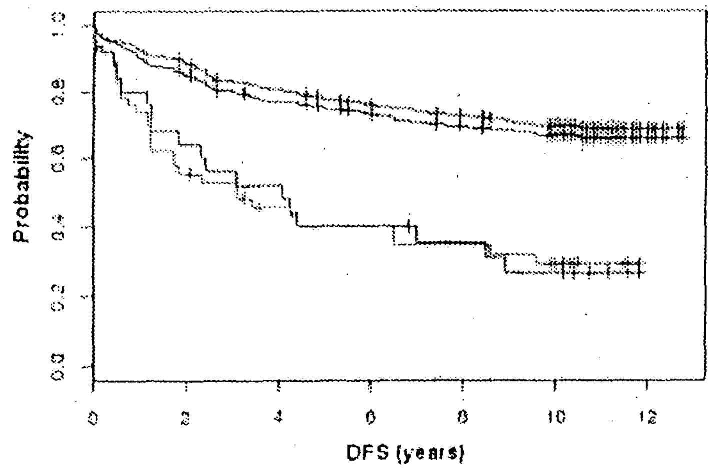


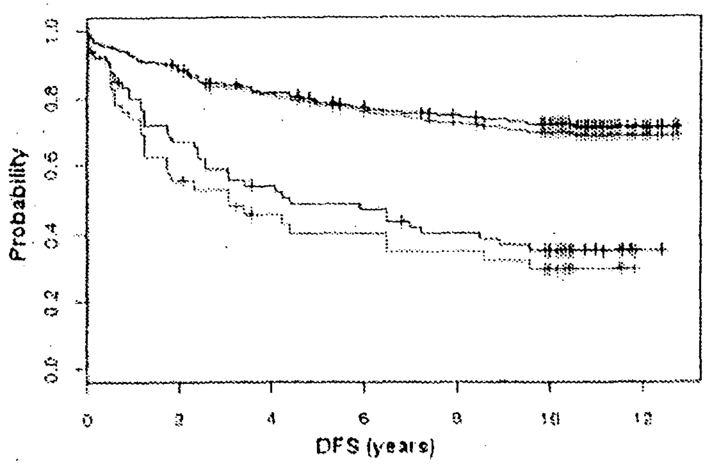


Top-left panel
Black lines: Survival curves of A.202154_x_at (TUBB3) grouping

Top-right panel
Black lines: Survival curves of A.213034_at (KIAA0999) grouping

Bottom-left panel
Black lines: Survival curves of 2D synergy grouping of
A.202154_x_at (TUBB3) - A.213034_at (KIAA0999)

Red lines: Survival curves of ratio grouping of
A.202154_x_at (TUBB3) - A.213034_at (KIAA0999)

Fig. 10

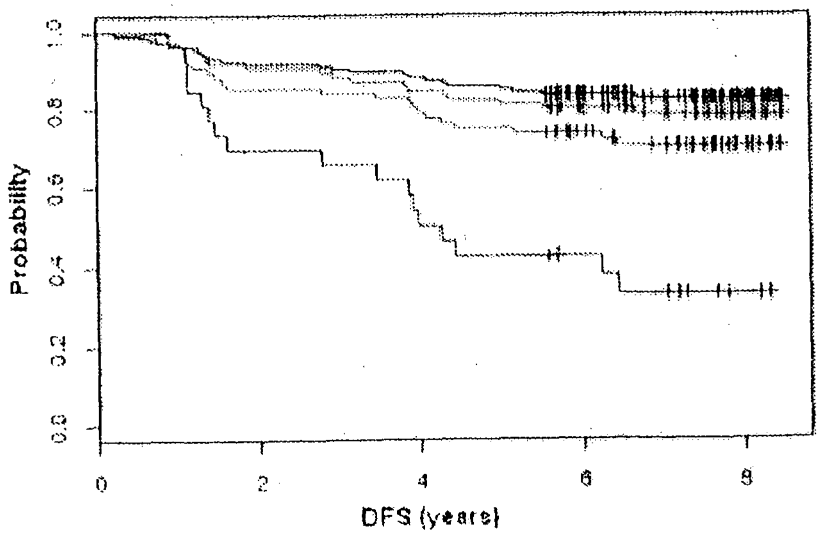


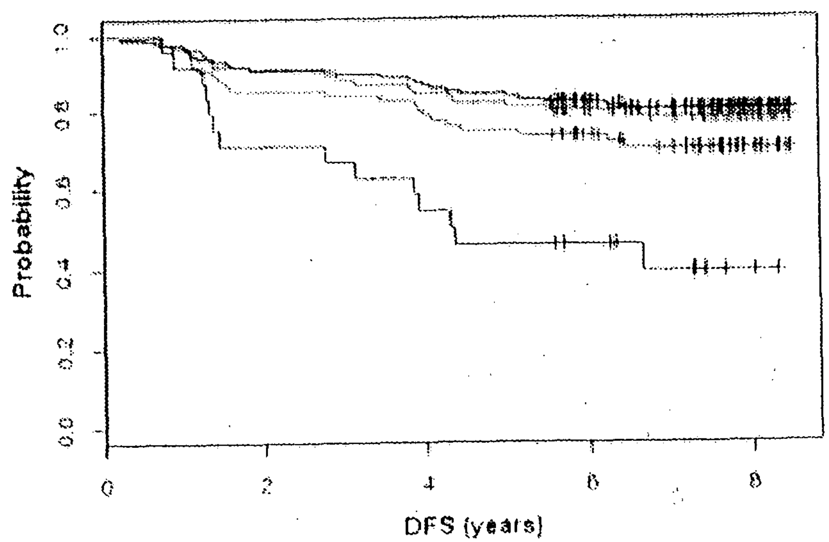


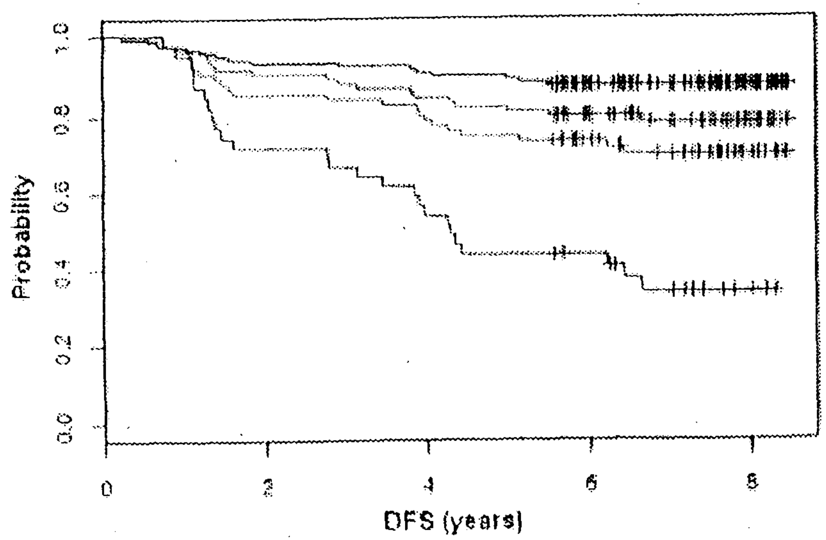


Top-left panel
Black lines: Survival curves of A.200853_at (H2AFZ) grouping

Top-right panel
Black lines: Survival curves of A.219395_at (RBM35B) grouping

Bottom-left panel
Black lines: Survival curves of 2D synergy grouping of
A.200853_at (H2AFZ) - A.219395_at (RBM35B)

Red lines: Survival curves of ratio grouping of
A.200853_at (H2AFZ) - A.219395_at (RBM35B)

Fig. 11

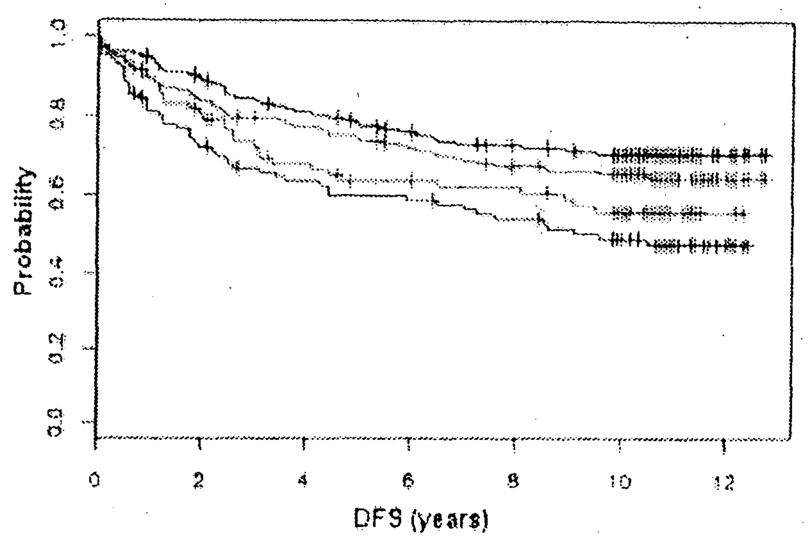


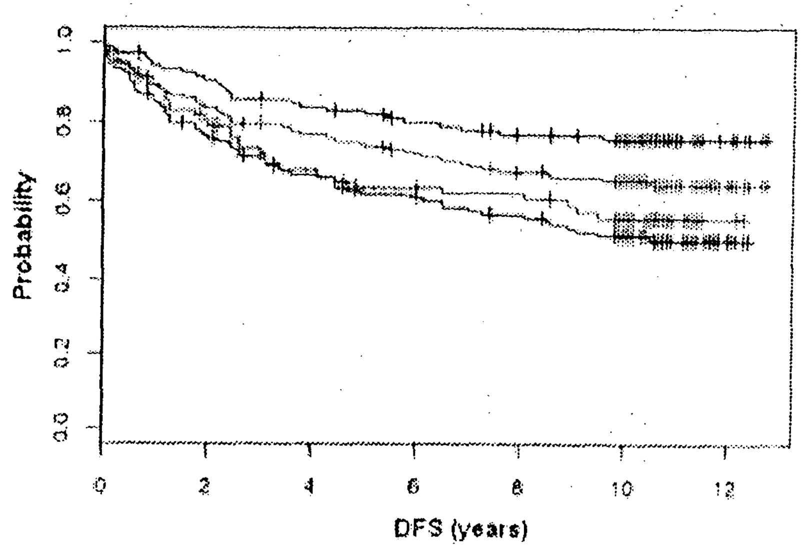


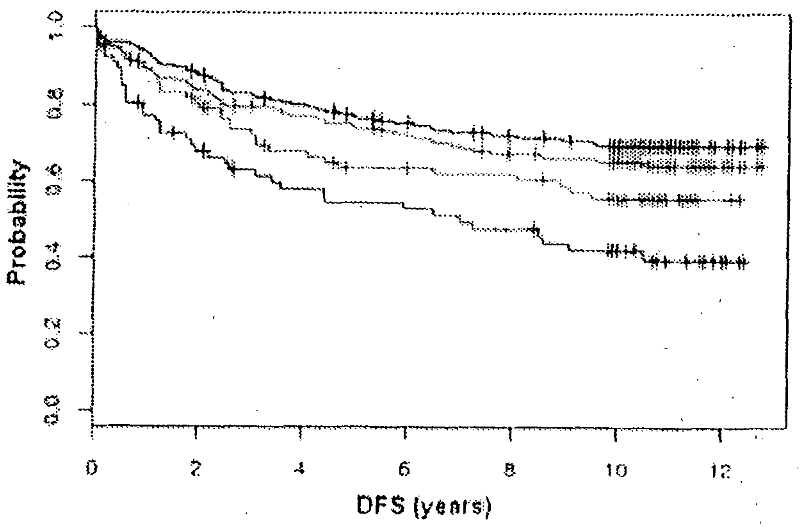


Top-left panel
Black lines: Survival curves of A.200853_at (H2AFZ) grouping

Top-right panel
Black lines: Survival curves of A.219395_at (RBM35B) grouping

Bottom-left panel
Black lines: Survival curves of 2D synergy grouping of
A.200853_at (H2AFZ) - A.219395_at (RBM35B)

Red lines: Survival curves of ratio grouping of
A.200853_at (H2AFZ) - A.219395_at (RBM35B)

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61158948 B
- SG 2009016825

### Non-patent literature cited in the description


- **SAMBROOK ; RUSSEL.** Molecular Cloning: A Laboratory Manual. Cold Springs Harbor Laboratory, 2001
- **ATA, N. ; SOZER, M.T.** Cox regression models with non-proportional hazards applied to lung cancer survival data. *Hacettepe Journal of Mathematics and Statistics,* 2007, vol. 36, 157-167
- **BENJAMINI, Y. ; HOCHBERG, Y.** Controlling the false discovery rate: a practical and powerful approach to multiple testing. *Journal of the Royal Statistical Society, Series B (Methodological),* 1995, vol. 57 (1), 289-300
- **BENJAMINI, Y. ; YEKUTIELI, D.** The control of the false discovery rate in multiple testing under dependency. *Annals of Statistics,* 2001, vol. 29 (4), 1165-1188
- **BRESLOW, N.E.** Covariance analysis of censored survival data. *Biometrics,* 1974, vol. 30, 89-99
- **BROET P. ; KUZNETSOV V.A. ; J. BERGH J. ; LIU E. T-B. ; MILLER L.D.** Identifying gene expression changes in breast cancer that distinguish early and late relapse among uncured patients. *Bioinformatics,* 2006, vol. 22, 1477-1485
- **CHEN CC ; CHANG TW ; CHEN FM ; HOU MF ; HUNG SY ; CHONG IW ; LEE SC ; ZHOU TH ; LIN SR.** Combination of multiple mRNA markers (PTTG1, Survivin, UbcH10 and TK1) in the diagnosis of Taiwanese patients with breast cancer by membrane array. *Oncology,* 2006, vol. 70 (6), 438-46
- **COX, D. R.** Regression Models and Life Tables (with Discussion). *Journal of the Royal Statistical Society,* 1972, vol. 34, 187-220
- **COX R.D. ; OAKES, D.** Analysis of Survival Data. Chapman and Hall, 1984
- **COX, D.R. ; SNELL, E.J.** A general definition of residuals (with discussion). *Journal of the Royal Statistical Society,* 1968, vol. 30, 248-265
- **CUI, X. ; CHURCHILL, G.A.** Statistical tests for differential expression in cDNA microarray experiments. *Genome Biology,* 2003, vol. 4 (4), 210
- **EFRON, B. ; TIBSHIRANI, R.J.** An Introduction to the Bootstrap. Chapman and Hall, 1994
- **GOETZ MP ; SUMAN VJ ; INGLE JN ; NIBBE AM ; VISSCHER DW ; REYNOLDS CA ; LINGLE WL ; ERLANDER M ; MA XJ ; SGROI DC.** A two-gene expression ratio of homeobox 13 and interleukin-17B receptor for prediction of recurrence and survival in women receiving adjuvant tamoxifen. *Clin Cancer Res,* 2006, vol. 12 (7), 2080-7
- **GRAMBSCH, M.P. ; THERNEAU, M.T.** Proportional Hazards Tests and Diagnostics Based on Weighted Residuals. *Biometrika,* 1994, vol. 81, 515-526
- **HUA S ; KALLEN CB ; DHAR R ; BAQUERO MT ; MASON CE ; RUSSELL BA ; SHAH PK ; LIU J ; KHRAMTSOV A ; TRETIAKOVA MS.** Genomic analysis of estrogen cascade reveals histone variant H2A.Z associated with breast cancer progression. *Mol Syst Biol.,* 2008, vol. 4, 188
- **IVSHINA AV ; GEORGE J ; SENKO OV. ; MOW B ; PUTTI TC ; SMEDS J ; NORDGREN H ; BERGH J ; LIU E T-B ; KUZNETSOV VA.** Genetic reclassification of histologic grade delineates new clinical subtypes of breast cancer. *Cancer Res.,* 2006, vol. 66, 10292-10301
- **KALBFLEISCH, J.D. ; PRENTICE, R.L.** The Statistical Analysis of Failure Time Data. John Wiley & Sons, Inc, 1980
- **KUZNETSOV V. A. ; SENKO O. V. ; MILLER L. D. ; IVSHINA ANNA V.** Statistically Weighted Voting Analysis of Microarrays for Molecular Pattern Selection and Discovery Cancer Genotypes. *Intern J of Computer Sciences and Network Security,* 2006, vol. 6, 73-83
- **KUZNETSOV, V.A. ; MOTAKIS E. ; IVSHINA, A.V.** Low- and High- Agressive Genetic Breast Cancer Subtypes and Significant Survival Gene Signatures. *IEEE Congress on Computational Intelligence,* 01 June 2008
- **LEBRUN D ; BAETZ T ; FOSTER C ; FARMER P ; SIDHU R ; GUO H ; HARRISON K ; SOMOGYI R ; GRELLER LD ; FEILOTTER H.** Predicting outcome in follicular lymphoma by using interactive gene pairs. *Clin Cancer Res.,* 2008, vol. 14 (2), 478-87

- **LINJIE TIAN ; PINGZHANG WANG ; 1 ; JINH GUO ; XINYU WANG ; WEIWEI DENG ; CHENYING ZHANG ; DONGXU FU ; XI GAO ; TAIPING SHI.** *Screening for novel human genes associated with CRE pathway activation with cell microarray,* July 2007, vol. 90 (1), 28-34
- **LOUGHIN, T.M.** A residual bootstrap for regression parameters in proportional hazards model. *J. of Statistical and Computational Simulations,* 1995, vol. 52, 367-384
- **MOTAKIS, E. ; IVSHINA, A.V. ; KUZNETSOV, V.A.** A data-driven method of identification of essential genes and gene pairs associated with survival time of cancer patients. *IEEE Engineering in Medicine and Biology,* 2008
- **RUTH M. R. ; ADRIAN L. ; HARRIS ; LIONEL TARASSENKO.** Non-linear survival analysis using neural networks. *Statistics in Medicine,* 2004, vol. 23, 825-842
- **SCHEMPER, M.** Cox analysis of survival data with non-proportional hazard functions. *Stafistician,* 1992, vol. 41, 455-465
- **SCHOENFELD D.** Residuals for the proportional hazards regresssion model. *Biometrika,* 1982, vol. 69 (1), 239-241
- **SHIBAYAMA H ; TAKAI E ; MATSUMURA I ; KOUNO M ; MORII E ; KITAMURA Y ; TAKEDA J ; KANAKURA Y.** Identification of a cytokine-induced antiapoptotic molecule anamorsin essential for definitive hematopoiesis. *J Exp Med.,* 16 February 2004, vol. 199 (4), 581-92
- **SJOBLOM T ; JONES S ; WOOD LD ; PARSONS DW ; LIN J ; BARBER TD ; MANDELKER D ; LEARY RJ ; PTAK J ; SILLIMAN N.** *Science,* 13 October 2006, vol. 314 (5797), 268-74
- **SOTIRIOU, T. ; PEROU, C.M. ; TIBSHIRANI, R. et al.** Gene expression patterns of breast carcinomas distinguish tumor subclasses with clinical implications. *Proc Natl Acad Sci USA,* 2001, vol. 98, 10869-10874
- **THERNEAU, M.T. ; GRAMBSCH, M.P.** Modeling Survival Data: Extending The Cox Model. Springer-Verlag, 2000
- **THOMPSON, A.L. ; CHHIKARA, S.R ; CONKIN, J.** Cox proportional hazards models for modeling the time to onset of decompression sickness in hypobaric environments. *NASA/TP-2003-210791,* 2003
- **TIBSHIRANI, R. ; HASTIE, T. ; NARASIMHAN, B. ; CHU, G.** Diagnosis of multiple cancer types by shrunken centroids of gene expression. *PNAS,* 2002, vol. 99, 6567-6572
- **VAN'T VEER, L.J. ; DAI, H. ; VAN DE VIJVER et al.** Gene expression profiling predicts clinical outcome of breast cancer. *Nature,* 2002, vol. 415, 530-536

SEQUENCE LISTING

<110> Agency for Science. Technology and Research

<120> A METHOD FOR IDENTIFICATION, PREDICTION AND PROGNOSIS OF CANCER AGGRESSIVENESS

<130> FP4966

<150> SG 200901682-5
<151> 2009-03-10

<160> 61

<170> PatentIn version 3.4

<210> 1
<211> 1736
<212> DNA
<213> Homo sapiens

<400> 1

cgaccctcag cagccagccc ggcccgcccg cgcccgtccg cagccgcccg ccagacgcgc 60
ccagtatgag ggagatcgtg cacatccagg ccggccagtg cggcaaccag atcggggcca 120
agttctggga agtcatcagt gatgagcatg gcatcgaccc cagcggcaac tacgtgggcg 180
actcggactt gcagctggag cggatcagcg tctactacaa cgaggcctct tctcacaagt 240
acgtgcctcg agccattctg gtggacctgg aacccggaac catggacagt gtccgctcag 300
gggcctttgg acatctcttc aggcctgaca atttcatctt tggtcagagt ggggccggca 360
acaactgggc caagggtcac tacacggagg gggcggagct ggtggattcg gtcctggatg 420
tggtgcggaa ggagtgtgaa aactgcgact gcctgcaggg cttccagctg acccactcgc 480
tgggggggcgg cacgggctcc ggcatgggca cgttgctcat cagcaaggtg cgtgaggagt 540
atcccgaccg catcatgaac accttcagcg tcgtgccctc acccaaggtg tcagacacgg 600
tggtggagcc ctacaacgcc acgctgtcca tccaccagct ggtggagaac acggatgaga 660
cctactgcat cgacaacgag gcgctctacg acatctgctt ccgcaccctc aagctggcca 720
cgcccaccta cggggacctc aaccacctgg tatcggccac catgagcgga gtcaccacct 780
ccttgcgctt cccgggccag ctcaacgctg acctgcgcaa gctggccgtc aacatggtgc 840
ccttcccgcg cctgcacttc ttcatgcccg gcttcgcccc cctcacagcc cggggcagcc 900
agcagtaccg ggccctgacc gtgcccgagc tcacccagca gatgttcgat gccaagaaca 960
tgatggccgc ctgcgacccg cgccacggcc gctacctgac ggtggccacc gtgttccggg 1020
gccgcatgtc catgaaggag gtggacgagc agatgctggc catccagagc aagaacagca 1080
gctacttcgt ggagtggatc cccaacaacg tgaaggtggc cgtgtgtgac atcccgcccc 1140

gcggcctcaa gatgtcctcc accttcatcg ggaacagcac ggccatccag gagctgttca 1200
agcgcatctc cgagcagttc acggccatgt tccggcgcaa ggccttcctg cactggtaca 1260
cgggcgaggg catggacgag atggagttca ccgaggccga gagcaacatg aacgacctgg 1320
tgtccgagta ccagcagtac caggacgcca cggccgagga agagggcgag atgtacgaag 1380
acgacgagga ggagtcggag gcccagggcc ccaagtgaag ctgctcgcag ctggagtgag 1440
aggcaggtgg cggccggggc cgaagccagc agtgtctaaa cccccggagc catcttgctg 1500
ccgacaccct gctttcccct cgccctaggg ctcccttgcc gccctcctgc agtatttatg 1560
gcctcgtcct ccccacctag gccacgtgtg agctgctcct gtctctgtct tattgcagct 1620
ccaggcctga cgttttacgg ttttgttttt tactggtttg tgtttatatt ttcggggata 1680
cttaataaat ctattgctgt cagataccct taaaaaaaaa aaaaaaaaaa aaaaaa 1736

<210> 2
<211> 6072
<212> DNA
<213> Homo sapiens

<400> 2

```
cagccgcgtc ccccagcccc ggcctcccgc ggacccatgc ccgcccgtat cggctactac     60
gagatcgacc gcaccatcgg caagggcaac ttcgcggtgg tcaagcgggc cacgcacctc    120
gtcaccaagg ccaaggttgc tatcaagatc atagataaga cccagctgga tgaagaaaac    180
ttgaagaaga ttttccggga agttcaaatt atgaagatgc tttgccaccc ccatatcatc    240
aggctctacc aggttatgga gacagaacgg atgatttatc tggtgacaga atatgctagt    300
ggaggggaaa tatttgacca cctggtggcc catggtagaa tggcagaaaa ggaggcacgt    360
cggaagttca aacagatcgt cacagctgtc tatttttgtc actgtcggaa cattgttcat    420
cgtgatttaa aagctgaaaa tttacttctg gatgccaatc tgaatatcaa aatagcagat    480
tttggtttca gtaacctctt cactcctggg cagctgctga agacctggtg tggcagccct    540
ccctatgctg cacctgaact ctttgaagga aaagaatatg atgggcccaa agtggacatc    600
tggagccttg gagttgtcct ctacgtgctt gtgtgcggtg ccctgccatt tgatggaagc    660
acactgcaga atctgcgggc ccgcgtgctg agtggaaagt tccgcatccc atttttttatg    720
tccacagaat gtgagcattt gatccgccat atgttggtgt tagatcccaa taagcgcctc    780
tccatggagc agatctgcaa gcacaagtgg atgaagctag ggacgccga tcccaacttt    840
gacaggttaa tagctgaatg ccaacaacta aaggaagaaa gacaggtgga cccccctgaat    900
gaggatgtcc tcttggccat ggaggacatg ggactggaca aagaacagac actgcagtca    960
```

ttaagatcag atgcctatga tcactatagt gcaatctaca gcctgctgtg tgatcgacat 1020
aagagacata aaaccctgcg tctcggagca cttcctagca tgcccgagc cctggccttt 1080
caagcaccag tcaatatcca ggcggagcag gcaggtactg ctatgaacat cagcgttccc 1140
caggtgcagc tgatcaaccc agagaaccaa attgtggagc cggatgggac actgaatttg 1200
gacagtgatg agggtgaaga gccttcccct gaagcattgg tgcgctattt gtcaatgagg 1260
aggcacacag tgggtgtggc tgacccacgc acggaagtta tggaagatct gcagaagctc 1320
ctacctggct ttcctggagt caacccccag gctccattcc tgcaggtggc ccctaatgtg 1380
aacttcatgc acaacctgtt gcctatgcaa aacttgcaac caaccgggca acttgagtac 1440
aaggagcagt ctctcctaca gccgcccacg ctacagctgt tgaatggaat gggccccctt 1500
ggccggaggg catcagatgg aggagccaac atccaactgc atgcccagca gctgctgaag 1560
cgcccacggg gaccctctcc gcttgtcacc atgacaccag cagtgccagc agttacccct 1620
gtggacgagg agagctcaga cggggagcca gaccaggaag ctgtgcagag ctctacctac 1680
aaggactcca acactctgca cctccctacg gagcgtttct cccctgtgcg ccggttctca 1740
gatggggctg cgagcatcca ggccttcaaa gctcacctgg aaaaaatggg caacaacagc 1800
agcatcaaac agctgcagca ggagtgtgag cagctgcaga agatgtacgg ggggcagatt 1860
gatgaaagaa ccctggagaa gacccagcag cagcatatgt tataccagca ggagcagcac 1920
catcaaattc tccagcaaca aattcaagac tctatctgtc ctcctcagcc atctccacct 1980
cttcaggctg catgtgaaaa tcagccagcc ctccttaccc atcagctcca gaggttaagg 2040
attcagcctt caagcccacc ccccaaccac cccaacaacc atctcttcag gcagcccagt 2100
aatagtcctc cccccatgag cagtgccatg atccagcctc acggggctgc atcttcttcc 2160
cagtttcaag gcttaccttc ccgcagtgca atctttcagc agcaacctga gaactgttcc 2220
tctcctccca acgtggcact aacctgcttg ggtatgcagc agcctgctca gtcacagcag 2280
gtcaccatcc aagtccaaga gcctgttgac atgctcagca acatgccagg cacagctgca 2340
ggctccagtg ggcgcggcat ctccatcagc cccagtgctg gtcagatgca gatgcagcac 2400
cgtaccaacc tgatggccac cctcagctat gggcaccgtc ccttgtccaa gcagctgagt 2460
gctgacagtg cagaggctca cagcttgaac gtgaatcggt tctcccctgc taactacgac 2520
caggcgcatt tacacccccca tctgttttcg gaccagtccc ggggttcccc cagcagctac 2580
agcccttcaa caggagtggg gttctctcca acccaagccc tgaaagtccc tccacttgac 2640
caattcccca ccttccctcc cagtgcacat cagcagccgc cacactatac cacgtcggca 2700

ctacagcagg ccctgctgtc tcccacgccg ccagactata caagacacca gcaggtaccc 2760
cacatccttc aaggactgct ttctccccgg cattcgctca ccggccactc ggacatccgg 2820
ctgcccccaa cagagtttgc acagctcatt aaaaggcagc agcaacaacg gcagcagcag 2880
cagcaacagc agcaacagca agaataccag gaactgttca ggcacatgaa ccaaggggat 2940
gcggggagtc tggctcccag ccttgggga cagagcatga cagagcgcca ggctttatct 3000
tatcaaaatg ctgactctta tcaccatcac accagccccc agcatctgct acaaatcagg 3060
gcacaagaat gtgtctcaca ggcttcctca cccaccccgc cccacgggta tgctcaccag 3120
ccggcactga tgcattcaga gagcatggag gaggactgct cgtgtgaggg ggccaaggat 3180
ggcttccaag acagtaagag ttcaagtaca ttgaccaaag gttgccatga cagccctctg 3240
ctcttgagta ccggtggacc tggggaccct gaatctttgc taggaactgt gagtcatgcc 3300
caagaattgg ggatacatcc ctatggtcat cagccaactg ctgcattcag taaaaataag 3360
gtgcccagca gagagcctgt catagggaac tgcatggata gaagttctcc aggacaagca 3420
gtggagctgc cggatcacaa tgggctcggg tacccagcac gcccctccgt ccatgagcac 3480
cacaggcccc gggccctcca gagacaccac acgatccaga acagcgacga tgcttatgta 3540
cagctggata acttgccagg aatgagtctc gtggctggga aagcacttag ctctgcccgg 3600
atgtcggatg cagttctcag tcagtcttcg ctcatgggca gccagcagtt tcaggatggg 3660
gaaaatgagg aatgtggggc aagcctggga ggtcatgagc acccagacct gagtgatggc 3720
agccagcatt taaactcctc ttgctatcca tctacgtgta ttacagacat tctgctcagc 3780
tacaagcacc ccgaagtctc cttcagcatg gagcaggcag gcgtgtaaca agaaacagag 3840
agttttgtgt acagcttggg aatgaaaagg ttgattgtaa acccacagta tctagcagcg 3900
ttgtgccaaa ttgcccttgt gtttctctcc acccaaaata tcacagctgc tttcctcaca 3960
tttggttcat ccgtgtgctg ttcttttggg ttctgagagg gttttgccat gtttgcttgt 4020
atgaccaagt caccaaggaa ataaacagga aggaaatcca tgttctccat cttttgtgaa 4080
agtatatttg agttggtggt tttttgtttt gtttgggggt ttgtgttttg ttttgttttt 4140
ggtatgtttt cttccagagg tgatatactt tctttttttt cttcctttct tttttttctt 4200
tcgttccttt tttgaaacag gagagcaaag cagttagagt tcagaggcca gcggcctcag 4260
ggccactccc tccctagcct tcatcagcag agcaccctcc atccccctgc attgctcttc 4320
tgtgaaagca aatactaaag gatgccatcc tctggaatcc taatggcagg caaagggaga 4380
gaggaagggt gacggcttct ggcacttaga aaacaaaaag aacaaaaaaa gagaaacccc 4440
caagcctgga acgcagagag gtctttactg ctgggatcca cggaaaacat gtctgtccta 4500

gccaagatca tatgaagagt ttggcacgga ggctgagaat gacctggcat agatggtttg 4560
ccagttagga tgtctcaatt tgagcctttg cttttggtgg ataactcagc tcccctcttg 4620
taacctggaa agttggttgc ctttatcatc ctgctggttt tatccatgga ctgaacaccc 4680
aacagcagtg cactatgctt tctatggcat ctttcattct cattttatat tgtgctataa 4740
aaaggattgt ttctccatat atatattata tatgtgtgta tatatataat ataatatatg 4800
tgtatatata tattatatat ataatatata atataatata ttatatatat attatatata 4860
taatatatat ataaaatata tatatatatg ctctcctctt tcagcctctt tgtcacaggg 4920
aagaagtgta ggaggttgcc ttgggccctg cctctctcct aacctcctct tccccactgg 4980
gtaccctcag cccctatatt ttaattcttg atcatgtaga aattgttttt ggtaaatgtt 5040
gatattattg ttattatcat tattaataaa taaagagaaa aggaattttt gtttaaatga 5100
gaaatgttta accagattct gttctatttg aattgtgact tgcacctttt gttcaaagta 5160
tttcctttag gcattgtaat tgtgaacagc tcttacttgt gccagtgaca gatgcagtgg 5220
tctcctttcc ccagttgaag cagtgcatac gcagtagcta ttatttgtgt tatctttatt 5280
tctcttcatt gttagaaacc aaagtcttct ctgctggctg gggctgagag agggtctggg 5340
ttatctcctt ctgatcttca aaacaagaga gagaccttga atacactgac tcttccaccc 5400
tttttttttc tgggaaagga gagcaagagg tcccgagtcc cctcctagtc tttcatcctg 5460
aatttgcaca gaggaaagcg ggtgcccggc atggccatcc tgatgttgct ggcgggatcc 5520
ccatgcacct tgtccttctc cactgatact ggcagctcgg ctcctggacc caagatccct 5580
tgagtggaat tctgcagtgc aagagccctt cgtgggagct gtcccatgtt tccatggtcc 5640
ccagtctccc ctccacttgg tggggtcacc aactactcac cagaaggggg cttaccaaga 5700
aagccctaaa aagctgttga cttatctgcg cttgttccaa ctcttatgcc cccaacctgc 5760
cctaccacca ccacgcgctc agcctgatgt gtttacatgg tactgtatgt atgggagagc 5820
agactgcacc ctccagcaac aacagatgaa agccagtgag cctactaacc gtgccatctt 5880
gcaaactaca ctttaaaaaa aactcattgc tttgtattgt agtaaccaat atgtgcagta 5940
tacgttgaat gtatatgaac atactttcct atttctgttc tttgaaaatg tcagaaatat 6000
ttttttcttt ctcattttat gttgaactaa aaaggattaa aaaaaaaatc tccagactca 6060
agttgctaaa aa 6072

<210> 3
<211> 5031
<212> DNA
<213> Homo sapiens

<400> 3

```
acgtgaccta ggctgcggcg gcggcgtgct gcgggctctg tggcgggagc gaggccgacg     60
ggcggggccg cgcggccgcg tgacgcgaag cgttcgagag cgcgcgtcgt ggaacgtctt    120
ggttgccacg gcaagcgcgc gcgcgaggcc ttgggaacct cgggaccggc cccggcgag     180
cgcagcggcg cccagtgtaa gggagtggga gctggtccgt gccgcggcgg ccgcgcaggg    240
agctctcgag gcaacgccgg ggcgcccgag gtctggaagg cgcagaaatg gagcaagagc    300
cacaaaatgg agaacctgct gaaattaaga tcatcagaga agcatataag aaggcctttt    360
tatttgttaa caaaggtctg aatacagatg aattaggtca gaaggaagaa gcaaagaact    420
actataagca aggaatagga cacctgctca gagggatcag catttcatca aaagagtctg    480
aacacacagg tcctgggtgg gaatctgcta gacagatgca acagaaaatg aaagaaactc    540
tacagaatgt acgcaccagg ctggaaattc tagagaaggg tcttgccact tctctgcaga    600
atgatcttca ggaggtgccc aagttatatc cagaatttcc acctaaagac atgtgtgaaa    660
aattaccaga gcctcagtct tttagttcag ctcctcagca tgctgaagta aatggaaaca    720
cctcaactcc aagtgcaggg gcagttgctg cacctgcttc tctgtcttta ccatcacaaa    780
gttgtccagc agaagctcct cctgcttata ctcctcaagc tgctgaaggt cactacactg    840
tatcctatgg aacagattct ggggagtttt catcagttgg agaggagttt tataggaatc    900
attctcagcc accgcctctt gagaccttag ggctggatgc agatgaattg attttgatac    960
caaatggagt acagattttt tttgtaaatc ctgcagggga ggttagtgca ccttcgtatc   1020
ctgggtacct tcgaattgtg aggtttttgg ataattctct cgatacggtt ctaaaccgtc   1080
ctcccgggtt tcttcaggtt tgtgactggt tatatcctct agttcctgat agatctccgg   1140
ttctgaaatg tactgcggga gcctacatgt ttcctgatac aatgctacaa gcagcaggat   1200
gctttgtggg ggtcgtcctg tcctctgagt taccagagga tgatagagag ctctttgagg   1260
atctgttaag gcaaatgtct gaccttcggc tccaggccaa ctggaacaga gcagaagaag   1320
aaaatgaatt ccaaatccct ggaagaacta gaccctcctc tgaccaacta aaagaagcct   1380
ctggcactga tgtgaaacag ttggaccaag gcaataagga tgtacgtcat aaaggaaaac   1440
gtggaaaaag ggctaaagat acttcaagtg aagaagttaa cctgagtcac attgtaccat   1500
gtgagccagt tccagaagaa aagccaaaag aattacctga atggagtgaa aaagtggctc   1560
acaacatttt gtcaggtgct tcctgggtga gttggggttt agtcaaaggt gctgagatta   1620
ctggtaaggc aatccagaaa ggtgcttcta aactccgaga gcggattcaa ccagaagaaa   1680
```

aacccgtgga agttagtcca gctgtcacca agggacttta tatagcgaag caagctacag 1740
gaggagcagc aaaagtcagt cagttcctgg ttgatggagt ttgcactgta gcaaattgcg 1800
ttggaaaaga actagctcca catgtcaaga agcatggaag caaacttgtt ccagaatctc 1860
ttaaaaaaga caaagatggg aaatctcctc tggatggtgc tatggttgta gcagcaagta 1920
gtgttcaagg attttcaact gtctggcaag gattggaatg tgcagctaaa tgcatcgtta 1980
acaatgtttc agcagaaact gtacaaactg tcagatacaa atacggatat aatgcaggag 2040
aagctaccca ccatgcggtg gattctgcgg tcaatgttgg cgtaactgcc tacaatatta 2100
acaacattgg tatcaaagca atggtgaaga aaactgcaac acaaacagga cacactctcc 2160
ttgaggacta tcagatagtt gataattctc agagggaaaa tcaagaagga gcagcaaatg 2220
tcaacgtgag aggggagaag gatgagcaga cgaaggaagt aaaggaggca aagaagaaag 2280
ataaatgatg aagtgctggg aatcacttat accaaagcct tatgaaatgg atgaaatttt 2340
gttaaatagg caaatgtgga attcctcaca gattaaccag tatttttaa atgtattcat 2400
tcctacaaat taactttcat aaattttatg gcatgtcttc tatttaaaag gaaaagaata 2460
agtattcttg catctggcct tagaaatgtg aagttatatt ctcaagttta ttttttcca 2520
agtgtagcta aaatattttt gcaggtaaaa taaagctgat agtacatgtg ttgttcaaac 2580
cttgttaaac ctaatattga actattttta tatctgctgt ctttcagaag gcaaatagga 2640
aactatatat ttgcttaaaa attggcattt agtaacctta attcttttta tagaaggaat 2700
gacttaaagt attgtcccct ctttttgcac taattgtgga tttttttaga tgcttctcaa 2760
aattttcagt gtgtaagcta aacaaaaact aaaactaaga attctcaaaa aaacttgttc 2820
aaaacaggga aagactgatg aaaagtaaaa tggactactt ttgtaactta cctgtttgtt 2880
aggaaatgga atggtctctt tgatttaaaa taaataaaaa tagattatta cgtcttttgt 2940
attgagactg tattgttatg agcctaggaa atttgggaac atgattgtat tgtattaaaa 3000
ttcgaagtga ttattatcag cttaattgga ttaaaaaagt acttcaagaa attattttat 3060
catatctgct tctgtttttc caaaaggtta aaacttgtaa aaaaaatata tataaacaat 3120
tgagtttact aatggtaaac atttttattc tgggattcgg tcattggaat ttatattaaa 3180
agacaagtta ttaaaaagga aaggttctat tcataatcag ggtaaagaat atgaaaacct 3240
tagacgtaat ccatggtgga taggcattat ggtttccact ttggcagaag gcagactatt 3300
cacagcccta tttacttaca taggctaaaa aactatgtaa ctaaatacct aatggtattt 3360
aatttttgtt tattgaattt aagagattgg tattagtttt catagctgta gtccattcta 3420
ataatttctg atcttctagt ggctacttaa ttagacatta tttgaagctg tctgaagaat 3480

```
gcactttatg aattaaaaaa ctgaattgcc tgacctcgtt atcacatgag cttatatttt   3540
gggaacacat agaactgatg gaggcttttc ctaaggccaa ggataatgta ctagttgtta   3600
aaatggaaat aaaagtgaag tggtaaatag cttacagaca gtctatgctt tgtttaagaa   3660
atggaatggc acttcatata ttctcatttg tgggagctaa aaattacaat aatcaaactt   3720
aaggagatag agagtagaat gatggttacc agaggctggg aggcattgaa gggatgggga   3780
gggaagtgag gttgattaat gggtacgaaa atatagttag aacgaatttg ttgtagtatt   3840
tgatagccca acagggtgac tacagtcaac aataacttta tacatttaaa aataaaagag   3900
tgtaattggg acgtttgtaa cacaaatgaa tgcttgaagc agtagataca ccctgatgta   3960
attattataa atcgcatacc tgtatcaaaa tatctcatgt atcccataaa tatatacacc   4020
taccatgtac ccataaaaat ttaaaaagaa atggaatggc aaataatgga acataaactt   4080
taaacagtta gctttgacca gaggtcagca aatatcctcc atgggctaaa tccagatgtc   4140
cacctgtttt cataaataac gttttattgg agcatagctg tacccatttg tttacatatt   4200
ggctatggct gctgtctagt tcaacgtgag agttgaatag ttgcatcaga gagtatatgg   4260
tccacaagcc taacttattc actctctggc cctttgcaga aaaagtgtgc tgccttagtc   4320
tatagaaaat ggacttttcc caccattcac tgactcatta ggagataggc atcttctaaa   4380
gatttttttt atattgtgta atgattgata tatagataaa attatatatt acttgtaagg   4440
tatgatgaat aacaacactt aaaaactcat cacacctaaa gacatggaaa gctgccaatt   4500
acctatatgt atgttcttcc tattttctta cctctttccc catgtcattt tttattgttc   4560
cttgccattt tttaatcaca catgtatgtt ctccaaacat actgtttcat tttttgaagc   4620
tttctacata tagtgtctga atgtgaagtc ttactcaact tgcttttttg agtttatgtt   4680
taaaattcat tcttgcgctt atatgcatgg atggtttact tgctctatag tattctgtgt   4740
gagtatacca caatttaagt gttcattttc ctatcagttg acatttgttt ttagtttttg   4800
ttggtagaaa cagtacactc aaaacatcct tgactgtccc ctggtacaca tatgcaagag   4860
actttatgcg taagagtgga gttgttggct tgtcaagttt tgcaaaataa tgccaggtat   4920
tttccaaaat gatgatattc tcttgagtat tacttccctt tgcaatgttt ttattgttaa   4980
tgtagcatat ataaaaaagt atctcataat tttaaaaaaa aaaaaaaaaa a            5031
```

<210> 4
<211> 5014
<212> DNA
<213> Homo sapiens

<400> 4

```
acgtgaccta ggctgcggcg gcggcgtgct gcgggctctg tggcgggagc gaggccgacg      60
ggcggggccg cgcggccgcg tgacgcgaag cgttcgagag cgcgcgtcgt ggaacgtctt     120
ggttgccacg gcaagcgcgc gcgcgaggcc ttgggaacct cgggaccggc cccggcgag      180
cgcagcggcg cccagtagtc atcttagtgg gatttgggga agcaacaggg ctgtgtgggg     240
taacctgcca cctttaagtg gaaatcagaa atggagcaag agccacaaaa tggagaacct     300
gctgaaatta agatcatcag agaagcatat aagaaggcct ttttatttgt taacaaaggt     360
ctgaatacag atgaattagg tcagaaggaa gaagcaaaga actactataa gcaaggaata     420
ggacacctgc tcagagggat cagcatttca tcaaaagagt ctgaacacac aggtcctggg     480
tgggaatctg ctagacagat gcaacagaaa atgaaagaaa ctctacagaa tgtacgcacc     540
aggctggaaa ttctagagaa gggtcttgcc acttctctgc agaatgatct tcaggaggtg     600
cccaagttat atccagaatt tccacctaaa gacatgtgtg aaaaattacc agagcctcag     660
tcttttagtt cagctcctca gcatgctgaa gtaaatggaa acacctcaac tccaagtgca     720
ggggcagttg ctgcacctgc ttctctgtct ttaccatcac aaagttgtcc agcagaagct     780
cctcctgctt atactcctca agctgctgaa ggtcactaca ctgtatccta tggaacagat     840
tctggggagt tttcatcagt tggagaggag ttttatagga atcattctca gccaccgcct     900
cttgagacct tagggctgga tgcagatgaa ttgattttga taccaaatgg agtacagatt     960
ttttttgtaa atcctgcagg ggaggttagt gcaccttcgt atcctgggta ccttcgaatt    1020
gtgaggtttt tggataattc tctcgatacg gttctaaacc gtcctcccgg gtttcttcag    1080
gtttgtgact ggttatatcc tctagttcct gatagatctc cggttctgaa atgtactgcg    1140
ggagcctaca tgtttcctga tacaatgcta caagcagcag gatgctttgt gggggtcgtc    1200
ctgtcctctg agttaccaga ggatgataga gagctctttg aggatctgtt aaggcaaatg    1260
tctgaccttc ggctccaggc caactggaac agagcagaag aagaaaatga attccaaatc    1320
cctggaagaa ctagaccctc ctctgaccaa ctaaaagaag cctctggcac tgatgtgaaa    1380
cagttggacc aaggcaataa ggatgtacgt cataaaggaa aacgtggaaa aagggctaaa    1440
gatacttcaa gtgaagaagt taacctgagt cacattgtac catgtgagcc agttccagaa    1500
gaaaagccaa aagaattacc tgaatggagt gaaaaagtgg ctcacaacat tttgtcaggt    1560
gcttcctggg tgagttgggg tttagtcaaa ggtgctgaga ttactggtaa ggcaatccag    1620
aaaggtgctt ctaaactccg agagcggatt caaccagaag aaaaacccgt ggaagttagt    1680
ccagctgtca ccaaggact ttatatagcg aagcaagcta caggaggagc agcaaaagtc    1740
```

agtcagttcc tggttgatgg agtttgcact gtagcaaatt gcgttggaaa agaactagct 1800
ccacatgtca agaagcatgg aagcaaactt gttccagaat ctcttaaaaa agacaaagat 1860
gggaaatctc ctctggatgg tgctatggtt gtagcagcaa gtagtgttca aggattttca 1920
actgtctggc aaggattgga atgtgcagct aaatgcatcg ttaacaatgt ttcagcagaa 1980
actgtacaaa ctgtcagata caaatacgga tataatgcag gagaagctac ccaccatgcg 2040
gtggattctg cggtcaatgt tggcgtaact gcctacaata ttaacaacat tggtatcaaa 2100
gcaatggtga agaaaactgc aacacaaaca ggacacactc tccttgagga ctatcagata 2160
gttgataatt ctcagaggga aaatcaagaa ggagcagcaa atgtcaacgt gagaggggag 2220
aaggatgagc agacgaagga agtaaaggag gcaaagaaga aagataaatg atgaagtgct 2280
gggaatcact tataccaaag ccttatgaaa tggatgaaat tttgttaaat aggcaaatgt 2340
ggaattcctc acagattaac cagtattttt taaatgtatt cattcctaca aattaacttt 2400
cataaatttt atggcatgtc ttctatttaa aaggaaaaga ataagtattc ttgcatctgg 2460
ccttagaaat gtgaagttat attctcaagt ttattttttt ccaagtgtag ctaaaatatt 2520
tttgcaggta aaataaagct gatagtacat gtgttgttca aaccttgtta aacctaatat 2580
tgaactattt ttatatctgc tgtctttcag aaggcaaata ggaaactata tatttgctta 2640
aaaattggca tttagtaacc ttaattcttt ttatagaagg aatgacttaa agtattgtcc 2700
cctctttttg cactaattgt ggattttttt agatgcttct caaaattttc agtgtgtaag 2760
ctaaacaaaa actaaaacta agaattctca aaaaaacttg ttcaaaacag ggaaagactg 2820
atgaaaagta aaatggacta cttttgtaac ttacctgttt gttaggaaat ggaatggtct 2880
ctttgattta aaataaataa aaatagatta ttacgtcttt tgtattgaga ctgtattgtt 2940
atgagcctag gaaatttggg aacatgattg tattgtatta aaattcgaag tgattattat 3000
cagcttaatt ggattaaaaa agtacttcaa gaaattattt tatcatatct gcttctgttt 3060
ttccaaaagg ttaaaacttg taaaaaaaat atatataaac aattgagttt actaatggta 3120
aacattttta ttctgggatt cggtcattgg aatttatatt aaaagacaag ttattaaaaa 3180
ggaaaggttc tattcataat cagggtaaag aatatgaaaa ccttagacgt aatccatggt 3240
ggataggcat tatggtttcc actttggcag aaggcagact attcacagcc ctatttactt 3300
acataggcta aaaaactatg taactaaata cctaatggta tttaattttt gtttattgaa 3360
tttaagagat tggtattagt tttcatagct gtagtccatt ctaataattt ctgatcttct 3420
agtggctact taattagaca ttatttgaag ctgtctgaag aatgcacttt atgaattaaa 3480
aaactgaatt gcctgacctc gttatcacat gagcttatat tttgggaaca catagaactg 3540

atggaggctt ttcctaaggc caaggataat gtactagttg ttaaaatgga aataaaagtg 3600
aagtggtaaa tagcttacag acagtctatg ctttgtttaa gaaatggaat ggcacttcat 3660
atattctcat ttgtgggagc taaaaattac aataatcaaa cttaaggaga tagagagtag 3720
aatgatggtt accagaggct gggaggcatt gaagggatgg ggagggaagt gaggttgatt 3780
aatgggtacg aaaatatagt tagaacgaat ttgttgtagt atttgatagc ccaacagggt 3840
gactacagtc aacaataact ttatacattt aaaaataaaa gagtgtaatt gggacgtttg 3900
taacacaaat gaatgcttga agcagtagat acaccctgat gtaattatta taaatcgcat 3960
acctgtatca aaatatctca tgtatcccat aaatatatac acctaccatg tacccataaa 4020
aatttaaaaa gaaatggaat ggcaaataat ggaacataaa ctttaaacag ttagctttga 4080
ccagaggtca gcaaatatcc tccatgggct aaatccagat gtccacctgt tttcataaat 4140
aacgttttat tggagcatag ctgtacccat ttgtttacat attggctatg gctgctgtct 4200
agttcaacgt gagagttgaa tagttgcatc agagagtata tggtccacaa gcctaactta 4260
ttcactctct ggccctttgc agaaaaagtg tgctgcctta gtctatagaa aatggacttt 4320
tcccaccatt cactgactca ttaggagata ggcatcttct aaagattttt tttatattgt 4380
gtaatgattg atatatagat aaaattatat attacttgta aggtatgatg aataacaaca 4440
cttaaaaact catcacacct aaagacatgg aaagctgcca attacctata tgtatgttct 4500
tcctattttc ttacctcttt ccccatgtca tttttattg ttccttgcca ttttttaatc 4560
acacatgtat gttctccaaa catactgttt catttttga agctttctac atatagtgtc 4620
tgaatgtgaa gtcttactca acttgctttt ttgagtttat gtttaaaatt cattcttgcg 4680
cttatatgca tggatggttt acttgctcta tagtattctg tgtgagtata ccacaattta 4740
agtgttcatt ttcctatcag ttgacatttg tttttagttt ttgttggtag aaacagtaca 4800
ctcaaaacat ccttgactgt cccctggtac acatatgcaa gagactttat gcgtaagagt 4860
ggagttgttg gcttgtcaag ttttgcaaaa taatgccagg tattttccaa aatgatgata 4920
ttctcttgag tattacttcc ctttgcaatg tttttattgt taatgtagca tatataaaaa 4980
agtatctcat aattttaaaa aaaaaaaaaa aaaa 5014

<210> 5
<211> 4962
<212> DNA
<213> Homo sapiens

<400> 5

cggaggcctc ggtttcgctg gagagaaaga gattgcgcag gcgcggagca gccgacgtgg 60

ccgcagggga ccgtgcctgg gggggtctgc gcggggccgc tgactcgcga ggttctctgt 120
cgcctgccgc cctcgctcct tttctcccct tctagagtgt tacggtgact cttgggtccg 180
aaggggacgg acgtcctccc acgtaccatt tagaagaaat ggagcaagag ccacaaaatg 240
gagaacctgc tgaaattaag atcatcagag aagcatataa gaaggccttt ttatttgtta 300
acaaaggtct gaatacagat gaattaggtc agaaggaaga agcaaagaac tactataagc 360
aaggaatagg acacctgctc agagggatca gcatttcatc aaaagagtct gaacacacag 420
gtcctgggtg ggaatctgct agacagatgc aacagaaaat gaaagaaact ctacagaatg 480
tacgcaccag gctggaaatt ctagagaagg gtcttgccac ttctctgcag aatgatcttc 540
aggaggtgcc caagttatat ccagaatttc cacctaaaga catgtgtgaa aaattaccag 600
agcctcagtc ttttagttca gctcctcagc atgctgaagt aaatggaaac acctcaactc 660
caagtgcagg ggcagttgct gcacctgctt ctctgtcttt accatcacaa agttgtccag 720
cagaagctcc tcctgcttat actcctcaag ctgctgaagg tcactacact gtatcctatg 780
gaacagattc tggggagttt tcatcagttg gagaggagtt ttataggaat cattctcagc 840
caccgcctct tgagacctta gggctggatg cagatgaatt gattttgata ccaaatggag 900
tacagatttt ttttgtaaat cctgcagggg aggttagtgc accttcgtat cctgggtacc 960
ttcgaattgt gaggtttttg gataattctc tcgatacggt tctaaaccgt cctcccgggt 1020
ttcttcaggt ttgtgactgg ttatatcctc tagttcctga tagatctccg gttctgaaat 1080
gtactgcggg agcctacatg tttcctgata caatgctaca agcagcagga tgctttgtgg 1140
gggtcgtcct gtcctctgag ttaccagagg atgatagaga gctctttgag gatctgttaa 1200
ggcaaatgtc tgaccttcgg ctccaggcca actggaacag agcagaagaa gaaaatgaat 1260
tccaaatccc tggaagaact agaccctcct ctgaccaact aaaagaagcc tctggcactg 1320
atgtgaaaca gttggaccaa ggcaataagg atgtacgtca taaaggaaaa cgtggaaaaa 1380
gggctaaaga tacttcaagt gaagaagtta acctgagtca cattgtacca tgtgagccag 1440
ttccagaaga aaagccaaaa gaattacctg aatggagtga aaaagtggct cacaacattt 1500
tgtcaggtgc ttcctgggtg agttggggtt tagtcaaagg tgctgagatt actggtaagg 1560
caatccagaa aggtgcttct aaactccgag agcggattca accagaagaa aaacccgtgg 1620
aagttagtcc agctgtcacc aagggacttt atatagcgaa gcaagctaca ggaggagcag 1680
caaaagtcag tcagttcctg gttgatggag tttgcactgt agcaaattgc gttggaaaag 1740
aactagctcc acatgtcaag aagcatggaa gcaaacttgt tccagaatct cttaaaaaag 1800

acaaagatgg gaaatctcct ctggatggtg ctatggttgt agcagcaagt agtgttcaag 1860
gattttcaac tgtctggcaa ggattggaat gtgcagctaa atgcatcgtt aacaatgttt 1920
cagcagaaac tgtacaaact gtcagataca aatacggata taatgcagga gaagctaccc 1980
accatgcggt ggattctgcg gtcaatgttg gcgtaactgc ctacaatatt aacaacattg 2040
gtatcaaagc aatggtgaag aaaactgcaa cacaaacagg acacactctc cttgaggact 2100
atcagatagt tgataattct cagagggaaa atcaagaagg agcagcaaat gtcaacgtga 2160
gaggggagaa ggatgagcag acgaaggaag taaaggaggc aaagaagaaa gataaatgat 2220
gaagtgctgg gaatcactta taccaaagcc ttatgaaatg gatgaaattt tgttaaatag 2280
gcaaatgtgg aattcctcac agattaacca gtattttta aatgtattca ttcctacaaa 2340
ttaactttca taaattttat ggcatgtctt ctatttaaaa ggaaaagaat aagtattctt 2400
gcatctggcc ttagaaatgt gaagttatat tctcaagttt atttttttcc aagtgtagct 2460
aaaatatttt tgcaggtaaa ataaagctga tagtacatgt gttgttcaaa ccttgttaaa 2520
cctaatattg aactattttt atatctgctg tctttcagaa ggcaaatagg aaactatata 2580
tttgcttaaa aattggcatt tagtaacctt aattcttttt atagaaggaa tgacttaaag 2640
tattgtcccc tctttttgca ctaattgtgg attttttag atgcttctca aaattttcag 2700
tgtgtaagct aaacaaaaac taaaactaag aattctcaaa aaaacttgtt caaaacaggg 2760
aaagactgat gaaaagtaaa atggactact tttgtaactt acctgtttgt taggaaatgg 2820
aatggtctct ttgatttaaa ataaataaaa atagattatt acgtcttttg tattgagact 2880
gtattgttat gagcctagga aatttgggaa catgattgta ttgtattaaa attcgaagtg 2940
attattatca gcttaattgg attaaaaaag tacttcaaga aattatttta tcatatctgc 3000
ttctgttttt ccaaaaggtt aaaacttgta aaaaaaatat atataaacaa ttgagtttac 3060
taatggtaaa catttttatt ctgggattcg gtcattggaa tttatattaa aagacaagtt 3120
attaaaaagg aaaggttcta ttcataatca gggtaaagaa tatgaaaacc ttagacgtaa 3180
tccatggtgg ataggcatta tggtttccac tttggcagaa ggcagactat tcacagccct 3240
atttacttac ataggctaaa aaactatgta actaaatacc taatggtatt taatttttgt 3300
ttattgaatt taagagattg gtattagttt tcatagctgt agtccattct aataatttct 3360
gatcttctag tggctactta attagacatt atttgaagct gtctgaagaa tgcactttat 3420
gaattaaaaa actgaattgc ctgacctcgt tatcacatga gcttatattt tgggaacaca 3480
tagaactgat ggaggctttt cctaaggcca aggataatgt actagttgtt aaaatggaaa 3540
taaaagtgaa gtggtaaata gcttacagac agtctatgct ttgtttaaga aatggaatgg 3600

cacttcatat attctcattt gtgggagcta aaaattacaa taatcaaact taaggagata 3660
gagagtagaa tgatggttac cagaggctgg gaggcattga agggatgggg agggaagtga 3720
ggttgattaa tgggtacgaa aatatagtta gaacgaattt gttgtagtat ttgatagccc 3780
aacagggtga ctacagtcaa caataacttt atacatttaa aaataaaaga gtgtaattgg 3840
gacgtttgta acacaaatga atgcttgaag cagtagatac accctgatgt aattattata 3900
aatcgcatac ctgtatcaaa atatctcatg tatcccataa atatatacac ctaccatgta 3960
cccataaaaa tttaaaaaga aatggaatgg caaataatgg aacataaact ttaaacagtt 4020
agctttgacc agaggtcagc aaatatcctc catgggctaa atccagatgt ccacctgttt 4080
tcataaataa cgttttattg gagcatagct gtacccattt gtttacatat tggctatggc 4140
tgctgtctag ttcaacgtga gagttgaata gttgcatcag agagtatatg gtccacaagc 4200
ctaacttatt cactctctgg ccctttgcag aaaaagtgtg ctgccttagt ctatagaaaa 4260
tggacttttc ccaccattca ctgactcatt aggagatagg catcttctaa agattttttt 4320
tatattgtgt aatgattgat atatagataa aattatatat tacttgtaag gtatgatgaa 4380
taacaacact taaaaactca tcacacctaa agacatggaa agctgccaat tacctatatg 4440
tatgttcttc ctattttctt acctctttcc ccatgtcatt ttttattgtt ccttgccatt 4500
ttttaatcac acatgtatgt tctccaaaca tactgtttca ttttttgaag ctttctacat 4560
atagtgtctg aatgtgaagt cttactcaac ttgctttttt gagtttatgt ttaaaattca 4620
ttcttgcgct tatatgcatg gatggtttac ttgctctata gtattctgtg tgagtatacc 4680
acaatttaag tgttcatttt cctatcagtt gacatttgtt tttagttttt gttggtagaa 4740
acagtacact caaaacatcc ttgactgtcc cctggtacac atatgcaaga gactttatgc 4800
gtaagagtgg agttgttggc ttgtcaagtt ttgcaaaata atgccaggta ttttccaaaa 4860
tgatgatatt ctcttgagta ttacttccct ttgcaatgtt tttattgtta atgtagcata 4920
tataaaaaag tatctcataa ttttaaaaaa aaaaaaaaaa aa 4962

<210> 6
<211> 4838
<212> DNA
<213> Homo sapiens

<400> 6

accagtacca gcccccacct tccgtctgtg catgttttct tcagtcctgg aaggaaatca 60
taagtgattt gccccaaaag gattgctgtt gaaaatggag caagagccac aaaatggaga 120
acctgctgaa attaagatca tcagagaagc atataagaag gccttttttat ttgttaacaa 180

aggtctgaat acagatgaat taggtcagaa ggaagaagca aagaactact ataagcaagg 240
aataggacac ctgctcagag ggatcagcat ttcatcaaaa gagtctgaac acacaggtcc 300
tgggtgggaa tctgctagac agatgcaaca gaaaatgaaa gaaactctac agaatgtacg 360
caccaggctg gaaattctag agaagggtct tgccacttct ctgcagaatg atcttcagga 420
ggtgcccaag ttatatccag aatttccacc taaagacatg tgtgaaaaat taccagagcc 480
tcagtctttt agttcagctc ctcagcatgc tgaagtaaat ggaaacacct caactccaag 540
tgcaggggca gttgctgcac ctgcttctct gtctttacca tcacaaagtt gtccagcaga 600
agctcctcct gcttatactc ctcaagctgc tgaaggtcac tacactgtat cctatggaac 660
agattctggg gagttttcat cagttggaga ggagttttat aggaatcatt ctcagccacc 720
gcctcttgag accttagggc tggatgcaga tgaattgatt ttgataccaa atggagtaca 780
gatttttttt gtaaatcctg caggggaggt tagtgcacct tcgtatcctg ggtaccttcg 840
aattgtgagg tttttggata attctctcga tacggttcta aaccgtcctc ccgggtttct 900
tcaggtttgt gactggttat atcctctagt tcctgataga tctccggttc tgaaatgtac 960
tgcgggagcc tacatgtttc ctgatacaat gctacaagca gcaggatgct ttgtgggggt 1020
cgtcctgtcc tctgagttac cagaggatga tagagagctc tttgaggatc tgttaaggca 1080
aatgtctgac cttcggctcc aggccaactg gaacagagca gaagaagaaa atgaattcca 1140
aatccctgga agaactagac cctcctctga ccaactaaaa gaagcctctg gcactgatgt 1200
gaaacagttg gaccaaggca ataaggatgt acgtcataaa ggaaaacgtg gaaaaagggc 1260
taaagatact tcaagtgaag aagttaacct gagtcacatt gtaccatgtg agccagttcc 1320
agaagaaaag ccaaaagaat tacctgaatg gagtgaaaaa gtggctcaca acattttgtc 1380
aggtgcttcc tgggtgagtt ggggtttagt caaaggtgct gagattactg gtaaggcaat 1440
ccagaaaggt gcttctaaac tccgagagcg gattcaacca gaagaaaaac ccgtggaagt 1500
tagtccagct gtcaccaagg gactttatat agcgaagcaa gctacaggag gagcagcaaa 1560
agtcagtcag ttcctggttg atggagtttg cactgtagca aattgcgttg gaaaagaact 1620
agctccacat gtcaagaagc atggaagcaa acttgttcca gaatctctta aaaaagacaa 1680
agatgggaaa tctcctctgg atggtgctat ggttgtagca gcaagtagtg ttcaaggatt 1740
ttcaactgtc tggcaaggat tggaatgtgc agctaaatgc atcgttaaca atgtttcagc 1800
agaaactgta caaactgtca gatacaaata cggatataat gcaggagaag ctacccacca 1860
tgcggtggat tctgcggtca atgttggcgt aactgcctac aatattaaca acattggtat 1920

caaagcaatg gtgaagaaaa ctgcaacaca aacaggacac actctccttg aggactatca 1980
gatagttgat aattctcaga gggaaaatca agaaggagca gcaaatgtca acgtgagagg 2040
ggagaaggat gagcagacga aggaagtaaa ggaggcaaag aagaaagata aatgatgaag 2100
tgctgggaat cacttatacc aaagccttat gaaatggatg aaattttgtt aaataggcaa 2160
atgtggaatt cctcacagat taaccagtat tttttaaatg tattcattcc tacaaattaa 2220
ctttcataaa ttttatggca tgtcttctat ttaaaaggaa aagaataagt attcttgcat 2280
ctggccttag aaatgtgaag ttatattctc aagtttattt ttttccaagt gtagctaaaa 2340
tatttttgca ggtaaaataa agctgatagt acatgtgttg ttcaaacctt gttaaaccta 2400
atattgaact atttttatat ctgctgtctt tcagaaggca aataggaaac tatatatttg 2460
cttaaaaatt ggcatttagt aaccttaatt ctttttatag aaggaatgac ttaaagtatt 2520
gtcccctctt tttgcactaa ttgtggattt ttttagatgc ttctcaaaat tttcagtgtg 2580
taagctaaac aaaaactaaa actaagaatt ctcaaaaaaa cttgttcaaa acagggaaag 2640
actgatgaaa agtaaaatgg actacttttg taacttacct gtttgttagg aaatggaatg 2700
gtctctttga tttaaaataa ataaaaatag attattacgt cttttgtatt gagactgtat 2760
tgttatgagc ctaggaaatt tgggaacatg attgtattgt attaaaattc gaagtgatta 2820
ttatcagctt aattggatta aaaaagtact tcaagaaatt attttatcat atctgcttct 2880
gtttttccaa aaggttaaaa cttgtaaaaa aaatatatat aaacaattga gtttactaat 2940
ggtaaacatt tttattctgg gattcggtca ttggaattta tattaaaaga caagttatta 3000
aaaaggaaag gttctattca taatcagggt aaagaatatg aaaaccttag acgtaatcca 3060
tggtggatag gcattatggt ttccactttg gcagaaggca gactattcac agccctattt 3120
acttacatag gctaaaaaac tatgtaacta aatacctaat ggtatttaat ttttgtttat 3180
tgaatttaag agattggtat tagttttcat agctgtagtc cattctaata atttctgatc 3240
ttctagtggc tacttaatta gacattattt gaagctgtct gaagaatgca ctttatgaat 3300
taaaaaactg aattgcctga cctcgttatc acatgagctt atattttggg aacacataga 3360
actgatggag gcttttccta aggccaagga taatgtacta gttgttaaaa tggaaataaa 3420
agtgaagtgg taaatagctt acagacagtc tatgctttgt ttaagaaatg gaatggcact 3480
tcatatattc tcatttgtgg gagctaaaaa ttacaataat caaacttaag gagatagaga 3540
gtagaatgat ggttaccaga ggctgggagg cattgaaggg atggggaggg aagtgaggtt 3600
gattaatggg tacgaaaata tagttagaac gaatttgttg tagtatttga tagcccaaca 3660
gggtgactac agtcaacaat aactttatac atttaaaaat aaaagagtgt aattgggacg 3720

```
tttgtaacac aaatgaatgc ttgaagcagt agatacaccc tgatgtaatt attataaatc   3780
gcatacctgt atcaaaatat ctcatgtatc ccataaatat atacacctac catgtaccca   3840
taaaaattta aaaagaaatg gaatggcaaa taatggaaca taaactttaa acagttagct   3900
ttgaccagag gtcagcaaat atcctccatg ggctaaatcc agatgtccac ctgttttcat   3960
aaataacgtt ttattggagc atagctgtac ccatttgttt acatattggc tatggctgct   4020
gtctagttca acgtgagagt tgaatagttg catcagagag tatatggtcc acaagcctaa   4080
cttattcact ctctggccct ttgcagaaaa agtgtgctgc cttagtctat agaaaatgga   4140
cttttcccac cattcactga ctcattagga gataggcatc ttctaaagat tttttttata   4200
ttgtgtaatg attgatatat agataaaatt atatattact tgtaaggtat gatgaataac   4260
aacacttaaa aactcatcac acctaaagac atggaaagct gccaattacc tatatgtatg   4320
ttcttcctat tttcttacct ctttccccat gtcatttttt attgttcctt gccatttttt   4380
aatcacacat gtatgttctc caaacatact gtttcatttt ttgaagcttt ctacatatag   4440
tgtctgaatg tgaagtctta ctcaacttgc ttttttgagt ttatgtttaa aattcattct   4500
tgcgcttata tgcatggatg gtttacttgc tctatagtat tctgtgtgag tataccacaa   4560
tttaagtgtt cattttccta tcagttgaca tttgttttta gtttttgttg gtagaaacag   4620
tacactcaaa acatccttga ctgtcccctg gtacacatat gcaagagact ttatgcgtaa   4680
gagtggagtt gttggcttgt caagttttgc aaaataatgc caggtatttt ccaaaatgat   4740
gatattctct tgagtattac ttccctttgc aatgttttta ttgttaatgt agcatatata   4800
aaaaagtatc tcataatttt aaaaaaaaaa aaaaaaa                            4838
```

<210> 7
<211> 1762
<212> DNA
<213> Homo sapiens

<400> 7

```
gctaatccca gtcggtgccg catccccagc ccgccgccat ggccgcctac aaactggtgc     60
tgatccggca cggcgagagc gcatggaacc tggagaaccg cttcagcggc tggtacgacg    120
ccgacctgag cccggcgggc cacgaggagg cgaagcgcgg cgggcaggcg ctacgagatg    180
ctggctatga gtttgacatc tgcttcacct cagtgcagaa gagagcgatc cggaccctct    240
ggacagtgct agatgccatt gatcagatgt ggctgccagt ggtgaggact tggcgcctca    300
atgagcggca ctatgggggt ctaaccggtc tcaataaagc agaaactgct gcaaagcatg    360
gtgaggccca ggtgaagatc tggaggcgct cctatgatgt cccaccacct ccgatggagc    420
```

ccgaccatcc tttctacagc aacatcagta aggatcgcag gtatgcagac ctcacagaag 480
atcagctacc ctcctgtgag agtctgaagg atactattgc cagagctctg cccttctgga 540
atgaagaaat agttccccag atcaaggagg ggaaacgtgt actgattgca gcccatggca 600
acagcctccg gggcattgtc aagcatctgg agggtctctc tgaagaggct atcatggagc 660
tgaacctgcc gactggtatt cccattgtct atgaattgga caagaacttg aagcctatca 720
agcccatgca gtttctgggg gatgaagaga cggtgcgcaa agccatggaa gctgtggctg 780
cccagggcaa ggccaagaag tgaaggccgg cggggaggat actgtcccca ggagcaccct 840
ccctgcccgt cttgtccctc tgcccctccc acctgcacat gtcacactga ccacatctgt 900
agacatcttg agttgtagct gcagacgggg accagtggct cccattttca ttttagccat 960
tttgtcgcct gcacccactc ccttcataca atctagtcag aatagcagtt ctagagcaca 1020
ggttctcagt ctaagctatg gaaaagctcc ccttatccaa cagagtttaa aagtagtgac 1080
ttgggttttt gcgagtgctt tgtttactaa ggactttggg gaggaaccat gctaagccat 1140
gaccagtgag gagaagcaac agagcctgtc tgtccccatg agcggagtct gtcctctgct 1200
cttctgcagt caggtcactg cctactgcct gggggctcta gtcattccag tggaagacga 1260
atgtaacctg cgtggtgatg tgacaactgt ttcctccctg accccagagg atctggctct 1320
aggttgggat caatcctgaa tttcgttatg tgttaattta cttttattaa aaaagtatag 1380
tatatataat acaaaacaat aacccttctg ggtttcttg tggcggttga aatagtccca 1440
catgtggtca tcagaaaata agccattcct cataccaata taggatcagc tccttgacct 1500
ctgaggggca ggagtgcttc ctggtgtgtg tattagaatc ccttcctgcc ttgtttcatg 1560
gcagtgaaat gcctcttggt cctgtccaag tgtatctttc actgatttct gaatcatgtt 1620
ctagttgctt gaccctgcca catgggtcca gtgttcatct gagcataact gtactaaatc 1680
ctttttccat atcagtataa taaaggagtg atgtgcaata aaaaaaaaaa aaaaaaaaaa 1740
aaaaaaaaaa aaaaaaaaaa aa 1762

<210> 8
<211> 951
<212> DNA
<213> Homo sapiens

<400> 8

```
attggtggga tgagcaatcc gagttcccgg atgagggaac attctgcagt ataaagggag    60
cagggaaggc gggagacagc gcagtttgaa tcgcggtgcg acgaaggagt aggtggtggg   120
atctcaccgt gggtccgatt agccttttct ctgccttgct tgcttgagct tcagcggaat   180
tcgaaatggc tggcggtaag gctggaaagg actccggaaa ggccaagaca aaggcggttt   240
cccgctcgca gagagccggc ttgcagttcc cagtgggccg tattcatcga cacctaaaat   300
ctaggacgac cagtcatgga cgtgtgggcg cgactgccgc tgtgtacagc gcagccatcc   360
tggagtacct caccgcagag gtacttgaac tggcaggaaa tgcatcaaaa gacttaaagg   420
taaagcgtat tacccctcgt cacttgcaac ttgctattcg tggagatgaa gaattggatt   480
ctctcatcaa ggctacaatt gctggtggtg gtgtcattcc acacatccac aaatctctga   540
ttgggaagaa aggacaacag aagactgtct aaaggatgcc tggattcctt gttatctcag   600
gactctaaat actctaacag ctgtccagtg ttggtgattc cagtggactg tatctctgtg   660
aaaaacacaa ttttgccttt ttgtaattct atttgagcaa gttggaagtt taattagctt   720
tccaaccaac caaatttctg cattcgagtc ttaaccatat ttaagtgtta ctgtggcttc   780
aaagaagcta ttgattctga agtagtgggt tttgattgag ttgactgttt ttaaaaaact   840
gtttggattt taattgtgat gcagaagtta tagtaacaaa catttggttt tgtacagaca   900
ttatttccac tctggtggat aagttcaata aaggtcatat cccaaacaaa a            951
```

<210> 9
<211> 4021
<212> DNA
<213> Homo sapiens

<400> 9

```
ggtagccgcc ccgccccgcg gggcgccacg ggcgggtctt ggcagcgccc actgagccag      60
ccgggccgca ggtgccgccc ccgatacacg gtgtcccgcc caagctgatc cgcgtctgcg     120
gtcggtcggt gcgtgcgtgc gcctcgtcgg tccgcgtgtc tggccgagag ccccccttcct    180
ctgcggccat gactccgccg ccgccgccgc cccctccccc gggccctgac cccgcggccg     240
accccgccgc ggacccctgc ccctggcccg gatcactggt cgtcctcttc ggggctacgg     300
cgggtgcgct gggacgggac ctgggctcgg acgagaccga cttaatcctc ctagtttggc     360
aagtggttga gccgcggagc cgccaggtgg ggacgctgca caaatcgctg gttcgtgccg     420
aggcggccgc actgagtacg cagtgccgcg aggcgagcgg cctgagcgcc gacagcctgg     480
cgcgggcaga gccgctggac aaggtgctgc agcagttctc acagctggtg aacggggatg     540
tggctttgct gggcgggggc ccctacatgc tctgcactga tgggcagcag ctattgcgac     600
aggtcctgca ccccgaggcc tccaggaaga acctggtgct ccccgacatg ttcttctcct     660
tctatgacct ccgaagagaa ttccatatgc agcatccaag cacctgccct gccagggacc     720
tcactgtggc caccatggca cagggtttag gactggagac agatgccaca gaggatgact     780
```

ttggggtctg ggaagtcaag acaatggtag ctgttatcct ccatctactc aaagagccca 840
gcagtcaatt gttttcgaag cccgaggtga taaagcagaa atacgagacg gggccttgca 900
gcaaggctga tgtggtggac agtgagactg tggtacgggc tcgtgggttg ccgtggcagt 960
catcagacca ggacgtggct cgcttcttca aagggctcaa cgtggccagg ggtggtgtag 1020
cactctgcct caacgcccag ggccgcagaa atggcgaggc cctcatccgc tttgtggaca 1080
gcgagcagcg ggacctagcg ctgcagagac acaagcacca catgggcgtc cgctatattg 1140
aggtgtataa agcgacaggg gaggagtttg taaagattgc agggggcaca tcactagagg 1200
tggctcgttt cttgtcacgg gaagaccaag tgatcctgcg gctgcgggga ctgcccttct 1260
cggctgggcc aacggacgtg cttggcttcc tggggccaga gtgcccagtg actgggggta 1320
ccgaggggct gctctttgtg cgccatcctg atggccggcc gactggtgat gccttcgccc 1380
tctttgcttg tgaggagctg gcacaggctg cactgcgcag gcacaagggc atgctgggta 1440
agcgatacat tgaactcttc cggagcactg cagccgaagt gcagcaggtc ttgaaccgct 1500
atgcatccgg cccactcctt cctacactga ctgccccact gctgcccatc cccttcccac 1560
tggcacctgg gactgggagg gactgtgtac gcctccgagg cctgccctac acggccacca 1620
ttgaagacat cctgagcttt ctgggggagg cagcagctga cattcggccc cacggtgtac 1680
acatggtgct caaccagcag ggccggccat cgggcgatgc cttcattcag atgacatcag 1740
cagagcgagc cctagctgct gctcagcgtt gccataagaa ggtgatgaag gagcgctacg 1800
tggaggtggt cccctgttcc acagaggaga tgagccgagt gctgatgggg ggcaccttgg 1860
gccgcagtgg catgtcccct ccaccctgca agctgccctg cctctcacca cctacctaca 1920
ccaccttcca agccacccca acgctcattc ccacggagac ggcagctcta tacccctctt 1980
cagcactgct cccagctgcc agggtgcctg ctgcccccac ccctgttgcc tactatccag 2040
ggccagccac tcaactctac ctgaactaca cagcctacta cccaagcccc ccagtctccc 2100
ccaccactgt gggctacctc actacaccca ctgctgccct ggcctctgct cccacctcag 2160
tgttgtccca gtcaggagcc ttggtccgca tgcagggtgt cccatacacg gctggtatga 2220
aggatctgct cagcgtcttc caggcctacc agctacccgc tgatgactac accagtctga 2280
tgcctgttgg tgacccacct cgcactgtgt tacaagcccc caaggaatgg gtgtgtttgt 2340
aggagagaaa gccaggaggt aagagccagc tgatatcctc ggcgaacatg tctctcctga 2400
gtccagaaga ccagcaccct caacctggta gcttctttct ggcttgtcaa agctctcaga 2460
aggtacctag aggagcccaa gccccagctc catcctccac ttattctgcc tgtttccccc 2520

aaagacaatg gctggaccct gcatgcaggg ctgggggtgg aatggggcta accagctcct 2580
gatggcctga gccaggcatc ttgactggca cctggagagc ccttaagtct gtcctggctg 2640
tggcccatgc cgacagatat cgtggggctg acaggtccac ggcaggcttg ctttctttta 2700
taaaatggaa gctctggtac cttcaatgta tgactcctgg gagaatcaag ggtccatctg 2760
agcctctgag taaagatccc aatgttctac ctctccctgt ccctcttgta ggggataggg 2820
aggcagagag agccagcccc taccctcaga gtatctggac ctcagagacc atgttgtgcc 2880
aggggtggtc ccacctaaag atgctagccc ctctccaggt gggcataagg agtaacagat 2940
ggcaaaacca caaactattt tgatggactg tgctgcagta tcaccagaag acattagggg 3000
gcagtaggcc cccacacaaa accttcaggc ttgaatttta aaggggagga ctttctgcca 3060
acttttcttg tatgccttgg gaaagccagt tgccctgaac ccagcagaca ccatggaatg 3120
tcctttgcac gcattaaatg gtacagaact gaagcctcgg aagcaatttg gaactcgatc 3180
ttctcttcct taaatgaaaa gttattgacc aaatggactt tttaaaagac acaggaccct 3240
taactttgcc ccaaagtgag gggctccaca ccaaccccag gcggaggaac actcagacag 3300
attaaggata ctgttgacct gtcactgttt attatttcag cactaaaact gaggagcctc 3360
aactgctggc tcttcttccc tttgtatttg tgtaaggagc actgcactcc cataaaaggt 3420
tttaaaatac aaaatgtaca agaacacaca attccaagtg ctgtaaacat aactgagaac 3480
cagttccttt actaaacatc cattttataa aacacaaggt ttcaatttga gcccatctga 3540
gccttaaaga tccattctga ataccaaaaa cagggcttca cagccaggcc cagaagaggt 3600
ctggtgataa tggctggccc tgggtgggga tagtttacac ccgggcagca gcaccacaca 3660
tgaacccaaa gacatgttct ttttaaagct gttttcagcc atgtttctct gtgcatctcc 3720
agtaagcaga aggctaccca ttccattcct caacccaaga gctagcacag ttagagtagg 3780
aggggtgcg tactagcacg tgcccagttg ctcagtgctg ctagtagaaa ttgatttgca 3840
tagtccaatg gatgtgtgct ttaacaccac tatgttgcac aaaaatttaa gtctttatct 3900
acaaagccaa aaaatattga ctcttaacac caaagctttt acaaagctga tataaaactg 3960
cttacatagt atacaaagct ctattttaaa atttaatgtt tattttaaat aggaaagcat 4020
t 4021

<210> 10
<211> 1636
<212> DNA
<213> Homo sapiens

<400> 10

```
cagtctacgc ttgcgcggcg caacagggcc gactgcagct ggaagatggc ggcgtccgtg     60
gtctgtcggg ccgctaccgc cggggcacaa gtgctattgc gcgcccgccg ctcgccggcc    120
ctgctgcgga cgccagcctt gcggagtacg gcaaccttcg ctcaggcgct ccagttcgtg    180
ccggagacgc aggttagcct gctggacaac ggcctgcgtg tggcctccga gcagtcctct    240
cagcccactt gcacggtggg agtgtggatt gatgttggca gccgttttga gactgagaag    300
aataatgggg caggctactt tttggagcat ctggctttca agggaacaaa gaatcggcct    360
ggcagtgccc tggagaagga ggtggagagc atgggggccc atcttaatgc ctacagcacc    420
cgggagcaca cagcttacta catcaaggcg ctgtccaagg atctgccgaa agctgtggag    480
ctcctgggtg acattgtgca gaactgtagt ctggaagact cacagattga gaaggaacgt    540
gatgtgatcc tgcgggagat gcaggagaat gatgcatcta tgcgagatgt ggtctttaac    600
tacctgcatg ccacagcatt ccagggcaca cctctagccc aggctgtgga ggggcccagt    660
gagaatgtca ggaagctgtc tcgtgcagac ttgaccgagt acctcagcac acattacaag    720
gcccctcgaa tggtgctggc agcagctgga ggagtggagc accagcaact gttagacctc    780
gcccagaagc acctcggtgg catcccatgg acatatgcag aggacgctgt gcccactctt    840
actccatgcc gcttcactgg cagtgagatc cgccaccgtg atgatgctct acctttgcc     900
cacgtggcca ttgcagtaga gggtcctggc tgggccagcc cggacaatgt ggccttgcaa    960
gtggccaatg ccatcatcgg ccactatgac tgcacttatg gtggtggcgt gcacctgtcc   1020
agcccactgg cttcaggtgc tgtggccaac aagctatgcc agagtttcca gaccttcagc   1080
atctgctatg cagagacggg cttgctgggt gcacactttg tctgtgaccg aatgaaaatc   1140
gatgacatga tgttcgtcct gcaagggcag tggatgcgcc tgtgtaccag tgccacggag   1200
agtgaggtgg cccggggcaa aaacatcctc agaaatgccc tggtatctca tctagatggc   1260
actactcctg tgtgtgagga catcggacgc agcctcctga cctatggccg ccgcatcccc   1320
ctggctgaat gggaaagccg gattgcggag gtggatgcca gtgtggtacg tgagatctgc   1380
tccaagtaca tctatgacca gtgcccagca gtggctggat atggccccat tgagcagctc   1440
ccagactaca accggatccg tagcggcatg ttctggctgc gcttctaggc gggaagccta   1500
tgtaagcaag agggcagggc cggggtttgt ggtccccccc ccaccacaaa cacagcactt   1560
cggctcctct aacctgtgcc acaggtgacc accaataaaa tcctctgctg agaagtgaaa   1620
aaaaaaaaaa aaaaaa                                                   1636
```

<210> 11
<211> 1591
<212> DNA

<213> Homo sapiens

<400> 11

atataagcgt tggcggagcg tcggttgtag cactctgcgc gcccgctctt ctgctgctgt 60
ttgtctactt cctcctgctt ccccgccgcc gccgccgcca tcatgaggga aatcgtgcac 120
ttgcaggccg ggcagtgcgg caaccaaatc ggcgccaagt tttgggaggt gatcagcgat 180
gagcacggca tcgaccccac gggcacctac cacggggaca gcgacctgca gctggaacgc 240
atcaacgtgt actacaatga ggccaccggc ggcaagtacg tgccccgcgc cgtgctcgtg 300
gatctggagc ccggcaccat ggactccgtg cgctcggggc ccttcgggca gatcttccgg 360
ccggacaact tcgttttcgg tcagagtggt gctgggaaca actgggccaa ggggcactac 420
acagaaggcg cggagctggt ggactcggtg ctggatgttg tgagaaagga ggctgagagc 480
tgtgactgcc tgcagggttt ccagctgacc cactccctgg gtgggggcac tgggtctggg 540
atgggtaccc tcctcatcag caagatccgg gaggagtacc cagacaggat catgaacacg 600
tttagtgtgg tgccttcgcc caaagtgtca gacacagtgg tggagcccta caacgccacc 660
ctctcagtcc accagctcgt agaaaacaca gacgagacct actgcattga taacgaagct 720
ctctacgaca tttgcttcag aaccctaaag ctgaccacgc ccacctatgg tgacctgaac 780
cacctggtgt ctgctaccat gagtggggtc accacctgcc tgcgcttccc aggccagctc 840
aatgctgacc tgcggaagct ggctgtgaac atggtcccgt ttccccggct gcacttcttc 900
atgcccggct ttgccccact gaccagccgg ggcagccagc agtaccgggc gctgaccgtg 960
cccgagctca cccagcagat gtttgatgcc aagaacatga tggctgcctg cgacccccgc 1020
catggccgct acctgacggt tgccgccgtg ttcaggggcc gcatgtccat gaaggaggtg 1080
gatgagcaaa tgcttaatgt ccaaaacaaa aacagcagct attttgttga gtggatcccc 1140
aacaatgtga aaacggctgt ctgtgacatc ccacctcggg ggctaaaaat gtccgccacc 1200
ttcattggca acagcacggc catccaggag ctgttcaagc gcatctccga gcagttcacg 1260
gccatgttcc ggcgcaaggc cttcctgcac tggtacacgg gcgagggcat ggacgagatg 1320
gagttcaccg aggccgagag caacatgaat gacctggtgt ccgagtacca gcagtaccag 1380
gatgccacag ccgaggagga gggcgagttc gaggaggagg ctgaggagga ggtggcctag 1440
agccttcagt cactggggaa agcagggaag cagtgtgaac tctttattca ctcccagcct 1500
gtcctgtggc ctgtcccact gtgtgcactt gctgttttcc ctgtccacat ccatgctgta 1560
cagacaccac cattaaagca ttttcatagt g 1591

<210> 12

<211> 2543
<212> DNA
<213> Homo sapiens

<400> 12

```
gctcttagac aaaccaacag cagcttcttc tgacatatac acacgcacac tcaccccgga    60
cacacactca gcacactttt cctccatttg attaacagtg ctgcacacac aatgattacg   120
ggaaagcgca aataaatacg gaaaggggtg cttattttga ctactggaag agctttgctg   180
ggtctcagcg caacttttgt tttttattcc tgagaaggtg atctctccat gcggttctct   240
cacacaagga ttctttaaaa gaggaagaga gacaagcaga gggggaggga cagtctttca   300
ctttaagaac ggctgggctc aaagataaaa ggaagggaaa agcagcagca gcagcagcag   360
cagcagcagc agcagcagca gcagcagcag cagcagcagg gaaaccaacg ctgcagcact   420
tccgaaaggc atttttgatc catttctgag tgttgcggcc cgtttctcca ccgaagttgg   480
ctccagctct agcagccgca ttggatccca cagcttactg cgagactccg gtgtacaatc   540
cggatctctg ccccaacatg attgcggccc aggccaagct ggtctaccat ctgaataaat   600
actacaacga aaaatgccaa gccaggaaag ctgccattgc caaaactatc cgggaagtct   660
gcaaagtagt ttccgacgta ctgaaggaag tggaagtgca ggagccgcgg ttcatcagct   720
ctctcaacga gatggacaat cgctacgagg gcctcgaggt catctccccc accgaatttg   780
aagtggtgct ttatctcaac caaatggggg tgttcaactt cgtggacgat ggctcactgc   840
ccggctgcgc ggtgctgaag ttgagcgacg ggcgcaagag gagcatgtcc ctctgggtgg   900
aattcattac cgcctccggc tacctctcgg cgcgcaaaat ccggtccagg tttcagacgc   960
tggtggctca agcggtagac aaatgtagct accgggatgt ggtaaagatg gtggcagaca  1020
ccagcgaagt gaaactgaga atccgagata ggtacgtggt gcagatcacg ccggccttta  1080
aatgcaccgg gatctggccg aggagtgctg cccactggcc acttccccac atcccctggc  1140
cgggacccaa ccgggtggcg gaggtcaagg cggaaggttt caatctcttg tccaaggagt  1200
gccactcctt ggccggcaag cagagctcgg cggagagcga cgcctgggtg ctgcagttcg  1260
cggaggcaga gaacagactg cagatggggg gctgcagaaa gaagtgcctc tccatcctca  1320
aaaccttaag ggatcgtcac cttgaactgc cgggccagcc cttgaacaat taccatatga  1380
agactctggt ttcctacgag tgtgaaaagc atccccgaga gtcggactgg gacgagtctt  1440
gcctgggtga tcggctgaac gggattttgc tgcaacttat ctcctgcctg cagtgccggc  1500
ggtgtcccca ctactttcta ccgaacttag atctgtttca aggcaaacct cactcagctc  1560
tggaaaacgc tgccaaacaa acgtggcgac tggcaagaga gatcctgacc aacccgaaaa  1620
```

gtttggaaaa actttagagg atgatttaat caagagccga aattattacc cttctcaaag 1680
tccttattaa gtgtaaactt ctgttcaatt cctaatattc cactccgcag tgcaaacaat 1740
ctcttccttt aaaaaggaat aataatacaa tatttaaaca tcatctcacc cacccccaca 1800
aggggagaaa aagtagggga agcggatgga gaaaaaccca aagccactag tattagaaga 1860
cttctttcca cacgatttcc tatctccctt gaaaagtaca ccgtaacact ccgtaaacag 1920
cccagctgta acgccagacc gagacgaaca ctctgcctaa ctatcaaagg attatagcaa 1980
tcctggtgat ttaggtgcat ctgtctgtga gtaaacacga tttggatatg ccatctgaaa 2040
gaaactgtaa tgtatatttt gatttgtaac aaatattgtg atctcacatt gtctttgaaa 2100
gtgtggatgt tggtgttttg tgatttggtg aacagaactt aaattgccat tctggatact 2160
tccagacatt ttccactaac aaagatatca tttaaaggta gatttcttcc tggtactttt 2220
atctgtcttt gaaagtgtct gaactttaaa aagtttacat tttgtttcaa atattgcttg 2280
ttctatttct aacattccat aaatatactt gaaatgttat ttaaatatat tcaaagaaat 2340
ttgaattcag cttatataat aacgcttgaa tatctgaatt atatatttga aaaatgcact 2400
tgaaatacac tggataatta cttttgtgat ttagatttta atttgttgct ggtttttatt 2460
taattagatg gtaataaatg aagtaaaata aaagttgttg tgtctcactt taaaaaaaaa 2520
aaaaaaaaaa aaaaaaaaaa aaa 2543

<210> 13
<211> 4177
<212> DNA
<213> Homo sapiens

<400> 13

```
aatttcgcta accgtgacgt catctcggtg cgccgttaca gcgcttaact ggaaactgcg     60
atttagtttt tggtgaaagg agtggaaaaa aaccatgaac tgacctaccc cgccccattt    120
tgcttttagt ccttcacact ggcttcgcct gcactttggg caatggagcg ggaaaggaaa    180
aggcacgcag gctgagctca aagtcgcgcc agccggggat gactcggcga cgccgccagg    240
cgcgttaccc ggcgtgctcc gcgcggcgcc ggcgaaggga cgtgggggaa ggggcaggga    300
ggaggaagcg gtggctgctg cggatgtcgg tgtgagcgag cggcgcctga acacacggcg    360
gctgccgagc gcctgacccg ggcctgcgcc agagcctgca ccgagctccg gggccccaca    420
cccgctacgg tggccctgcg cccgttgcta ctgaggcggc gtgctctgca ttcttcgctg    480
tccaggcctg ccggctctgg tgtctgctgg ctcctccttg ctcgcctgct ccctcctgct    540
tgcctgagtc accgccgccg ccgccgccac agccatggcc gagagtggtg aaagcggcgg    600
```

tcctccgggc tcccaggata gcgccgccgg agccgaaggt gctggcgccc ccgcggccgc 660
tgcctccgcg gagcccaaaa tcatgaaagt caccgtgaag accccgaagg aaaaggagga 720
attcgccgtg cccgagaata gctccgtcca gcagtttaag gaagaaatct ctaaacgttt 780
taaatcacat actgaccaac ttgtgttgat atttgctgga aaaattttga aagatcaaga 840
taccttgagt cagcatggaa ttcatgatgg acttactgtt caccttgtca ttaaaacaca 900
aaacaggcct caggatcatt cagctcagca aacaaataca gctggaagca atgttactac 960
atcatcaact cctaatagta actctacatc tggttctgct actagcaacc cttttggttt 1020
aggtggcctt gggggacttg caggtctgag tagcttgggt ttgaatacta ccaacttctc 1080
tgaactacag agtcagatgc agcgacaact tttgtctaac cctgaaatga tggtccagat 1140
catggaaaat ccctttgttc agagcatgct ctcaaatcct gacctgatga gacagttaat 1200
tatggccaat ccacaaatgc agcagttgat acagagaaat ccagaaatta gtcatatgtt 1260
gaataatcca gatataatga gacaaacgtt ggaacttgcc aggaatccag caatgatgca 1320
ggagatgatg aggaaccagg accgagcttt gagcaaccta gaaagcatcc caggggggata 1380
taatgcttta aggcgcatgt acacagatat tcaggaacca atgctgagtg ctgcacaaga 1440
gcagtttggt ggtaatccat ttgcttcctt ggtgagcaat acatcctctg gtgaaggtag 1500
tcaaccttcc cgtacagaaa atagagatcc actacccaat ccatgggctc cacagacttc 1560
ccagagttca tcagcttcca gcggcactgc cagcactgtg ggtggcacta ctggtagtac 1620
tgccagtggc acttctgggc agagtactac tgcgccaaat ttggtgcctg gagtaggagc 1680
tagtatgttc aacacaccag gaatgcagag cttgttgcaa caaataactg aaaacccaca 1740
actgatgcaa aacatgttgt ctgcccccta catgagaagc atgatgcagt cactaagcca 1800
gaatcctgac cttgctgcac agatgatgct gaataatccc ctatttgctg gaaatcctca 1860
gcttcaagaa caaatgagac aacagctccc aactttcctc caacaaatgc agaatcctga 1920
tacactatca gcaatgtcaa accctagagc aatgcaggcc ttgttacaga ttcagcaggg 1980
tttacagaca ttagcaacgg aagccccggg cctcatccca gggtttactc ctggcttggg 2040
ggcattagga agcactggag gctcttcggg aactaatgga tctaacgcca cacctagtga 2100
aaacacaagt cccacagcag gaaccactga acctggacat cagcagttta ttcagcagat 2160
gctgcaggct cttgctggag taaatcctca gctacagaat ccagaagtca gatttcagca 2220
acaactggaa caactcagtg caatgggatt tttgaaccgt gaagcaaact tgcaagctct 2280
aatagcaaca ggaggtgata tcaatgcagc tattgaaagg ttactgggct cccagccatc 2340

atagcagcat ttctgtatct tgaaaaaatg taatttattt ttgataacgg ctcttaaact 2400
ttaaaatacc tgctttattt cattttgact cttggaattc tgtgctgtta taaacaaacc 2460
caatatgatg cattttaagg tggagtacag taagatgtgt gggtttttct gtatttttct 2520
tttctggaac agtgggaatt aaggctactg catgcatcac ttctgcattt attgtaattt 2580
tttaaaaaca tcacctttta tagttgggtg accagatttt gtcctgcatc tgtccagttt 2640
atttgctttt taaacattag cctatggtag taatttatgt agaataaaag cattaaaaag 2700
aagcaaatca tttgcactct ataatttgtg gtacagtatt gcttattgtg actttggcat 2760
gcatttttgc aaacaatgct gtaagattta tactactgat aattttgttt tatttgtata 2820
caatatagag tatgcacatt tgggactgca tttctggaaa catactgcaa taggctctct 2880
gagcaaaaca cctgtaacta aaaaagtgaa gataagaaaa tactcttaaa gctgagtatt 2940
tcctaattgt atagaatctt acagcatctt tgacaaacat ctcccagcaa aagtgccggt 3000
tagtcaggtt tgttgaaaat acagtagaaa agctgattct ggttatctct ttaaggacaa 3060
ttaattgtac agacacataa tgtaacattg tctcaacatt cattcacaga ttgactgtaa 3120
attaccttaa tctttgtgca gactgaagga acactgtagt ataccccaaa gtgcatttgc 3180
ctaggacttc tcagcttctc ccataggtag tttaacaggc attaaaattt gtaattgaaa 3240
tgttgctttc actgaaaaag tgtcttgatg tttcagttat ttttaatcgc cataaaaaaa 3300
tagaactatc ttttgggttt atctgttttc tcatgcacag gcaatacaca aatttaaaat 3360
gagttgtgag ccaattgttt ctgaagtgtt ttggtagttc tattaagaaa tagttaaata 3420
ttgtgctttt cagagcctca gagaaagggg gacggggtgg gggggtgggg cagcggaatc 3480
tgtcctggat ggggccagct taaataatac tggcaaccaa gattctgtta ggatttctgt 3540
gcatatagtg tagtaaagaa gtatcattca ggggtgaaaa acaaagagcc gttttaatga 3600
tgttgagtac atttggctgt tttatagcct ttttcttccc tcccccaaag aattctgttt 3660
gcctaactcc caaactgttg ggtggtaca ttcctttagg accaattaaa acataattga 3720
gggtcagtga tacatttggc tgactctggt tcagtattct cttaggtgat tatattctct 3780
catgtacagt tacaggaaat taaaatgtta aagtaaccta aaatgaattc agaccaataa 3840
aatcaaggga aatacaagtt gattgcatta cttctgtatg ttgcttgcta ttaaaaaggt 3900
taagaggcca ggttacccac cagtccttgc actgttctga cactttcccc aggaggaaaa 3960
caagtacaaa ggttacggtg gaggcataag tagaagagat tgttaagaag ggtattcatg 4020
tgtctttgct ctttctgctt tatgcctcag tttggtttaa aaacttctgt actggcaaat 4080
ggtggtattc agtgtgggat agtgtcataa ctaatttgac aatttattaa tcataaaata 4140

acaataaatc tctagctttt acacttgaaa aaaaaaa 4177

<210> 14
<211> 4093
<212> DNA
<213> Homo sapiens

<400> 14

aatttcgcta accgtgacgt catctcggtg cgccgttaca gcgcttaact ggaaactgcg 60
atttagtttt tggtgaaagg agtggaaaaa aaccatgaac tgacctaccc cgccccattt 120
tgcttttagt ccttcacact ggcttcgcct gcactttggg caatggagcg ggaaaggaaa 180
aggcacgcag gctgagctca aagtcgcgcc agccggggat gactcggcga cgccgccagg 240
cgcgttaccc ggcgtgctcc gcgcggcgcc ggcgaaggga cgtgggggaa ggggcaggga 300
ggaggaagcg gtggctgctg cggatgtcgg tgtgagcgag cggcgcctga acacacggcg 360
gctgccgagc gcctgacccg ggcctgcgcc agagcctgca ccgagctccg gggccccaca 420
cccgctacgg tggccctgcg cccgttgcta ctgaggcggc gtgctctgca ttcttcgctg 480
tccaggcctg ccggctctgg tgtctgctgg ctcctccttg ctcgcctgct ccctcctgct 540
tgcctgagtc accgccgccg ccgccgccac agccatggcc gagagtggtg aaagcggcgg 600
tcctccgggc tcccaggata gcgccgccgg agccgaaggt gctggcgccc ccgcggccgc 660
tgcctccgcg gagcccaaaa tcatgaaagt caccgtgaag accccgaagg aaaaggagga 720
attcgccgtg cccgagaata gctccgtcca gcagtttaag gaagaaatct ctaaacgttt 780
taaatcacat actgaccaac ttgtgttgat atttgctgga aaaattttga aagatcaaga 840
taccttgagt cagcatggaa ttcatgatgg acttactgtt caccttgtca ttaaaacaca 900
aaacaggcct caggatcatt cagctcagca aacaaataca gctggaagca atgttactac 960
atcatcaact cctaatagta actctacatc tggttctgct actagcaacc cttttggttt 1020
aggtggcctt gggggacttg caggtctgag tagcttgggt ttgaatacta ccaacttctc 1080
tgaactacag agtcagatgc agcgacaact tttgtctaac cctgaaatga tggtccagat 1140
catggaaaat ccctttgttc agagcatgct ctcaaatcct gacctgatga gacagttaat 1200
tatggccaat ccacaaatgc agcagttgat acagagaaat ccagaaatta gtcatatgtt 1260
gaataatcca gatataatga gacaaacgtt ggaacttgcc aggaatccag caatgatgca 1320
ggagatgatg aggaaccagg accgagcttt gagcaaccta gaaagcatcc caggggggata 1380
taatgcttta aggcgcatgt acacagatat tcaggaacca atgctgagtg ctgcacaaga 1440
gcagtttggt ggtaatccat ttgcttcctt ggtgagcaat acatcctctg gtgaaggtag 1500

tcaaccttcc cgtacagaaa atagagatcc actacccaat ccatgggctc cacagacttc 1560
ccagagttca tcagcttcca gcggcactgc cagcactgtg ggtggcacta ctggtagtac 1620
tgccagtggc acttctgggc agagtactac tgcgccaaat ttggtgcctg gagtaggagc 1680
tagtatgttc aacacaccag gaatgcagag cttgttgcaa caaataactg aaaacccaca 1740
actgatgcaa aacatgttgt ctgcccccta catgagaagc atgatgcagt cactaagcca 1800
gaatcctgac cttgctgcac agatgcagaa tcctgataca ctatcagcaa tgtcaaaccc 1860
tagagcaatg caggccttgt tacagattca gcagggttta cagacattag caacggaagc 1920
cccgggcctc atcccagggt ttactcctgg cttgggggca ttaggaagca ctggaggctc 1980
ttcgggaact aatggatcta acgccacacc tagtgaaaac acaagtccca cagcaggaac 2040
cactgaacct ggacatcagc agtttattca gcagatgctg caggctcttg ctggagtaaa 2100
tcctcagcta cagaatccag aagtcagatt tcagcaacaa ctggaacaac tcagtgcaat 2160
gggattttg aaccgtgaag caaacttgca agctctaata gcaacaggag gtgatatcaa 2220
tgcagctatt gaaaggttac tgggctccca gccatcatag cagcatttct gtatcttgaa 2280
aaaatgtaat ttattttga taacggctct taaactttaa aatacctgct ttatttcatt 2340
ttgactcttg gaattctgtg ctgttataaa caaacccaat atgatgcatt ttaaggtgga 2400
gtacagtaag atgtgtgggt ttttctgtat ttttcttttc tggaacagtg ggaattaagg 2460
ctactgcatg catcacttct gcatttattg taattttta aaaacatcac cttttatagt 2520
tgggtgacca gattttgtcc tgcatctgtc cagtttattt gctttttaaa cattagccta 2580
tggtagtaat ttatgtagaa taaaagcatt aaaaagaagc aaatcatttg cactctataa 2640
tttgtggtac agtattgctt attgtgactt tggcatgcat ttttgcaaac aatgctgtaa 2700
gatttatact actgataatt ttgttttatt tgtatacaat atagagtatg cacatttggg 2760
actgcatttc tggaaacata ctgcaatagg ctctctgagc aaaacacctg taactaaaaa 2820
agtgaagata agaaaatact cttaaagctg agtatttcct aattgtatag aatcttacag 2880
catctttgac aaacatctcc cagcaaaagt gccggttagt caggtttgtt gaaaatacag 2940
tagaaaagct gattctggtt atctctttaa ggacaattaa ttgtacagac acataatgta 3000
acattgtctc aacattcatt cacagattga ctgtaaatta ccttaatctt tgtgcagact 3060
gaaggaacac tgtagtatac cccaaagtgc atttgcctag gacttctcag cttctcccat 3120
aggtagttta acaggcatta aaatttgtaa ttgaaatgtt gctttcactg aaaaagtgtc 3180
ttgatgtttc agttattttt aatcgccata aaaaaataga actatctttt gggtttatct 3240

gttttctcat gcacaggcaa tacacaaatt taaaatgagt tgtgagccaa ttgtttctga 3300
agtgttttgg tagttctatt aagaaatagt taaatattgt gcttttcaga gcctcagaga 3360
aagggggacg gggtgggggg gtggggcagc ggaatctgtc ctggatgggg ccagcttaaa 3420
taatactggc aaccaagatt ctgttaggat ttctgtgcat atagtgtagt aaagaagtat 3480
cattcagggg tgaaaaacaa agagccgttt taatgatgtt gagtacattt ggctgtttta 3540
tagccttttt cttccctccc ccaaagaatt ctgtttgcct aactcccaaa ctgttggggt 3600
ggtacattcc tttaggacca attaaaacat aattgagggt cagtgataca tttggctgac 3660
tctggttcag tattctctta ggtgattata ttctctcatg tacagttaca ggaaattaaa 3720
atgttaaagt aacctaaaat gaattcagac caataaaatc aagggaaata caagttgatt 3780
gcattacttc tgtatgttgc ttgctattaa aaaggttaag aggccaggtt acccaccagt 3840
ccttgcactg ttctgacact ttccccagga ggaaaacaag tacaaaggtt acggtggagg 3900
cataagtaga agagattgtt aagaagggta ttcatgtgtc tttgctcttt ctgctttatg 3960
cctcagtttg gtttaaaaac ttctgtactg gcaaatggtg gtattcagtg tgggatagtg 4020
tcataactaa tttgacaatt tattaatcat aaaataacaa taaatctcta gcttttacac 4080
ttgaaaaaaa aaa 4093

<210> 15
<211> 2005
<212> DNA
<213> Homo sapiens

<400> 15

```
gcggattccc gccccgaggt ttctaaatcc agacattccc gtttggctga gcactctagg      60
cctacatcca tgaagctagg agagcgacac tcaaagactg cactattgag agaagctaac     120
gttaaaggca gtgaatatat tcgggagtcc agctccggaa cccgggagct cttttagtgg     180
gaggggcggc gctgatggcg cttctggcct ccgaatgcta gggggcgctg tgatcgccgg     240
gcggcctctt gggcgctggg agtccaccgc gcaagcgcat cctggccttt cttcagtccc     300
cacgtgcgat ccttcccggc aactttttcg agaaaaatgc ccaaattcaa ggcggcccgt     360
ggggtggggg gtcaggaaaa acatgcgccc ctggccgatc agatcctggc tgggaatgcg     420
gtgcgggcgg gggtccggga gaagcggcgg ggtcgcggga caggagaagc ggaggaagag     480
tatgtggggc cccggctgag ccgacggatt ttgcagcaag cacggcagca acaggaggaa     540
ctcgaggccg agcatgggac tggggacaag cccgcggcgc cgcgggaacg caccacgcgg     600
ctgggtccaa gaatgcctca ggatggatca gatgacgagg acgaggagtg gcccaccctg     660
```

gagaaggctg ccacaatgac agcagcgggc catcatgcag aggtggttgt ggaccctgag 720
gatgagcgtg ccatagagat gttcatgaac aagaaccctc ctgccaggcg caccctggct 780
gacatcatca tggagaagct gactgagaag cagacagagg ttgagacagt catgtcagag 840
gtgtcgggct tccctatgcc ccagctggac ccccgggtcc tagaagtgta caggggggtc 900
cgggaggtat tatctaagta ccgcagtgga aaactgccca aggcatttaa gatcatccct 960
gcactctcca actgggagca aatcctctac gtcacagagc cggaggcctg gactgcagct 1020
gccatgtacc aggccaccag gatttttgcc tctaacctga aggaacgcat ggcccagcgc 1080
ttctacaacc ttgtcctgct ccctcgagta cgagatgacg ttgctgaata caaacgactc 1140
aacttccatc tctacatggc tctcaagaag gccctttttca aacctggagc ctggttcaaa 1200
gggatcctga ttccactgtg cgagtctggc acttgtaccc tccgggaagc catcattgtg 1260
ggtagcatca tcaccaagtg ctccatccct gtgttgcact ccagtgcggc catgctgaaa 1320
attgctgaga tggaatacag cggtgccaac agcatcttcc tgcgactgct gctggataag 1380
aagtatgcac tgccttaccg ggtgctggat gccctagtct tccacttcct ggggttccgg 1440
acagagaagc gtgaactgcc tgtgctgtgg caccagtgcc tcctgacttt ggtccagcgc 1500
tacaaggccg acttggccac agaccagaaa gaggccctct tagaactgct ccggctgcag 1560
ccccatccac agctatcgcc cgaaatcagg cgtgagcttc agagtgcagt cccccgcgat 1620
gtggaagatg ttcccatcac cgtggagtga ggaaaacagt cagctgtcct ggccaaaggg 1680
gtttggaagg acaccaagac ccccgttggt gactgaagat gacactgagc tttaatggct 1740
gaagacccag atcagggcag tgacagatca cagggacatc tgtggctccc agtccaggac 1800
aggaaggact gagggtctgg ctggttccct cttccattct aggcccttat ccctgtttag 1860
ttctgagagc caacttgaga taccatatgc tagcattccc agtccccagc tggggcttgg 1920
tgtgagtact ttttctatgg ctattgtgtc aggtcactgt ggataaaggc aaagacagat 1980
atttattgaa aaaaaaaaaa aaaaa 2005

<210> 16
<211> 990
<212> DNA
<213> Homo sapiens

<400> 16

agtctcgcgc gcgggcggag gtgggccggt gcggcgctgt cacgtaggtt cagtgggcgg 60
aagaggtggc cccggatgct gcggcgcccg ctggccgggc tggctgcggc cgccctgggc 120
cgggccccac cggacggcat gtcgggcccc aggcctgtgg tgctgagcgg gccttcggga 180

gctgggaaga gcaccctgct gaagaggctg ctccaggagc acagcggcat ctttggcttc 240
agcgtgtccc ataccacgag gaacccgagg cccggcgagg agaacggcaa agattactac 300
tttgtaacca gggaggtgat gcagcgtgac atagcagccg gcgacttcat cgagcatgcc 360
gagttctcgg ggaacctgta tggcacgagc aaggtggcgg tgcaggccgt gcaggccatg 420
aaccgcatct gtgtgctgga cgtggacctg cagggtgtgc ggaacatcaa ggccaccgat 480
ctgcggccca tctacatctc tgtgcagccg ccttcactgc acgtgctgga gcagcggctg 540
cggcagcgca acactgaaac cgaggagagc ctggtgaagc ggctggctgc tgcccaggcc 600
gacatggaga gcagcaagga gcccggcctg tttgatgtgg tcatcattaa cgacagcctg 660
gaccaggcct acgcagagct gaaggaggcg ctctctgagg aaatcaagaa agctcaaagg 720
accggcgcct gaggcttgct gtctgttctc ggcaccccgg gcccatacag gaccagggca 780
gcagcattga gccacccccct tggcaggcga tacggcagct ctgtgccctt ggccagcatg 840
tggagtggag gagatgctgc ccctgtggtt ggaacatcct ggggtgaccc ccgacccagc 900
ctcgctgggc tgtcccctgt ccctatctct cactctggac ccagggctga catcctaata 960
aaataactgt tggattagaa actccataaa 990

<210> 17
<211> 1120
<212> DNA
<213> Homo sapiens

<400> 17

```
agtctcgcgc gcgggcggag gtgggccggt gcggcgctgt cacgtaggtt cagtgggcgg      60
aagaggtggc cccggatgct gcggcgcccg ctggccgggc tggctgcggc cgccctgggc     120
cgggccccac cggacggctt gctctgctct ttacctgggg ttgctgtcga ggaccctgtg     180
caagactcgg ccggtttttc tttctccctg atggacagac ccaaacatag ccgcgcagca     240
tcgtgaaggg ctggggcctt cactcctctg tggctctgga agagcccgat ttcctcagga     300
ggcatgtcgg gccccaggcc tgtggtgctg agcgggcctt cgggagctgg gaagagcacc     360
ctgctgaaga ggctgctcca ggagcacagc ggcatctttg gcttcagcgt gtcccatacc     420
acgaggaacc cgaggcccgg cgaggagaac ggcaaagatt actactttgt aaccagggag     480
gtgatgcagc gtgacatagc agccggcgac ttcatcgagc atgccgagtt ctcggggaac     540
ctgtatggca cgagcaaggt ggcggtgcag gccgtgcagg ccatgaaccg catctgtgtg     600
ctggacgtgg acctgcaggg tgtgcggaac atcaaggcca ccgatctgcg gcccatctac     660
atctctgtgc agccgccttc actgcacgtg ctggagcagc ggctgcggca gcgcaacact     720

gaaaccgagg agagcctggt gaagcggctg gctgctgccc aggccgacat ggagagcagc     780
aaggagcccg gcctgtttga tgtggtcatc attaacgaca gcctggacca ggcctacgca     840
gagctgaagg aggcgctctc tgaggaaatc aagaaagctc aaaggaccgg cgcctgaggc     900
ttgctgtctg ttctcggcac cccgggccca tacaggacca gggcagcagc attgagccac     960
ccccttggca ggcgatacgg cagctctgtg cccttggcca gcatgtggag tggaggagat    1020
gctgcccctg tggttggaac atcctggggt gacccccgac ccagcctcgc tgggctgtcc    1080
cctgtcccta tctctcactc tggacccagg gctgacatcc                          1120
```

<210> 18
<211> 904
<212> DNA
<213> Homo sapiens

<400> 18

cagacagacc caccccaggc tgactacaga ggctgcacct cagcaaacag gcatgtcggg 60

ccccaggcct gtggtgctga gcgggccttc gggagctggg aagagcaccc tgctgaagag 120

gctgctccag gagcacagcg gcatctttgg cttcagcgtg tcccatacca cgaggaaccc 180

gaggcccggc gaggagaacg gcaaagatta ctactttgta accagggagg tgatgcagcg 240

tgacatagca gccggcgact tcatcgagca tgccgagttc tcggggaacc tgtatggcac 300

gagcaaggtg gcggtgcagg ccgtgcaggc catgaaccgc atctgtgtgc tggacgtgga 360

cctgcagggt gtgcggaaca tcaaggccac cgatctgcgg cccatctaca tctctgtgca 420

gccgccttca ctgcacgtgc tggagcagcg gctgcggcag cgcaacactg aaaccgagga 480

gagcctggtg aagcggctgg ctgctgccca ggccgacatg gagagcagca aggagcccgg 540

cctgtttgat gtggtcatca ttaacgacag cctggaccag gcctacgcag agctgaagga 600

ggcgctctct gaggaaatca agaaagctca aaggaccggc gcctgaggct tgctgtctgt 660

tctcggcacc ccgggcccat acaggaccag ggcagcagca ttgagccacc cccttggcag 720

gcgatacggc agctctgtgc ccttggccag catgtggagt ggaggagatg ctgcccctgt 780

ggttggaaca tcctggggtg acccccgacc cagcctcgct gggctgtccc ctgtccctat 840

ctctcactct ggacccaggg ctgacatcct aataaaataa ctgttggatt agaaactcca 900

taaa 904

<210> 19
<211> 6255
<212> DNA
<213> Homo sapiens

<400> 19

```
actcgcctgg agcgcgcggg caaggcgggc ggagcgcact ggaactcaag ggggcgcaca     60
gcggcgcgct cgcaccgctc ggctccgcgc ggctctagga ggtggcggcg gtggcggtgg    120
cggcggtggc ggcggcggcg gcggcggggc gcagggctga gcgagcgtcc gggttccggg    180
gctccgggga aggcggttgc agctcctgag tgcagcgcgg cttcctgcca ctgtcccggc    240
ccggccacct ctctgtcatg gctctggcgg acagcacacg tggattaccc aacgggggcg    300
gcggcggggg cggcagtggc tcctcgtcgt cctccgcgga gccaccgctc ttccccgaca    360
tcgtggagct gaacgtgggg ggccaggtgt acgtgacccg gcgctgcacg gtggtgtcgg    420
tgcccgactc gctgctctgg cgcatgttca cgcagcagca gccgcaggag ctggcccggg    480
acagcaaagg ccgcttcttt ctggaccggg acggcttcct cttccgctac atcctggatt    540
acctgcggga cttgcagctc gtgctgcccg actacttccc cgagcgcagc cggctgcagc    600
gcgaggccga gtacttcgag ctgccagagc tcgtgcgccg cctcggggcg ccccagcagc    660
ccggcccggg gccgccgccc tcgcggcgcg gggtgcacaa ggagggctcg ctgggtgacg    720
agctgctgcc gcttggctac tcggagcccg aacagcagga gggcgcctct gccggggcgc    780
cgtcgcccac gctggagctg gctagccgca gtccgtccgg gggcgcggcg ggcccgctgc    840
tcacgccgtc ccagtcgctg gacggcagcc ggcgctcggg ctacatcacc atcggctacc    900
gcggctccta caccatcggg cgggacgcgc aggcggacgc caagttccgg cgagtggcgc    960
gcatcaccgt ttgcggaaag acgtcgctgg ccaaggaggt gtttggggac accctgaacg   1020
aaagccggga ccccgaccgt cccccggagc gctacacctc gcgctattac ctcaagttca   1080
acttcctgga gcaggccttc gacaagctgt ccgagtcggg cttccacatg gtggcgtgca   1140
gctccacggg cacctgcgcc tttgccagca gcaccgacca gagcgaggac aagatctgga   1200
ccagctacac cgagtacgtc ttctgcaggg agtgagctcc ccagaccccc tcgccactcc   1260
agcgcccagt ccttctcctg cccgagagat gattacagag cctcttgtcc cacctttgtc   1320
ccctggctgc tgccctccca ttctccccct ccagtagtag ctgggtgaga cctgtccgcc   1380
caccttccct ccactacaga acctgcagcc gcaaatcctc tgggctgctt cgtcttcttt   1440
ggacctcctg aaccgagaga acccagagga acccccaccc cacccccacc taccactcca   1500
tgctttctct actccctgcc tcaaaccacc cctcccccag atggtacttc agtttggatc   1560
tattggggga gtgtggccac agaccggggg atgattgaat tgttcagaac ctgattggac   1620
cgtgtccaat gtgcggaaga tttccttgaa atcttctcaa gctcttatga ctcactgggg   1680
gtttaagaga tcaggattgg ttccactgtc tggggttagt gttttacaag gtcattacac   1740
```

agtctttttg acctcttttg aaggtagagt tttagaaggc tggatggaag attctgagcc 1800
tggaattagg accccatgga ggcagttcag taactaaact aataaagttt tgaaaagtta 1860
cacgtaaagt agaagaatct agtgcgtggg acagtaaagg atcctttctc gtacagaata 1920
aaaggtctca gcctgtagct taaacttata gaaagtgatc cgcctgcctg cagaggcgcc 1980
cttttcagct gctgctcgcc agaagccctt gattccactg gttgacatgg cagcagttac 2040
tggcaagagg gagaaaggac gctgccgcct aagagtgcaa ggctgctcag gtctccaagc 2100
gccgtaggag gtcacctggc agtgactgta gggagctggg tcatagtgca cgtcgtgggt 2160
attaggaaag cctgtattct ttcaatgaat gtcagtagga ccttccttta gctgtaagac 2220
ttggtgggcg gggtggggtg gggagggagg aaagggtagg aagggtggga agggagaagc 2280
agacatagtc atttatgatt tgaaagttgg aagtttgtac catctgtttg agtatatgca 2340
catttaaaaa atatcatata gtaaatgcaa acatgccaag tattttataa agattaataa 2400
cagacctact cttacctggc agtttactta acttactgtt ttgagtccta aacttagagt 2460
tgttaatgct tatatataat ctaaccaaag agttacccag tagggtttta gtttttgaac 2520
ttttattttc ttgttgatta taaatcctga ttttggaatc tattgcgcaa aagaagtttc 2580
attttggtta cttagaccta agatcactta ttaaaaatcc ttattttctc caagcccagc 2640
aaacgttgac ttctgggcaa acctgaaaac ctgaaaatgc cactttcatg cagtttgttt 2700
gaagttaagt ggaatccttt caaatgacga gctgcagaga actcagcacc aagggctgcc 2760
tatctgtaga tagctgtaaa atggaatatt tttaaatgaa ggcaaataag tacttaaaag 2820
tgagctgagc aataaaatgg tccaataata ggtaaatgca acagaaacag aaggagacct 2880
ggttgcctta tgcctttact cttacatgga ataaattccc aatgcatatc ctatgtaaac 2940
cataagtgaa gggaaataaa cctcgtcatg ctccatgctg tgaggtgtcc tttggatatt 3000
ctgtgatgac agagaagcct attttgtttt gttttcagca tctttctctg atgtacgttt 3060
ttaaggattt tgtaagagct gttttcagtg tttaaattag tgctattttt ccttgttttt 3120
aaaaatgaat ctcgtactgt atcttactat gtccatacag atgttacaaa tcgacagttt 3180
tattcttaga ctcatgtgat ccaagctgta tataccatat ataaacattt tacatgaatc 3240
atttagtttt ttaattcatt tactaatgct ataaaatttc ctatattacc ccagtaattt 3300
gcatcagctg gtttatatac taaagcaaca tgttttgatg agtttcttac atccttatcg 3360
aggaattggg ttaggaaaaa atacataatt gtaaaactga gtttgctgta ttatactttt 3420
tttcttgagt attagttgta ttactaatca tatgttgatt aactgtctac ttaaagtcaa 3480
ggtacctgta tttttaatcc actaattttt ttttagttgg gaaatagatt tcaggtcttt 3540

```
tattagacta acatttttg agaagtaaaa ttgacttcat atacaaagcc tgtaatttta   3600
ggcgaaatgg aagcagaaat ctaggaagtt gtgcttgctt gtatgttgag tttggtctca   3660
gactaagtaa tgcatcagaa ttcatctgtt tgaagcctga aataatttag gactctgatt   3720
cactgaccaa aagtcagtgt tgcagagatt tctctacccc gtatggtatt ttgttagatt   3780
gttcaacagg aagcacatga ttgagaacat cttgggacag accaaaacca ctgacagatg   3840
gcaaggctcg gcgattctga tttcccttct caaatctgct caactccaag agtcttgaga   3900
aactgctaaa attttgcctc tgtcactcaa gtcttacaaa tgttatcttg taaacctttg   3960
aggtgaacta ttccactgtc ttgtacatag gcatcttatt cactgcaccc tgtcacaccc   4020
agcacccccc gccccgcaca ttatttgaaa gactgggaat ttaatggtta gggacagtaa   4080
atctacttct tttccaggg acgactgtcc cctctaaagt taaagtcaat acaagaaaac   4140
tgtctatttt tagcctaaag taaaggctgt gaagaaaatt cattttacat tgggtagaca   4200
gtaaaaaaca agtaaaataa cttgacatga gcacctttag atcccttccc ctccatgggc   4260
tttgggccac agaatgaacc tttgaggcct gtaaagtgga ttgtaatttc ctataagctg   4320
taatagtgga ggtattgtgg gttcatttga gtaagccctc caaagatacc attcaaataa   4380
cctgggagaa tgtcataaat tattcagata attaacactg catgaatctg attcagaggc   4440
atgcatttac atatgttgcc ctaattacca tttgatgatc ataaatacaa gtgaatgaca   4500
ttggactttt agtaacaaac ttaattttta aaaaggtgta gacaatggtg gttaaaaaaa   4560
aaaaaaaaac aggtaccagg ttctgtgtgt ttgcaccaag taattgacat gtttttttaat   4620
acatgtggac catgaacagt attcattcta ctttttcaaa tgatatgctg tagaaaatat   4680
tccttgaaga tgtgagattt aaaaattttt ccctttcaat gttgttatgt attctactgg   4740
tttttgattg atagcacagt gataaatcat aatactagac aaaattgtct tctctttcaa   4800
accagagcca tatatatgtc tgtatatatg ggacctactg cttctctgag gaaatgcata   4860
atctgttaat atcagacaaa atgagcaatt ggcagtgctc ataatatatt ccaattttta   4920
ttggaatttt cgatggaatg ttatttcaat aaagccatgt aaggtgaaac tttgataact   4980
ttttactctt caagttaggg taaattctga tccaatattc aattcatttg tgtactccca   5040
catgcaaaat gctaaattac aatgcagaca ttaagaaaaa gtattgactg gaggggttga   5100
attccttgag aatttatttt atagtctaaa tcacaaatac tttactcaat ttagttttta   5160
aaatagtaaa ctgaatattt ttgttgtaag cctatcagag tcaatccttc gtttggaatt   5220
gttttcctgt tttccttac tataaatcat ttaaaaactg aattcatttt cttagatggc   5280
```

ataagtctgt ctcttgagaa ataagtaaaa tactcctatt ttcagtatct gtagcacctg 5340
aaataggtct ttgtatagcc agaaacaagt tatgttgaag ttagcttttc tttgtcaaca 5400
gttttggaca ataaaaatct gaaagtatta acacttgatt ttctactggg gcccttcaaa 5460
cttggttgga agaaattcaa ccagaatatc tacattagag tataatcatg tgtggtagga 5520
agatggacta gttaatcaag atttgttgtc acttaaattt tttgtgattt ttttccaagc 5580
cagttttttt aaattctaaa tgtgttttga ggtatgggta cattaattgt aatgtaaact 5640
attatacaac tgtttttgcg actttatagg caggtaaatt ttgctattac tattgaatac 5700
aaatgacaat tcatttatga ccactcaaac agcgttagta accatttagt gacaaaggat 5760
taaaacatcc atctggatgt taattttgaa gatgtaaatt atatgttgtt taaatttttc 5820
caggcatctg aaaaccttat ctgctagaca atgtaagatt cacacagagt tatctgggat 5880
tctgattttt taaatagtac atatcattaa accattttct ctaaatgtaa gaagagcaga 5940
aaaaatctta taagattatc agatttttct aatgacacag aaatgtaaga aaaaaatccc 6000
tttatattga aaaaagatgc agtcaaagtc ttttcagaca tgcccaaact ttgagaattt 6060
cttcaaccat ctaatgctat aaagattttt gttcttcctg ttcacaacca gttgtataac 6120
agaaatacta gctactgttt tccttcctgt gtgtgaagta atgaatcatt gattatgtga 6180
cttgttatgt attcaattaa acactaaaga ataaaacatt cactccttta attattcaaa 6240
aaaaaaaaaa aaaaa 6255

<210> 20
<211> 2020
<212> DNA
<213> Homo sapiens

<400> 20

gcccgcgcgt ggacggttgg gattagcggc cgcgggggcc ggtgggggtc gtggggcctc 60
ccccacccga gtacaggcga gcgagcggca tccgggcgga cccccgcctc ggggaccggg 120
aaactgaggc tcgggcggcc tgagacgccg tcaaggtcat gtaggtggtg ggggcttgca 180
tcttcgcctc ccgactccat ccagttcgtg tttgctgtga agacggtgtc cctggccagt 240
gtccttccac ctgtccacaa gcatggggaa catcttcgcc aacctcttca agggcctttt 300
tggcaaaaaa gaaatgcgca tcctcatggt gggcctggat gctgcaggga agaccacgat 360
cctctacaag cttaagctgg gtgagatcgt gaccaccatt cccaccatag gcttcaacgt 420
ggaaaccgtg gagtacaaga acatcagctt cactgtgtgg gacgtgggtg gccaggacaa 480
gatccggccc ctgtggcgcc actacttcca gaacacacaa ggcctgatct tcgtggtgga 540

```
cagcaatgac agagagcgtg tgaacgaggc ccgtgaggag ctcatgagga tgctggccga     600
ggacgagctc cgggatgctg tcctcctggt gttcgccaac aagcaggacc tccccaacgc     660
catgaatgcg gccgagatca cagacaagct ggggctgcac tcactacgcc acaggaactg     720
gtacattcag gccacctgcg ccaccagcgg cgacgggctc tatgaaggac tggactggct     780
gtccaatcag ctccggaacc agaagtgaac gcgacccccc tccctctcac tcctcttgcc     840
ctctgcttta ctctcatgtg gcaaacgtgc ggctcgtggt gtgagtgcca gaagctgcct     900
ccgtggtttg gtcaccgtgt gcatcgcacc gtgctgtaaa tgtggcagac gcagcctgcg     960
gccaggcttt ttatttaatg taaatagttt ttgtttccaa tgaggcagtt tctggtactc    1020
ctatgcaata ttactcagct ttttttattg taaaaagaaa aatcaactca ctgttcagtg    1080
ctgagagggg atgtaggccc atgggcacct ggcctccagg agtcgctgtg ttgggagagc    1140
cggccacgcc cttggcttta gagctgtgtt gaaatccatt ttggtggttg gtttttaacc    1200
caaactcagt gcattttttta aaatagttaa gaatccaagt cgagaacact tgaacacaca    1260
gaagggagac cccgcctagc atagatttgc agttacggcc tggatgccag tcgccagccc    1320
agctgttccc ctcgggaaca tgaggtggtg gtggcgcagc agactgcgat caattctgca    1380
tggtcacagt agagatcccc gcaactcgct tgtccttggg tcaccctgca ttccatagcc    1440
atgtgcttgt ccctgtgctc ccacggttcc caggggccag gctgggagcc cacagccacc    1500
ccactatgcc gcaggccgcc ctacccacct tcaggcagcc tatgggacgc agggccccat    1560
ctgtccctcg gtcgccgtgt ggccagagtg ggtccgtcgt ccccaacact cgtgctcgct    1620
cagacacttt ggcaggatgt ctggggcctc accagcagga gcgcgtgcaa gccgggcagg    1680
cggtccacct agacccacag cccctcggga gcaccccacc tctgtgtgtg atgtagcttt    1740
ctctccctca gcctgcaagg gtccgatttg ccatcgaaaa agacaacctc tactttttc    1800
ttttgtattt tgataaacac tgaagctgga gctgttaaat ttatcttggg gaaacctcag    1860
aactggtcta tttggtgtcg tggaacctct tactgctttc aatacacgat tagtaatcaa    1920
ctgttttgta tacttgtttt cagttttcat ttcgacaaac aagcactgta attatagcta    1980
ttagaataaa atctcttaac tatttcaaaa aaaaaaaaaa                          2020
```

<210> 21
<211> 1998
<212> DNA
<213> Homo sapiens

<400> 21

gcccgcgcgt ggacggttgg gattagcggc cgcgggggcc ggtgggggtc gtggggcctc 60

```
ccccacccga gtacaggcga gcgagcggca tccgggcgga cccccgcctc ggggaccggg    120
aaactgaggc tcgggcggcc tgagacgccg tcaaggtcat gtaggtggtg gggcttgca     180
tcttcgcctc ccgactccat ccagtgtccc tggccagtgt ccttccacct gtccacaagc    240
atggggaaca tcttcgccaa cctcttcaag ggcctttttg gcaaaaaaga aatgcgcatc    300
ctcatggtgg gcctggatgc tgcagggaag accacgatcc tctacaagct taagctgggt    360
gagatcgtga ccaccattcc caccataggc ttcaacgtgg aaaccgtgga gtacaagaac    420
atcagcttca ctgtgtggga cgtgggtggc caggacaaga tccggcccct gtggcgccac    480
tacttccaga acacacaagg cctgatcttc gtggtggaca gcaatgacag agagcgtgtg    540
aacgaggccc gtgaggagct catgaggatg ctggccgagg acgagctccg ggatgctgtc    600
ctcctggtgt tcgccaacaa gcaggacctc cccaacgcca tgaatgcggc cgagatcaca    660
gacaagctgg ggctgcactc actacgccac aggaactggt acattcaggc cacctgcgcc    720
accagcggcg acgggctcta tgaaggactg gactggctgt ccaatcagct ccggaaccag    780
aagtgaacgc gaccccctc cctctcactc ctcttgccct ctgctttact ctcatgtggc     840
aaacgtgcgg ctcgtggtgt gagtgccaga agctgcctcc gtggtttggt caccgtgtgc    900
atcgcaccgt gctgtaaatg tggcagacgc agcctgcggc caggcttttt atttaatgta    960
aatagttttt gtttccaatg aggcagtttc tggtactcct atgcaatatt actcagcttt   1020
ttttattgta aaaagaaaaa tcaactcact gttcagtgct gagaggggat gtaggcccat   1080
gggcacctgg cctccaggag tcgctgtgtt gggagagccg gccacgccct tggctttaga   1140
gctgtgttga aatccatttt ggtggttggt ttttaaccca aactcagtgc attttttaaa   1200
atagttaaga atccaagtcg agaacacttg aacacacaga agggagaccc cgcctagcat   1260
agatttgcag ttacggcctg gatgccagtc gccagcccag ctgttcccct cgggaacatg   1320
aggtggtggt ggcgcagcag actgcgatca attctgcatg gtcacagtag agatccccgc   1380
aactcgcttg tccttgggtc accctgcatt ccatagccat gtgcttgtcc ctgtgctccc   1440
acggttccca ggggccaggc tgggagccca cagccacccc actatgccgc aggccgccct   1500
acccaccttc aggcagccta tgggacgcag ggccccatct gtccctcggt cgccgtgtgg   1560
ccagagtggg tccgtcgtcc ccaacactcg tgctcgctca gacactttgg caggatgtct   1620
ggggcctcac cagcaggagc gcgtgcaagc cgggcaggcg gtccacctag acccacagcc   1680
cctcgggagc accccacctc tgtgtgtgat gtagctttct ctccctcagc ctgcaagggt   1740
ccgatttgcc atcgaaaaag acaacctcta ctttttctt ttgtattttg ataaacactg    1800
aagctggagc tgttaaattt atcttgggga aacctcagaa ctggtctatt tggtgtcgtg   1860
```

```
gaacctctta ctgctttcaa tacacgatta gtaatcaact gttttgtata cttgttttca   1920
gttttcattt cgacaaacaa gcactgtaat tatagctatt agaataaaat ctcttaacta   1980
tttcaaaaaa aaaaaaaa                                                  1998
```

<210> 22
<211> 1986
<212> DNA
<213> Homo sapiens

<400> 22

```
ccttacccgg cgtgccccgc gcccggaggc gctgacgtgg ccgccgtcag agccgccatc     60
ttgtgggagc aaaaccaacg cctggctcgg agcagcagcc tctgaggtga gggcgagggg    120
cgcgggccgg tgtgggccgc agagacgttg gagccggcgg gggctgggga ctggcctcgg    180
ggcgacttga ggtgtccctg gccagtgtcc ttccacctgt ccacaagcat ggggaacatc    240
ttcgccaacc tcttcaaggg cctttttggc aaaaaagaaa tgcgcatcct catggtgggc    300
ctggatgctg cagggaagac cacgatcctc tacaagctta agctgggtga gatcgtgacc    360
accattccca ccataggctt caacgtggaa accgtggagt acaagaacat cagcttcact    420
gtgtgggacg tgggtggcca ggacaagatc cggcccctgt ggcgccacta cttccagaac    480
acacaaggcc tgatcttcgt ggtggacagc aatgacagag agcgtgtgaa cgaggcccgt    540
gaggagctca tgaggatgct ggccgaggac gagctccggg atgctgtcct cctggtgttc    600
gccaacaagc aggacctccc caacgccatg aatgcggccg agatcacaga caagctgggg    660
ctgcactcac tacgccacag gaactggtac attcaggcca cctgcgccac cagcggcgac    720
gggctctatg aaggactgga ctggctgtcc aatcagctcc ggaaccagaa gtgaacgcga    780
ccccccctccc tctcactcct cttgccctct gctttactct catgtggcaa acgtgcggct    840
cgtggtgtga gtgccagaag ctgcctccgt ggtttggtca ccgtgtgcat cgcaccgtgc    900
tgtaaatgtg gcagacgcag cctgcggcca ggctttttat ttaatgtaaa tagtttttgt    960
ttccaatgag gcagtttctg gtactcctat gcaatattac tcagcttttt ttattgtaaa   1020
aagaaaaatc aactcactgt tcagtgctga gaggggatgt aggcccatgg gcacctggcc   1080
tccaggagtc gctgtgttgg gagagccggc cacgcccttg gctttagagc tgtgttgaaa   1140
tccattttgg tggttggttt ttaacccaaa ctcagtgcat tttttaaaat agttaagaat   1200
ccaagtcgag aacacttgaa cacacagaag ggagaccccg cctagcatag atttgcagtt   1260
acggcctgga tgccagtcgc cagcccagct gttcccctcg ggaacatgag gtggtggtgg   1320
cgcagcagac tgcgatcaat tctgcatggt cacagtagag atccccgcaa ctcgcttgtc   1380
```

```
cttgggtcac cctgcattcc atagccatgt gcttgtccct gtgctcccac ggttcccagg   1440
ggccaggctg ggagcccaca gccaccccac tatgccgcag gccgccctac ccaccttcag   1500
gcagcctatg ggacgcaggg ccccatctgt ccctcggtcg ccgtgtggcc agagtgggtc   1560
cgtcgtcccc aacactcgtg ctcgctcaga cactttggca ggatgtctgg ggcctcacca   1620
gcaggagcgc gtgcaagccg ggcaggcggt ccacctagac ccacagcccc tcgggagcac   1680
cccacctctg tgtgtgatgt agctttctct ccctcagcct gcaagggtcc gatttgccat   1740
cgaaaaagac aacctctact tttttctttt gtattttgat aaacactgaa gctggagctg   1800
ttaaatttat cttggggaaa cctcagaact ggtctatttg gtgtcgtgga acctcttact   1860
gctttcaata cacgattagt aatcaactgt tttgtatact tgttttcagt tttcatttcg   1920
acaaacaagc actgtaatta tagctattag aataaaatct cttaactatt tcaaaaaaaa   1980
aaaaaa                                                              1986
```

<210> 23
<211> 1901
<212> DNA
<213> Homo sapiens

<400> 23

```
ccttacccgg cgtgccccgc gcccggaggc gctgacgtgg ccgccgtcag agccgccatc      60
ttgtgggagc aaaaccaacg cctggctcgg agcagcagcc tctgaggtgt ccctggccag     120
tgtccttcca cctgtccaca agcatgggga acatcttcgc caacctcttc aagggccttt     180
ttggcaaaaa agaaatgcgc atcctcatgg tgggcctgga tgctgcaggg aagaccacga     240
tcctctacaa gcttaagctg ggtgagatcg tgaccaccat tcccaccata ggcttcaacg     300
tggaaaccgt ggagtacaag aacatcagct tcactgtgtg ggacgtgggt ggccaggaca     360
agatccggcc cctgtggcgc cactacttcc agaacacaca aggcctgatc ttcgtggtgg     420
acagcaatga cagagagcgt gtgaacgagg cccgtgagga gctcatgagg atgctggccg     480
aggacgagct ccgggatgct gtcctcctgg tgttcgccaa caagcaggac ctccccaacg     540
ccatgaatgc ggccgagatc acagacaagc tggggctgca ctcactacgc cacaggaact     600
ggtacattca ggccacctgc gccaccagcg gcgacgggct ctatgaagga ctggactggc     660
tgtccaatca gctccggaac cagaagtgaa cgcgaccccc ctccctctca ctcctcttgc     720
cctctgcttt actctcatgt ggcaaacgtg cggctcgtgg tgtgagtgcc agaagctgcc     780
tccgtggttt ggtcaccgtg tgcatcgcac cgtgctgtaa atgtggcaga cgcagcctgc     840
ggccaggctt tttatttaat gtaaatagtt tttgtttcca atgaggcagt ttctggtact     900
```

```
cctatgcaat attactcagc ttttttatt gtaaaaagaa aaatcaactc actgttcagt     960
gctgagaggg gatgtaggcc catgggcacc tggcctccag gagtcgctgt gttgggagag   1020
ccggccacgc ccttggcttt agagctgtgt tgaaatccat tttggtggtt ggtttttaac   1080
ccaaactcag tgcatttttt aaaatagtta agaatccaag tcgagaacac ttgaacacac   1140
agaagggaga ccccgcctag catagatttg cagttacggc ctggatgcca gtcgccagcc   1200
cagctgttcc cctcgggaac atgaggtggt ggtggcgcag cagactgcga tcaattctgc   1260
atggtcacag tagagatccc cgcaactcgc ttgtccttgg gtcaccctgc attccatagc   1320
catgtgcttg tccctgtgct cccacggttc ccaggggcca ggctgggagc ccacagccac   1380
cccactatgc cgcaggccgc cctacccacc ttcaggcagc ctatgggacg cagggcccca   1440
tctgtccctc ggtcgccgtg tggccagagt gggtccgtcg tccccaacac tcgtgctcgc   1500
tcagacactt tggcaggatg tctggggcct caccagcagg agcgcgtgca agccgggcag   1560
gcggtccacc tagacccaca gcccctcggg agcaccccac ctctgtgtgt gatgtagctt   1620
tctctccctc agcctgcaag ggtccgattt gccatcgaaa aagacaacct ctactttttt   1680
cttttgtatt ttgataaaca ctgaagctgg agctgttaaa tttatcttgg ggaaacctca   1740
gaactggtct atttggtgtc gtggaacctc ttactgcttt caatacacga ttagtaatca   1800
actgttttgt atacttgttt tcagttttca tttcgacaaa caagcactgt aattatagct   1860
attagaataa aatctcttaa ctatttcaaa aaaaaaaaaa a                       1901
```

<210> 24
<211> 3509
<212> DNA
<213> Homo sapiens

<400> 24

```
ggcgccctga aacgttcggc gagccgactg cggctgcgcg gggtattcga atcggcggcg      60
gcttctagtt tgcggttcag gtttggccgc tgccggccag cgtcctctgg ccatggacac     120
cccggaaaat gtccttcaga tgcttgaagc ccacatgcag agctacaagg gcaatgaccc     180
tcttggtgaa tgggaaagat acatacagtg ggtagaagag aattttcctg agaataaaga     240
atacttgata actttactag aacatttaat gaaggaattt ttagataaga agaaatacca     300
caatgaccca agattcatca gttattgttt aaaatttgct gagtacaaca gtgacctcca     360
tcaatttttt gagtttctgt acaaccatgg gattggaacc ctgtcatccc ctctgtacat     420
tgcctgggcg gggcatctgg aagcccaagg agagctgcag catgccagtg ctgtccttca     480
gagaggaatt caaaaccagg ctgaacccag agagttcctg caacaacaat acaggttatt     540
```

tcagacacgc ctcactgaaa cccatttgcc agctcaagct agaacctcag aacctctgca 600
taatgttcag gttttaaatc aaatgataac atcaaaatca aatccaggaa ataacatggc 660
ctgcatttct aagaatcagg gttcagagct ttctggagtg atatcttcag cttgtgataa 720
agagtcaaat atggaacgaa gagtgatcac gatttctaaa tcagaatatt ctgtgcactc 780
atctttggca tccaaagttg atgttgagca ggttgttatg tattgcaagg agaagcttat 840
tcgtggggaa tcagaatttt cctttgaaga attgagagcc cagaaataca atcaacggag 900
aaagcatgag caatgggtaa atgaagacag acattatatg aaaaggaaag aagcaaatgc 960
ttttgaagaa cagctattaa aacagaaaat ggatgaactt cataagaagt tgcatcaggt 1020
ggtggagaca tcccatgagg atctgcccgc ttcccaggaa aggtccgagg ttaatccagc 1080
acgtatgggg ccaagtgtag gctcccagca ggaactgaga gcgccatgtc ttccagtaac 1140
ctatcagcag acaccagtga acatggaaaa gaacccaaga gaggcacctc ctgttgttcc 1200
tcctttggca aatgctattt ctgcagcttt ggtgtcccca gccaccagcc agagcattgc 1260
tcctcctgtt cctttgaaag cccagacagt aacagactcc atgtttgcag tggccagcaa 1320
agatgctgga tgtgtgaata agagtactca tgaattcaag ccacagagtg gagcagagat 1380
caaagaaggg tgtgaaacac ataaggttgc caacacaagt tcttttcaca caactccaaa 1440
cacatcactg ggaatggttc aggcaacgcc atccaaagtg cagccatcac ccaccgtgca 1500
cacaaaagaa gcattaggtt tcatcatgaa tatgtttcag gctcctacac ttcctgatat 1560
ttctgatgac aaagatgaat ggcaatctct agatcaaaat gaagatgcat ttgaagccca 1620
gtttcaaaaa aatgtaaggt catctggggc ttggggagtc aataagatca tctcttcttt 1680
gtcatctgct tttcatgtgt ttgaagatgg aaacaaagaa aattatggat taccacagcc 1740
taaaaataaa cccacaggag ccaggacctt tggagaacgc tctgtcagca gacttccttc 1800
aaaaccaaag gaggaagtgc ctcatgctga agagtttttg gatgactcaa ctgtatgggg 1860
tattcgctgc aacaaaaccc tggcacccag tcctaagagc ccaggagact tcacatctgc 1920
tgcacaactt gcgtctacac cattccacaa gcttccagtg gagtcagtgc acattttaga 1980
agataaagaa aatgtggtag caaaacagtg tacccaggcg actttggatt cttgtgagga 2040
aaacatggtg gtgccttcaa gggatggaaa attcagtcca attcaagaga aaagcccaaa 2100
acaggccttg tcgtctcaca tgtattcagc atccttactt cgtctgagcc agcctgctgc 2160
aggtggggta cttacctgtg aggcagagtt gggcgttgag gcttgcagac tcacagacac 2220
tgacgctgcc attgcagaag atccaccaga tgctattgct gggctccaag cagaatggat 2280

gcagatgagt tcacttggga ctgttgatgc tccaaacttc attgttggga acccatggga 2340
tgataagctg attttcaaac ttttatctgg gctttctaaa ccagtgagtt cctatccaaa 2400
tacttttgaa tggcaatgta aacttccagc catcaagccc aagactgaat ttcaattggg 2460
ttctaagctg gtctatgtcc atcaccttct tggagaagga gcctttgccc aggtgtacga 2520
agctacccag ggagatctga atgatgctaa aaataaacag aaatttgttt taaaggtcca 2580
aaagcctgcc aacccctggg aattctacat tgggacccag ttgatggaaa gactaaagcc 2640
atctatgcag cacatgttta tgaagttcta ttctgcccac ttattccaga atggcagtgt 2700
attagtagga gagctctaca gctatggaac attattaaat gccattaacc tctataaaaa 2760
tacccctgaa aaagtgatgc ctcaaggtct tgtcatctct tttgctatga gaatgcttta 2820
catgattgag caagtgcatg actgtgaaat cattcatgga gacattaaac cagacaattt 2880
catacttgga aacggatttt tggaacagga tgatgaagat gatttatctg ctggcttggc 2940
actgattgac ctgggtcaga gtatagatat gaaacttttt ccaaaaggaa ctatattcac 3000
agcaaagtgt gaaacatctg gttttcagtg tgttgagatg ctcagcaaca aaccatggaa 3060
ctaccagatc gattactttg ggttgctgc aacagtatat tgcatgctct ttggcactta 3120
catgaaagtg aaaaatgaag gaggagagtg taagcctgaa ggtcttttta gaaggcttcc 3180
tcatttggat atgtggaatg aatttttca tgttatgttg aatattccag attgtcatca 3240
tcttccatct ttggatttgt taaggcaaaa gctgaagaaa gtatttcaac aacactatac 3300
taacaagatt agggccctac gtaataggct aattgtactg ctcttagaat gtaagcgttc 3360
acgaaaataa aatttggata tagacagtcc ttaaaaatca cactgtaaat atgaatctgc 3420
tcactttaaa cctgtttttt tttcatttat tgtttatgta aatgtttgtt aaaaataaat 3480
cccatggaat atttccatgt aaaaaaaaa 3509

<210> 25
<211> 2501
<212> DNA
<213> Homo sapiens

<400> 25

cgaaaagatt cttaggaacg ccgtaccagc cgcgtctctc aggacagcag gcccctgtcc 60
ttctgtcggg cgccgctcag ccgtgccctc cgcccctcag gttctttttc taattccaaa 120
taaacttgca agaggactat gaaagattat gatgaacttc tcaaatatta tgaattacat 180
gaaactattg ggacaggtgg ctttgcaaag gtcaaacttg cctgccatat ccttactgga 240
gagatggtag ctataaaaat catggataaa aacacactag ggagtgattt gccccggatc 300

```
aaaacggaga ttgaggcctt gaagaacctg agacatcagc atatatgtca actctaccat    360
gtgctagaga cagccaacaa aatattcatg gttcttgagt actgccctgg aggagagctg    420
tttgactata taatttccca ggatcgcctg tcagaagagg agacccgggt tgtcttccgt    480
cagatagtat ctgctgttgc ttatgtgcac agccagggct atgctcacag ggacctcaag    540
ccagaaaatt tgctgtttga tgaatatcat aaattaaagc tgattgactt tggtctctgt    600
gcaaaaccca agggtaacaa ggattaccat ctacagacat gctgtgggag tctggcttat    660
gcagcacctg agttaataca aggcaaatca tatcttggat cagaggcaga tgtttggagc    720
atgggcatac tgttatatgt tcttatgtgt ggatttctac catttgatga tgataatgta    780
atggctttat acaagaagat tatgagagga aaatatgatg ttcccaagtg gctctctccc    840
agtagcattc tgcttcttca acaaatgctg caggtggacc caaagaaacg gatttctatg    900
aaaaatctat tgaaccatcc ctggatcatg caagattaca actatcctgt tgagtggcaa    960
agcaagaatc cttttattca cctcgatgat gattgcgtaa cagaactttc tgtacatcac   1020
agaaacaaca ggcaaacaat ggaggattta atttcactgt ggcagtatga tcacctcacg   1080
gctacctatc ttctgcttct agccaagaag gctcggggaa aaccagttcg tttaaggctt   1140
tcttctttct cctgtggaca agccagtgct accccattca cagacatcaa gtcaaataat   1200
tggagtctgg aagatgtgac cgcaagtgat aaaaattatg tggcgggatt aatagactat   1260
gattggtgtg aagatgattt atcaacaggt gctgctactc cccgaacatc acagtttacc   1320
aagtactgga cagaatcaaa tggggtggaa tctaaatcat taactccagc cttatgcaga   1380
acacctgcaa ataaattaaa gaacaaagaa aatgtatata ctcctaagtc tgctgtaaag   1440
aatgaagagt actttatgtt tcctgagcca aagactccag ttaataagaa ccagcataag   1500
agagaaatac tcactacgcc aaatcgttac actacaccct caaaagctag aaaccagtgc   1560
ctgaaagaaa ctccaattaa aataccagta aattcaacag gaacagacaa gttaatgaca   1620
ggtgtcatta gccctgagag gcggtgccgc tcagtggaat tggatctcaa ccaagcacat   1680
atggaggaga ctccaaaaag aaagggagcc aaagtgtttg ggagccttga aagggggttg   1740
gataaggtta tcactgtgct caccaggagc aaaaggaagg gttctgccag agacgggccc   1800
agaagactaa agcttcacta taatgtgact acaactagat tagtgaatcc agatcaactg   1860
ttgaatgaaa taatgtctat tcttccaaag aagcatgttg actttgtaca aaagggttat   1920
acactgaagt gtcaaacaca gtcagatttt gggaaagtga caatgcaatt tgaattagaa   1980
gtgtgccagc ttcaaaaacc cgatgtggtg ggtatcagga ggcagcggct taagggcgat   2040
gcctgggttt acaaaagatt agtggaagac atcctatcta gctgcaaggt ataattgatg   2100
```

gattcttcca tcctgccgga tgagtgtggg tgtgatacag cctacataaa gactgttatg 2160
atcgctttga ttttaaagtt cattggaact accaacttgt ttctaaagag ctatcttaag 2220
accaatatct ctttgttttt aaacaaaaga tattattttg tgtatgaatc taaatcaagc 2280
ccatctgtca ttatgttact gtctttttta atcatgtggt tttgtatatt aataattgtt 2340
gactttctta gattcacttc catatgtgaa tgtaagctct taactatgtc tctttgtaat 2400
gtgtaatttc tttctgaaat aaaaccattt gtgaatataa aaaaaaaaaa aaaaaaaaaa 2460
aaaaaaaaaa aaaaaaaaaa aaaaaaaaaa aaaaaaaaaa a 2501

<210> 26
<211> 890
<212> DNA
<213> Homo sapiens

<400> 26

atcttcaccc aggctggccc ctttggtgaa actacaactc ccaggggtct gtgcgcgaga 60
aggcaggcgg gtttttctac cggaagtccg ctctagctct gggccctaca actgcaccct 120
gagccggagc tgcccagtcg ccgcgggacc ggggccgctg ggtctggac ggggtcgcc 180
atgatccgct ttatcctcat ccagaaccgg gcaggcaaga cgcgcctggc caagtggtac 240
atgcagtttg atgatgatga gaaacagaag ctgatcgagg aggtgcatgc cgtggtcacc 300
gtccgagacg ccaaacacac caactttgtg gagttccgga actttaagat catttaccgc 360
cgctatgctg gcctctactt ctgcatctgt gtggatgtca atgacaacaa cctggcttac 420
ctggaggcca ttcacaactt cgtggaggtc ttaaacgaat atttccacaa tgtctgtgaa 480
ctggacctgg tgttcaactt ctacaaggtt tacacggtcg tggacgagat gttcctggct 540
ggcgaaatcc gagagaccag ccagacgaag gtgctgaaac agctgctgat gctacagtcc 600
ctggagtgag ggcaggcgag ccccaccccg gccccggccc ctcctggact cgcctgctcg 660
cttccccttc ccaggcccgt ggccaaccca gcagtccttc cctcagctgc ctaggaggaa 720
gggacccagc tgggtctggg ccacaaggga ggagactgca ccccactgcc tctgggccct 780
ggctgtgggc agaggccacc gtgtgtgtcc cgagtaaccg tgccgttgtc gtgtgatgcc 840
ataagcgtct gtgcgtggag tccccaataa acctgtggtc ctgcctggcc 890

<210> 27
<211> 776
<212> DNA
<213> Homo sapiens

<400> 27

```
atcttcaccc aggctggccc ctttggtgaa actacaactc ccaggggtct gtgcgcgaga     60
aggcaggcgg gtttttctac cggaagtccg ctctagctct gggccctaca actgcaccct    120
gagccggagc tgcccagtcg ccgcgggacc ggggccgctg ggtctggac ggggtcgcc     180
atgatccgct ttatcctcat ccagaaccgg gcaggcaaga cgcgcctggc caagtggtac    240
atgcagtttg atgatgatga gaaacagaag ctgatcgagg aggtgcatgc cgtggtcacc    300
gtccgagacg ccaaacacac caactttgtg gaggtcttaa acgaatattt ccacaatgtc    360
tgtgaactgg acctggtgtt caacttctac aaggtttaca cggtcgtgga cgagatgttc    420
ctggctggcg aaatccgaga gaccagccag acgaaggtgc tgaaacagct gctgatgcta    480
cagtccctgg agtgagggca ggcgagcccc accccggccc cggcccctcc tggactcgcc    540
tgctcgcttc cccttcccag gcccgtggcc aacccagcag tccttccctc agctgcctag    600
gaggaaggga cccagctggg tctgggccac aagggaggag actgcacccc actgcctctg    660
ggccctggct gtgggcagag gccaccgtgt gtgtcccgag taaccgtgcc gttgtcgtgt    720
gatgccataa gcgtctgtgc gtggagtccc caataaacct gtggtcctgc ctggcc        776
```

<210> 28
<211> 2554
<212> DNA
<213> Homo sapiens

<400> 28

```
ggattggctg ttcggagcgg cgaggggctt ggcgggagct tccagtcgct cgagagcgga    60
gagcggcacc atggccccgc gggggcggcg gcggccgcgg cctcacaggt ctgagggcgc   120
aagacgttca aagaacactt tagaaagaac acattccatg aaagataaag ctggtcaaaa   180
gtgcaagcct attgacgtgt tcgactttcc tgataattct gatgtctcaa gcattggcag   240
gctgggtgaa aatgagaaag atgaagaaac ttatgagacc tttgatcctc ctttacatag   300
cacagctata tatgctgatg aagaagaatt ctccaaacat tgtggactgt ctctctcttc   360
aactcctcca ggaaaagaag caaaaagaag ttcagacact tctggaaatg aagcaagtga   420
aatcgaatct gtaaaaatta gtgcaaaaaa gccaggaaga aagctcaggc ccattagtga   480
tgactctgaa agcattgaag aaagtgatac aaggagaaaa gttaaatcag cagagaaaat   540
aagtacacaa cgtcatgagg ttattcgaac cacagcgtct tcagaacttt cagagaaacc   600
agctgagtct gtcacttcta aaaagacagg accccttagt gcccagccct ctgttgaaaa   660
agagaacttg gcaatagaaa gtcaatcgaa aactcagaaa aaagggaaga tatctcatga   720
caaaaggaag aaatcaagaa gtaaagccat aggctcagat acttctgaca ttgtgcacat   780
```

```
ttggtgtcca gaaggaatga aaaccagtga catcaaggag ttgaatattg ttttgcctga    840
atttgagaaa acccacctag agcatcaaca aagaatagaa tctaaagttt gtaaggcagc    900
catcgccaca ttttatgtta atgttaaaga acaattcatc aaaatgctta aagaaagcca    960
gatgttgaca aatctgaaaa ggaagaatgc taagatgatt tcagatatcg aaaagaaaag   1020
gcagcgtatg attgaagtcc aggatgaact gcttcggtta gagccacagc tgaaacaact   1080
acaaacaaaa tatgatgaac ttaaagagag aaagtcttcc cttaggaatg cagcatattt   1140
cttatctaat ttaaaacagc tttatcaaga ttattcagat gttcaagctc aagaaccaaa   1200
cgtaaaggaa acgtatgatt catccagcct tccagctctg ttatttaaag caagaacact   1260
tctgggagcc gaaagccatc tgcgaaatat caaccatcag ttagagaagc tccttgacca   1320
gggatgagaa gagcagtcta ctaaaatgtg cctataggaa gactagtctc atgctgttac   1380
cttctgaaac tgtaccttta taaatcaatt gttttgcaaa gaagttatgg cctacttaga   1440
atctaaaatt tgttattcaa attaaatggc tgtgaacaat gttaaatagc atcagtttgt   1500
ccaatagttt taaaggccat aatcatcttt tctggttaat atcttgagta attttaaaat   1560
gttgacacct taatcggtcc caggtatgag ctataataaa cttgtaaaat taagttgatg   1620
tgaacataat tttgattaat taataaggcg atttctcctg aatttacacc aaagctaatt   1680
tttaatgaaa ttggtttaca gaagtaaaaa acaaaaattg gaaagcaaag taataaaact   1740
tagtttatat aaacaggttg aatgatatat ttatcaaatc tcacagacat caggcaaatt   1800
atagcctggt gacaaaagtg ttcatagtga attagttact cttgtaatac ttctataatt   1860
agttcatcag gaatttcatc cacttcactg ttataactga gaagactgtt ctctgcagct   1920
tcagctaatt cagcatcttc agtagcttct aaaaaataag catcatcaat gccattatcc   1980
cagacagcat cagcagatgc acctgttgac agcctgctag gtgatggttt atgaggattc   2040
tgggtttcat tgctcctagt ttcatctgct tcatctgttg taaactcttc ttcctttatt   2100
tcagtggtga agggatagag agtgggatag gaaaatattt actcaggata tgtgatttaa   2160
ccttatactc tatgttgaag taaggtatta agtgacagat actaaagtga atatgcagga   2220
ggaatgctgt ctccgatatc tcaccgtggg aatgagtgca ctgattcaaa cgttgctgca   2280
ctgaagctca gacacacttg aaactccaaa tttgaaatta cctacagttc tgtgcacata   2340
cttttcaata ctccccgacg gaagagcaag ggtggattta atttttaac aagtggacag    2400
tccagctgaa gacaaatcag aagataaatt tgctatcttg acaatggact tagtacccat   2460
gctttaaatt ttaaagtatt tagcaaatcg taaacatgga ttgaaaaaag attaaaaaca   2520
gttgccaaaa tatgactgga ttgtatatta aaaa                               2554
```

<210> 29
<211> 1431
<212> DNA
<213> Homo sapiens

<400> 29

```
ccgtgaagtg ggcggagcga gcgatttgaa cgcgagcggc gcggacttct gccaagcacc      60
ggctcatgtg aggctcgcgg cacagcgttc tctgggctcc ccagaagcca gcctttcgct     120
cccggacccg gcagcccgag caggagccgt gggaccgggc gccagcaccc tctgcggcgt     180
gtcatgggcc cgcgccgccg gagccgaaag cccgaggccc cgaggaggcg cagcccgagc     240
ccgaccccga cccccggccc ctcccggcgg ggcccctcct taggcgcttc ctcccatcaa     300
cacagtcggc ggagacaagg ttggctaaag gagatccgaa agcttcagaa gagcacacac     360
ctcttgataa ggaagctgcc cttcagccgc ctggcaagag aaatatgtgt taaattcact     420
cgtggtgtgg acttcaattg gcaagcccag gccctattgg ccctacaaga ggcagcagaa     480
gcatttctag ttcatctctt tgaggacgcc tatctcctca ccttacatgc aggccgagtt     540
actctcttcc caaaggatgt gcaactggcc cggaggatcc ggggccttga ggagggactc     600
ggctgagctc ctgcacccag tgtttctgtc agtctttcct gctcagccag gggggatgat     660
accggggact ctccagagcc atgactagat ccaatggatt ctgcgatgct gtctggactt     720
tgctgtctct gaacagtatg tgtgtgttgc tttaaatatt tttctttttt ttgagaagga     780
gaagactgca tgactttcct ctgtaacaga ggtaatatat gagacaatca acaccgttcc     840
aaaggcctga aaataatttt cagataaaga gactccaagg ttgactttag tttgtgagtt     900
actcatgtga ctatttgagg attttgaaaa catcagattt gctgtggtat gggagaaaag     960
gctatgtact tattatttta gctctttctg taatatttac attttttacc atatgtacat    1020
ttgtactttt attttacaca taagggaaaa aataagacca ctttgagcag ttgcctggaa    1080
ggctgggcat ttccatcata tagacctctg cccttcagag tagcctcacc attagtggca    1140
gcatcatgta actgagtgga ctgtgcttgt caacggatgt gtagcttttc agaaacttaa    1200
ttggggatga atagaaaacc tgtaagcttt gatgttctgg ttacttctag taaattcctg    1260
tcaaaatcaa ttcagaaatt ctaacttgga gaatttaaca ttttactctt gtaaatcata    1320
gaagatgtat cataacagtt cagaatttta aagtacattt tcgatgcttt tatgggtatt    1380
tttgtagttt ctttgtagag agataataaa aatcaaaata tttaatgaaa a             1431
```

<210> 30
<211> 1352
<212> DNA
<213> Homo sapiens

<400> 30

```
cgtgaagtgg gcggagcgag cgatttgaac gcgagcggcg cggacttctg ccaagcaccg      60
gctcatgtga ggctcgcggc acagcgttct ctgggctccc cagaagccag cctttcgctc     120
ccggacccgg cagcccgagc aggagccgtg ggaccgggcg ccagcaccct ctgcggcgtg     180
tcatgggccc gcgccgccgg agccgaaagc ccgaggcccc gaggaggcgc agcccgagcc     240
cgacccccgac ccccggcccc tcccggcggg gcccctcctt aggcgcttcc tcccatcaac     300
acagtcggcg gagacaaggt tggctaaagg agatccgaaa gcttcagaag agcacacacc     360
tcttgataag gaagctgccc ttcagccgcc tggcagcaga agcatttcta gttcatctct     420
ttgaggacgc ctatctcctc accttacatg caggccgagt tactctcttc ccaaaggatg     480
tgcaactggc ccggaggatc cggggccttg aggagggact cggctgagct cctgcaccca     540
gtgtttctgt cagtctttcc tgctcagcca gggggatga taccggggac tctccagagc     600
catgactaga tccaatggat tctgcgatgc tgtctggact ttgctgtctc tgaacagtat     660
gtgtgtgttg ctttaaatat ttttcttttt tttgagaagg agaagactgc atgactttcc     720
tctgtaacag aggtaatata tgagacaatc aacaccgttc caaaggcctg aaaataattt     780
tcagataaag agactccaag gttgacttta gtttgtgagt tactcatgtg actatttgag     840
gattttgaaa acatcagatt tgctgtggta tgggagaaaa ggctatgtac ttattatttt     900
agctctttct gtaatattta catttttac catatgtaca tttgtacttt tattttacac     960
ataagggaaa aaataagacc actttgagca gttgcctgga aggctgggca tttccatcat    1020
atagacctct gcccttcaga gtagcctcac cattagtggc agcatcatgt aactgagtgg    1080
actgtgcttg tcaacggatg tgtagctttt cagaaactta attggggatg aatagaaaac    1140
ctgtaagctt tgatgttctg gttacttcta gtaaattcct gtcaaaatca attcagaaat    1200
tctaacttgg agaatttaac attttactct tgtaaatcat agaagatgta tcataacagt    1260
tcagaatttt aaagtacatt ttcgatgctt ttatgggtat ttttgtagtt tctttgtaga    1320
gagataataa aaatcaaaat atttaatgaa aa                                  1352
```

<210> 31
<211> 916
<212> DNA
<213> Homo sapiens

<400> 31

```
ggacgcagag aagccagaga ctttcgcttc cggctgccgc aggcgcttcg ctggtgcagg      60
```

taagctccgc acactctcgg ccggtcccga gtccgactcc ctcaagggtg acgcgagctc 120

tgccctttaa ccggaaacgt ctccctgctc accccacccc cgcgcagacg cagtgctgag 180

cacacagcta ccggacaaag agtgacgccc ggagctggag ttatggcggc tacggagccg 240

atcttggcgg ccactgggag tcccgcggcg gtgccaccgg agaaactgga aggagccggt 300

tcgagctcag cccctgagcg taactgtgtg ggctcctcgc tgccagaggc ctcaccgcct 360

gcccctgagc cttccagtcc caacgccgcg gtccctgaag ccatccctac gccccgagct 420

gcggcctccg cggccctgga gctgcctctc gggcccgcac ccgtgagcgt agcgcctcag 480

gccgaagctg aagcgcgctc cacaccaggc cccgccggct ctagactcgg tcccgagacg 540

ttccgccagc gtttccggca gttccgctac caggatgcgg cgggtccccg ggaggctttc 600

cggcagctgc gggagctgtc ccgccagtgg ctgcggcctg acatccgcac caaggagcag 660

atcgtggaga tgctggtgca agagcagctg ctcgccatcc tgcccgaggc ggctcgggcc 720

cggcggatcc gccgccgcac ggatgtgcgc atcactggct gagcggtgga gctgcgggcg 780

gccagggccg ggcgctctgt gcggactggg gccatgatcg ggcccggggg cctgagcctg 840

ggaccccacc ccgtgttaat gaaaaatgag ttttggcagc gccaaaaaaa aaaaaaaaaa 900

aaaaaaaaaa aaaaaa 916

<210> 32
<211> 840
<212> DNA
<213> Homo sapiens

<400> 32

ggacgcagag aagccagaga ctttcgcttc cggctgccgc aggcgcttcg ctggtgcaga 60
cgcagtgctg agcacacagc taccggacaa agagtgacgc ccggagctgg agttatggcg 120
gctacggagc cgatcttggc ggccactggg agtcccgcgg cggtgccacc ggagaaactg 180
gaaggagccg gttcgagctc agcccctgag cgtaactgtg tgggctcctc gctgccagag 240
gcctcaccgc ctgcccctga gccttccagt cccaacgccg cggtccctga agccatccct 300
acgccccgag ctgcggcctc cgcggccctg gagctgcctc tcgggcccgc acccgtgagc 360
gtagcgcctc aggccgaagc tgaagcgcgc tccacaccag gccccgccgg ctctagactc 420
ggtcccgaga cgttccgcca gcgtttccgg cagttccgct accaggatgc ggcgggtccc 480
cgggaggctt tccggcagct gcgggagctg tcccgccagt ggctgcggcc tgacatccgc 540
accaaggagc agatcgtgga gatgctggtg caagagcagc tgctcgccat cctgcccgag 600
gcggctcggg cccggcggat ccgccgccgc acggatgtgc gcatcactgg ctgagcggtg 660

gagctgcggg cggccagggc cgggcgctct gtgcggactg gggccatgat cgggcccggg 720
ggcctgagcc tgggacccca ccccgtgtta atgaaaaatg agttttggca gcgccaaaaa 780
aaaaaaaaaa aaaaaaaaaa aaaaaaaaaa aaaaaaaaaa aaaaaaaaaa aaaaaaaaaa 840

<210> 33
<211> 3995
<212> DNA
<213> Homo sapiens

<400> 33

```
ccccgccccg ctcctctccg gccgcgcagc cgctgccgcc cacccgcacc cgccgtcatg      60
ctccgggccg cgctgcccgc gctcctgctg ccgttgctgg gcctcgccgc tgctgccgtc     120
gcggactgtc cttcatctac ttggattcag ttccaagaca gttgttacat tttctccaa      180
gaagccatca aagtagaaag catagaggat gtcagaaatc agtgtactga ccatggagcg     240
gacatgataa gcatacataa tgaagaagaa aatgctttta tactggatac tttgaaaaag     300
caatggaaag gcccagatga tatcctacta ggcatgtttt atgacacaga tgatgcgagt     360
ttcaagtggt ttgataattc aaatatgaca tttgataagt ggacagacca agatgatgat     420
gaggatttag ttgacacctg tgcttttctg cacatcaaga caggtgaatg gaaaaaagga     480
aattgtgaag tttcttctgt ggaaggaaca ctatgcaaaa cagctatccc atacaaaagg     540
aaatatttat cagataacca cattttaata tcagcattgg tgattgctag cacggtaatt     600
ttgacagttt tgggagcaat catttggttc ctgtacaaaa aacattctga ttctcgtttc     660
accacagttt tttcaaccgc accccaatca ccttataatg aagactgtgt tttggtagtt     720
ggagaagaaa atgaatatcc tgttcaattt gactaagttt ttggtaatct tgcactaaga     780
catcaacaaa atgccctggc agagataact tgggaaagat tttaatataa aacttgacat     840
tggatattag agctttaatg gtattcctta ttccagtaac atttttatgt actcatctgc     900
tgtgaaaagt ctttaggttc attaaaaaaa caggttttag aaatgatctt agatctaata     960
tagtgatttt aagcatcccg tcaaaggcag aatctgtcac ttgaatgaag gaaagcttaa    1020
agcccaagca gataaaaata aaagcccagc ctatttgtct tgcctgctgt atcttcccta    1080
tttagttgac ccactttagt ttatatgttt attagtaaac atgaaatggg gaataagtga    1140
ttttaagtac atcccataca tttaaatatc tttgataatt gttatttttt tggcagataa    1200
ttcctctaga atgtgtatct ttttatgatt tagatgaaga aaattttaca acttttaaca    1260
ccccacacca attttagttt cattactttt acacacacca ttttatcaca aatgactcaa    1320
gttttaatga atgtttataa attatttgaa acaaaatatg atcgctgtgt ccaggatggc    1380
```

atagagaaag ctggcaatta ggttaacact tacatattat agtgcccctt taaggatttc 1440
tctcttgcca ccataccttt tgtactttcc cctatacaag atgtatctca ttctcctcaa 1500
gcatttataa atttttcctt caatgacatg aaaactgtgc aagcaaaaac cgaagaaaaa 1560
cacttaagta caactgtagt gacagtgatc aaagttttca gtgcatttat tgtacatttt 1620
aagaaaaagg tgaaaatcat ttggggagta aaaaaatgaa aaagctgaaa cgagtaattt 1680
tcctcaccat caataaacca aaaacaggaa agataaagaa tgtataaatt tcacgtaaat 1740
tagtcacgta tcacttatca atggggatac gttctaagaa atgcatagtt agggaatctt 1800
gtgtgaaaat cagcttgtat ttacacaaac ccagatggta gagcctattt tgtcccaaac 1860
ctacacagca tgttactgtg ctgaatactg cagacaattg taacacaata tttgtgtatc 1920
taaatataga aaaggtacag taaaaatatg gtctactaag gaaacactgt tctatatgtg 1980
gtccattact gactgaagta tactgtctag aagtctgagg ctcaaagaaa agtaatccct 2040
cttctgaatc cacaccccat caattatctt actttcttct ggggagatag atagatatac 2100
tatctcacta gcttgactaa tggcaacaaa gttccagctt gtgtagtctc tttttattga 2160
ccacatgaat cgaaaacact catcacaatt aatggcacta tcattaatga gacatgagta 2220
actaaaaagt gatagaaaac tattaacagt gcggctacat ggtactgaaa atgcaggcat 2280
tacaccagct gttacacaag cacaagcatg ctctgtaaga gctttacatt tctgagattt 2340
tgtatagtga ttgagatgtc tattttatta ttgatagact attactaatg tcaatattga 2400
acactaccct ggaattcctg cctggttttc ctacccaaat tgtaccactc cttgaagaac 2460
tacaggcaca gtaaaaaaaa tatggcgtat tatgtgaact aaaagagttc taaaggagtt 2520
cttaaaggag tggtagaatt tgggtaggaa agtgattaag tccaacttaa aaccaacagt 2580
ctcaaacgtc tacaactaca atgtccaatg agccactagc cacatgaggc tatttaagta 2640
aatttagttt aaaatccagt tttcgaatta cattagccac attgtcaagt gttcaaatca 2700
caggtggtta gtggctactg tactgggcaa catacattat agaacatttt cattatagga 2760
agttttattg ggcagtgctg ctcttaaatc ctaccttcca ctcaactccc atacaacttt 2820
cttttgtaca ttttgatact ttctacctaa tggcagctct tccaaaatag ctgctttaaa 2880
ctctgattta attttcaata tttggtttca tttttcaaca ggccaagagg cctctggtaa 2940
tgaagtgcta tatatatata tatgatggag tctcactgtg ctgcccaggc tacagtgcag 3000
tggctcgatc ttggctctct ccaatctccg ccttgcaggt tttcaagcaa ttctcctgcc 3060
tcagcctcct tagtagctgg gaccacagac atctgtcacc acacccagct aactttttgt 3120
atttttggta gagacggggt ttcgccatat tgactgggct ggtctcaaac tcctgacctc 3180

aagtgatcca cccaccttgg tctcccaaag tgctgggatt acatgcgtga gccaccacac 3240
ttggcctaca tttttctttt atataccaga acatctataa caggcacctt atctactcat 3300
tagtgaagag ataattggat tacacaggca ggcttgttta ctacatccag aatgtagaaa 3360
ctgctttctt caacatcttg gttctagcta gtaataacaa tataattctt tggcagatat 3420
tcagaataac attttaaact acattttctt agaaaattgc attcttgtag tgagcagtgt 3480
atggtctctt ttgttcagaa tttaaaactg ataaccaatg aaagcctttt ctcttattcc 3540
tctaccgtca tttacatgat aatctgaagc taatatgaca atatttaaat actaagtggt 3600
actagggaac tacaagaata ctgtaaagct taagccattg ttatcactgt catttagcat 3660
ttaataacaa aactatacag aattatgtgc ataccaatga atgttttgta ccatctagtt 3720
aaatttttta aataaagttt tatgggttaa gcagaagaca actgtcatac tgaattttat 3780
taaaagtata tatacttcaa attcaaagca tcccttagga cccacagaat atattaaaac 3840
taccaccctt aaattttata tttttgcttt aagacagaca atgcaaaggt aactggcaag 3900
aggtgagcaa atgttttaga acatttatat tattgcttaa aatgagattt gaaattgtaa 3960
taaaattctt ggttatgaaa aaaaaaaaaa aaaaa 3995

<210> 34
<211> 2742
<212> DNA
<213> Homo sapiens

<400> 34

```
cccgcctctt cctcccttcc ttctttcctt gctttcgccg cgcactccgc cgccatggag     60
cagcgccgcg tcaccgactt cttcgcgcgc cgccgccccg ggcccccccg catcgcgccg    120
cccaagctgg cctgccgcac ccccagcccc gccaggcccg cactccgcgc cccggcctcc    180
gctaccagtg gcagccgcaa gcgcgcccgc ccgcccgccg cccccggacg cgaccaggcc    240
aggccaccgg cccgcaggag actgcggctg tcggtggacg aggtttccag ccccagtacc    300
cccgaggccc cagacatccc agcctgccct tctccgggcc agaagataaa gaaatccacc    360
ccggcagcag gtcagccgcc ccacctgaca tccgcgcagg accaggacac catctctgag    420
cttgcgtcat gcctgcaacg ggcccgggag ctgggggcaa gagtccgggc gctgaaggcc    480
agtgcccagg atgctgggga gtcctgcacc ccagaggccg agggccgccc tgaggagcca    540
tgtggcgaga aggcgcccgc ctaccagcgc ttccatgccc tggcccagcc cggcctgccg    600
ggactcgtgc tgccctacaa gtaccaggtg ctggcggaga tgttccgcag catggacacc    660
atcgtgggca tgctccacaa ccgctccgag acgcccacct ttgccaaggt ccagcggggc    720
```

gtccaggaca tgatgcgtag gcgttttgag gagtgcaatg ttggccagat caaaaccgtg 780
tacccggcct cctaccgctt ccgccaggag cgcagtgtcc ccaccttcaa ggatggcacc 840
aggaggtcag attaccagct caccatcgag ccactgctgg agcaggaggc tgacggagca 900
gcccccccagc tcacggcctc gcgcctcctg cagcgacggc agatcttcag ccagaagctg 960
gtggagcatg tcaaggagca ccacaaggcc ttcctggcct ccctgagccc cgccatggtg 1020
gtgccggagg accagctgac ccgctggcac ccgcgcttca acgtggatga agtacccgac 1080
atcgagccgg ccgcgctgcc ccagccaccc gccacggaga agctcaccac tgctcaggag 1140
gtgctggccc gggcccgcaa cctgatttca cccaggatgg agaaggcctt gagtcaattg 1200
gccctgcgct ctgctgcgcc cagcagcccc gggtctccca ggccagcact gccggctacc 1260
ccaccagcca ccccgcctgc agcctctccc agtgctctga aggggtgtc ccaggatctg 1320
ctggagcgga tccgagccaa ggaggcacag aagcagctgg cacagatgac gcggtgcccg 1380
gagcaggagc agcggctgca gcgcttagaa cggctgcctg agctggcccg cgtgctgcgg 1440
agcgtctttg tgtccgaacg caagcctgcg ctcagcatgg aggtggcctg tgccaggatg 1500
gtgggcagct gttgtactat catgagccct ggggaaatgg agaagcacct gctgctcctc 1560
tccgagctgc tgccggactg gctcagcctc caccgcatcc gcaccgacac ctacgtcaag 1620
ctggacaagg ccgcggacct cgcccacatc actgcacgcc tggcccacca gacacgtgct 1680
gaggaggggc tgtgagcctg gggccactg tggacagacg tgggcttcag aagctcgctg 1740
gcctgggccc accagcattt tcttttatga acatgataca ctttggcctt cctttcccca 1800
gcgcccctga gggccagagg cagatgtggg ctgcaggctg cacagcccga gggtctctgg 1860
ctgcgggcgg tgggcccctt catggggctc acctggtgga ttcacattaa accggtttct 1920
gtgggcacct ctgtccttgc tgctggtggg gaagggaagc cagatccagc accccctggg 1980
gggccatcgg gagtgtggct gggggtgaag ggggctctgt ggcaatatgg ggttgggtag 2040
tgtgggtggc aggccatccc ctctaatctt ggaacctctg aatatgggac ctcccacagc 2100
aaagggtgac ttttgtcatt aagaaagact ggggtgggtg tggtggctca cgcctgtaac 2160
cccagcactt tgggaggcca aggtgggcag atcacgaggt caagagatcg agaccatcct 2220
ggcgaacatg gtgaaacccc atctctacta aaaatacaaa aaattagccg ggtgtggtgg 2280
tgggcacctg tcgtcccagc tactagggag gctgaggcag gagaatggtg tgaacccagg 2340
aggcacagct tgcagtgagc gaagatcgca ccactgcacg cactccagcc tgggtgacag 2400
agcgagactc cgtctcaaaa aaaaaaattt caagactgga gaggtgatcc tgaattgtcc 2460

agctacgccc catgtcatca cagggccttc atgacagggc cagagccagc cagctttgaa 2520

gacgcggccc tgccccgaca caggcagcct ggagaagctg ggcaggacaa gtaggacatc 2580

cctggagcct ccagaaggga ctggcctctg cccacacctt gacttcagta tttctgacct 2640

cctaaactct aataaagtca tgcttacagc cactaaaaaa aaaaaaaaaa aaaaaaaaaa 2700

aaaaaaaaaa aaaaaaaaaa aaaaaaaaaa aaaaaaaaaa aa 2742

<210> 35
<211> 8449
<212> DNA
<213> Homo sapiens

<400> 35

gcgacctccg gcgccatttt gtagagaaac aagcggagtt aaccgaagag ggggtcgagg 60

agagccggag tcggggaccc aggagtttcc tgtgtccagc gctgccggag ccgcctgagg 120

tgccatgttt cagaacagag taagacccct ggtaaagaag aactgaagat attatacaga 180

taccagatat agcctaatta caaagaaagc attaacctgc ctctgaggtg actaaagggg 240

aataatggtg attttgcgcc gggctcggcc gcctgcttcc gccccaacca gcaatgaatc 300

ttgactcgct ctcgctggcc ttgtctcaaa tcagctacct ggtggacaat ttaaccaaga 360

aaaattaccg agccagccag caggaaatac agcatattgt gaatcggcac ggtcctgagg 420

cagacaggca tttattacgc tgcctatttt cgcatgtgga tttcagtggc gatggtaaaa 480

gcagtggcaa agatttccat cagactcagt ttctgattca ggagtgtgcg ttgctgatta 540

caaagccaaa ttttatctcg acgctgtcct atgccattga taatccattg cactatcaga 600

agagtttaaa gcctgcaccc cacttatttg cccagctgag taaagtgctc aaattaagca 660

aagtacaaga ggtaattttt ggccttgccc tgttgaattc ttccagctca gatcttagag 720

gtttcgctgc ccagtttatc aaacagaagc ttccagatct tctgcgttct tacattgacg 780

cagacgtcag tggaaatcaa gaaggtggct tccaagatat agcaatagag gtcctacacc 840

tcctcctctc ccatctcctc tttgggcaga agggagcctt tggagttgga caagaacaga 900

tagacgcttt tcttaagacg ctgcgcagag attttcccca agaacgctgt cccgtggtgc 960

tcgcaccact tttataccct gaaaaacggg acattctaat ggacaggatc ctgcctgatt 1020

ccggaggggt agctaaaacc atgatggaga gctctttggc tgatttcatg caagaagtag 1080

gctatggctt ttgtgcaagt attgaagaat gtcgcaatat aatcgtgcag tttggtgttc 1140

gggaggtcac agctgcccag gttgcaaggg ttttgggaat gatggctcga actcattcag 1200

gattaacaga tggcattcca ttacagagta tttctgctcc gggcagtggg atctggagtg 1260

atgggaaaga taaaagtgat ggagcacagg cacacacatg gaatgtagaa gtcttgattg 1320
acgttcttaa agaactgaat ccaagtttga atttcaagga agtaacttat gaactggacc 1380
atcctggatt tcaaattcgt gacagtaaag gacttcataa tgtggtttat ggcattcaga 1440
ggggtttggg tatggaagtg ttcccagtag acctcatata tagaccttgg aaacatgctg 1500
aaggccagct ctccttcatt caacattccc ttataaatcc agagatcttc tgttttgctg 1560
actatccctg tcatactgtt gccactgata ttctgaaagc accaccagag gatgacaatc 1620
gagaaattgc cacatggaag agcttggatt tgattgaatc tctgctgagg cttgcagagg 1680
ttgggcagta tgagcaagtc aaacagctct tcagcttccc tatcaaacac tgtccagaca 1740
tgctggtatt ggccttacta caaattaaca cctcttggca taccttgcgc catgaactta 1800
tctccactct gatgccaatt ttccttggaa accatcctaa ctcagctatt attttgcact 1860
atgcatggca tgggcaggga cagtctccct caattcgcca acttatcatg catgcaatgg 1920
cagaatggta catgagaggg gagcagtatg atcaggccaa attgtctcga atacttgatg 1980
tggcccagga cttgaaggcc ttgtcaatgc tgctaaatgg tactccattt gcctttgtta 2040
ttgaccttgc tgcacttgct tcacgtcgtg aatacctcaa acttgataag tggctcacag 2100
ataaaattcg agagcatggg gagcctttta tccaggcgtg tatgactttt ttaaagagac 2160
ggtgtccttc tattttgggc ggacttgccc cagaaaaaga ccagcccaaa agtgctcaac 2220
ttcctccaga aactttggcg acaatgttgg cctgtctgca agcttgtgca gggagtgttt 2280
ctcaggagct atcagaaact atcctcacca tggtagccaa ttgcagtaat gttatgaata 2340
aggccagaca accaccacct ggagttatgc caaaaggacg tcctcctagt gctagcagct 2400
tagatgccat ttctcctgtt cagattgacc ctcttgctgg aatgacatct cttagtatag 2460
gtggttcagc tgcccctcac acccagagta tgcagggttt tcctccaaat ttgggttctg 2520
cattcagtac ccctcagtca ccagcaaaag catttccacc cctttcaacc cccaatcaga 2580
ccactgcatt cagtggtatt ggaggacttt catcacagct tccagtaggt ggtcttggca 2640
caggcagcct gactggtata ggaactggtg ctcttggact ccctgcagtg aataacgacc 2700
cttttgtaca gaggaaactg ggcacctctg gactgaatca gcctacattc cagcagagta 2760
agatgaaacc ttcggacttg tctcaggtgt ggccagaggc aaaccagcac tttagtaaag 2820
agatagatga tgaagcaaac agctatttcc agcgaatata taatcatcca ccacatccaa 2880
ccatgtctgt tgatgaggta ttagaaatgc tgcagagatt taaagactct actataaaga 2940
gggaacgaga agtatttaac tgtatgctaa ggaacttgtt tgaagaatat cgtttttttc 3000
cccagtatcc tgataaagag ttacatataa cagcctgcct atttggtggt ataattgaga 3060

aaggactggt cacttacatg gcactaggtc tggctctacg atatgttctt gaagccttac 3120

gcaagccttt tggatccaaa atgtattatt tcgggattgc tgcactagat agatttaaaa 3180

acagattgaa ggactatccc cagtattgtc agcatttggc ttctatcagt cactttatgc 3240

aatttccaca tcatttacag gagtatattg agtatggaca gcagtctaga gatcctcctg 3300

tgaaaatgca aggctctatc acaacccctg gaagtattgc actggctcag gcccaggctc 3360

aggcccaggt tccagcaaaa gctcctcttg ctggtcaagt tagcactatg gtaaccacct 3420

caacaactac cactgttgct aaaacggtta cggtcaccag gccaactgga gtcagcttta 3480

agaaagatgt gccacccttct attaatacta caaatataga tacgttgctt gtggccacag 3540

atcaaactga gagaattgtg gagcccccag aaaatatcca ggagaaaatt gcttttattt 3600

tcaataatct ctcacagtca aatatgacac aaaaggttga agagctaaag gaaacggtga 3660

aagaagaatt tatgccttgg gtttcacagt atctggttat gaagagagtc agtattgagc 3720

caaactttca tagcctgtat tcaaacttcc ttgacacgct gaagaatcct gaatttaaca 3780

agatggttct gaatgagacc tacagaaaca ttaaagtgct cctgacctct gataaagctg 3840

cagccaattt ctcagatcgt tctttgctga agaacttggg acattggcta ggaatgatca 3900

cattagctaa aaacaaaccc atcttacaca ctgacttgga tgtgaaatca ttgctgctag 3960

aggcttatgt taaaggacaa caagaattgc tctatgtagt gcccttgtt gccaaagtct 4020

tagaatctag cattcgtagt gtggttttta ggccaccaaa cccttggaca atggcaatta 4080

tgaatgtatt agctgagcta catcaggagc atgacttaaa gttaaacttg aagtttgaaa 4140

tcgaggttct ctgcaagaac cttgcattag acatcaatga gctaaaacct ggaaacctcc 4200

taaaggataa agatcgcctg aagaatttag atgagcaact ctctgctcca aagaaagatg 4260

tcaagcagcc agaagaactc cctcccatca caaccacaac aacttctact acaccagcta 4320

ccaacaccac ttgtacagcc acggttccac cacagccaca gtacagctac cacgacatca 4380

atgtctattc ccttgcgggc ttggcaccac acattactct gaatccaaca attcccttgt 4440

ttcaggccca tccacagttg aagcagtgtg tgcgtcaggc aattgaacgg gctgtccagg 4500

agctggtcca tcctgtggtg gatcgatcaa ttaagattgc catgactact tgtgagcaaa 4560

tagtcaggaa ggattttgcc ctggattcgg aggaatctcg aatgcgaata gcagctcatc 4620

acatgatgcg taacttgaca gctggaatgg ctatgattac atgcagggaa cctttgctca 4680

tgagcatatc taccaactta aaaaacagtt ttgcctcagc ccttcgtact gcttccccac 4740

aacaaagaga aatgatggat caggcagctg ctcaattagc tcaggacaat tgtgagttgg 4800

cttgctgttt tattcagaag actgcagtag aaaaagcagg ccctgagatg gacaagagat 4860
tagcaactga atttgagctg agaaaacatg ctaggcaaga aggacgcaga tactgtgatc 4920
ctgttgtttt aacatatcaa gctgaacgga tgccagagca aatcaggctg aaagttggtg 4980
gtgtggaccc aaagcagttg gctgtttacg aagagtttgc acgcaatgtt cctggcttct 5040
tacctacaaa tgacttaagt cagcccacgg gatttttagc ccagcccatg aagcaagctt 5100
gggcaacaga tgatgtagct cagatttatg ataagtgtat tacagaactg gagcaacatc 5160
tacatgccat cccaccaact ttggccatga accctcaagc tcaggctctt cgaagtctct 5220
tggaggttgt agttttatct cgaaactctc gggatgccat agctgctctt ggattgctcc 5280
aaaaggctgt agagggctta ctagatgcca caagtggtgc tgatgctgac cttctgctgc 5340
gctacaggga atgccacctc ttggtcctaa aagctctgca ggatggccgg gcatatgggt 5400
ctccatggtg caacaaacag atcacaaggt gcctaattga atgtcgagat gaatataaat 5460
ataatgtgga ggctgtggag ctgctaattc gcaatcattt ggttaatatg cagcagtatg 5520
atcttcacct agcgcagtca atggagaatg gcttaaacta catggctgtg gcatttgcta 5580
tgcagttagt aaaaatcctg ctggtggatg aaaggagtgt tgctcatgtt actgaggcag 5640
atctgttcca caccattgaa accctcatga ggattaatgc tcattccaga ggcaatgctc 5700
cagaaggatt gccccagctg atggaagtag tgcgatccaa ctatgaagca atgattgatc 5760
gtgctcatgg aggcccaaac tttatgatgc attctgggat ctctcaagcc tcagagtatg 5820
atgaccctcc aggcctgagg gagaaggcag agtatcttct gagggaatgg gtgaatctct 5880
accattcagc agcagctggc cgcgacagta ccaaagcttt ctctgcattt gttggacaga 5940
tgcaccagca aggaatactg aagaccgatg atctcataac aaggttcttt cgtctgtgta 6000
ctgaaatgtg tgttgaaatc agttaccgtg ctcaggctga gcagcagcac aatcctgctg 6060
ccaatcccac catgatccga gccaagtgct atcacaacct ggatgccttt gttcgactca 6120
ttgcactgct cgtgaaacac tcaggggagg ccaccaacac tgtcacaaag attaatctgc 6180
tgaacaaggt ccttggtata gtagtgggag ttctccttca ggatcatgat gttcgtcaga 6240
gtgaatttca gcaacttccc taccatcgaa tttttatcat gcttctcttg gaactcaatg 6300
cacctgagca tgtgttggaa accattaatt tccagacact tacagctttc tgcaatacat 6360
tccacatctt gaggcctacc aaagctcctg gctttgtata tgcctggctt gaactgattt 6420
cccatcggat atttattgca agaatgctgg cacatacgcc acagcagaag gggtggccta 6480
tgtatgcaca gctactgatt gatttattca aatatttagc gcctttcctt agaaatgtgg 6540
aactcaccaa acctatgcaa atcctctaca agggcacttt aagagtgctg ctggttcttt 6600

tgcatgattt cccagagttc ctttgtgatt accattatgg gttctgtgat gtgatcccac 6660
ctaattgtat ccagttaaga aatttgatcc tgagtgcctt tccaagaaac atgaggctcc 6720
ccgacccatt cactcctaat ctaaaggtgg acatgttgag tgaaattaac attgctcccc 6780
ggattctcac caatttcact ggagtaatgc cacctcagtt caaaaaggat ttggattcct 6840
atcttaaaac tcgatcacca gtcactttcc tgtctgatct gcgcagcaac ctacaggtat 6900
ccaatgaacc tgggaatcgc tacaacctcc agctcatcaa tgcactggtg ctctatgtcg 6960
ggactcaggc cattgcgcac atccacaaca agggcagcac accttcaatg agcaccatca 7020
ctcactcagc acacatggat atcttccaga atttggctgt ggacttggac actgagggtc 7080
gctatctctt tttgaatgca attgcaaatc agctccggta cccaaatagc cacactcact 7140
acttcagttg caccatgctg taccttttg cagaggccaa tacggaagcc atccaagaac 7200
agatcacaag agttctcttg gaacggttga ttgtaaatag gccacatcct tggggtcttc 7260
ttattacctt cattgagctg attaaaaacc cagcgtttaa gttctggaac catgaatttg 7320
tacactgtgc cccagaaatc gaaaagttat tccagtcggt cgcacagtgc tgcatgggac 7380
agaagcaggc ccagcaagta atggaaggga caggtgccag ttagacgaaa ctgcatctct 7440
gttgtacgtg tcagtctaga ggtctcactg caccgagttc ataaactgac tgaagaatcc 7500
tttcagctct tcctgacttt cccagccctt tggtttgtgg gtatctgccc caactactgt 7560
tgggatcagc ctcctgtctt atgtgggcac gttccaaagt ttaaatgcat ttttttgact 7620
cttggccaaa atttagaaga tgctgtgaat atcattttga acttgtgtaa atacatgaaa 7680
gagaaaacct ttgtctggaa cttcttggct ttgtgcaagc tgtgtccaag gcaagtacat 7740
aaactggtac cttgtaatga agaggcagct gatgccatgc acttgtctga gggcatagct 7800
ccatgtcttc tgacattcct ggtgtcccaa agaatagcaa aaagccagtt tgaatattat 7860
gtaacttatt tttttaatgt ggacagggga ccttgaaaat cactaagtta ttaaaaatgt 7920
ggatgtgcta gaattggata tgtccaggaa catgggaagg gctcactatt ggaatcccat 7980
gagtttccat tttgtctcta cccaaacgta ttccaaagct gactgcattt gtaccatctt 8040
atttcttttg gggattatac acctcagccg cctgagatgg gggtcagctc tttatataaa 8100
gggaaaccag accaggccta aagcccaccc ctaccctcac ccccccaatc ctctcctgaa 8160
acttaaaaac agtgggaata taggaaaggg aaccaaatct cattaattaa ttgttctccc 8220
ccattacccc actgaatgaa tggccataca ggctaagctg aataatgaca aagttgaaag 8280
gaccaataca gcccctttta taaggatttt gaatgttttg caaatgtatt ggtccctgtg 8340

ttgtattttg tagccttttc ctgggcttca gctcccctac ttcttgtatg tgtatgcata 8400

ctgtagctaa ccattaaagt catgacacac aaaaaaaaa aaaaaaaaa 8449

<210> 36
<211> 5019
<212> DNA
<213> Homo sapiens

<400> 36

gcgacctccg gcgccatttt gtagagaaac aagcggagtt aaccgaagag ggggtcgagg 60

agagccggag tcggggaccc aggagtttcc tgtgtccagc gctgccggag ccgcctgagg 120

tgccatgttt cagaacagag taagacccct ggtaaagaag aactgaagat attatacaga 180

taccagatat agcctaatta caaagaaagc attaacctgc ctctgaggtg actaaagggg 240

aataatggtg attttgcgcc gggctcggcc gcctgcttcc gccccaacca gcaatgaatc 300

ttgactcgct ctcgctggcc ttgtctcaaa tcagctacct ggtggacaat ttaaccaaga 360

aaaattaccg agccagccag caggaaatac agcatattgt gaatcggcac ggtcctgagg 420

cagacaggca tttattacgc tgcctatttt cgcatgtgga tttcagtggc gatggtaaaa 480

gcagtggcaa agatttccat cagactcagt ttctgattca ggagtgtgcg ttgctgatta 540

caaagccaaa ttttatctcg acgctgtcct atgccattga taatccattg cactatcaga 600

agagtttaaa gcctgcaccc cacttatttg cccagctgag taaagtgctc aaattaagca 660

aagtacaaga ggtaattttt ggccttgccc tgttgaattc ttccagctca gatcttagag 720

gtttcgctgc ccagtttatc aaacagaagc ttccagatct tctgcgttct tacattgacg 780

cagacgtcag tggaaatcaa gaaggtggct tccaagatat agcaatagag gtcctacacc 840

tcctcctctc ccatctcctc tttgggcaga agggagcctt tggagttgga caagaacaga 900

tagacgcttt tcttaagacg ctgcgcagag attttcccca agaacgctgt cccgtggtgc 960

tcgcaccact tttataccct gaaaaacggg acattctaat ggacaggatc ctgcctgatt 1020

ccggaggggt agctaaaacc atgatggaga gctctttggc tgatttcatg caagaagtag 1080

gctatggctt ttgtgcaagt attgaagaat gtcgcaatat aatcgtgcag tttggtgttc 1140

gggaggtcac agctgcccag gttgcaaggg ttttgggaat gatggctcga actcattcag 1200

gattaacaga tggcattcca ttacagagta tttctgctcc gggcagtggg atctggagtg 1260

atgggaaaga taaaagtgat ggagcacagg cacacacatg gaatgtagaa gtcttgattg 1320

acgttcttaa agaactgaat ccaagtttga atttcaagga agtaacttat gaactggacc 1380

atcctggatt tcaaattcgt gacagtaaag gacttcataa tgtggtttat ggcattcaga 1440

gggtttggg tatggaagtg ttcccagtag acctcatata tagaccttgg aaacatgctg 1500
aaggccagct ctccttcatt caacattccc ttataaatcc agagatcttc tgttttgctg 1560
actatccctg tcatactgtt gccactgata ttctgaaagc accaccagag gatgacaatc 1620
gagaaattgc cacatggaag agcttggatt tgattgaatc tctgctgagg cttgcagagg 1680
ttgggcagta tgagcaagtc aaacagctct tcagcttccc tatcaaacac tgtccagaca 1740
tgctggtatt ggccttacta caaattaaca cctcttggca taccttgcgc catgaactta 1800
tctccactct gatgccaatt ttccttggaa accatcctaa ctcagctatt attttgcact 1860
atgcatggca tgggcaggga cagtctccct caattcgcca acttatcatg catgcaatgg 1920
cagaatggta catgagaggg gagcagtatg atcaggccaa attgtctcga atacttgatg 1980
tggcccagga cttgaaggcc ttgtcaatgc tgctaaatgg tactccattt gcctttgtta 2040
ttgaccttgc tgcacttgct tcacgtcgtg aatacctcaa acttgataag tggctcacag 2100
ataaaattcg agagcatggg gagcctttta tccaggcgtg tatgactttt ttaaagagac 2160
ggtgtccttc tattttgggc ggacttgccc cagaaaaaga ccagcccaaa agtgctcaac 2220
ttcctccaga aactttggcg acaatgttgg cctgtctgca agcttgtgca gggagtgttt 2280
ctcaggagct atcagaaact atcctcacca tggtagccaa ttgcagtaat gttatgaata 2340
aggccagaca accaccacct ggagttatgc caaaaggacg tcctcctagt gctagcagct 2400
tagatgccat ttctcctgtt cagattgacc ctcttgctgg aatgacatct cttagtatag 2460
gtggttcagc tgcccctcac acccagagta tgcagggttt tcctccaaat ttgggttctg 2520
cattcagtac ccctcagtca ccagcaaaag catttccacc cctttcaacc cccaatcaga 2580
ccactgcatt cagtggtatt ggaggacttt catcacagct tccagtaggt ggtcttggca 2640
caggcagcct gactggtata ggaactggtg ctcttggact ccctgcagtg aataacgacc 2700
cttttgtaca gaggaaactg ggcacctctg gactgaatca gcctacattc cagcagagta 2760
agatgaaacc ttcggacttg tctcaggtgt ggccagaggc aaaccagcac tttagtaaag 2820
agatagatga tgaagcaaac agctatttcc agcgaatata taatcatcca ccacatccaa 2880
ccatgtctgt tgatgaggta ttagaaatgc tgcagagatt taaagactct actataaaga 2940
gggaacgaga agtatttaac tgtatgctaa ggaacttgtt tgaagaatat cgtttttttc 3000
cccagtatcc tgataaagag ttacatataa cagcctgcct atttggtggt ataattgaga 3060
aaggactggt cacttacatg gcactaggtc tggctctacg atatgttctt gaagccttac 3120
gcaagccttt tggatccaaa atgtattatt tcgggattgc tgcactagat agatttaaaa 3180
acagattgaa ggactatccc cagtattgtc agcatttggc ttctatcagt cactttatgc 3240

aatttccaca tcatttacag gagtatattg agtatggaca gcagtctaga gatcctcctg 3300
tgaaaatgca aggctctatc acaacccctg gaagtattgc actggctcag gcccaggctc 3360
aggcccaggt tccagcaaaa gctcctcttg ctggtcaagt tagcactatg gtaaccacct 3420
caacaactac cactgttgct aaaacggtta cggtcaccag gccaactgga gtcagcttta 3480
agaaagatgt gccaccttct attaatacta caaatataga tacgttgctt gtggccacag 3540
atcaaactga gagaattgtg gagcccccag aaaatatcca ggagaaaatt gcttttattt 3600
tcaataatct ctcacagtca aatatgacac aaaaggttga agagctaaag gaaacggtga 3660
aagaagaatt tatgccttgg gtttcacagt atctggttat gaagagagtc agtattgagc 3720
caaactttca tagcctgtat tcaaacttcc ttgacacgct gaagaatcct gaatttaaca 3780
agatggttct gaatgagacc tacagaaaca ttaaagtgct cctgacctct gataaagctg 3840
cagccaattt ctcagatcgt tctttgctga agaacttggg acattggcta ggaatgatca 3900
cattagctaa aaacaaaccc atcttacaca ctgacttgga tgtgaaatca ttgctgctag 3960
aggcttatgt taaaggacaa caagaattgc tctatgtagt gcccttttgtt gccaaagtct 4020
tagaatctag cattcgtagt gtggttttta ggccaccaaa cccttggaca atggcaatta 4080
tgaatgtatt agctgagcta catcaggagc atgacttaaa gttaaacttg aagtttgaaa 4140
tcgaggttct ctgcaagaac cttgcattag acatcaatga gctaaaacct ggaaacctcc 4200
taaaggataa agatcgcctg aagaatttag atgagcaact ctctgctcca aagaaagatg 4260
tcaagcagcc agaagaactc cctcccatca caaccacaac aacttctact acaccagcta 4320
ccaacaccac ttgtacagcc acggttccac cacagccaca gtacagctac cacgacatca 4380
atgtctattc ccttgcgggc ttggcaccac acattactct gaatccaaca attcccttgt 4440
ttcaggccca tccacagttg aagcagtgtg tgcgtcaggc aattgaacgg gctgtccagg 4500
agctggtcca tcctgtggtg gatcgatcaa ttaagattgc catgactact tgtgagcaaa 4560
tagtcaggaa ggattttgcc ctggattcgg aggaatctcg aatgcgaata gcagctcatc 4620
acatgatgcg taacttgaca gctggaatgg ctatgattac atgcagggaa cctttgctca 4680
tgagcatatc taccaactta aaaaacagtt ttgcctcagc ccttcgtgta agttggctat 4740
ttccttggta taggtacaaa acgtattact gcttgtctgt aataattttt ttctttgtct 4800
atatatggca ctgggcgtta ccacttattc ttaataatca ccatatttgt ttgatgtctt 4860
ccatcatttt agattgtaat tctgtgaggc aaagcatcat gtctgtgtgt tttttttttt 4920
ttctgttata ttctcaacac gatgtttgac agatagtaga tacccaaata tttgttggtt 4980

taaataaatt aagatttaaa aaggaaaaaa aaaaaaaaa 5019

<210> 37
<211> 1616
<212> DNA
<213> Homo sapiens

<400> 37

```
gcgcacgtcc cggattcctc ccacgagggg gcgggctgcg gccaaatctc ccgccaggtc      60
agcggccggg cgctgattgg ccccatggcg gcggggccgg ctcgtgattg gccagcacgc     120
cgtggtttaa agcggtcggc gcgggaacca ggggcttact gcgggacggc cttggagagt     180
actcgggttc gtgaacttcc cggaggcgca atgagctgca ttaacctgcc cactgtgctg     240
cctggctccc ccagcaagac ccgggggcag atccaggtga ttctcgggcc gatgttctca     300
ggaaaaagca cagagttgat gagacgcgtc cgtcgcttcc agattgctca gtacaagtgc     360
ctggtgatca agtatgccaa agacactcgc tacagcagca gcttctgcac acatgaccgg     420
aacaccatgg aggcactgcc cgcctgcctg ctccgagacg tggcccagga ggccctgggc     480
gtggctgtca taggcatcga cgaggggcag tttttccctg acatcgtgga gttctgcgag     540
gccatggcca acgccgggaa gaccgtaatt gtggctgcac tggatgggac cttccagagg     600
aagccatttg ggccatcct  gaacctggtg ccgctggccg agagcgtggt gaagctgacg     660
gcggtgtgca tggagtgctt ccgggaagcc gcctatacca agaggctcgg cacagagaag     720
gaggtcgagg tgattggggg agcagacaag taccactccg tgtgtcggct ctgctacttc     780
aagaaggcct caggccagcc tgccgggccg gacaacaaag agaactgccc agtgccagga     840
aagccagggg aagccgtggc tgccaggaag ctctttgccc cacagcagat tctgcaatgc     900
agccctgcca actgagggac ctgcgagggc cgcccgctcc cttcctgcca ctgccgccta     960
ctggacgctg ccctgcatgc tgcccagcca ctccaggagg aagtcgggag gcgtggaggg    1020
tgaccacacc ttggccttct gggaactctc ctttgtgtgg ctgccccacc tgccgcatgc    1080
tccctcctct cctacccact ggtctgctta aagcttccct ctcagctgct gggacgatcg    1140
cccaggctgg agctggcccc gcttggtggc ctgggatctg gcacactccc tctccttggg    1200
gtgagggaca gagccccacg ctgttgacat cagcctgctt cttcccctct gcggctttca    1260
ctgctgagtt tctgttctcc ctgggaagcc tgtgccagca cctttgagcc ttggcccaca    1320
ctgaggctta ggcctctctg cctgggatgg gctcccaccc tcccctgagg atggcctgga    1380
ttcacgccct cttgtttcct tttgggctca aagcccttcc tacctctggt gatggtttcc    1440
acaggaacaa cagcatcttt caccaagatg ggtggcacca accttgctgg gacttggatc    1500

ccaggggctt atctcttcaa gtgtggagag ggcagggtcc acgcctctgc tgtagcttat    1560
gaaattaact aattgaaaat tcactggttg gtggaaaaaa aaaaaaaaaa aaaaaa        1616
```

<210> 38
<211> 3839

<212> DNA
<213> Homo sapiens

<400> > 38

```
agacaggcgg agcctcacct ggggctgccc gccagcccag acaagctcag actgggtgcc     60
tgtggccctg ggaggaggtg gaaggggagg agcaggccac acaggcacag gccggtgagg    120
gacctgccca gacctggagg gtctcgctct gtcacacagg ctggagtgca gtggtgtgat    180
cttggctcat cgtaacctcc acctcccggg ttcaagtgat tctcatgcct cagcctcccg    240
agtagctggg attacaggtg gtgacttcca agagtgactc cgtcggagga aaatgactcc    300
ccagtcgctg ctgcagacga cactgttcct gctgagtctg ctcttcctgg tccaaggtgc    360
ccacggcagg ggccacaggg aagactttcg cttctgcagc cagcggaacc agacacacag    420
gagcagcctc cactacaaac ccacaccaga cctgcgcatc tccatcgaga actccgaaga    480
ggccctcaca gtccatgccc ctttccctgc agcccacccct gcttcccgat ccttccctga    540
ccccaggggc ctctaccact tctgcctcta ctggaaccga catgctggga gattacatct    600
tctctatggc aagcgtgact tcttgctgag tgacaaagcc tctagcctcc tctgcttcca    660
gcaccaggag gagagcctgg ctcagggccc cccgctgtta gccacttctg tcacctcctg    720
gtggagccct cagaacatca gcctgcccag tgccgccagc ttcaccttct ccttccacag    780
tcctccccac acggccgctc acaatgcctc ggtggacatg tgcgagctca aaagggacct    840
ccagctgctc agccagttcc tgaagcatcc ccagaaggcc tcaaggaggc cctcggctgc    900
ccccgccagc cagcagttgc agagcctgga gtcgaaactg acctctgtga gattcatggg    960
ggacatggtg tccttcgagg aggaccggat caacgccacg gtgtggaagc tccagcccac   1020
agccggcctc caggacctgc acatccactc ccggcaggag gaggagcaga gcgagatcat   1080
ggagtactcg gtgctgctgc ctcgaacact cttccagagg acgaaaggcc ggagcgggga   1140
ggctgagaag agactcctcc tggtggactt cagcagccaa gccctgttcc aggacaagaa   1200
ttccagccaa gtcctgggtg agaaggtctt ggggattgtg gtacagaaca ccaaagtagc   1260
caacctcacg gagcccgtgg tgctcacttt ccagcaccag ctacagccga agaatgtgac   1320
tctgcaatgt gtgttctggg ttgaagaccc cacattgagc agcccggggc attggagcag   1380
tgctgggtgt gagaccgtca ggagagaaac ccaaacatcc tgcttctgca accacttgac   1440
```

```
ctactttgca gtgctgatgg tctcctcggt ggaggtggac gccgtgcaca agcactacct   1500
gagcctcctc tcctacgtgg gctgtgtcgt ctctgccctg gcctgccttg tcaccattgc   1560
cgcctacctc tgctccaggg tgcccctgcc gtgcaggagg aaacctcggg actacaccat   1620
caaggtgcac atgaacctgc tgctggccgt cttcctgctg gacacgagct tcctgctcag   1680
cgagccggtg gccctgacag gctctgaggc tggctgccga gccagtgcca tcttcctgca   1740
cttctccctg ctcacctgcc tttcctggat gggcctcgag gggtacaacc tctaccgact   1800
cgtggtggag gtctttggca cctatgtccc tggctaccta ctcaagctga gcgccatggg   1860
ctggggcttc cccatctttc tggtgacgct ggtggccctg gtggatgtgg acaactatgg   1920
ccccatcatc ttggctgtgc ataggactcc agagggcgtc atctaccctt ccatgtgctg   1980
gatccgggac tccctggtca gctacatcac caacctgggc ctcttcagcc tggtgtttct   2040
gttcaacatg gccatgctag ccaccatggt ggtgcagatc ctgcggctgc gcccccacac   2100
ccaaaagtgg tcacatgtgc tgacactgct gggcctcagc ctggtccttg gcctgccctg   2160
ggccttgatc ttcttctcct ttgcttctgg caccttccag cttgtcgtcc tctacctttt   2220
cagcatcatc acctccttcc aaggcttcct catcttcatc tggtactggt ccatgcggct   2280
gcaggcccgg ggtggcccct cccctctgaa gagcaactca gacagcgcca ggctccccat   2340
cagctcgggc agcacctcgt ccagccgcat ctaggcctcc agcccacctg cccatgtgat   2400
gaagcagaga ttcggcctcg tcgcacactg cctgtggccc ccgagcccgg cccagcccca   2460
ggccagtcag ccgcagactt tggaaagccc aacgaccatg gagagatggg ccgttgccat   2520
ggtggacgga ctcccgggct gggcttttga attggccttg gggactactc ggctctcact   2580
cagctcccac gggactcaga agtgcgccgc catgctgcct agggtactgt ccccacatct   2640
gtcccaaccc agctggaggc ctggtctctc cttacaaccc ctgggcccag ccctcattgc   2700
tgggggccag gccttggatc ttgagggtct ggcacatcct taatcctgtg cccctgcctg   2760
ggacagaaat gtggctccag ttgctctgtc tctcgtggtc accctgaggg cactctgcat   2820
cctctgtcat ttaacctca ggtggcaccc agggcgaatg gggcccaggg cagaccttca   2880
gggccagagc cctggcggag gagaggccct ttgccaggag cacagcagca gctcgcctac   2940
ctctgagccc aggccccctc cctccctcag ccccccagtc ctccctccat cttccctggg   3000
gttctcctcc tctcccaggg cctccttgct ccttcgttca cagctggggg tccccgattc   3060
caatgctgtt ttttggggag tggtttccag gagctgcctg gtgtctgctg taaatgtttg   3120
tctactgcac aagcctcggc ctgcccctga gccaggctcg gtaccgatgc gtgggctggg   3180
ctaggtccct ctgtccatct gggcctttgt atgagctgca ttgcccttgc tcaccctgac   3240
```

caagcacacg cctcagaggg gccctcagcc tctcctgaag ccctcttgtg gcaagaactg 3300

tggaccatgc cagtcccgtc tggtttccat cccaccactc caaggactga gactgacctc 3360

ctctggtgac actggcctag ggcctgacac tctcctaaga ggttctctcc aagcccccaa 3420

atagctccag gcgccctcgg ccgcccatca tggttaattc tgtccaacaa acacacacgg 3480

gtagattgct ggcctgttgt aggtggtagg gacacagatg accgacctgg tcactcctcc 3540

tgccaacatt cagtctggta tgtgaggcgt gcgtgaagca agaactcctg gagctacagg 3600

gacagggagc catcattcct gcctgggaat cctggaagac ttcctgcagg agtcagcgtt 3660

caatcttgac cttgaagatg ggaaggatgt tctttttacg taccaattct tttgtctttt 3720

gatattaaaa agaagtacat gttcattgta gagaatttgg aaactgtaga agagaatcaa 3780

gaagaaaaat aaaaatcagc tgttgtaatc acctagcaaa ctggaaaaaa aaaaaaaaa 3839

<210> 39
<211> 3982
<212> DNA
<213> Homo sapiens

<400> 39

gagaaatcca tgactcacat atatcgtccg tcatccccag tcaagctcag ggaggaggga 60

ggagcccagc tggggcagga gaggggtgtc tccccaccaa acaaacccgg tccctccctc 120

tccgcactag ctgtctgccc tgccctgccg taggagatgg gctgggagcc tcccacgctc 180

tccagctcac tcggcaggca gcggggacca gggctggcag gttaagcctc tgggggtgga 240

tcctgaaagg tggtccagcc gcctggccct gcgtgggacc ctccacctgg cagcagacag 300

ggtctcgctc tgtcacacag gctggagtgc agtggtgtga tcttggctca tcgtaacctc 360

cacctcccgg gttcaagtga ttctcatgcc tcagcctccc gagtagctgg gattacaggt 420

ggtgacttcc aagagtgact ccgtcggagg aaaatgactc cccagtcgct gctgcagacg 480

acactgttcc tgctgagtct gctcttcctg gtccaaggtg cccacggcag ggccacagg 540

gaagactttc gcttctgcag ccagcggaac cagacacaca ggagcagcct ccactacaaa 600

cccacaccag acctgcgcat ctccatcgag aactccgaag aggccctcac agtccatgcc 660

cctttccctg cagcccaccc tgcttcccga tccttccctg accccagggg cctctaccac 720

ttctgcctct actggaaccg acatgctggg agattacatc ttctctatgg caagcgtgac 780

ttcttgctga gtgacaaagc ctctagcctc ctctgcttcc agcaccagga ggagagcctg 840

gctcagggcc ccccgctgtt agccacttct gtcacctcct ggtggagccc tcagaacatc 900

agcctgccca gtgccgccag cttcaccttc tccttccaca gtcctcccca cacggccgct 960

cacaatgcct cggtggacat gtgcgagctc aaaagggacc tccagctgct cagccagttc 1020
ctgaagcatc cccagaaggc ctcaaggagg ccctcggctg cccccgccag ccagcagttg 1080
cagagcctgg agtcgaaact gacctctgtg agattcatgg gggacatggt gtccttcgag 1140
gaggaccgga tcaacgccac ggtgtggaag ctccagccca cagccggcct ccaggacctg 1200
cacatccact cccggcagga ggaggagcag agcgagatca tggagtactc ggtgctgctg 1260
cctcgaacac tcttccagag gacgaaaggc cggagcgggg aggctgagaa gagactcctc 1320
ctggtggact tcagcagcca agccctgttc caggacaaga attccagcca agtcctgggt 1380
gagaaggtct tggggattgt ggtacagaac accaaagtag ccaacctcac ggagcccgtg 1440
gtgctcactt tccagcacca gctacagccg aagaatgtga ctctgcaatg tgtgttctgg 1500
gttgaagacc ccacattgag cagcccgggg cattggagca gtgctgggtg tgagaccgtc 1560
aggagagaaa cccaaacatc ctgcttctgc aaccacttga cctactttgc agtgctgatg 1620
gtctcctcgg tggaggtgga cgccgtgcac aagcactacc tgagcctcct ctcctacgtg 1680
ggctgtgtcg tctctgccct ggcctgcctt gtcaccattg ccgcctacct ctgctccagg 1740
aggaaacctc gggactacac catcaaggtg cacatgaacc tgctgctggc cgtcttcctg 1800
ctggacacga gcttcctgct cagcgagccg gtggccctga caggctctga ggctggctgc 1860
cgagccagtg ccatcttcct gcacttctcc ctgctcacct gcctttcctg gatgggcctc 1920
gaggggtaca acctctaccg actcgtggtg gaggtctttg gcacctatgt ccctggctac 1980
ctactcaagc tgagcgccat gggctggggc ttccccatct ttctggtgac gctggtggcc 2040
ctggtggatg tggacaacta tggccccatc atcttggctg tgcataggac tccagagggc 2100
gtcatctacc cttccatgtg ctggatccgg gactccctgg tcagctacat caccaacctg 2160
ggcctcttca gcctggtgtt tctgttcaac atggccatgc tagccaccat ggtggtgcag 2220
atcctgcggc tgcgccccca cacccaaaag tggtcacatg tgctgacact gctgggcctc 2280
agcctggtcc ttggcctgcc ctgggccttg atcttcttct cctttgcttc tggcaccttc 2340
cagcttgtcg tcctctacct tttcagcatc atcacctcct tccaaggctt cctcatcttc 2400
atctggtact ggtccatgcg gctgcaggcc cggggtggcc cctcccctct gaagagcaac 2460
tcagacagcg ccaggctccc catcagctcg ggcagcacct cgtccagccg catctaggcc 2520
tccagcccac ctgcccatgt gatgaagcag agattcggcc tcgtcgcaca ctgcctgtgg 2580
cccccgagcc cggcccagcc ccaggccagt cagccgcaga ctttggaaag cccaacgacc 2640
atggagagat gggccgttgc catggtggac ggactcccgg gctgggcttt tgaattggcc 2700

ttggggacta ctcggctctc actcagctcc cacgggactc agaagtgcgc cgccatgctg 2760

cctagggtac tgtccccaca tctgtcccaa cccagctgga ggcctggtct ctccttacaa 2820

cccctgggcc cagccctcat tgctgggggc caggccttgg atcttgaggg tctggcacat 2880

ccttaatcct gtgcccctgc ctgggacaga aatgtggctc cagttgctct gtctctcgtg 2940

gtcaccctga gggcactctg catcctctgt cattttaacc tcaggtggca cccagggcga 3000

atggggccca gggcagacct tcagggccag agccctggcg gaggagaggc cctttgccag 3060

gagcacagca gcagctcgcc tacctctgag cccaggcccc ctccctccct cagcccccca 3120

gtcctccctc catcttccct ggggttctcc tcctctccca gggcctcctt gctccttcgt 3180

tcacagctgg gggtccccga ttccaatgct gtttttgggg gagtggtttc caggagctgc 3240

ctggtgtctg ctgtaaatgt ttgtctactg cacaagcctc ggcctgcccc tgagccaggc 3300

tcggtaccga tgcgtgggct gggctaggtc cctctgtcca tctgggcctt tgtatgagct 3360

gcattgccct tgctcaccct gaccaagcac acgcctcaga ggggccctca gcctctcctg 3420

aagccctctt gtggcaagaa ctgtggacca tgccagtccc gtctggtttc catcccacca 3480

ctccaaggac tgagactgac ctcctctggt gacactggcc tagggcctga cactctccta 3540

agaggttctc tccaagcccc caaatagctc caggcgccct cggccgccca tcatggttaa 3600

ttctgtccaa caaacacaca cgggtagatt gctggcctgt tgtaggtggt agggacacag 3660

atgaccgacc tggtcactcc tcctgccaac attcagtctg gtatgtgagg cgtgcgtgaa 3720

gcaagaactc ctggagctac agggacaggg agccatcatt cctgcctggg aatcctggaa 3780

gacttcctgc aggagtcagc gttcaatctt gaccttgaag atgggaagga tgttcttttt 3840

acgtaccaat tcttttgtct tttgatatta aaaagaagta catgttcatt gtagagaatt 3900

tggaaactgt agaagagaat caagaagaaa aataaaaatc agctgttgta atcacctagc 3960

aaactggaaa aaaaaaaaaa aa 3982

<210> 40
<211> 3860
<212> DNA
<213> Homo sapiens

<400> 40

gagaaatcca tgactcacat atatcgtccg tcatccccag tcaagctcag ggaggaggga 60

ggagcccagc tggggcagga gaggggtgtc tccccaccaa acaaacccgg tccctccctc 120

tccgcactag ctgtctgccc tgccctgccg taggagatgg gctgggagcc tcccacgctc 180

tccagctcac tcggcaggca gcggggacca gggctggcag gttaagcctc tgggggtgga 240

tcctgaaagg tggtccagcc gcctggccct gcgtgggacc ctccacctgg cagcaggtgg 300
tgacttccaa gagtgactcc gtcggaggaa aatgactccc cagtcgctgc tgcagacgac 360
actgttcctg ctgagtctgc tcttcctggt ccaaggtgcc cacggcaggg gccacaggga 420
agactttcgc ttctgcagcc agcggaacca gacacacagg agcagcctcc actacaaacc 480
cacaccagac ctgcgcatct ccatcgagaa ctccgaagag gccctcacag tccatgcccc 540
tttccctgca gcccaccctg cttcccgatc cttccctgac cccaggggcc tctaccactt 600
ctgcctctac tggaaccgac atgctgggag attacatctt ctctatggca agcgtgactt 660
cttgctgagt gacaaagcct ctagcctcct ctgcttccag caccaggagg agagcctggc 720
tcagggcccc ccgctgttag ccacttctgt cacctcctgg tggagccctc agaacatcag 780
cctgcccagt gccgccagct tcaccttctc cttccacagt cctccccaca cggccgctca 840
caatgcctcg gtggacatgt gcgagctcaa aagggacctc cagctgctca gccagttcct 900
gaagcatccc cagaaggcct caaggaggcc ctcggctgcc cccgccagcc agcagttgca 960
gagcctggag tcgaaactga cctctgtgag attcatgggg gacatggtgt ccttcgagga 1020
ggaccggatc aacgccacgg tgtggaagct ccagcccaca gccggcctcc aggacctgca 1080
catccactcc cggcaggagg aggagcagag cgagatcatg gagtactcgg tgctgctgcc 1140
tcgaacactc ttccagagga cgaaaggccg gagcggggag gctgagaaga gactcctcct 1200
ggtggacttc agcagccaag ccctgttcca ggacaagaat tccagccaag tcctgggtga 1260
gaaggtcttg ggattgtgg tacagaacac caaagtagcc aacctcacgg agcccgtggt 1320
gctcactttc cagcaccagc tacagccgaa gaatgtgact ctgcaatgtg tgttctgggt 1380
tgaagacccc acattgagca gcccggggca ttggagcagt gctgggtgtg agaccgtcag 1440
gagagaaacc caaacatcct gcttctgcaa ccacttgacc tactttgcag tgctgatggt 1500
ctcctcggtg gaggtggacg ccgtgcacaa gcactacctg agcctcctct cctacgtggg 1560
ctgtgtcgtc tctgccctgg cctgccttgt caccattgcc gcctacctct gctccaggag 1620
gaaacctcgg gactacacca tcaaggtgca catgaacctg ctgctggccg tcttcctgct 1680
ggacacgagc ttcctgctca gcgagccggt ggccctgaca ggctctgagg ctggctgccg 1740
agccagtgcc atcttcctgc acttctccct gctcacctgc ctttcctgga tgggcctcga 1800
ggggtacaac ctctaccgac tcgtggtgga ggtctttggc acctatgtcc ctggctacct 1860
actcaagctg agcgccatgg gctggggctt ccccatcttt ctggtgacgc tggtggccct 1920
ggtggatgtg gacaactatg gccccatcat cttggctgtg cataggactc cagagggcgt 1980
catctaccct tccatgtgct ggatccggga ctccctggtc agctacatca ccaacctggg 2040

cctcttcagc ctggtgtttc tgttcaacat ggccatgcta gccaccatgg tggtgcagat 2100
cctgcggctg cgcccccaca cccaaaagtg gtcacatgtg ctgacactgc tgggcctcag 2160
cctggtcctt ggcctgccct gggccttgat cttcttctcc tttgcttctg gcaccttcca 2220
gcttgtcgtc ctctaccttt tcagcatcat cacctccttc caaggcttcc tcatcttcat 2280
ctggtactgg tccatgcggc tgcaggcccg gggtggcccc tcccctctga agagcaactc 2340
agacagcgcc aggctcccca tcagctcggg cagcacctcg tccagccgca tctaggcctc 2400
cagcccacct gcccatgtga tgaagcagag attcggcctc gtcgcacact gcctgtggcc 2460
cccgagcccg gcccagcccc aggccagtca gccgcagact ttggaaagcc caacgaccat 2520
ggagagatgg gccgttgcca tggtggacgg actcccgggc tgggcttttg aattggcctt 2580
ggggactact cggctctcac tcagctccca cgggactcag aagtgcgccg ccatgctgcc 2640
tagggtactg tccccacatc tgtcccaacc cagctggagg cctggtctct ccttacaacc 2700
cctgggccca gccctcattg ctgggggcca ggccttggat cttgagggtc tggcacatcc 2760
ttaatcctgt gcccctgcct gggacagaaa tgtggctcca gttgctctgt ctctcgtggt 2820
caccctgagg gcactctgca tcctctgtca ttttaacctc aggtggcacc cagggcgaat 2880
ggggcccagg gcagaccttc agggccagag ccctggcgga ggagaggccc tttgccagga 2940
gcacagcagc agctcgccta cctctgagcc caggccccct ccctccctca gcccccagt 3000
cctccctcca tcttccctgg ggttctcctc ctctcccagg gcctccttgc tccttcgttc 3060
acagctgggg gtccccgatt ccaatgctgt tttttgggga gtggtttcca ggagctgcct 3120
ggtgtctgct gtaaatgttt gtctactgca caagcctcgg cctgcccctg agccaggctc 3180
ggtaccgatg cgtgggctgg gctaggtccc tctgtccatc tgggcctttg tatgagctgc 3240
attgcccttg ctcaccctga ccaagcacac gcctcagagg ggccctcagc ctctcctgaa 3300
gccctcttgt ggcaagaact gtggaccatg ccagtcccgt ctggtttcca tcccaccact 3360
ccaaggactg agactgacct cctctggtga cactggccta gggcctgaca ctctcctaag 3420
aggttctctc caagccccca aatagctcca ggcgccctcg gccgcccatc atggttaatt 3480
ctgtccaaca aacacacacg ggtagattgc tggcctgttg taggtggtag ggacacagat 3540
gaccgacctg gtcactcctc ctgccaacat tcagtctggt atgtgaggcg tgcgtgaagc 3600
aagaactcct ggagctacag ggacagggag ccatcattcc tgcctgggaa tcctggaaga 3660
cttcctgcag gagtcagcgt tcaatcttga ccttgaagat gggaaggatg ttctttttac 3720
gtaccaattc ttttgtcttt tgatattaaa aagaagtaca tgttcattgt agagaatttg 3780

gaaactgtag aagagaatca agaagaaaaa taaaaatcag ctgttgtaat cacctagcaa 3840

actggaaaaa aaaaaaaaaa 3860

<210> 41
<211> 3996
<212> DNA
<213> Homo sapiens

<400> 41

gagaaatcca tgactcacat atatcgtccg tcatccccag tcaagctcag ggaggaggga 60
ggagcccagc tggggcagga gaggggtgtc tccccaccaa acaaacccgg tccctccctc 120
tccgcactag ctgtctgccc tgccctgccg taggagatgg gctgggagcc tcccacgctc 180
tccagctcac tcggcaggca gcggggacca gggctggcag gttaagcctc tgggggtgga 240
tcctgaaagg tggtccagcc gcctggccct gcgtgggacc ctccacctgg cagcagggtc 300
tcgctctgtc acacaggctg gagtgcagtg gtgtgatctt ggctcatcgt aacctccacc 360
tcccgggttc aagtgattct catgcctcag cctcccgagt agctgggatt acaggtggtg 420
acttccaaga gtgactccgt cggaggaaaa tgactcccca gtcgctgctg cagacgacac 480
tgttcctgct gagtctgctc ttcctggtcc aaggtgccca cggcaggggc cacagggaag 540
actttcgctt ctgcagccag cggaaccaga cacacaggag cagcctccac tacaaaccca 600
caccagacct gcgcatctcc atcgagaact ccgaagaggc cctcacagtc catgcccctt 660
tccctgcagc ccaccctgct tcccgatcct tccctgaccc caggggcctc taccacttct 720
gcctctactg gaaccgacat gctgggagat tacatcttct ctatggcaag cgtgacttct 780
tgctgagtga caaagcctct agcctcctct gcttccagca ccaggaggag agcctggctc 840
agggcccccc gctgttagcc acttctgtca cctcctggtg gagccctcag aacatcagcc 900
tgcccagtgc cgccagcttc accttctcct tccacagtcc tccccacacg gccgctcaca 960
atgcctcggt ggacatgtgc gagctcaaaa gggacctcca gctgctcagc cagttcctga 1020
agcatcccca gaaggcctca aggaggccct cggctgcccc cgccagccag cagttgcaga 1080
gcctggagtc gaaactgacc tctgtgagat tcatgggga catggtgtcc ttcgaggagg 1140
accggatcaa cgccacggtg tggaagctcc agcccacagc cggcctccag gacctgcaca 1200
tccactcccg gcaggaggag gagcagagcg agatcatgga gtactcggtg ctgctgcctc 1260
gaacactctt ccagaggacg aaaggccgga gcggggaggc tgagaagaga ctcctcctgg 1320
tggacttcag cagccaagcc ctgttccagg acaagaattc cagccaagtc ctgggtgaga 1380
aggtcttggg gattgtggta cagaacacca aagtagccaa cctcacggag cccgtggtgc 1440

```
tcactttcca gcaccagcta cagccgaaga atgtgactct gcaatgtgtg ttctgggttg   1500
aagaccccac attgagcagc ccggggcatt ggagcagtgc tgggtgtgag accgtcagga   1560
gagaaaccca aacatcctgc ttctgcaacc acttgaccta ctttgcagtg ctgatggtct   1620
cctcggtgga ggtggacgcc gtgcacaagc actacctgag cctcctctcc tacgtgggct   1680
gtgtcgtctc tgccctggcc tgccttgtca ccattgccgc ctacctctgc tccagggtgc   1740
ccctgccgtg caggaggaaa cctcgggact acaccatcaa ggtgcacatg aacctgctgc   1800
tggccgtctt cctgctggac acgagcttcc tgctcagcga gccggtggcc ctgacaggct   1860
ctgaggctgg ctgccgagcc agtgccatct tcctgcactt ctccctgctc acctgccttt   1920
cctggatggg cctcgagggg tacaacctct accgactcgt ggtggaggtc tttggcacct   1980
atgtccctgg ctacctactc aagctgagcg ccatgggctg gggcttcccc atctttctgg   2040
tgacgctggt ggccctggtg gatgtggaca actatggccc catcatcttg gctgtgcata   2100
ggactccaga gggcgtcatc taccctccca tgtgctggat ccgggactcc ctggtcagct   2160
acatcaccaa cctgggcctc ttcagcctgg tgtttctgtt caacatggcc atgctagcca   2220
ccatggtggt gcagatcctg cggctgcgcc cccacaccca aaagtggtca catgtgctga   2280
cactgctggg cctcagcctg gtccttggcc tgccctgggc cttgatcttc ttctcctttg   2340
cttctggcac cttccagctt gtcgtcctct accttttcag catcatcacc tccttccaag   2400
gcttcctcat cttcatctgg tactggtcca tgcggctgca ggcccggggt ggcccctccc   2460
ctctgaagag caactcagac agcgccaggc tccccatcag ctcgggcagc acctcgtcca   2520
gccgcatcta ggcctccagc ccacctgccc atgtgatgaa gcagagattc ggcctcgtcg   2580
cacactgcct gtggcccccg agcccggccc agccccaggc cagtcagccg cagactttgg   2640
aaagcccaac gaccatggag agatgggccg ttgccatggt ggacggactc ccgggctggg   2700
cttttgaatt ggccttgggg actactcggc tctcactcag ctcccacggg actcagaagt   2760
gcgccgccat gctgcctagg gtactgtccc cacatctgtc ccaacccagc tggaggcctg   2820
gtctctcctt acaacccctg ggcccagccc tcattgctgg gggccaggcc ttggatcttg   2880
agggtctggc acatccttaa tcctgtgccc ctgcctggga cagaaatgtg gctccagttg   2940
ctctgtctct cgtggtcacc ctgagggcac tctgcatcct ctgtcatttt aacctcaggt   3000
ggcacccagg gcgaatgggg cccagggcag accttcaggg ccagagccct ggcggaggag   3060
aggccctttg ccaggagcac agcagcagct cgcctacctc tgagcccagg ccccctccct   3120
ccctcagccc cccagtcctc cctccatctt ccctggggtt ctcctcctct cccagggcct   3180
ccttgctcct tcgttcacag ctgggggtcc ccgattccaa tgctgttttt tggggagtgg   3240
```

tttccaggag ctgcctggtg tctgctgtaa atgtttgtct actgcacaag cctcggcctg 3300

cccctgagcc aggctcggta ccgatgcgtg ggctgggcta ggtccctctg tccatctggg 3360

cctttgtatg agctgcattg cccttgctca ccctgaccaa gcacacgcct cagaggggcc 3420

ctcagcctct cctgaagccc tcttgtggca agaactgtgg accatgccag tcccgtctgg 3480

tttccatccc accactccaa ggactgagac tgacctcctc tggtgacact ggcctagggc 3540

ctgacactct cctaagaggt tctctccaag cccccaaata gctccaggcg ccctcggccg 3600

cccatcatgg ttaattctgt ccaacaaaca cacacgggta gattgctggc ctgttgtagg 3660

tggtagggac acagatgacc gacctggtca ctcctcctgc caacattcag tctggtatgt 3720

gaggcgtgcg tgaagcaaga actcctggag ctacagggac agggagccat cattcctgcc 3780

tgggaatcct ggaagacttc ctgcaggagt cagcgttcaa tcttgacctt gaagatggga 3840

aggatgttct ttttacgtac caattctttt gtcttttgat attaaaaaga agtacatgtt 3900

cattgtagag aatttggaaa ctgtagaaga gaatcaagaa gaaaaataaa aatcagctgt 3960

tgtaatcacc tagcaaactg gaaaaaaaaa aaaaaa 3996

<210> 42
<211> 3821
<212> DNA
<213> Homo sapiens

<400> 42

agacaggcgg agcctcacct ggggctgccc gccagcccag acaagctcag actgggtgcc 60

tgtggccctg ggaggaggtg gaaggggagg agcaggccac acaggcacag gccggtgagg 120

gacctgccca gacctggagg gtctcgctct gtcacacagg ctggagtgca gtggtgtgat 180

cttggctcat cgtaacctcc acctcccggg ttcaagtgat tctcatgcct cagcctcccg 240

agtagctggg attacaggtg gtgacttcca agagtgactc cgtcggagga aaatgactcc 300

ccagtcgctg ctgcagacga cactgttcct gctgagtctg ctcttcctgg tccaaggtgc 360

ccacggcagg ggccacaggg aagactttcg cttctgcagc cagcggaacc agacacacag 420

gagcagcctc cactacaaac ccacaccaga cctgcgcatc tccatcgaga actccgaaga 480

ggccctcaca gtccatgccc ctttccctgc agcccaccct gcttcccgat ccttccctga 540

ccccaggggc ctctaccact tctgcctcta ctggaaccga catgctggga gattacatct 600

tctctatggc aagcgtgact tcttgctgag tgacaaagcc tctagcctcc tctgcttcca 660

gcaccaggag gagagcctgg ctcagggccc cccgctgtta gccacttctg tcacctcctg 720

gtggagccct cagaacatca gcctgcccag tgccgccagc ttcaccttct ccttccacag 780

tcctccccac acggccgctc acaatgcctc ggtggacatg tgcgagctca aaagggacct 840
ccagctgctc agccagttcc tgaagcatcc ccagaaggcc tcaaggaggc cctcggctgc 900
ccccgccagc cagcagttgc agagcctgga gtcgaaactg acctctgtga gattcatggg 960
ggacatggtg tccttcgagg aggaccggat caacgccacg gtgtggaagc tccagcccac 1020
agccggcctc caggacctgc acatccactc ccggcaggag gaggagcaga gcgagatcat 1080
ggagtactcg gtgctgctgc ctcgaacact cttccagagg acgaaaggcc ggagcgggga 1140
ggctgagaag agactcctcc tggtggactt cagcagccaa gccctgttcc aggacaagaa 1200
ttccagccaa gtcctgggtg agaaggtctt ggggattgtg gtacagaaca ccaaagtagc 1260
caacctcacg gagcccgtgg tgctcacttt ccagcaccag ctacagccga agaatgtgac 1320
tctgcaatgt gtgttctggg ttgaagaccc cacattgagc agcccggggc attggagcag 1380
tgctgggtgt gagaccgtca ggagagaaac ccaaacatcc tgcttctgca accacttgac 1440
ctactttgca gtgctgatgg tctcctcggt ggaggtggac gccgtgcaca agcactacct 1500
gagcctcctc tcctacgtgg gctgtgtcgt ctctgccctg gcctgccttg tcaccattgc 1560
cgcctacctc tgctccagga ggaaacctcg ggactacacc atcaaggtgc acatgaacct 1620
gctgctggcc gtcttcctgc tggacacgag cttcctgctc agcgagccgg tggccctgac 1680
aggctctgag gctggctgcc gagccagtgc catcttcctg cacttctccc tgctcacctg 1740
cctttcctgg atgggcctcg aggggtacaa cctctaccga ctcgtggtgg aggtctttgg 1800
cacctatgtc cctggctacc tactcaagct gagcgccatg ggctggggct tccccatctt 1860
tctggtgacg ctggtggccc tggtggatgt ggacaactat ggccccatca tcttggctgt 1920
gcataggact ccagagggcg tcatctaccc ttccatgtgc tggatccggg actccctggt 1980
cagctacatc accaacctgg gcctcttcag cctggtgttt ctgttcaaca tggccatgct 2040
agccaccatg gtggtgcaga tcctgcggct gcgcccccac acccaaaagt ggtcacatgt 2100
gctgacactg ctgggcctca gcctggtcct tggcctgccc tgggccttga tcttcttctc 2160
ctttgcttct ggcaccttcc agcttgtcgt cctctacctt ttcagcatca tcacctcctt 2220
ccaaggcttc ctcatcttca tctggtactg gtccatgcgg ctgcaggccc ggggtggccc 2280
ctcccctctg aagagcaact cagacagcgc caggctcccc atcagctcgg gcagcacctc 2340
gtccagccgc atctaggcct ccagcccacc tgcccatgtg atgaagcaga gattcggcct 2400
cgtcgcacac tgcctgtggc ccccgagccc ggcccagccc caggccagtc agccgcagac 2460
tttggaaagc ccaacgacca tggagagatg ggccgttgcc atggtggacg gactcccggg 2520

ctgggctttt gaattggcct tggggactac tcggctctca ctcagctccc acgggactca 2580
gaagtgcgcc gccatgctgc ctagggtact gtccccacat ctgtcccaac ccagctggag 2640
gcctggtctc tccttacaac ccctgggccc agccctcatt gctgggggcc aggccttgga 2700
tcttgagggt ctggcacatc cttaatcctg tgcccctgcc tgggacagaa atgtggctcc 2760
agttgctctg tctctcgtgg tcaccctgag ggcactctgc atcctctgtc attttaacct 2820
caggtggcac ccagggcgaa tggggcccag ggcagacctt cagggccaga gccctggcgg 2880
aggagaggcc ctttgccagg agcacagcag cagctcgcct acctctgagc ccaggccccc 2940
tccctccctc agccccccag tcctccctcc atcttccctg gggttctcct cctctcccag 3000
ggcctccttg ctccttcgtt cacagctggg ggtccccgat tccaatgctg tttttggggg 3060
agtggtttcc aggagctgcc tggtgtctgc tgtaaatgtt tgtctactgc acaagcctcg 3120
gcctgcccct gagccaggct cggtaccgat gcgtgggctg ggctaggtcc ctctgtccat 3180
ctgggccttt gtatgagctg cattgccctt gctcaccctg accaagcaca cgcctcagag 3240
gggccctcag cctctcctga agccctcttg tggcaagaac tgtggaccat gccagtcccg 3300
tctggtttcc atcccaccac tccaaggact gagactgacc tcctctggtg acactggcct 3360
agggcctgac actctcctaa gaggttctct ccaagccccc aaatagctcc aggcgccctc 3420
ggccgcccat catggttaat tctgtccaac aaacacacac gggtagattg ctggcctgtt 3480
gtaggtggta gggacacaga tgaccgacct ggtcactcct cctgccaaca ttcagtctgg 3540
tatgtgaggc gtgcgtgaag caagaactcc tggagctaca gggacaggga gccatcattc 3600
ctgcctggga atcctggaag acttcctgca ggagtcagcg ttcaatcttg accttgaaga 3660
tgggaaggat gttcttttta cgtaccaatt cttttgtctt ttgatattaa aaagaagtac 3720
atgttcattg tagagaattt ggaaactgta gaagagaatc aagaagaaaa ataaaaatca 3780
gctgttgtaa tcacctagca aactggaaaa aaaaaaaaaa a 3821

<210> 43
<211> 4004
<212> DNA
<213> Homo sapiens

<400> 43

gagaaatcca tgactcacat atatcgtccg tcatccccag tcaagctcag ggaggaggga 60

ggagcccagc tggggcagga gaggggtgtc tccccaccaa acaaacccgg tccctccctc 120

tccgcactag ctgtctgccc tgccctgccg taggagatgg gctgggagcc tcccacgctc 180

tccagctcac tcggcaggca gcggggacca gggctggcag gttaagcctc tgggggtgga 240

tcctgaaagg tggtccagcc gcctggccct gcgtgggacc ctccacctgg cagcaggtac 300
ccaaacaagg gctggacagc agggtctcgc tctgtcacac aggctggagt gcagtggtgt 360
gatcttggct catcgtaacc tccacctccc gggttcaagt gattctcatg cctcagcctc 420
ccgagtagct gggattacag gtggtgactt ccaagagtga ctccgtcgga ggaaaatgac 480
tccccagtcg ctgctgcaga cgacactgtt cctgctgagt ctgctcttcc tggtccaagg 540
tgcccacggc aggggccaca gggaagactt tcgcttctgc agccagcgga accagacaca 600
caggagcagc ctccactaca aacccacacc agacctgcgc atctccatcg agaactccga 660
agaggccctc acagtccatg cccctttccc tgcagcccac cctgcttccc gatccttccc 720
tgaccccagg ggcctctacc acttctgcct ctactggaac cgacatgctg ggagattaca 780
tcttctctat ggcaagcgtg acttcttgct gagtgacaaa gcctctagcc tcctctgctt 840
ccagcaccag gaggagagcc tggctcaggg ccccccgctg ttagccactt ctgtcacctc 900
ctggtggagc cctcagaaca tcagcctgcc cagtgccgcc agcttcacct tctccttcca 960
cagtcctccc cacacggccg ctcacaatgc ctcggtggac atgtgcgagc tcaaaaggga 1020
cctccagctg ctcagccagt tcctgaagca tccccagaag gcctcaagga ggccctcggc 1080
tgcccccgcc agccagcagt tgcagagcct ggagtcgaaa ctgacctctg tgagattcat 1140
gggggacatg gtgtccttcg aggaggaccg gatcaacgcc acggtgtgga agctccagcc 1200
cacagccggc ctccaggacc tgcacatcca ctcccggcag gaggaggagc agagcgagat 1260
catggagtac tcggtgctgc tgcctcgaac actcttccag aggacgaaag gccggagcgg 1320
ggaggctgag aagagactcc tcctggtgga cttcagcagc caagccctgt tccaggacaa 1380
gaattccagc caagtcctgg gtgagaaggt cttggggatt gtggtacaga acaccaaagt 1440
agccaacctc acggagcccg tggtgctcac tttccagcac cagctacagc cgaagaatgt 1500
gactctgcaa tgtgtgttct gggttgaaga ccccacattg agcagcccgg ggcattggag 1560
cagtgctggg tgtgagaccg tcaggagaga aacccaaaca tcctgcttct gcaaccactt 1620
gacctacttt gcagtgctga tggtctcctc ggtggaggtg gacgccgtgc acaagcacta 1680
cctgagcctc ctctcctacg tgggctgtgt cgtctctgcc ctggcctgcc ttgtcaccat 1740
tgccgcctac ctctgctcca ggaggaaacc tcgggactac accatcaagg tgcacatgaa 1800
cctgctgctg gccgtcttcc tgctggacac gagcttcctg ctcagcgagc cggtggccct 1860
gacaggctct gaggctggct gccgagccag tgccatcttc ctgcacttct ccctgctcac 1920
ctgccttttcc tggatgggcc tcgaggggta caacctctac cgactcgtgg tggaggtctt 1980
tggcacctat gtccctggct acctactcaa gctgagcgcc atgggctggg gcttccccat 2040

ctttctggtg acgctggtgg ccctggtgga tgtggacaac tatggcccca tcatcttggc 2100
tgtgcatagg actccagagg gcgtcatcta cccttccatg tgctggatcc gggactccct 2160
ggtcagctac atcaccaacc tgggcctctt cagcctggtg tttctgttca acatggccat 2220
gctagccacc atggtggtgc agatcctgcg gctgcgcccc cacacccaaa agtggtcaca 2280
tgtgctgaca ctgctgggcc tcagcctggt ccttggcctg ccctgggcct tgatcttctt 2340
ctcctttgct tctggcacct tccagcttgt cgtcctctac cttttcagca tcatcacctc 2400
cttccaaggc ttcctcatct tcatctggta ctggtccatg cggctgcagg cccggggtgg 2460
cccctcccct ctgaagagca actcagacag cgccaggctc cccatcagct cgggcagcac 2520
ctcgtccagc cgcatctagg cctccagccc acctgcccat gtgatgaagc agagattcgg 2580
cctcgtcgca cactgcctgt ggcccccgag cccggcccag ccccaggcca gtcagccgca 2640
gactttggaa agcccaacga ccatggagag atgggccgtt gccatggtgg acggactccc 2700
gggctgggct tttgaattgg ccttggggac tactcggctc tcactcagct cccacgggac 2760
tcagaagtgc gccgccatgc tgcctagggt actgtcccca catctgtccc aacccagctg 2820
gaggcctggt ctctccttac aacccctggg cccagccctc attgctgggg gccaggcctt 2880
ggatcttgag ggtctggcac atccttaatc ctgtgcccct gcctgggaca gaaatgtggc 2940
tccagttgct ctgtctctcg tggtcaccct gagggcactc tgcatcctct gtcattttaa 3000
cctcaggtgg cacccagggc gaatggggcc cagggcagac cttcagggcc agagccctgg 3060
cggaggagag gccctttgcc aggagcacag cagcagctcg cctacctctg agcccaggcc 3120
ccctccctcc ctcagccccc cagtcctccc tccatcttcc ctggggttct cctcctctcc 3180
cagggcctcc ttgctccttc gttcacagct gggggtcccc gattccaatg ctgtttttg 3240
gggagtggtt tccaggagct gcctggtgtc tgctgtaaat gtttgtctac tgcacaagcc 3300
tcggcctgcc cctgagccag gctcggtacc gatgcgtggg ctgggctagg tccctctgtc 3360
catctgggcc tttgtatgag ctgcattgcc cttgctcacc ctgaccaagc acacgcctca 3420
gaggggccct cagcctctcc tgaagccctc ttgtggcaag aactgtggac catgccagtc 3480
ccgtctggtt tccatcccac cactccaagg actgagactg acctcctctg gtgacactgg 3540
cctagggcct gacactctcc taagaggttc tctccaagcc cccaaatagc tccaggcgcc 3600
ctcggccgcc catcatggtt aattctgtcc aacaaacaca cacgggtaga ttgctggcct 3660
gttgtaggtg gtagggacac agatgaccga cctggtcact cctcctgcca acattcagtc 3720
tggtatgtga ggcgtgcgtg aagcaagaac tcctggagct acagggacag ggagccatca 3780

ttcctgcctg ggaatcctgg aagacttcct gcaggagtca gcgttcaatc ttgaccttga 3840

agatgggaag gatgttcttt ttacgtacca attcttttgt cttttgatat taaaaagaag 3900

tacatgttca ttgtagagaa tttggaaact gtagaagaga atcaagaaga aaaataaaaa 3960

tcagctgttg taatcaccta gcaaactgga aaaaaaaaaa aaaa 4004

<210> 44
<211> 4058
<212> DNA
<213> Homo sapiens

<400> 44

acttgggaac aggacaagtt acggagccac gttgctttgc tgggtctgag ccggggtgtg 60

acgtagtccc tgcagctgcc aacggttgcc agggcaacgg ttgccagggg ctgctgtcac 120

ctgcgcccct tctcccgcgc tggcggctgg ggcttctcag cctctattcc ctggctgtcc 180

cctttgtttg aagctccagt gagggagcag tggctggggt ggcccagctt caaagtctct 240

gtcctcttga aaaaaggtgg tcgggggaca ttgacccacc agccccgcag gctactgcct 300

gcaaacaaga acccctcatc tgccacgcac gttcttaatg tatctatagt ctgcctgatg 360

ccacaaagga gtccagggtc tcgctctgtc acacaggctg gagtgcagtg gtgtgatctt 420

ggctcatcgt aacctccacc tcccgggttc aagtgattct catgcctcag cctcccgagt 480

agctgggatt acaggtggtg acttccaaga gtgactccgt cggaggaaaa tgactcccca 540

gtcgctgctg cagacgacac tgttcctgct gagtctgctc ttcctggtcc aaggtgccca 600

cggcaggggc cacagggaag actttcgctt ctgcagccag cggaaccaga cacacaggag 660

cagcctccac tacaaaccca caccagacct gcgcatctcc atcgagaact ccgaagaggc 720

cctcacagtc catgcccctt tccctgcagc ccaccctgct tcccgatcct tccctgaccc 780

caggggcctc taccacttct gcctctactg gaaccgacat gctgggagat tacatcttct 840

ctatggcaag cgtgacttct tgctgagtga caaagcctct agcctcctct gcttccagca 900

ccaggaggag agcctggctc agggcccccc gctgttagcc acttctgtca cctcctggtg 960

gagccctcag aacatcagcc tgcccagtgc cgccagcttc accttctcct tccacagtcc 1020

tccccacacg gccgctcaca atgcctcggt ggacatgtgc gagctcaaaa gggacctcca 1080

gctgctcagc cagttcctga agcatcccca gaaggcctca aggaggccct cggctgcccc 1140

cgccagccag cagttgcaga gcctggagtc gaaactgacc tctgtgagat tcatggggga 1200

catggtgtcc ttcgaggagg accggatcaa cgccacggtg tggaagctcc agcccacagc 1260

cggcctccag gacctgcaca tccactcccg gcaggaggag gagcagagcg agatcatgga 1320

gtactcggtg ctgctgcctc gaacactctt ccagaggacg aaaggccgga gcggggaggc 1380
tgagaagaga ctcctcctgg tggacttcag cagccaagcc ctgttccagg acaagaattc 1440
cagccaagtc ctgggtgaga aggtcttggg gattgtggta cagaacacca aagtagccaa 1500
cctcacggag cccgtggtgc tcactttcca gcaccagcta cagccgaaga atgtgactct 1560
gcaatgtgtg ttctgggttg aagaccccac attgagcagc ccggggcatt ggagcagtgc 1620
tgggtgtgag accgtcagga gagaaaccca aacatcctgc ttctgcaacc acttgaccta 1680
ctttgcagtg ctgatggtct cctcggtgga ggtggacgcc gtgcacaagc actacctgag 1740
cctcctctcc tacgtgggct gtgtcgtctc tgccctggcc tgccttgtca ccattgccgc 1800
ctacctctgc tccaggagga aacctcggga ctacaccatc aaggtgcaca tgaacctgct 1860
gctggccgtc ttcctgctgg acacgagctt cctgctcagc gagccggtgg ccctgacagg 1920
ctctgaggct ggctgccgag ccagtgccat cttcctgcac ttctccctgc tcacctgcct 1980
ttcctggatg ggcctcgagg ggtacaacct ctaccgactc gtggtggagg tctttggcac 2040
ctatgtccct ggctacctac tcaagctgag cgccatgggc tggggcttcc ccatctttct 2100
ggtgacgctg gtggccctgg tggatgtgga caactatggc cccatcatct tggctgtgca 2160
taggactcca gagggcgtca tctacccttc catgtgctgg atccgggact ccctggtcag 2220
ctacatcacc aacctgggcc tcttcagcct ggtgtttctg ttcaacatgg ccatgctagc 2280
caccatggtg gtgcagatcc tgcggctgcg cccccacacc caaaagtggt cacatgtgct 2340
gacactgctg ggcctcagcc tggtccttgg cctgccctgg gccttgatct tcttctcctt 2400
tgcttctggc accttccagc ttgtcgtcct ctacctttc agcatcatca cctccttcca 2460
aggcttcctc atcttcatct ggtactggtc catgcggctg caggcccggg gtggccccctc 2520
ccctctgaag agcaactcag acagcgccag gctccccatc agctcgggca gcacctcgtc 2580
cagccgcatc taggcctcca gcccacctgc ccatgtgatg aagcagagat tcggcctcgt 2640
cgcacactgc ctgtggcccc cgagcccggc ccagccccag gccagtcagc cgcagacttt 2700
ggaaagccca acgaccatgg agagatgggc cgttgccatg gtggacggac tcccgggctg 2760
ggcttttgaa ttggccttgg ggactactcg gctctcactc agctcccacg ggactcagaa 2820
gtgcgccgcc atgctgccta gggtactgtc cccacatctg tcccaaccca gctggaggcc 2880
tggtctctcc ttacaacccc tgggcccagc cctcattgct gggggccagg ccttggatct 2940
tgagggtctg gcacatcctt aatcctgtgc ccctgcctgg gacagaaatg tggctccagt 3000
tgctctgtct ctcgtggtca ccctgagggc actctgcatc ctctgtcatt ttaacctcag 3060
gtggcaccca gggcgaatgg ggcccagggc agaccttcag ggccagagcc ctggcggagg 3120

agaggccctt tgccaggagc acagcagcag ctcgcctacc tctgagccca ggccccctcc 3180

ctccctcagc cccccagtcc tccctccatc ttccctgggg ttctcctcct ctcccagggc 3240

ctccttgctc cttcgttcac agctgggggt ccccgattcc aatgctgttt tttggggagt 3300

ggtttccagg agctgcctgg tgtctgctgt aaatgtttgt ctactgcaca agcctcggcc 3360

tgcccctgag ccaggctcgg taccgatgcg tgggctgggc taggtccctc tgtccatctg 3420

ggcctttgta tgagctgcat tgcccttgct caccctgacc aagcacacgc ctcagagggg 3480

ccctcagcct ctcctgaagc cctcttgtgg caagaactgt ggaccatgcc agtcccgtct 3540

ggtttccatc ccaccactcc aaggactgag actgacctcc tctggtgaca ctggcctagg 3600

gcctgacact ctcctaagag gttctctcca agcccccaaa tagctccagg cgccctcggc 3660

cgcccatcat ggttaattct gtccaacaaa cacacacggg tagattgctg gcctgttgta 3720

ggtggtaggg acacagatga ccgacctggt cactcctcct gccaacattc agtctggtat 3780

gtgaggcgtg cgtgaagcaa gaactcctgg agctacaggg acagggagcc atcattcctg 3840

cctgggaatc ctggaagact tcctgcagga gtcagcgttc aatcttgacc ttgaagatgg 3900

gaaggatgtt ctttttacgt accaattctt ttgtcttttg atattaaaaa gaagtacatg 3960

ttcattgtag agaatttgga aactgtagaa gagaatcaag aagaaaaata aaaatcagct 4020

gttgtaatca cctagcaaac tggaaaaaaa aaaaaaaa 4058

<210> 45
<211> 3875
<212> DNA
<213> Homo sapiens

<400> 45

```
gagaaatcca tgactcacat atatcgtccg tcatccccag tcaagctcag ggaggaggga     60
ggagcccagc tggggcagga gaggggtgtc tccccaccaa acaaacccgg tccctccctc    120
tccgcactag ctgtctgccc tgccctgccg taggagatgg gctgggagcc tcccacgctc    180
tccagctcac tcggcaggca gcggggacca gggctggcag gttaagcctc tgggggtgga    240
tcctgaaagg tggtccagcc gcctggccct gcgtgggacc ctccacctgg cagcaggtgg    300
tgacttccaa gagtgactcc gtcggaggaa aatgactccc cagtcgctgc tgcagacgac    360
actgttcctg ctgagtctgc tcttcctggt ccaagcctct gcctcctcag gtgcccacgg    420
caggggccac agggaagact ttcgcttctg cagccagcgg aaccagacac acaggagcag    480
cctccactac aaacccacac cagacctgcg catctccatc gagaactccg aagaggccct    540
cacagtccat gcccctttcc ctgcagccca ccctgcttcc cgatccttcc ctgaccccag    600
```

gggcctctac cacttctgcc tctactggaa ccgacatgct gggagattac atcttctcta 660
tggcaagcgt gacttcttgc tgagtgacaa agcctctagc ctcctctgct tccagcacca 720
ggaggagagc ctggctcagg gcccccccgct gttagccact tctgtcacct cctggtggag 780
ccctcagaac atcagcctgc ccagtgccgc cagcttcacc ttctccttcc acagtcctcc 840
ccacacggcc gctcacaatg cctcggtgga catgtgcgag ctcaaaaggg acctccagct 900
gctcagccag ttcctgaagc atccccagaa ggcctcaagg aggccctcgg ctgcccccgc 960
cagccagcag ttgcagagcc tggagtcgaa actgacctct gtgagattca tgggggacat 1020
ggtgtccttc gaggaggacc ggatcaacgc cacggtgtgg aagctccagc ccacagccgg 1080
cctccaggac ctgcacatcc actcccggca ggaggaggag cagagcgaga tcatggagta 1140
ctcggtgctg ctgcctcgaa cactcttcca gaggacgaaa ggccggagcg gggaggctga 1200
gaagagactc ctcctggtgg acttcagcag ccaagccctg ttccaggaca agaattccag 1260
ccaagtcctg ggtgagaagg tcttggggat tgtggtacag aacaccaaag tagccaacct 1320
cacggagccc gtggtgctca ctttccagca ccagctacag ccgaagaatg tgactctgca 1380
atgtgtgttc tgggttgaag accccacatt gagcagcccg gggcattgga gcagtgctgg 1440
gtgtgagacc gtcaggagag aaacccaaac atcctgcttc tgcaaccact tgacctactt 1500
tgcagtgctg atggtctcct cggtggaggt ggacgccgtg cacaagcact acctgagcct 1560
cctctcctac gtgggctgtg tcgtctctgc cctggcctgc cttgtcacca ttgccgccta 1620
cctctgctcc aggaggaaac ctcgggacta caccatcaag gtgcacatga acctgctgct 1680
ggccgtcttc ctgctggaca cgagcttcct gctcagcgag ccggtggccc tgacaggctc 1740
tgaggctggc tgccgagcca gtgccatctt cctgcacttc tccctgctca cctgcctttc 1800
ctggatgggc ctcgaggggt acaacctcta ccgactcgtg gtggaggtct ttggcaccta 1860
tgtccctggc tacctactca agctgagcgc catgggctgg ggcttcccca tctttctggt 1920
gacgctggtg gccctggtgg atgtggacaa ctatggcccc atcatcttgg ctgtgcatag 1980
gactccagag ggcgtcatct acccttccat gtgctggatc cgggactccc tggtcagcta 2040
catcaccaac ctgggcctct tcagcctggt gtttctgttc aacatggcca tgctagccac 2100
catggtggtg cagatcctgc ggctgcgccc ccacacccaa aagtggtcac atgtgctgac 2160
actgctgggc ctcagcctgg tccttggcct gccctgggcc ttgatcttct tctcctttgc 2220
ttctggcacc ttccagcttg tcgtcctcta ccttttcagc atcatcacct ccttccaagg 2280
cttcctcatc ttcatctggt actggtccat gcggctgcag gcccggggtg gcccctcccc 2340

```
tctgaagagc aactcagaca gcgccaggct ccccatcagc tcgggcagca cctcgtccag    2400
ccgcatctag gcctccagcc cacctgccca tgtgatgaag cagagattcg gcctcgtcgc    2460
acactgcctg tggcccccga gcccggccca gccccaggcc agtcagccgc agactttgga    2520
aagcccaacg accatggaga gatgggccgt tgccatggtg gacggactcc cgggctgggc    2580
ttttgaattg gccttgggga ctactcggct ctcactcagc tcccacggga ctcagaagtg    2640
cgccgccatg ctgcctaggg tactgtcccc acatctgtcc caacccagct ggaggcctgg    2700
tctctcctta caacccctgg gcccagccct cattgctggg ggccaggcct tggatcttga    2760
gggtctggca catccttaat cctgtgcccc tgcctgggac agaaatgtgg ctccagttgc    2820
tctgtctctc gtggtcaccc tgagggcact ctgcatcctc tgtcatttta acctcaggtg    2880
gcacccaggg cgaatggggc ccagggcaga ccttcagggc cagagccctg gcggaggaga    2940
ggccctttgc caggagcaca gcagcagctc gcctacctct gagcccaggc cccctccctc    3000
cctcagcccc ccagtcctcc ctccatcttc cctggggttc tcctcctctc ccagggcctc    3060
cttgctcctt cgttcacagc tgggggtccc cgattccaat gctgtttttt ggggagtggt    3120
ttccaggagc tgcctggtgt ctgctgtaaa tgtttgtcta ctgcacaagc ctcggcctgc    3180
ccctgagcca ggctcggtac cgatgcgtgg gctgggctag gtccctctgt ccatctgggc    3240
ctttgtatga gctgcattgc ccttgctcac cctgaccaag cacacgcctc agaggggccc    3300
tcagcctctc ctgaagccct cttgtggcaa gaactgtgga ccatgccagt cccgtctggt    3360
ttccatccca ccactccaag gactgagact gacctcctct ggtgacactg gcctagggcc    3420
tgacactctc ctaagaggtt ctctccaagc cccaaatag ctccaggcgc cctcggccgc    3480
ccatcatggt taattctgtc caacaaacac acacgggtag attgctggcc tgttgtaggt    3540
ggtagggaca cagatgaccg acctggtcac tcctcctgcc aacattcagt ctggtatgtg    3600
aggcgtgcgt gaagcaagaa ctcctggagc tacaggaca gggagccatc attcctgcct    3660
gggaatcctg gaagacttcc tgcaggagtc agcgttcaat cttgaccttg aagatgggaa    3720
ggatgttctt tttacgtacc aattcttttg tcttttgata ttaaaaagaa gtacatgttc    3780
attgtagaga atttggaaac tgtagaagag aatcaagaag aaaaataaaa atcagctgtt    3840
gtaatcacct agcaaactgg aaaaaaaaaa aaaaa                               3875
```

<210> 46
<211> 2655
<212> DNA
<213> Homo sapiens

<400> 46

```
cccagaaggc cgcgggggt ggaccgccta agagggcgtg cgctcccgac atgccccgcg      60
gcgcgccatt aaccgccaga tttgaatcgc gggacccgtt ggcagaggtg gcggcggcgg    120
catgggtgcc ccgacgttgc cccctgcctg gcagcccttt ctcaaggacc accgcatctc    180
tacattcaag aactggccct tcttggaggg ctgcgcctgc accccggagc ggatggccga    240
ggctggcttc atccactgcc ccactgagaa cgagccagac ttggcccagt gtttcttctg    300
cttcaaggag ctggaaggct gggagccaga tgacgacccc atagaggaac ataaaaagca    360
ttcgtccggt tgcgctttcc tttctgtcaa gaagcagttt gaagaattaa cccttggtga    420
atttttgaaa ctggacagag aaagagccaa gaacaaaatt gcaaaggaaa ccaacaataa    480
gaagaaagaa tttgaggaaa ctgcggagaa agtgcgccgt gccatcgagc agctggctgc    540
catggattga ggcctctggc cggagctgcc tggtcccaga gtggctgcac cacttccagg    600
gtttattccc tggtgccacc agccttcctg tgggcccctt agcaatgtct taggaaagga    660
gatcaacatt ttcaaattag atgtttcaac tgtgctcttg ttttgtcttg aaagtggcac    720
cagaggtgct tctgcctgtg cagcgggtgc tgctggtaac agtggctgct tctctctctc    780
tctctctttt ttgggggctc atttttgctg ttttgattcc cgggcttacc aggtgagaag    840
tgagggagga agaaggcagt gtcccttttg ctagagctga cagctttgtt cgcgtgggca    900
gagccttcca cagtgaatgt gtctggacct catgttgttg aggctgtcac agtcctgagt    960
gtggacttgg caggtgcctg ttgaatctga gctgcaggtt ccttatctgt cacacctgtg   1020
cctcctcaga ggacagtttt tttgttgttg tgttttttg ttttttttt tttggtagat     1080
gcatgacttg tgtgtgatga gagaatggag acagagtccc tggctcctct actgtttaac   1140
aacatggctt tcttattttg tttgaattgt taattcacag aatagcacaa actacaatta   1200
aaactaagca caaagccatt ctaagtcatt ggggaaacgg ggtgaacttc aggtggatga   1260
ggagacagaa tagagtgata ggaagcgtct ggcagatact ccttttgcca ctgctgtgtg   1320
attagacagg cccagtgagc cgcggggcac atgctggccg ctcctccctc agaaaaaggc   1380
agtggcctaa atccttttta aatgacttgg ctcgatgctg tgggggactg gctgggctgc   1440
tgcaggccgt gtgtctgtca gcccaacctt cacatctgtc acgttctcca cacgggggag   1500
agacgcagtc cgcccaggtc cccgctttct ttggaggcag cagctcccgc agggctgaag   1560
tctggcgtaa gatgatggat ttgattcgcc ctcctccctg tcatagagct gcagggtgga   1620
ttgttacagc ttcgctggaa acctctggag gtcatctcgg ctgttcctga gaaataaaaa   1680
gcctgtcatt tcaaacactg ctgtggaccc tactgggttt ttaaaatatt gtcagttttt   1740
catcgtcgtc cctagcctgc caacagccat ctgcccagac agccgcagtg aggatgagcg   1800
```

```
tcctggcaga gacgcagttg tctctgggcg cttgccagag ccacgaaccc cagacctgtt   1860
tgtatcatcc gggctccttc cgggcagaaa caactgaaaa tgcacttcag acccacttat   1920
ttctgccaca tctgagtcgg cctgagatag acttttccct ctaaactggg agaatatcac   1980
agtggttttt gttagcagaa aatgcactcc agcctctgta ctcatctaag ctgcttattt   2040
ttgatatttg tgtcagtctg taaatggata cttcacttta ataactgttg cttagtaatt   2100
ggctttgtag agaagctgga aaaaaatggt tttgtcttca actcctttgc atgccaggcg   2160
gtgatgtgga tctcggcttc tgtgagcctg tgctgtgggc agggctgagc tggagccgcc   2220
cctctcagcc cgcctgccac ggcctttcct taaaggccat ccttaaaacc agaccctcat   2280
ggctaccagc acctgaaagc ttcctcgaca tctgttaata aagccgtagg cccttgtcta   2340
agtgcaaccg cctagacttt ctttcagata catgtccaca tgtccatttt tcaggttctc   2400
taagttggag tggagtctgg gaagggttgt gaatgaggct tctgggctat gggtgaggtt   2460
ccaatggcag gttagagccc ctcgggccaa ctgccatcct ggaaagtaga gacagcagtg   2520
cccgctgccc agaagagacc agcaagccaa actggagccc ccattgcagg ctgtcgccat   2580
gtggaaagag taactcacaa ttgccaataa agtctcatgt ggttttatct aaaaaaaaaa   2640
aaaaaaaaaa aaaaa                                                    2655
```

<210> 47
<211> 2537
<212> DNA
<213> Homo sapiens

<400> 47

```
cccagaaggc cgcgggggt ggaccgccta agagggcgtg cgctcccgac atgccccgcg      60
gcgcgccatt aaccgccaga tttgaatcgc gggacccgtt ggcagaggtg gcggcggcgg    120
catgggtgcc ccgacgttgc cccctgcctg gcagcccttt ctcaaggacc accgcatctc    180
tacattcaag aactggccct tcttggaggg ctgcgcctgc accccggagc ggatggccga    240
ggctggcttc atccactgcc ccactgagaa cgagccagac ttggcccagt gtttcttctg    300
cttcaaggag ctggaaggct gggagccaga tgacgacccc atgcaaagga aaccaacaat    360
aagaagaaag aatttgagga aactgcggag aaagtgcgcc gtgccatcga gcagctggct    420
gccatggatt gaggcctctg gccggagctg cctggtccca gagtggctgc accacttcca    480
gggtttattc cctggtgcca ccagccttcc tgtgggcccc ttagcaatgt cttaggaaag    540
gagatcaaca ttttcaaatt agatgtttca actgtgctct tgttttgtct tgaaagtggc    600
accagaggtg cttctgcctg tgcagcgggt gctgctggta acagtggctg cttctctctc    660
```

tctctctctt ttttgggggc tcatttttgc tgttttgatt cccgggctta ccaggtgaga 720
agtgagggag gaagaaggca gtgtcccttt tgctagagct gacagctttg ttcgcgtggg 780
cagagccttc cacagtgaat gtgtctggac ctcatgttgt tgaggctgtc acagtcctga 840
gtgtggactt ggcaggtgcc tgttgaatct gagctgcagg ttccttatct gtcacacctg 900
tgcctcctca gaggacagtt tttttgttgt tgtgtttttt tgtttttttt tttttggtag 960
atgcatgact tgtgtgtgat gagagaatgg agacagagtc cctggctcct ctactgttta 1020
acaacatggc tttcttattt tgtttgaatt gttaattcac agaatagcac aaactacaat 1080
taaaactaag cacaaagcca ttctaagtca ttggggaaac ggggtgaact tcaggtggat 1140
gaggagacag aatagagtga taggaagcgt ctggcagata ctccttttgc cactgctgtg 1200
tgattagaca ggcccagtga gccgcggggc acatgctggc cgctcctccc tcagaaaaag 1260
gcagtggcct aaatcctttt taaatgactt ggctcgatgc tgtgggggac tggctgggct 1320
gctgcaggcc gtgtgtctgt cagcccaacc ttcacatctg tcacgttctc cacacggggg 1380
agagacgcag tccgcccagg tccccgcttt ctttggaggc agcagctccc gcagggctga 1440
agtctggcgt aagatgatgg atttgattcg ccctcctccc tgtcatagag ctgcagggtg 1500
gattgttaca gcttcgctgg aaacctctgg aggtcatctc ggctgttcct gagaaataaa 1560
aagcctgtca tttcaaacac tgctgtggac cctactgggt ttttaaaata ttgtcagttt 1620
ttcatcgtcg tccctagcct gccaacagcc atctgcccag acagccgcag tgaggatgag 1680
cgtcctggca gagacgcagt tgtctctggg cgcttgccag agccacgaac cccagacctg 1740
tttgtatcat ccgggctcct tccgggcaga aacaactgaa aatgcacttc agacccactt 1800
atttctgcca catctgagtc ggcctgagat agacttttcc ctctaaactg ggagaatatc 1860
acagtggttt ttgttagcag aaaatgcact ccagcctctg tactcatcta agctgcttat 1920
ttttgatatt tgtgtcagtc tgtaaatgga tacttcactt taataactgt tgcttagtaa 1980
ttggctttgt agagaagctg gaaaaaaatg gttttgtctt caactccttt gcatgccagg 2040
cggtgatgtg gatctcggct tctgtgagcc tgtgctgtgg gcagggctga gctggagccg 2100
cccctctcag cccgcctgcc acggcctttc cttaaaggcc atccttaaaa ccagaccctc 2160
atggctacca gcacctgaaa gcttcctcga catctgttaa taaagccgta ggcccttgtc 2220
taagtgcaac cgcctagact ttctttcaga tacatgtcca catgtccatt tttcaggttc 2280
tctaagttgg agtggagtct gggaagggtt gtgaatgagg cttctgggct atgggtgagg 2340
ttccaatggc aggttagagc ccctcgggcc aactgccatc ctggaaagta gagacagcag 2400

tgcccgctgc ccagaagaga ccagcaagcc aaactggagc ccccattgca ggctgtcgcc 2460
atgtggaaag agtaactcac aattgccaat aaagtctcat gtggttttat ctaaaaaaaa 2520
aaaaaaaaaa aaaaaaa 2537

<210> 48
<211> 2724
<212> DNA
<213> Homo sapiens

<400> 48

cccagaaggc cgcggggggt ggaccgccta agagggcgtg cgctcccgac atgccccgcg 60
gcgcgccatt aaccgccaga tttgaatcgc gggacccgtt ggcagaggtg gcggcggcgg 120
catgggtgcc ccgacgttgc cccctgcctg gcagcccttt ctcaaggacc accgcatctc 180
tacattcaag aactggccct tcttggaggg ctgcgcctgc accccggagc ggatggccga 240
ggctggcttc atccactgcc ccactgagaa cgagccagac ttggcccagt gtttcttctg 300
cttcaaggag ctggaaggct gggagccaga tgacgacccc attgggccgg gcacggtggc 360
ttacgcctgt aataccagca ctttgggagg ccgaggcggg cggatcacga gagaggaaca 420
taaaaagcat tcgtccggtt gcgctttcct ttctgtcaag aagcagtttg aagaattaac 480
ccttggtgaa tttttgaaac tggacagaga aagagccaag aacaaaattg caaaggaaac 540
caacaataag aagaaagaat ttgaggaaac tgcggagaaa gtgcgccgtg ccatcgagca 600
gctggctgcc atggattgag gcctctggcc ggagctgcct ggtcccagag tggctgcacc 660
acttccaggg tttattccct ggtgccacca gccttcctgt gggcccctta gcaatgtctt 720
aggaaaggag atcaacattt tcaaattaga tgtttcaact gtgctcttgt tttgtcttga 780
aagtggcacc agaggtgctt ctgcctgtgc agcgggtgct gctggtaaca gtggctgctt 840
ctctctctct ctctcttttt tgggggctca tttttgctgt tttgattccc gggcttacca 900
ggtgagaagt gagggaggaa gaaggcagtg tcccttttgc tagagctgac agctttgttc 960
gcgtgggcag agccttccac agtgaatgtg tctggacctc atgttgttga ggctgtcaca 1020
gtcctgagtg tggacttggc aggtgcctgt tgaatctgag ctgcaggttc cttatctgtc 1080
acacctgtgc ctcctcagag gacagttttt ttgttgttgt gttttttttgt ttttttttt 1140
ttggtagatg catgacttgt gtgtgatgag agaatggaga cagagtccct ggctcctcta 1200
ctgtttaaca acatggcttt cttattttgt ttgaattgtt aattcacaga atagcacaaa 1260
ctacaattaa aactaagcac aaagccattc taagtcattg gggaaacggg gtgaacttca 1320
ggtggatgag gagacagaat agagtgatag gaagcgtctg gcagatactc cttttgccac 1380

tgctgtgtga ttagacaggc ccagtgagcc gcggggcaca tgctggccgc tcctccctca 1440
gaaaaaggca gtggcctaaa tcctttttaa atgacttggc tcgatgctgt gggggactgg 1500
ctgggctgct gcaggccgtg tgtctgtcag cccaaccttc acatctgtca cgttctccac 1560
acgggggaga gacgcagtcc gcccaggtcc ccgctttctt tggaggcagc agctcccgca 1620
gggctgaagt ctggcgtaag atgatggatt tgattcgccc tcctccctgt catagagctg 1680
cagggtggat tgttacagct tcgctggaaa cctctggagg tcatctcggc tgttcctgag 1740
aaataaaaag cctgtcattt caaacactgc tgtggaccct actgggtttt taaaatattg 1800
tcagtttttc atcgtcgtcc ctagcctgcc aacagccatc tgcccagaca gccgcagtga 1860
ggatgagcgt cctggcagag acgcagttgt ctctgggcgc ttgccagagc cacgaacccc 1920
agacctgttt gtatcatccg ggctccttcc gggcagaaac aactgaaaat gcacttcaga 1980
cccacttatt tctgccacat ctgagtcggc ctgagataga cttttccctc taaactggga 2040
gaatatcaca gtggtttttg ttagcagaaa atgcactcca gcctctgtac tcatctaagc 2100
tgcttatttt tgatatttgt gtcagtctgt aaatggatac ttcactttaa taactgttgc 2160
ttagtaattg gctttgtaga gaagctggaa aaaaatggtt ttgtcttcaa ctcctttgca 2220
tgccaggcgg tgatgtggat ctcggcttct gtgagcctgt gctgtgggca gggctgagct 2280
ggagccgccc ctctcagccc gcctgccacg gcctttcctt aaaggccatc cttaaaacca 2340
gaccctcatg gctaccagca cctgaaagct tcctcgacat ctgttaataa agccgtaggc 2400
ccttgtctaa gtgcaaccgc ctagactttc tttcagatac atgtccacat gtccattttt 2460
caggttctct aagttggagt ggagtctggg aagggttgtg aatgaggctt ctgggctatg 2520
ggtgaggttc caatggcagg ttagagcccc tcgggccaac tgccatcctg gaaagtagag 2580
acagcagtgc ccgctgccca gaagagacca gcaagccaaa ctggagcccc cattgcaggc 2640
tgtcgccatg tggaaagagt aactcacaat tgccaataaa gtctcatgtg gttttatcta 2700
aaaaaaaaaa aaaaaaaaaa aaaa 2724

<210> 49
<211> 637
<212> DNA
<213> Homo sapiens

<400> 49

```
gcgtgaccag cggcgggtca cgtgacgcgg tgcctggcgc cgagcctccc aagatggcgg      60
tgtgcatcgc ggtgattgcc aaggagaatt accccctcta cattcgcagc acccctacgg     120
agaacgagct gaagttccac tacatggtgc acacatctct ggacgtggtg gatgagaaga     180
tctccgcaat ggggaaggcc ctggtcgacc agagggagct gtacctgggc ctgctctacc     240
ccacggagga ctacaaggta tacggctacg tcaccaactc caaggtgaag tttgtcatgg     300
tggtagattc ctccaacaca gcccttcgag acaacgaaat tcgcagcatg ttccggaagc     360
tacacaactc ctacacagac gtgatgtgca accccttcta caacccgggg gaccgcatcc     420
agtccagcag ggcctttgat aacatggtga cgtcgatgat gatacaggtg tgctgagtga     480
gctgtgctgc cagccatcgc agaggagccc gcgcacgact gtggtggggc cgtcggtctg     540
ttctggttgc ctcttcctga atggacgccc tggggctttc agggcaggca gctgtgcatg     600
ttctctcaac taaaggtctt gtgagaggaa aaaaaaa                              637
```

<210> 50
<211> 2838
<212> DNA
<213> Homo sapiens

<400> 50

```
ggaagtgccg aatggggacc aagatggcgg accttgattc gcctccgaag ctgtcagggg      60
tgcagcagcc gtctgagggg gtgggaggtg gccgctgctc cgaaatctcc gctgagctca     120
ttcgctccct gacagagctg caggagctgg aggctgtata cgaacggctc tgcggcgagg     180
agaaagtggt ggagagagag ctggatgctc ttttggaaca gcaaaacacc attgaaagta     240
agatggtcac tctccaccga atgggtccta atctgcagct gattgaggga gatgcaaagc     300
agctggctgg aatgatcacc tttacctgca acctggctga gaatgtgtcc agcaaagttc     360
gtcagcttga cctggccaag aaccgcctct atcaggccat tcagagagct gatgacatct     420
tggacctgaa gttctgcatg gatggagttc agactgcttt gaggagtgaa gattatgagc     480
aggctgcagc acatactcat cgctacttgt gcctggacaa gtcggtcatt gagctcagcc     540
gacagggcaa agaggggagc atgattgatg ccaacctgaa attgctgcag gaagctgagc     600
aacgtctcaa agccattgtg gcagagaagt ttgccattgc caccaaggaa ggtgatctgc     660
cccaggtgga gcgcttcttc aagatcttcc cactgctggg tttgcatgag gagggattaa     720
gaaagttctc ggagtacctt tgcaagcagg tggccagtaa agctgaggag aatctgctca     780
tggtgctggg gacagacatg agtgatcgga gagctgcagt catctttgca gatacactta     840
ctcttctgtt tgaagggatt gcccgcattg tggagaccca ccagccaata gtggagacct     900
attatgggcc agggagactc tataccctga tcaaatatct gcaggtggaa tgtgacagac     960
aggtggagaa ggtggtagac aagttcatca agcaaaggga ctaccaccag cagttccggc    1020
atgttcagaa caacctgatg agaaattcta caacagaaaa aatcgaacca agagaactgg    1080
```

accccatcct gactgaggtc accctgatga atgcccgcag tgagctatac ttacgcttcc 1140
tcaagaagag gattagctct gattttgagg tgggagactc catggcctca gaggaagtaa 1200
agcaagagca ccagaagtgt ctggacaaac tcctcaataa ctgccttttg agctgtacca 1260
tgcaggagct aattggctta tatgttacca tggaggagta cttcatgagg gagactgtca 1320
ataaggctgt ggctctggac acctatgaga agggccagct gacatccagc atggtggatg 1380
atgtcttcta cattgttaag aagtgcattg ggcgggctct gtccagctcc agcattgact 1440
gtctctgtgc catgatcaac ctcgccacca cagagctgga gtctgacttc agggatgttc 1500
tgtgtaataa gctgcggatg ggctttcctg ccaccacctt ccaggacatc cagcgcgggg 1560
tgacaagtgc cgtgaacatc atgcacagca gcctccagca aggcaaattt gacacaaaag 1620
gcatcgagag tactgacgag gcgaagatgt ccttcctggt gactctgaac aacgtggaag 1680
tctgcagtga aaacatctcc actctgaaga agacactgga gagtgactgc accaagctct 1740
tcagccaggg cattggaggg gagcaggccc aggccaagtt tgacagctgc ctttctgact 1800
tggccgccgt gtccaacaaa ttccgagacc tcttgcagga agggctgacg gagctcaaca 1860
gcacagccat caagccacag gtgcagcctt ggatcaacag ctttttctcc gtctcccaca 1920
acatcgagga ggaagaattc aatgactatg aggccaacga cccttgggta caacagttca 1980
tccttaacct ggagcagcaa atggcagagt tcaaggccag cctgtccccg gtcatctacg 2040
acagcctaac cggcctcatg actagccttg ttgccgtcga gttggagaaa gtggtgctga 2100
aatccacctt taaccggctg ggtggtctgc agtttgacaa ggagctgagg tcgctcattg 2160
cctaccttac cacggtgacc acctggacca tccgagacaa gtttgcccgg ctctcccaga 2220
tggccaccat cctcaatctg gagcgggtga ccgagatcct cgattactgg ggacccaatt 2280
ccggcccatt gacgtggcgc ctcacccctg ctgaagtgcg ccaggtgctg gccctgcgga 2340
tagacttccg cagtgaagat atcaagaggc tgcgcctgta gctgcctgga tgagcacacc 2400
tggctcatca cacttgcagg cctgttccct aaggggcccc agccaaggag ctgagcgagg 2460
ctgtctggct tgggggagat ctgacagccc agacctttct acggctggca gcagagaaac 2520
aaagtctgga cccactccat gctctgccct cagacctggc caggtgatgc tctgggggca 2580
gcatctcccc accgagagaa gcgggctcct aatgaggtgg gaaagccacg gcaggcagcg 2640
agcagcccag gccagctttc tgcatggatg gtcagtctct tgccctcaaa cactacagca 2700
aacaagctac ccctgccagt cctagacaac ttgggtacat ctggggacct agcagttagg 2760
cttgactttg aggagaggct gtgatgttta tgatccctga ataaagctac tccttggaga 2820
gaaaaaaaaa aaaaaaa 2838

<210> 51
<211> 1978
<212> DNA
<213> Homo sapiens

<400> 51

```
cgctctcagc gcgtgacgca gcacgctttg atataaatgc agaccgcgcg gccgtagctt     60
cctctctgct ctcgcggccg actcgcaaga tggcgccgca gaaagacagg aagcccaaga    120
ggtcaacctg gaggtttaat ttggacctta ctcatccagt agaagatgga atttttgatt    180
ctggaaattt tgagcaattt ctacgggaga aggttaaagt caatggcaaa actggaaatc    240
tcgggaatgt tgttcacatt gaacgcttca agaataaaat cacagttgtt tctgagaaac    300
agttctctaa aaggtatttg aaatacctta ccaagaaata ccttaagaag aacaatcttc    360
gtgattggct tcgagtggtt gcatctgaca aggagaccta cgaacttcgt tacttccaga    420
ttagtcaaga tgaagatgaa tcagagtcgg aggactaggc aaaggctccc cttacagggc    480
tttgcttatt aataaaataa atgaagtata catgagaaat accaagaaat tggcttttag    540
tttatcagtg aataaaaaat attatactct tgaacttttg tctcattttt ttgagtatgc    600
tgtttatatg attttgattt ccctctgata actatcaaca gtatttaaat agcttatagc    660
tggtataatt ttttcccacg atttccaaaa tcttttatgt actcaggtaa aagtagcgtt    720
atataggaaa tctttttttt agacactctc gttctgtcac ccaggctgga gtgcagtgac    780
tcagcttcct aaatagctgg aattacaggt gtgagccacc atgcccggct aatttttttgt   840
acttttagta gagtagggtt tggccatgtt ggccaggctg gtttcaaact cctgacctca    900
agtgatctac ccacctcggc ttcccaaagt gctgattata gctgtgaacc accatgcccg    960
gccaggaaat cttactgtag aacaattttt tatatagctg tataaaatgt atatgattgt   1020
cttgacagtc tcaaatactg tttttaatag cttgtaaatg taatctcaag tgcttagaac   1080
agttcttaca tataagttgc tctgtagttt gctcttatag ttagcccaaa gactctgggt   1140
gtgaggcctg ctgtaaacca atgttaaact gcttattaga aagccctaac cacctgcttt   1200
gtaggcacca gaaactcaaa accaaatctc aactcagcta cagaatctac tgtggtcctt   1260
gtctgaaaaa attagttcac tcggttggaa tcttgtctca gagcatcctc atctctttct   1320
caaaagcccc taccccaaca ccggcgtgtt ggttgtctat tgaaacttac aagtggatgg   1380
acccttttctc ccgaataaac tggcctttga aagctctaat cgaaatggtt tggcaaaatc  1440
catactgcag gagattaggg aggacaagaa tgatgtgcct ttttgtactg ctgagcctga   1500
tggtggtgcc actacttcag gtacttagat gagtcttgat gctaatagaa ttgtgtcgcc   1560
```

aaacatatct ggacagttac aacctaatct atgcattaat tggtttggga attgcttgaa 1620

attattgttt aattcaatgt tttaattcgt tttcctaaaa atttaagtgc ccccatcatc 1680

gtgcaatacc tcagtgcagc aactccttga ttcttggatg actgaacttc ctaacttggc 1740

tctgccccat tgttcccatt tttcatgttt ttcacaaata gttaaccagg tacctactac 1800

tgtgcaccgc tgcagagcat tgaggatgta tgtgatgagt aaaaacaccc agcctgctct 1860

gctgtgttag tattatgacg gaaactgatc aaatcacatg tgaacaaatt tactgctaca 1920

aaagggaggg cttaataaaa ggaatttcat ctgggaaggc aaaaaaaaaa aaaaaaaa 1978

<210> 52
<211> 17821
<212> DNA
<213> Homo sapiens

<400> 52

cacgctcccc tcgactgcgc tccagcctgg ggcgcgcccg gccgccgccg ccttcgctgc 60

cgccacgggc ccgtcttctt cctccttcgg ctcccaggat gaagaaactg agtctcagag 120

aggtgaagtg acttgcccaa gatcacagca attatcactt ctccctgggc tcccaggccc 180

tcctgcagca gcccccgcct gggccatgtc ttcctcagat gaagagacgc tcagtgagcg 240

gtcatgtcgg agtgagcggt cttgtcggag tgagcgatct tacaggagcg agcggtcggg 300

gagcctgtct ccctgtcccc caggggacac cttgccctgg aacctgccac tgcatgagca 360

gaaaaagcgg aaaagccagg attcggtgct ggaccctgca gagcgtgctg tggtcagagt 420

cgctgatgaa cgggaccggg ttcagaagaa aacgttcacc aagtgggtca acaagcactt 480

aatgaaggtc cgcaagcaca tcaatgatct ttatgaagat ctgcgggatg gccataacct 540

gatctctctg ttggaggtcc tctcaggcat caaactgccc cgggagaagg gcaggatgcg 600

ttttcatagg ctgcagaatg tgcagattgc cctggacttc ctaaagcagc gacaggtgaa 660

actagtgaat attcgcaatg atgacatcac agatggcaac cccaagttga ccctgggtct 720

gatctggacc attattttgc atttccagat ctctgacatc tacattagtg gagaatcagg 780

ggatatgtca gccaaggaga aactactcct gtggacccag aaggtgacag ctggttacac 840

aggaatcaaa tgcaccaact ttcctcctg ctggagtgat gggaagatgt tcaatgcact 900

cattcaccga taccgacccg atctagtaga catggagagg gtgcaaatcc aaagtaaccg 960

agagaatctg gaacaggctt ttgaagtggc agaaagactg ggggtcactc gcctgctgga 1020

tgcagaagat gtggatgtgc catctccaga tgaaaagtct gtaatcactt atgtgtcttc 1080

gatttatgat gccttcccta aagttcctga gggtggagaa gggatcagtg ctacggaagt 1140

ggactccagg tggcaagaat accaaagccg agtggactcc ctcattccct ggatcaaaca 1200
gcatacaata ctgatgtcag ataaaacttt tccccaaaac cctgttgaac taaaggcact 1260
ttataaccaa tatatacact tcaaagaaac agaaattctg gccaaggaga gagaaaaagg 1320
aagaattgag gaattatata aattactaga ggtgtggatt gaatttggcc gaattaaact 1380
gcctcaaggt tatcacccta atgatgtgga agaagagtgg ggaaagctca tcatagagat 1440
gctggaacga gagaaatcac ttcggccggc tgtggagagg ctggaattgc tgctacagat 1500
tgcaaacaaa atccagaatg gtgctttgaa ctgtgaagaa aaactgacac tagctaagaa 1560
tacactgcag gctgatgctg ctcacctgga atcaggacaa ccggtacaat gtgagtcaga 1620
tgtcattatg tacattcagg agtgtgaagg tctcatcagg cagctgcagg tggatctcca 1680
gatcctgcgg gatgagaatt actaccagct agaagagctg gcttttaggg tcatgcgtct 1740
tcaggatgag ctggtcacct tgcgtctaga gtgtacaaac ctgtaccgga agggtcattt 1800
cacttcactt gaattggttc caccctctac tttaaccacc actcatctga aagcagaacc 1860
cttaaccaag gcaacccatt cttcttctac ctcctggttc cgaaagccta tgactcgggc 1920
tgaacttgtg gccatcagct cctctgaaga tgaaggcaat ctccgatttg tgtatgaact 1980
actgtcttgg gtagaagaga tgcagatgaa actggagcga gcagagtggg gcaatgacct 2040
gcctagtgtg gagttgcagc tagaaacaca gcagcacatc catacgagtg tagaagagct 2100
gggctcaagt gtcaaggagg ccaggttgta tgagggaaag atgtcccaga atttccatac 2160
cagctatgct gaaactcttg gaaagctgga gacacagtat tgtaaattga aggaaacttc 2220
tagcttccgg atgaggcacc ttcagagcct gcataaattt gtttccagag ctacagctga 2280
gttgatctgg ttgaatgaga aggaggagga ggaactagca tatgactgga gtgacaacaa 2340
ttccaatatc tcagccaaga gaaattactt ctctgagttg acaatggaac tggaggagaa 2400
acaggatgtg tttcgttctc tacaagatac agcagaacta ctttcacttg agaaccaccc 2460
agccaagcag acagtggagg cttacagtgc tgctgtccag tcccagttgc agtggatgaa 2520
gcagctgtgc ctgtgtgttg agcagcatgt gaaagagaat actgcttatt ttcagttctt 2580
cagtgatgca cgagagctgg agtcattctt gaggaacctc caagattcca ttaaacgaaa 2640
atattcctgt gaccacaaca ccagcttatc ccgccttgaa gacctgctcc aggactccat 2700
ggatgaaaag gagcagctta tacagtccaa gagttccgtt gccagtctcg ttgggagatc 2760
aaaaaccatc gttcagctaa aaccacgcag tccagaccat gtgttaaaga acaccatttc 2820
tgtcaaggct gtctgtgact acaggcagat cgagattact atttgcaaaa atgatgaatg 2880

tgtgctagaa gataattctc agcggaccaa atggaaagtg atcagcccca cagggaacga 2940
ggcaatggtg ccgtcagtct gcttcctcat ccccccaccc aataaggatg ccattgagat 3000
ggccagcagg gtcgaacaat cttatcagaa ggttatggcc ctttggcatc agctgcatgt 3060
taacaccaaa agccttatct cttggaacta tctgcgtaaa gaccttgacc ttgtacagac 3120
ctggaaccta gaaaagcttc gatcctcagc accaggggag tgccatcaga ttatgaagaa 3180
ccttcaggcc cactatgaag actttctgca ggatagtcgt gactctgtgc tgttctcagt 3240
ggctgatcgc ttgcgcttgg aagaggaggt ggaagcttgt aaagcccgct tccagcacct 3300
gatgaagtcc atggagaatg aggacaaaga ggagactgtg gccaagatgt acatttcaga 3360
gttgaagaac atccggctac gcctggagga gtatgaacag agggtggtca aacgaattca 3420
gtctctagcc agctctagga ctgacagaga tgcctggcag gacaatgcat taaggattgc 3480
agagcaagag cacacccagg aggatttaca gcaattgagg tcagacttgg atgcagtttc 3540
tatgaaatgt gacagctttc tccatcagtc tccatctagt tcaagtgtcc caactctgcg 3600
ctcagaactg aatctgctgg tggagaagat ggaccatgtc tatggtctct ctactgtata 3660
tctgaataag ttaaagacag ttgatgttat agtacgtagc atacaggatg ctgaactctt 3720
ggtcaaaggt tatgagatta agctgagtca agaagaagta gtactggcag atctctcagc 3780
tctggaggcc cattggtcga cattacggca ctggcttagt gatgtgaagg acaagaattc 3840
agtgttttca gtcctggatg aggaaattgc caaggccaag gtagtggcag agcagatgag 3900
tcgtctgaca ccagagcgaa atctggattt ggagcgctat caggaaaaag gctcccagct 3960
gcaggagcgt tggcaccgag tcattgccca gctcgagatt cgccaatctg agctagaaag 4020
tatccaggaa gttctgggag attaccgagc ctgccatgga actctcatca agtggattga 4080
ggaaaccact gcccagcagg aaatgatgaa gccaggccag gcagaggata gcagagtgct 4140
ttcggagcag ctcagccagc agacggccct atttgcagaa attgagagaa atcagacaaa 4200
actggatcaa tgtcaaaaat ttcccagca gtactctact attgtaaagg actatgaatt 4260
gcaactgatg acatacaagg cctttgtgga atcgcagcag aaatcccctg gcaagcgccg 4320
tcgcatgctt tcctcttcag atgccatcac tcaagagttc atggacttaa ggactcgcta 4380
cacggcattg gtgactttaa caactcagca cgtgaaatac atcagtgatg cactccggcg 4440
tctggaggag gaggagaaag tggtagaaga ggagaaacaa gaacatgtgg agaaggttaa 4500
agaacttttg ggctgggtgt ctaccctagc gaggaataca caaggaaaag ctacctcatc 4560
cgagaccaaa gaatcaacag acattgaaaa agctattttg gaacagcagg ttctgtcaga 4620
agagctgaca acaaagaaag aacaagtctc tgaagctatt aaaacatcac agatcttctt 4680

ggccaagcat ggtcataagc tctcagaaaa agagaagaaa caaatatctg agcaattgaa 4740
tgccctaaac aaggcttacc atgacctttg tgatggttct gcaaatcagc ttcagcagct 4800
tcagagccag ttggctcacc agacagaaca aaagaccctg cagaaacaac aaaatacctg 4860
tcaccagcaa ctggaggatc tttgcagttg ggtaggacag gcagaaagag cactggcagg 4920
ccaccaaggc agaaccaccc agcaggatct ctctgctttg cagaagaacc aaagtgactt 4980
gaaggattta caggatgaca ttcagaatcg tgccacctca tttgccactg ttgtcaagga 5040
cattgagggg ttcatggaag agaatcagac caagctgagc ccacgtgagt tgacagctct 5100
tcgggaaaag cttcatcagg ctaaggagca atatgaggcg ctccaggaag agacacgtgt 5160
ggcccagaag gaactggagg aagcagtgac ctccgcctta cagcaggaga ctgaaaagag 5220
taaagcagca aaggaactgg cagagaacaa gaagaagatc gatgctctcc tggattgggt 5280
aacttcagta ggatcatctg gtggacagct gctgaccaac cttccaggaa tggagcagct 5340
ctcgggagct agcttggaga aaggagcctt ggacaccact gatggttaca tgggggtgaa 5400
tcaagcccca gagaaactgg acaagcaatg tgagatgatg aaggcccgtc accaagaatt 5460
gctgtcccag cagcaaaatt tcattctggc cacccagtca gctcaggcct tcttggatca 5520
gcatggccac aatctcacac ctgaggagca acagatgctg caacagaagc tgggagagct 5580
aaaggaacaa tactctactt ccctggccca atcagaggca gaactgaagc aggtgcagac 5640
acttcaggat gagttgcaga aatttctgca ggatcataaa gagtttgaaa gctggttgga 5700
acgatccgag aaagagctgg agaacatgca taagggaggc agcagccccg agacccttcc 5760
ctccctgcta aagcggcaag gaagcttctc agaggatgtc atttcccaca aaggagactt 5820
gagatttgtg actatctcag gacagaaagt cttggacatg gaaaacagtt ttaaggaagg 5880
caaagaacca tcagaaattg gaaacttagt aaaggacaag ttgaaggatg caacagaaag 5940
atacactgct ctccactcaa agtgtacacg attaggatct cacctgaata tgctgttagg 6000
ccagtatcat caattccaaa acagtgctga cagcctgcag gcctggatgc aggcttgtga 6060
ggccaacgtg gagaagctcc tctcagatac tgttgcctct gaccctggag ttctccagga 6120
gcagcttgca acaacaaagc agttgcagga ggaattggct gagcaccaag tacctgtgga 6180
aaaactccaa aaagtagctc gtgacataat ggaaattgaa ggggagccag ccccagacca 6240
caggcatgtt caagaaacta cagattccat actcagccac ttccaaagcc tctcctatag 6300
cctggctgag cgatcttctc tgctgcagaa agcaattgcc caatctcaga gtgtccagga 6360
aagcctggag agcctgttgc agtctattgg ggaagttgaa caaaacctgg aaggaagca 6420

ggtgtcatca ctctcatcag gagtcatcca ggaagcctta gccacaaata tgaaattgaa 6480
gcaggacatt gctcggcaaa agagcagctt ggaggccacc cgtgagatgg tgacccgatt 6540
catggagaca gcagacagta ctacagcagc agtgctgcag ggcaaactgg cagaggtgag 6600
ccagcggttc gaacagctct gtctacagca gcaagaaaag gagagctccc taaagaagct 6660
tctaccccag gcagagatgt ttgaacacct ctctggtaag ctgcagcagt tcatggaaaa 6720
caaaagtcgg atgctggcct ctggaaatca gccagatcaa gatattacac atttcttcca 6780
acagatccag gagctcaatt tggaaatgga agaccaacag gagaacctag atactcttga 6840
gcacctggtc actgaactga gctcttgtgg ctttgcgctg gacttgtgcc agcatcagga 6900
cagggtacag aatctaagaa aagacttcac agagctacag aagacagtta aagagagaga 6960
gaaagatgca tcatcttgcc aggaacagtt ggatgaattc cggaagctgg tcaggacctt 7020
ccagaaatgg ttgaaagaaa ctgaagggag tattccacct acggaaactt ctatgagtgc 7080
taaagagtta gaaaagcaga ttgaacacct gaagagtcta ctagatgact gggcaagtaa 7140
gggaactctg gtggaagaaa tcaattgcaa aggtacttct ttagaaaatc tcatcatgga 7200
aatcacagca cctgattccc aaggcaagac aggttccata ctgccctctg taggaagctc 7260
tgtaggcagt gtaaacggat accacacctg caaagatctg acggagatcc agtgtgacat 7320
gtcagatgta aacttgaagt atgagaaact agggggagta cttcatgaac gccaggaaag 7380
ccttcaggct atcctcaaca gaatggagga ggttcacaag gaggcaaact ctgtgctgca 7440
gtggctggaa tcaaaagagg aagtcctgaa atccatggat gccatgtcat ctccaaccaa 7500
gacagaaaca gtgaaagccc aagctgaatc taacaaggcc ttcctggctg agttggaaca 7560
gaattctcca aaaattcaaa aagtaaagga agccctggct ggattactgg tgacatatcc 7620
caactcacag gaagcagaaa attggaagaa aattcaggaa gaactcaatt cccgatggga 7680
aagggccact gaggttactg tggctcggca aaggcagcta gaggaatctg caagtcatct 7740
ggcctgcttc caggctgcag aatcccagct ccggccgtgg ctgatggaga aagaactgat 7800
gatgggagtg ctggggcccc tgtctattga ccccaacatg ttgaatgcac aaaagcaaca 7860
ggtccagttt atgctaaagg aatttgaagc acgcaggcaa cagcatgagc aactgaatga 7920
ggcagctcag ggcatcctaa caggccctgg agatgtctct ctgtccacca gccaagtaca 7980
gaaagaactc cagagcatca atcagaaatg ggttgagctg actgacaaac tcaactcccg 8040
ttccagccaa attgaccaag ctattgttaa gagcacccag taccaggaac tgctccagga 8100
cttatcagag aaggtgaggg cagttggaca acggctgagt gtccagtcag ctatcagcac 8160
ccaaccagag gctgtaaagc agcaattgga agagaccagt gaaattcgat ctgacttgga 8220

gcagttagac cacgaggtta aggaggctca gacactgtgc gatgaactct cagtgctcat 8280
tggtgagcag tacctcaagg atgaactgaa gaagcgtttg gagacagttg ccctgcctct 8340
ccaaggttta gaagaccttg cagccgatcg cattaacaga ctccaggcag ctcttgccag 8400
cacccagcag ttccagcaaa tgtttgatga gttgaggacc tggttggatg ataaacaaag 8460
ccagcaagca aaaaactgcc caatttctgc aaaattggag cggctacagt ctcagctaca 8520
ggagaatgaa gagtttcaga aaagtcttaa tcaacacagt ggctcctatg aggtgattgt 8580
ggctgaaggg gaatctctac ttctttctgt acctcctgga gaagagaaaa ggactctaca 8640
aaaccagttg gttgagctca aaaaccattg ggaagagctt agtaaaaaaa ctgcagacag 8700
acaatccagg ctcaaggatt gtatgcagaa agctcagaaa tatcagtggc atgtggaaga 8760
ccttgtgcca tggatagaag attgtaaagc taagatgtct gagttgcgag tcactctgga 8820
tccagtgcag ctagagtcca gtctcctaag atcaaaggct atgctgaatg aggtggagaa 8880
gcgccgctcc ctgctggaaa tattgaatag tgctgctgac attctgatca attcttcaga 8940
agcagatgag gatggaatcc gggatgagaa ggctgggatc aaccagaaca tggatgctgt 9000
tacagaagag ctgcaggcca aaacagggtc actcgaagaa atgactcaga ggctcaggga 9060
gttccaggaa agctttaaga atattgaaaa gaaggttgaa ggagccaaac accaacttga 9120
gatctttgat gctctgggtt ctcaagcctg tagcaacaag aacctggaga agctaagagc 9180
tcaacaggaa gtgctgcagg ccctagagcc tcaggtagac tatctgagga actttactca 9240
gggtctggta gaagatgccc cagatggatc tgatgcttct caacttctcc accaagctga 9300
ggtcgcccag caagagttcc tcgaagttaa gcaaagagtg aacagtggtt gtgtgatgat 9360
ggaaaacaag ctggaggga ttggccagtt tcactgccgg gtccgagaga tgttctctca 9420
attggcagac ctggatgatg agctagatgg catgggtgct attggcagag acactgatag 9480
cctccagtcc caaatcgagg atgtccggct attccttaac aaaattcacg tcctcaaatt 9540
agacatagag gcctctgaag cagagtgtcg acatatgcta gaagaagagg ggactctgga 9600
tttgttaggt ctcaaaaggg agctagaagc cctgaacaaa cagtgtggca aactgacaga 9660
gagggggaaa gctcgtcagg aacagctgga actgacacta ggccgtgtag aggacttcta 9720
caggaaattg aaaggactca atgacgcgac cacagcagca gaggaggcag aggccctcca 9780
gtgggtagtg gggaccgaag tggaaatcat caaccaacaa ttagcagatt ttaaaatgtt 9840
tcagaaagaa caagtggatc ctcttcagat gaaattgcag caggtgaatg gacttggcca 9900
gggattaatt cagagtgcag gaaaagactg tgatgtacag ggtttagaac atgacatgga 9960

agagatcaat gctcgatgga atacattgaa taaaaaggtc gcacaaagaa ttgcacagct 10020
acaggaagct ttgttgcatt gtgggaagtt tcaagatgcc ttggagccat tgctcagctg 10080
gttggcagat accgaggagc tcatagccaa tcagaaacct ccatctgctg agtataaagt 10140
ggtgaaagca cagatccaag aacagaagtt gctccagcgg ctcctagatg atcgaaaggc 10200
cacagtagac atgcttcaag cagaaggagg cagaatagcc cagtcagcag agctggctga 10260
tagagagaaa atcactggac agctggagag tcttgaaagt agatggactg aactactcag 10320
taaggcagca gccaggcaaa aacagctgga agacatcctg gttctggcca aacagttcca 10380
tgagacagct gagcctattt ctgacttctt atctgtcaca gagaaaaagc ttgctaactc 10440
agaacctgtt ggcactcaga ctgccaaaat acagcagcag atcattcggc acaaggctct 10500
ggaagaagac atagaaaacc atgcaacaga tgtgcaccag gcagtcaaaa ttgggcagtc 10560
cctctcctcc ctgacatctc ctgcagaaca gggtgtgctg tcagaaaaga tagactcatt 10620
gcaggcccga tacagtgaaa ttcaagaccg ctgttgtcgg aaggcagccc tacttgacca 10680
agctctgtct aatgctaggc tgtttgggga ggatgaggtg gaggtgctca actggctggc 10740
tgaggttgag gacaagctca gttcagtgtt cgtaaaggat ttcaaacagg atgtcctgca 10800
caggcagcat gctgaccacc tggctttaaa tgaagaaatt gttaatagaa agaagaatgt 10860
agatcaagct attaaaaatg gtcaggctct tctaaaacaa accacaggtg aggaggtgtt 10920
acttatccag gaaaaactag atggtataaa gactcgttac gcagacatca cagttactag 10980
ctccaaggcc ctcagaactt tagagcaagc ccggcagctg gccaccaagt tccagtctac 11040
ttatgaggaa ctgaccgggt ggctgaggga ggtggaggag gagctggcaa ccagtggagg 11100
acagtctccc acaggggaac agataccccca gtttcagcag agacagaagg aattaaagaa 11160
ggaggtcatg gagcacaggc tggtgttgga cacagtgaat gaggtgagcc gtgctctctt 11220
agagctggtg ccctggagag ccagagaagg gctggataaa cttgtgtccg atgctaacga 11280
gcagtacaaa ctagtcagtg acactattgg acaaagggtg gatgaaattg atgctgctat 11340
tcagagatca caacagtatg agcaagctgc cgatgcagaa ctagcttggg ttgctgaaac 11400
aaaacggaaa ctgatggctc tgggtccaat tcgcctggaa caggaccaga ccacagctca 11460
gcttcaggta cagaaggctt tctccattga cattattcga cacaaagatt caatggatga 11520
actcttcagt caccgtagtg aaatctttgg cacatgtggg gaggagcaaa aactgtatt 11580
acaggaaaag acagagtctc taatacagca atatgaagcc attagcctac tcaattcaga 11640
gcgttatgcc cgcctagagc gggcccaggt cttagtaaac cagttttggg aaacttatga 11700
agagctcagc ccctggattg aggaaactcg ggcactaata gcacagttac cctctccagc 11760

cattgatcat gagcagctca ggcagcaaca agaggaaatg aggcaattaa gggaatctat 11820
tgctgaacac aaacctcata ttgacaaact actaaagata ggcccacaac taaaggaatt 11880
aaaccctgag gaaggggaaa tggtggaaga aaaataccag aaagcagaaa acatgtatgc 11940
ccaaataaag gaggaggtgc gccagcgagc cctggctctg gatgaagccg tgtcccagtc 12000
cacacagatt acagagtttc atgataaaat tgagcctatg ttggagacac tggagaatct 12060
ttcctctcgc ctgcgtatgc caccactgat ccctgctgaa gtagacaaga tcagagagtg 12120
catcagtgac aataagagtg ccaccgtgga gctagaaaaa ctgcagccat cctttgaggc 12180
cttgaagcgc cgtggagagg agcttattgg acgatctcag ggagcagaca aggatctggc 12240
tgcaaaagaa atccaggata aattggatca aatggtattc ttctgggagg acatcaaagc 12300
tcgggctgaa gaacgagaaa tcaaatttct tgatgtcctt gaattagcag agaagttctg 12360
gtatgacatg gcagctctcc tgaccaccat caaagacacc caggatattg tccatgactt 12420
ggaaagccca ggcattgatc cttccatcat caaacaacag gttgaagctg ctgagactat 12480
taaggaagag acagatggtc tgcatgaaga gctggagttt attcggatcc ttggagcaga 12540
tttgattttt gcctgtggag aaactgagaa gcctgaagtg aggaagagca ttgatgagat 12600
gaataatgct tgggagaact taaacaaaac atggaaagag aggctagaaa aacttgagga 12660
tgctatgcaa gctgctgtgc agtatcagga cactcttcag gctatgtttg actggctaga 12720
taacactgtg attaaactct gcaccatgcc ccctgttggc actgacctca atactgttaa 12780
agatcagtta aatgaaatga aggagttcaa agtagaagtt taccaacagc aaattgagat 12840
ggagaagctt aatcaccagg gtgaactgat gttaaagaaa gctactgatg agacggacag 12900
agacattata cgagaaccac tgacagaact caaacacctc tgggagaacc tgggtgagaa 12960
aattgcccac cgacagcaca aactagaagg ggctctgttg gccccttggtc agttccagca 13020
tgccttagag gaactaatga gttggctgac tcataccgaa gagttgttag atgctcagag 13080
accaataagt ggagacccaa aagtcattga agttgagctc gcaaagcacc atgtcctaaa 13140
aaatgatgtt ttggctcatc aagccacagt ggaaacagtc aacaaagctg gcaatgagct 13200
tcttgaatcc agtgctggag atgatgccag cagcttaagg agccgtttgg aagccatgaa 13260
ccaatgctgg gagtcagtgt tacagaaaac agaggagagg gagcagcagc ttcagtcaac 13320
tctgcagcag gcccagggct tccacagtga aattgaagat ttcctcttgg aacttactag 13380
aatggagagc cagctttctg catctaagcc cacaggagga cttcctgaaa ctgctaggga 13440
acagcttgat acacatatgg aactctattc ccagctgaaa gccaaggaag agacttataa 13500

tcaactactt gacaagggca gactcatgct tctaagccgt gacgactctg ggtctggctc 13560
caagacagaa cagagtgtag cacttttgga gcagaagtgg catgtggtca gcagtaagat 13620
ggaagaaaga aagtcaaagc tggaagaggc cctcaacttg gcaacagaat tccagaattc 13680
cctacaagaa tttatcaact ggctcactct agcagagcag agtttaaaca tcgcttctcc 13740
accaagcctg attctaaata ctgtcctttc ccagatagaa gagcacaagg tttttgctaa 13800
tgaagtaaat gctcatcgag accagatcat tgagctggat caaactggga atcaattaaa 13860
gttccttagc caaaagcagg atgttgttct gatcaagaat ttgttggtga gcgtgcagtc 13920
tcgatgggag aaggttgtcc agcgatctat tgaaagaggg cgatcactag atgatgccag 13980
gaagcgggca aaacaattcc atgaagcttg gaaaaaactg attgactggc tagaagatgc 14040
agagagtcac ctggactcag aactagagat atccaatgac ccagacaaaa ttaaacttca 14100
gctttctaag cataaggagt ttcagaagac tcttggtggc aagcagcctg tgtatgatac 14160
cacaattaga actggcagag cactgaaaga aaagactttg cttcccgaag atagtcagaa 14220
acttgacaat ttcctaggag aagtcagaga caaatgggat actgtttgtg gcaagtctgt 14280
ggagcggcag cacaagttgg aggaagccct gctcttttcg ggtcagttca tggatgcttt 14340
gcaggcattg gttgactggt tatacaaggt ggagccacag ctggctgagg accagcccgt 14400
gcacggggac cttgacctcg tcatgaacct catggatgca cacaaggttt tccagaagga 14460
actgggaaag cgaacaggaa ccgttcaggt cctgaagcgg tcaggccgag agctgattga 14520
gaatagtcga gatgacacca cttgggtaaa aggacagctc caggaactga gcactcgctg 14580
ggacactgtc tgtaaactct ctgtttccaa acaaagccgg cttgagcagg ccttaaaaca 14640
agcggaagtg tttcgagaca cagtccacat gctgttggag tggctttctg aagcagagca 14700
aacgcttcgc tttcggggag cacttcctga tgacacagag gccctgcagt ctctcattga 14760
cacccataag gaattcatga agaaagtaga agaaaagcga gtggacgtta actcagcagt 14820
agccatggga gaagtcatcc tggctgtctg ccaccccgat tgcatcacaa ccatcaaaca 14880
ctggatcacc atcatccgag ctcgcttcga ggaggtcctg acatgggcta agcagcacca 14940
gcagcgtctt gaaacggcct tgtcagaact ggtggctaat gctgagctcc tggaagaact 15000
tctggcatgg atccagtggg ctgagaccac cctcattcag cgggatcagg agccaatccc 15060
gcagaacatt gaccgagtta aagcccttat cgctgagcat cagacattta tggaggagat 15120
gactcgcaaa cagcctgacg tggaccgggt caccaagaca tacaaaagga aaaacataga 15180
gcctactcac gcgcctttca tagagaaatc ccgcagcgga ggcaggaaat ccctaagtca 15240
gccaacccct cctcccatgc caatcctttc acagtctgaa gcaaaaaacc cacggatcaa 15300

ccagctttct gcccgctggc agcaggtgtg gctgttagca ctggagcggc aaaggaaact 15360
gaatgatgcc ttggatcggc tggaggagtt gaaagaattt gccaactttg actttgatgt 15420
ctggaggaaa aagtatatgc gttggatgaa tcacaaaaag tctcgagtga tggatttctt 15480
ccggcgcatt gataaggacc aggatgggaa gataacacgt caggagttta tcgatggcat 15540
tttagcatcc aagttcccca ccaccaagtt agagatgact gctgtggctg acattttcga 15600
ccgagatggg gatggttaca ttgattatta tgaatttgtg gctgctcttc atcccaacaa 15660
ggatgcgtat cgaccaacaa ccgatgcaga taaaatcgaa gatgaggtta caagacaagt 15720
ggctcagtgc aaatgtgcaa aaaggtttca ggtggagcag atcggagaga ataaataccg 15780
gtttggggat tctcagcagt tgcggctggt ccgtattctg cgcagcaccg tgatggttcg 15840
cgttggtgga ggatggatgg ccttggatga atttttagtg aaaaatgatc cctgccgagc 15900
acgaggtaga actaacattg aacttagaga gaaattcatc ctaccagagg gagcatccca 15960
gggaatgacc cccttccgct cacggggtcg aaggtccaaa ccatcttccc gggcagcttc 16020
ccctactcgt tccagctcca gtgctagtca gagtaaccac agctgtacat ccatgccatc 16080
ttctccagcc accccagcca gtggaaccaa ggttatccca tcatcaggta gcaagttgaa 16140
acgaccaaca ccaacttttc attctagtcg gacatccctt gctggtgata ccagcaatag 16200
ttcttccccg gcctccacag gtgccaaaac taatcgggca gaccctaaaa agtctgccag 16260
tcgccctggg agtcgggctg ggagtcgagc cgggagtcga gccagcagcc ggcgaggaag 16320
tgacgcttct gactttgacc tcttagagac gcagtctgct tgttccgaca cttcagaaag 16380
cagcgctgca gggggccaag gcaactccag gagagggcta aacaaacctt ccaaaatccc 16440
aaccatgtct aagaagacca ccactgcctc ccccaggact ccaggtccca agcgataaca 16500
ctgtctaagc acccccaagc cactatccac tttgaatcct gctccataca ttgggtgtat 16560
atttattctg aacgggagaa gttatattgt taaaagtgta aaagaataat tgtgttatga 16620
agctgcctta tttttttct ttttgtaagt tactattttc atgtgaatat ttatgtagat 16680
aaaatttgcc tcctggtaac cctgtaatgg atggggccca gaaatgaaat atttgagaaa 16740
aacaagtgaa aaggtcaaga tacaaatgtg tattaaaaaa aaaaaagcct attaataggg 16800
tttctgcgcg gtgcagggtt gtaaacctgc tttatctttt aggattattc ctaaatgcat 16860
cttctttata aacttgactt gctatctcag caagataaat tatattaaaa aaataagaat 16920
cctgcagtgt ttaaggaact cttttttgt aaatcacgga cacctcaatt agcaagaact 16980
gaggggaggg ctttttccat tgtttaatgt tttgtgattt ttagctaaag agagggaacc 17040

tcatctaagt aacatttgca catgatacag caaaaggagt tcattgcaat actgtctttg 17100
gatattgttt cagtactggg tgtttaaagg acaaatagct gctagaattc aggggtaaat 17160
gtaagtgttc agaaaacgtc agaacatttg gggttttaaa ctgatttgtt gctccctatc 17220
cagcctagac accagtaact cttgtgttca ccaggaccca gacccttggc aagggatagg 17280
ctcgttggtg acattgtgaa tttcagattt gttttatcca cttttttgc tatttattta 17340
aatggtcgat caacttccca caaactgagg aatgaattcc acgagcctgt tctgaaaatg 17400
tggacgtaag acaaacacgt gctcgtcctt taatggagtt caccagcaca cttgttaacc 17460
agtcctgttt gctttcgtct ttttttgtgc gtaataaagt caactgacca agtgaccatg 17520
aaaaggggct gtctggggct cctgtttttt agctgctgtt cttcagctcc gaccatgttg 17580
ctgtgtgatt atctcaattg gttttaattg aggcagaaac tgaagctcta ccaatgaact 17640
gtttagaaac aagacacact tttgtattaa aattgcttgc agtaacaaat attttgtatt 17700
tcctgatttt cttttcaact attaccttat ctataaatgt taccctgggg tataatcatg 17760
ttgtaggtac ttaaatgcat tccgcaaatc aaaatatctt gatggataaa ttatagagct 17820
t 17821

<210> 53
<211> 19271
<212> DNA
<213> Homo sapiens

<400> 53

ggctctgatt cagttttctt tttcttctct tttccttttt tttttttttt tttttttttt 60
tttaggaatg cagagcagtt gctggggtga ttgacctagg cacagtggag atatttccca 120
tcttcaaagc catgcaaaag ggcctccttg accaagacac aggcctagtg cttctggaat 180
ctcaggttat catgtctggc ctcattgccc ctgagacggg tgaaaacctc tctttggagg 240
agggcatagc cagaaacctc attaatcccc agatgtacca gcagctccgg gagctacagg 300
atgccctggc cttaataagc aggcttactg agagcagagg ccctctttct gtggtggaag 360
caattgaaaa gagaataatc agtgagacag ttggactgaa aatcttagaa gctcacctgg 420
caactggagg tttcagtctt tcccctagtg agaactgtat taacctggaa gaggcttttc 480
atcaaggcct catttctgca tggcttcatt cagtattaga gtcttatctt agaacatcca 540
agaatttgat agaccctaac acagctgaga aaattggttt gctggatctg atgcagcgat 600
gtattgtcca ccaggaatca ggattcaaat tactgcctgt caaacaattg gcagggggga 660
tggtgagctt gaaatcaggc cggaaggtta gcattttccg tgcagttcag gaagggctaa 720

```
tagataggca ggtcactgtc cggttgctgg aagctcagct ttttgctggt ggcatagtag    780
atccaagaac aggacacaga cttacagtgg aagaggctgt aagacataat ctgattgacc    840
aagatatggc ctgtgctatc ctcataaggc agcttcagac aggaggcatc atagacactg    900
tcacggggca aaggctaaca atagatgaag cagtgagcaa tgatctagta gctgctaaga    960
tcgcccttgt gattctggag tccctctggt cattcatggg gttgctgtgg cctgaatctg   1020
gagagatcct cccaattaca gatgccctag aacaaggtat tgtgtctact gaactagcac   1080
ataaaatcct tagtaaccga cagcatatta aggctctgtt tctaccagca accacagaga   1140
ttttgtcctg gaagaaagca atagaaagtg gtatcctgga tagagatctt gccaataact   1200
taaaatcgat ttgtatacct gatgtgatgc cccacatgca actagcagac tctgcagaac   1260
aaaatattaa tcctggagca gcagttctac cgtgcagcaa gagccaccct aaggccacag   1320
caagccagag tgagaatctg ttgttccagc tgatgactca cagctatatt aatgtgcaaa   1380
atggacagag gctgcttctg ttagataaag agctgatgga gacactaaca tccagagatg   1440
agtatcaaac aagtcctcca aaagtggttg aaattgggca tcaaaggcaa aaaactcctg   1500
agggattgca agaatcagct aatgtgaaaa tctcaggaac tttcagcagt gggtggactg   1560
tgaggctgcc tgagttccag ttttcttctc agaacaaaga atatcccgat cgggaagatt   1620
gcactacaga aaaaggcaaa aagaccactg tagaaacaga agattcttct gtagagaacc   1680
ctgaacagga tctgtttgta gaacaaaaag agagaaatcc aaacattgat gctttgaagg   1740
taataaataa agtcaaatta gaggtacaaa ggcagttgat aggtacccaa agggaagacc   1800
aaacagcagt gtctgtcaga gaaaatgcca gcaggggaca cctcctgacc atacctcctg   1860
ctgaggcgga aggtgtgccg ttggtggttg acaaagatgt tttttctgtt gaaacaccaa   1920
agaaagaaca tcaacctcta agaaacactt cctttacatg tcagaatgaa caagcacaca   1980
ctcttgagac tgaatatatt catgatgaaa ctggaggatc tcacataaaa ccccaaagca   2040
aaaagttaca agttcaggta aagaaaactc taggtataaa gttagaacta aagtctgaaa   2100
ctgatgggaa tgttcatcct ctggacaaaa aggaaatgtt aaagaaaaca tttctggcta   2160
aggatgacca taaagaaagt caagaagcac agaacatcgc aggtggtagt atgatgatgt   2220
cagaaaagac cgatgaggaa gatagtggca gggaaatttt tctgtcatgc agtcatccat   2280
tagaattgct tgaagaagct accttaaatg tattatctgc acagttacta gatggtggta   2340
tctttcatga acaaacaggt caaaagctct tactaaatga agcaatatcc cgaggcattg   2400
tgccaagtca cactgccgtg aagcttatgg agaagctgaa catgtttcag ggggttcttg   2460
actctcagac ttgtgagtct ttgacaactg aagaagtcat taatgaaggt ctgatggatg   2520
```

agaaattatt acataatgtc ctcatggcag acaaagctat aagtggtgtc ttagaccccc 2580
gtacccagac actgtgctct gtaaaggatg cagttacagt tggacttctt gacaaggaaa 2640
cagccaccag aattttagag aggcaggtgg tgactggtgg aattattgat ctgaaacgag 2700
gcaaaaaagt ttcagtaact ttggcctcaa ctcttggctt ggtggacgtt gctgaccagc 2760
cagaacttat aaatctggag aaagcttcca aaggtagaga tgctgaaaaa acagttaggg 2820
agagattaat tagtttacaa atggaaacaa caggacttat agaccctgat agtaaagcac 2880
ctttaacagt tgtgcagtcc attgacagag gtcttttgga gagagaggag gccgttcgtt 2940
tgttgactaa gcaagtggta gatggaggta tcattcacca tatatctggg atgagacttt 3000
ctgttgataa tgccttcaga catggcttaa ttggtgaaga tttagccgag aaactcaaaa 3060
gagttgagaa cttaaacatc catcagattt ttaatcctga aacgaaggaa aatatttccc 3120
tccctaaagc cataaaatta gatcttatta cctcagacct gaaaagagaa atccaggagg 3180
ttcaggcctt tactggaaac tttgtggatc tcatttctgg tcagagattg accttggcag 3240
aagctaaaaa agaaggactg ttaactaatg aagcagtatt gtctccagga atgatgcatg 3300
gcattgtaga tcccgagaac tgcagaattg tcccctactc agaattagtc aagaaatgta 3360
agattgatat tgaatctgga cagagatatc tagaagtaat tcccttctca gacattaaag 3420
atggggtgag cgacaaagtg cttacattgt ctcaagcaat tcagcttgga aaagtagact 3480
ttgcatctac gctgaaggtt ctagaagccc aggcaaatac tggtggaatc atagatactg 3540
ctactggaaa aagactgaca ttggcatcag ctttggaaga gaaactggtg gatgaaaaca 3600
tggtcagaat tattgcatct catcaggtgt taaatggagg aattgttgac atatttagtg 3660
atcagagagt gactttagta gaagctattg agaaaagact gatcagccct gaactggcaa 3720
atatgatcca aatagatagt tcagagttca gcgatcacag ggctcagatt gaaaagcaag 3780
aagggattga agtgtgtgca ttacaaaatg aatttctagg aaaggatatg ttaattgctt 3840
gtaatcagac tgctgaaatg agttgtaata aagtagaaga gagtgagaga ttatttcaag 3900
ttgaaaatca gtctgcacaa gaaaaggtta aagtgagagt ttctgatggg gagcaggcaa 3960
aaaagagcag ggaaatttcc ttaaaggaat ttgggtgcaa ggatcaacgt aagccaagaa 4020
tgtcttcaga tgctaaagaa tttatcagta tcataaatcc tcataatctt aaaggtaaat 4080
ccttgggcca agtgtcattg acacaccctt actctgaatg tgattttaaa cttaaagaag 4140
tggctagaaa taacatggga aatgatacaa atgaagagca ggaaaaagca gtgacaaaaa 4200
tagaaattat ttctcatatg aagcagtcta cctcatgtct agattctgaa gaaataagag 4260

aaaatcaagg ggaagtgatt ttggaagtac aagaaacata ttgtgaaacg tcaggcaaat 4320
tgccgagtga gcaggttttg cagcaaccaa tgaatgctcg ggtgaaaagt aagagagaga 4380
agagggaggt gattgtagaa gaaagtatca gaacatgcaa accagcattt ctttctgaag 4440
aaaagttgta tcaggaaact gccattagag atgagcatga ctcccatata aagagccaac 4500
ctagggaaat gacctcaagt gaaaaaggga aagaagctga tacagaaatg ggattttcta 4560
ttacttttaa aattgaagag tcctcttccc aagtggtacc tcaaggaatt tctgtaaaac 4620
atttagatgc tttaacactc ttcagctcta aacaggccaa tgaaggaaaa gtaaacaatt 4680
taagtctctg cttgacttta aaaccagaag aaaacttatc tcgagaaatt gcctgtgggg 4740
cccagagtga accattccct tgtatgaccc caagacctga aggattgcac taccaggaat 4800
cagatggaaa agcccaagtg acaggcccat cccaaatttc caaaacagac aagtctttcc 4860
aaggaaccac cagacaggag accaactatc aagattcctg ggttacttcg aaaactaagg 4920
aaaccaaaca tcaaatttcc tcatctaatg aatgtaaaga aaagtcatac caagaagtat 4980
cttttgaccc agcaagaggt cttaaattgg aagaaatcac agtttctaga ccagattcaa 5040
aagaagtcag gtatctagaa ttctcagaca gaaaagacct tcatcatcag ggcagcaaaa 5100
gtgatgataa actttgtgga actctcaaat ctgaaatagc aacacaggaa ctaactggag 5160
agaaatttct agaaatggca aaccctaatg ttgcaggtct agaagcagga tccattgagg 5220
acatagtgac tcagagaggt tccagagtct tgggatcctt tcttccagag aaactgttca 5280
aaggagtgtc tcaaaaagag aatacagggc aacagaatgc catcattagt cctactgttc 5340
tagagaccag tgaagaaaag acagtgtccc taacagtatg ctctgcagtg aagacagaga 5400
agacaccaca ggaaaagctc agagaaagcc ctggcagtga acaaactccc ttcatgactg 5460
cacctgaagg aaagggaaat ggaggtgtaa acccagagcc cttcagagca actcagaatg 5520
tatttacccg gcaactctgt ttagaacatg atgaaaagct agtatcctat ctgtctctgt 5580
tacggaacat tgaaatgagg accaaacaga ttcaaccttt ggagctaaac ctggcagaac 5640
tacaggatct gctgtgtcag gccaaggtat tagaaaggga gttaaaggat ctgaccacct 5700
tggtcagtca ggagctggag tgtgtgaatc agattatcat cagccagcct caagaagttc 5760
ctgctcaact gttgaaggct ctagagaaag atgccaagaa tcttcagaag tctctcagct 5820
ctgtgagtga cacttggaat tccaggctac tccacttcca gaatgctgtg gaaatagaaa 5880
agactaaagt gttaaatcag cacacacagc tagaaggccg acttcaagat ctgagagcct 5940
gggttggcaa taaaaatctt attctgaaca gcaagggatc taacagtgaa atagatgttg 6000
acagcctgaa cctctgcctc caacagtatg aggatttgaa acagcccatg gctgaaagga 6060

aagctcagct ggatgctctt gcttttgata ttcagttctt tatctcagaa catgcccagg 6120
acttgtcccc tcagcagaat cgacagatgc tgaggcttct gaatgaactg cagaggtcct 6180
tccaggacat tttggaacag actgccgctc aggtggatgc cttgcagggc catcttcaac 6240
aaatggagca ggaagccctg gtgaagaccc tgcagaaaca acaaaatacc tgtcaccagc 6300
aactggagga tctttgcagt tgggtaggac aggcagaaag agcactggca ggccaccaag 6360
gcagaaccac ccagcaggat ctctctgctt tgcagaagaa ccaaagtgac ttgaaggatt 6420
tacaggatga cattcagaat cgtgccacct catttgccac tgttgtcaag gacattgagg 6480
ggttcatgga agagaatcag accaagctga gcccacgtga gttgacagct cttcgggaaa 6540
agcttcatca ggctaaggag caatatgagg cgctccagga agagacacgt gtggcccaga 6600
aggaactgga ggaagcagtg acctccgcct tacagcagga gactgaaaag agtaaagcag 6660
caaaggaact ggcagagaac aagaagaaga tcgatgctct cctggattgg gtaacttcag 6720
taggatcatc tggtggacag ctgctgacca accttccagg aatggagcag ctctcgggag 6780
ctagcttgga gaaaggagcc ttggacacca ctgatggtta catgggggtg aatcaagccc 6840
cagagaaact ggacaagcaa tgtgagatga tgaaggcccg tcaccaagaa ttgctgtccc 6900
agcagcaaaa tttcattctg gccacccagt cagctcaggc cttcttggat cagcatggcc 6960
acaatctcac acctgaggag caacagatgc tgcaacagaa gctgggagag ctaaaggaac 7020
aatactctac ttccctggcc caatcagagg cagaactgaa gcaggtgcag acacttcagg 7080
atgagttgca gaaatttctg caggatcata aagagtttga aagctggttg gaacgatccg 7140
agaaagagct ggagaacatg cataagggag gcagcagccc cgagaccctt ccctccctgc 7200
taaagcggca aggaagcttc tcagaggatg tcatttccca caaaggagac ttgagatttg 7260
tgactatctc aggacagaaa gtcttggaca tggaaaacag ttttaaggaa ggcaaagaac 7320
catcagaaat tggaaactta gtaaaggaca agttgaagga tgcaacagaa agatacactg 7380
ctctccactc aaagtgtaca cgattaggat ctcacctgaa tatgctgtta ggccagtatc 7440
atcaattcca aaacagtgct gacagcctgc aggcctggat gcaggcttgt gaggccaacg 7500
tggagaagct cctctcagat actgttgcct ctgaccctgg agttctccag gagcagcttg 7560
caacaacaaa gcagttgcag gaggaattgg ctgagcacca agtacctgtg gaaaaactcc 7620
aaaaagtagc tcgtgacata atggaaattg aaggggagcc agccccagac cacaggcatg 7680
ttcaagaaac tacagattcc atactcagcc acttccaaag cctctcctat agcctggctg 7740
agcgatcttc tctgctgcag aaagcaattg cccaatctca gagtgtccag gaaagcctgg 7800

agagcctgtt gcagtctatt ggggaagttg aacaaaacct ggaagggaag caggtgtcat 7860
cactctcatc aggagtcatc caggaagcct tagccacaaa tatgaaattg aagcaggaca 7920
ttgctcggca aaagagcagc ttggaggcca cccgtgagat ggtgacccga ttcatggaga 7980
cagcagacag tactacagca gcagtgctgc agggcaaact ggcagaggtg agccagcggt 8040
tcgaacagct ctgtctacag cagcaagaaa aggagagctc cctaaagaag cttctacccc 8100
aggcagagat gtttgaacac ctctctggta agctgcagca gttcatggaa aacaaaagtc 8160
ggatgctggc ctctggaaat cagccagatc aagatattac acatttcttc caacagatcc 8220
aggagctcaa tttggaaatg gaagaccaac aggagaacct agatactctt gagcacctgg 8280
tcactgaact gagctcttgt ggctttgcgc tggacttgtg ccagcatcag gacagggtac 8340
agaatctaag aaaagacttc acagagctac agaagacagt taaagagaga gagaaagatg 8400
catcatcttg ccaggaacag ttggatgaat tccggaagct ggtcaggacc ttccagaaat 8460
ggttgaaaga aactgaaggg agtattccac ctacggaaac ttctatgagt gctaaagagt 8520
tagaaaagca gattgaacac ctgaagagtc tactagatga ctgggcaagt aagggaactc 8580
tggtggaaga aatcaattgc aaaggtactt ctttagaaaa tctcatcatg gaaatcacag 8640
cacctgattc ccaaggcaag acaggttcca tactgccctc tgtaggaagc tctgtaggca 8700
gtgtaaacgg ataccacacc tgcaaagatc tgacggagat ccagtgtgac atgtcagatg 8760
taaacttgaa gtatgagaaa ctaggggggag tacttcatga acgccaggaa agccttcagg 8820
ctatcctcaa cagaatggag gaggttcaca aggaggcaaa ctctgtgctg cagtggctgg 8880
aatcaaaaga ggaagtcctg aaatccatgg atgccatgtc atctccaacc aagacagaaa 8940
cagtgaaagc ccaagctgaa tctaacaagg ccttcctggc tgagttggaa cagaattctc 9000
caaaaattca aaaagtaaag gaagccctgg ctggattact ggtgacatat cccaactcac 9060
aggaagcaga aaattggaag aaaattcagg aagaactcaa ttcccgatgg gaaagggcca 9120
ctgaggttac tgtggctcgg caaaggcagc tagaggaatc tgcaagtcat ctggcctgct 9180
tccaggctgc agaatcccag ctccggccgt ggctgatgga gaaagaactg atgatgggag 9240
tgctggggcc cctgtctatt gaccccaaca tgttgaatgc acaaaagcaa caggtccagt 9300
ttatgctaaa ggaatttgaa gcacgcaggc aacagcatga gcaactgaat gaggcagctc 9360
agggcatcct aacaggccct ggagatgtct ctctgtccac cagccaagta cagaaagaac 9420
tccagagcat caatcagaaa tgggttgagc tgactgacaa actcaactcc cgttccagcc 9480
aaattgacca agctattgtt aagagcaccc agtaccagga actgctccag gacttatcag 9540
agaaggtgag ggcagttgga caacggctga gtgtccagtc agctatcagc acccaaccag 9600

aggctgtaaa gcagcaattg gaagagacca gtgaaattcg atctgacttg gagcagttag 9660
accacgaggt taaggaggct cagacactgt gcgatgaact ctcagtgctc attggtgagc 9720
agtacctcaa ggatgaactg aagaagcgtt tggagacagt tgccctgcct ctccaaggtt 9780
tagaagacct tgcagccgat cgcattaaca gactccaggc agctcttgcc agcacccagc 9840
agttccagca aatgtttgat gagttgagga cctggttgga tgataaacaa agccagcaag 9900
caaaaaactg cccaatttct gcaaaattgg agcggctaca gtctcagcta caggagaatg 9960
aagagtttca gaaaagtctt aatcaacaca gtggctccta tgaggtgatt gtggctgaag 10020
gggaatctct acttctttct gtacctcctg gagaagagaa aaggactcta caaaaccagt 10080
tggttgagct caaaaaccat tgggaagagc ttagtaaaaa aactgcagac agacaatcca 10140
ggctcaagga ttgtatgcag aaagctcaga aatatcagtg gcatgtggaa gaccttgtgc 10200
catggataga agattgtaaa gctaagatgt ctgagttgcg agtcactctg gatccagtgc 10260
agctagagtc cagtctccta agatcaaagg ctatgctgaa tgaggtggag aagcgccgct 10320
ccctgctgga aatattgaat agtgctgctg acattctgat caattcttca gaagcagatg 10380
aggatggaat ccgggatgag aaggctggga tcaaccagaa catggatgct gttacagaag 10440
agctgcaggc caaaacaggg tcactcgaag aaatgactca gaggctcagg gagttccagg 10500
aaagctttaa gaatattgaa aagaaggttg aaggagccaa acaccaactt gagatctttg 10560
atgctctggg ttctcaagcc tgtagcaaca agaacctgga gaagctaaga gctcaacagg 10620
aagtgctgca ggccctagag cctcaggtag actatctgag gaactttact cagggtctgg 10680
tagaagatgc cccagatgga tctgatgctt ctcaacttct ccaccaagct gaggtcgccc 10740
agcaagagtt cctcgaagtt aagcaaagag tgaacagtgg ttgtgtgatg atggaaaaca 10800
agctggaggg gattggccag tttcactgcc gggtccgaga gatgttctct caattggcag 10860
acctggatga tgagctagat ggcatgggtg ctattggcag agacactgat agcctccagt 10920
cccaaatcga ggatgtccgg ctattcctta acaaaattca cgtcctcaaa ttagacatag 10980
aggcctctga agcagagtgt cgacatatgc tagaagaaga ggggactctg gatttgttag 11040
gtctcaaaag ggagctagaa gccctgaaca aacagtgtgg caaactgaca gagaggggga 11100
aagctcgtca ggaacagctg gaactgacac taggccgtgt agaggacttc tacaggaaat 11160
tgaaaggact caatgacgcg accacagcag cagaggaggc agaggccctc cagtgggtag 11220
tggggaccga agtggaaatc atcaaccaac aattagcaga ttttaaaatg tttcagaaag 11280
aacaagtgga tcctcttcag atgaaattgc agcaggtgaa tggacttggc cagggattaa 11340

ttcagagtgc aggaaaagac tgtgatgtac agggtttaga acatgacatg gaagagatca 11400
atgctcgatg gaatacattg aataaaaagg tcgcacaaag aattgcacag ctacaggaag 11460
ctttgttgca ttgtgggaag tttcaagatg ccttggagcc attgctcagc tggttggcag 11520
ataccgagga gctcatagcc aatcagaaac ctccatctgc tgagtataaa gtggtgaaag 11580
cacagatcca agaacagaag ttgctccagc ggctcctaga tgatcgaaag gccacagtag 11640
acatgcttca agcagaagga ggcagaatag cccagtcagc agagctggct gatagagaga 11700
aaatcactgg acagctggag agtcttgaaa gtagatggac tgaactactc agtaaggcag 11760
cagccaggca aaaacagctg gaagacatcc tggttctggc caaacagttc catgagacag 11820
ctgagcctat ttctgacttc ttatctgtca cagagaaaaa gcttgctaac tcagaacctg 11880
ttggcactca gactgccaaa atacagcagc agatcattcg gcacaaggct ctggaagaag 11940
acatagaaaa ccatgcaaca gatgtgcacc aggcagtcaa aattgggcag tccctctcct 12000
ccctgacatc tcctgcagaa cagggtgtgc tgtcagaaaa gatagactca ttgcaggccc 12060
gatacagtga aattcaagac cgctgttgtc ggaaggcagc cctacttgac caagctctgt 12120
ctaatgctag gctgtttggg gaggatgagg tggaggtgct caactggctg gctgaggttg 12180
aggacaagct cagttcagtg ttcgtaaagg atttcaaaca ggatgtcctg cacaggcagc 12240
atgctgacca cctggcttta aatgaagaaa ttgttaatag aaagaagaat gtagatcaag 12300
ctattaaaaa tggtcaggct cttctaaaac aaaccacagg tgaggaggtg ttacttatcc 12360
aggaaaaact agatggtata aagactcgtt acgcagacat cacagttact agctccaagg 12420
ccctcagaac tttagagcaa gcccggcagc tggccaccaa gttccagtct acttatgagg 12480
aactgaccgg gtggctgagg gaggtggagg aggagctggc aaccagtgga ggacagtctc 12540
ccacagggga acagataccc cagtttcagc agagacagaa ggaattaaag aaggaggtca 12600
tggagcacag gctggtgttg gacacagtga atgaggtgag ccgtgctctc ttagagctgg 12660
tgccctggag agccagagaa gggctggata aacttgtgtc cgatgctaac gagcagtaca 12720
aactagtcag tgacactatt ggacaaaggg tggatgaaat tgatgctgct attcagagat 12780
cacaacagta tgagcaagct gccgatgcag aactagcttg ggttgctgaa acaaaacgga 12840
aactgatggc tctgggtcca attcgcctgg aacaggacca gaccacagct cagcttcagg 12900
tacagaaggc tttctccatt gacattattc gacacaaaga ttcaatggat gaactcttca 12960
gtcaccgtag tgaaatcttt ggcacatgtg gggaggagca aaaaactgta ttacaggaaa 13020
agacagagtc tctaatacag caatatgaag ccattagcct actcaattca gagcgttatg 13080
cccgcctaga gcgggcccag gtcttagtaa accagttttg ggaaacttat gaagagctca 13140

gcccctggat tgaggaaact cgggcactaa tagcacagtt accctctcca gccattgatc 13200
atgagcagct caggcagcaa caagaggaaa tgaggcaatt aagggaatct attgctgaac 13260
acaaacctca tattgacaaa ctactaaaga taggcccaca actaaaggaa ttaaaccctg 13320
aggaagggga aatggtggaa gaaaaatacc agaaagcaga aaacatgtat gcccaaataa 13380
aggaggaggt gcgccagcga gccctggctc tggatgaagc cgtgtcccag tccacacaga 13440
ttacagagtt tcatgataaa attgagccta tgttggagac actggagaat ctttcctctc 13500
gcctgcgtat gccaccactg atccctgctg aagtagacaa gatcagagag tgcatcagtg 13560
acaataagag tgccaccgtg gagctagaaa aactgcagcc atcctttgag gccttgaagc 13620
gccgtggaga ggagcttatt ggacgatctc agggagcaga caaggatctg gctgcaaaag 13680
aaatccagga taaattggat caaatggtat tcttctggga ggacatcaaa gctcgggctg 13740
aagaacgaga aatcaaattt cttgatgtcc ttgaattagc agagaagttc tggtatgaca 13800
tggcagctct cctgaccacc atcaaagaca cccaggatat tgtccatgac ttggaaagcc 13860
caggcattga tccttccatc atcaaacaac aggttgaagc tgctgagact attaaggaag 13920
agacagatgg tctgcatgaa gagctggagt ttattcggat ccttggagca gatttgattt 13980
ttgcctgtgg agaaactgag aagcctgaag tgaggaagag cattgatgag atgaataatg 14040
cttgggagaa cttaaacaaa acatggaaag agaggctaga aaaacttgag gatgctatgc 14100
aagctgctgt gcagtatcag gacactcttc aggctatgtt tgactggcta gataacactg 14160
tgattaaact ctgcaccatg ccccctgttg gcactgacct caatactgtt aaagatcagt 14220
taaatgaaat gaaggagttc aaagtagaag tttaccaaca gcaaattgag atggagaagc 14280
ttaatcacca gggtgaactg atgttaaaga aagctactga tgagacggac agagacatta 14340
tacgagaacc actgacagaa ctcaaacacc tctgggagaa cctgggtgag aaaattgccc 14400
accgacagca caaactagaa ggggctctgt tggcccttgg tcagttccag catgccttag 14460
aggaactaat gagttggctg actcataccg aagagttgtt agatgctcag agaccaataa 14520
gtggagaccc aaaagtcatt gaagttgagc tcgcaaagca ccatgtccta aaaaatgatg 14580
ttttggctca tcaagccaca gtggaaacag tcaacaaagc tggcaatgag cttcttgaat 14640
ccagtgctgg agatgatgcc agcagcttaa ggagccgttt ggaagccatg aaccaatgct 14700
gggagtcagt gttacagaaa acagaggaga gggagcagca gcttcagtca actctgcagc 14760
aggcccaggg cttccacagt gaaattgaag atttcctctt ggaacttact agaatggaga 14820
gccagctttc tgcatctaag cccacaggag gacttcctga aactgctagg gaacagcttg 14880

atacacatat ggaactctat tcccagctga aagccaagga agagacttat aatcaactac 14940

ttgacaaggg cagactcatg cttctaagcc gtgacgactc tgggtctggc tccaagacag 15000

aacagagtgt agcacttttg gagcagaagt ggcatgtggt cagcagtaag atggaagaaa 15060

gaaagtcaaa gctggaagag gccctcaact tggcaacaga attccagaat tccctacaag 15120

aatttatcaa ctggctcact ctagcagagc agagtttaaa catcgcttct ccaccaagcc 15180

tgattctaaa tactgtcctt tcccagatag aagagcacaa ggtttttgct aatgaagtaa 15240

atgctcatcg agaccagatc attgagctgg atcaaactgg gaatcaatta aagttcctta 15300

gccaaaagca ggatgttgtt ctgatcaaga atttgttggt gagcgtgcag tctcgatggg 15360

agaaggttgt ccagcgatct attgaaagag ggcgatcact agatgatgcc aggaagcggg 15420

caaaacaatt ccatgaagct tggaaaaaac tgattgactg gctagaagat gcagagagtc 15480

acctggactc agaactagag atatccaatg acccagacaa aattaaactt cagctttcta 15540

agcataagga gtttcagaag actcttggtg gcaagcagcc tgtgtatgat accacaatta 15600

gaactggcag agcactgaaa gaaaagactt tgcttcccga agatagtcag aaacttgaca 15660

atttcctagg agaagtcaga gacaaatggg atactgtttg tggcaagtct gtggagcggc 15720

agcacaagtt ggaggaagcc ctgctctttt cgggtcagtt catggatgct ttgcaggcat 15780

tggttgactg gttatacaag gtggagccac agctggctga ggaccagccc gtgcacgggg 15840

accttgacct cgtcatgaac ctcatggatg cacacaaggt tttccagaag gaactgggaa 15900

agcgaacagg aaccgttcag gtcctgaagc ggtcaggccg agagctgatt gagaatagtc 15960

gagatgacac cacttgggta aaggacagc tccaggaact gagcactcgc tgggacactg 16020

tctgtaaact ctctgtttcc aaacaaagcc ggcttgagca ggccttaaaa caagcggaag 16080

tgtttcgaga cacagtccac atgctgttgg agtggctttc tgaagcagag caaacgcttc 16140

gctttcgggg agcacttcct gatgacacag aggccctgca gtctctcatt gacacccata 16200

aggaattcat gaagaaagta gaagaaaagc gagtggacgt taactcagca gtagccatgg 16260

gagaagtcat cctggctgtc tgccaccccg attgcatcac aaccatcaaa cactggatca 16320

ccatcatccg agctcgcttc gaggaggtcc tgacatgggc taagcagcac cagcagcgtc 16380

ttgaaacggc cttgtcagaa ctggtggcta atgctgagct cctggaagaa cttctggcat 16440

ggatccagtg ggctgagacc accctcattc agcgggatca ggagccaatc ccgcagaaca 16500

ttgaccgagt taaagccctt atcgctgagc atcagacatt tatggaggag atgactcgca 16560

aacagcctga cgtggaccgg gtcaccaaga catacaaaag gaaaaacata gagcctactc 16620

acgcgccttt catagagaaa tcccgcagcg gaggcaggaa atccctaagt cagccaaccc 16680

ctcctcccat gccaatcctt tcacagtctg aagcaaaaaa cccacggatc aaccagcttt 16740
ctgcccgctg gcagcaggtg tggctgttag cactggagcg gcaaaggaaa ctgaatgatg 16800
ccttggatcg gctggaggag ttgaaagaat ttgccaactt tgactttgat gtctggagga 16860
aaaagtatat gcgttggatg aatcacaaaa agtctcgagt gatggatttc ttccggcgca 16920
ttgataagga ccaggatggg aagataacac gtcaggagtt tatcgatggc attttagcat 16980
ccaagttccc caccaccaag ttagagatga ctgctgtggc tgacattttc gaccgagatg 17040
gggatggtta cattgattat tatgaatttg tggctgctct tcatcccaac aaggatgcgt 17100
atcgaccaac aaccgatgca gataaaatcg aagatgaggt tacaagacaa gtggctcagt 17160
gcaaatgtgc aaaaaggttt caggtggagc agatcggaga gaataaatac cggttcttcc 17220
tcggcaatca gtttggggat tctcagcagt tgcggctggt ccgtattctg cgcagcaccg 17280
tgatggttcg cgttggtgga ggatggatgg ccttggatga atttttagtg aaaaatgatc 17340
cctgccgagc acgaggtaga actaacattg aacttagaga gaaattcatc ctaccagagg 17400
gagcatccca gggaatgacc cccttccgct cacggggtcg aaggtccaaa ccatcttccc 17460
gggcagcttc ccctactcgt tccagctcca gtgctagtca gagtaaccac agctgtacat 17520
ccatgccatc ttctccagcc accccagcca gtggaaccaa ggttatccca tcatcaggta 17580
gcaagttgaa acgaccaaca ccaacttttc attctagtcg gacatccctt gctggtgata 17640
ccagcaatag ttcttccccg gcctccacag gtgccaaaac taatcgggca gaccctaaaa 17700
agtctgccag tcgccctggg agtcgggctg ggagtcgagc cgggagtcga gccagcagcc 17760
ggcgaggaag tgacgcttct gactttgacc tcttagagac gcagtctgct tgttccgaca 17820
cttcagaaag cagcgctgca gggggccaag gcaactccag gagagggcta aacaaacctt 17880
ccaaaatccc aaccatgtct aagaagacca ccactgcctc ccccaggact ccaggtccca 17940
agcgataaca ctgtctaagc acccccaagc cactatccac tttgaatcct gctccataca 18000
ttgggtgtat atttattctg aacgggagaa gttatattgt taaaagtgta aaagaataat 18060
tgtgttatga agctgcctta tttttttct ttttgtaagt tactattttc atgtgaatat 18120
ttatgtagat aaaatttgcc tcctggtaac cctgtaatgg atggggccca gaaatgaaat 18180
atttgagaaa aacaagtgaa aaggtcaaga tacaaatgtg tattaaaaaa aaaaaagcct 18240
attaataggg tttctgcgcg gtgcagggtt gtaaacctgc tttatctttt aggattattc 18300
ctaaatgcat cttctttata aacttgactt gctatctcag caagataaat tatattaaaa 18360
aaataagaat cctgcagtgt ttaaggaact cttttttgt aaatcacgga cacctcaatt 18420

agcaagaact gaggggaggg ctttttccat tgtttaatgt tttgtgattt ttagctaaag 18480

agagggaacc tcatctaagt aacatttgca catgatacag caaaaggagt tcattgcaat 18540

actgtctttg gatattgttt cagtactggg tgtttaaagg acaaatagct gctagaattc 18600

aggggtaaat gtaagtgttc agaaaacgtc agaacatttg gggttttaaa ctgatttgtt 18660

gctccctatc cagcctagac accagtaact cttgtgttca ccaggaccca gacccttggc 18720

aagggatagg ctcgttggtg acattgtgaa tttcagattt gttttatcca ctttttttgc 18780

tatttattta aatggtcgat caacttccca caaactgagg aatgaattcc acgagcctgt 18840

tctgaaaatg tggacgtaag acaaacacgt gctcgtcctt taatggagtt caccagcaca 18900

cttgttaacc agtcctgttt gctttcgtct ttttttgtgc gtaataaagt caactgacca 18960

agtgaccatg aaaaggggct gtctggggct cctgtttttt agctgctgtt cttcagctcc 19020

gaccatgttg ctgtgtgatt atctcaattg gttttaattg aggcagaaac tgaagctcta 19080

ccaatgaact gtttagaaac aagacacact tttgtattaa aattgcttgc agtaacaaat 19140

attttgtatt tcctgatttt cttttcaact attaccttat ctataaatgt taccctgggg 19200

tataatcatg ttgtaggtac ttaaatgcat tccgcaaatc aaaatatctt gatggataaa 19260

ttatagagct t 19271

<210> 54
<211> 2688
<212> DNA
<213> Homo sapiens

<400> 54

catctcccgg cggcgggccg cggaagcagt gcagacgcgg ctcctagcgg atgggtgcta 60

ttgtgaggcg gttgtagaag agtttcgtga gtgctcgcag ctcatacctg tggctgtgta 120

tccgtggcca cagctggttg gcgtcgcctt gaaatcccag gccgtgagga gttagcgagc 180

cctgctcaca ctcggcgctc tggttttcgg tgggtgtgcc ctgcacctgc ctcttccccc 240

attctcatta ataaaggtat ccatggagaa cactgaaaac tcagtggatt caaaatccat 300

taaaaatttg gaaccaaaga tcatacatgg aagcgaatca atggactctg gaatatccct 360

ggacaacagt tataaaatgg attatcctga gatgggttta tgtataataa ttaataataa 420

gaattttcat aaaagcactg gaatgacatc tcggtctggt acagatgtcg atgcagcaaa 480

cctcagggaa acattcagaa acttgaaata tgaagtcagg aataaaaatg atcttacacg 540

tgaagaaatt gtggaattga tgcgtgatgt ttctaaagaa gatcacagca aaaggagcag 600

ttttgtttgt gtgcttctga gccatggtga agaaggaata atttttggaa caaatggacc 660

tgttgacctg aaaaaaataa caaacttttt cagaggggat cgttgtagaa gtctaactgg 720
aaaacccaaa cttttcatta ttcaggcctg ccgtggtaca gaactggact gtggcattga 780
gacagacagt ggtgttgatg atgacatggc gtgtcataaa ataccagtgg aggccgactt 840
cttgtatgca tactccacag cacctggtta ttattcttgg cgaaattcaa aggatggctc 900
ctggttcatc cagtcgcttt gtgccatgct gaaacagtat gccgacaagc ttgaatttat 960
gcacattctt acccgggtta accgaaaggt ggcaacagaa tttgagtcct tttcctttga 1020
cgctactttt catgcaaaga aacagattcc atgtattgtt tccatgctca caaaagaact 1080
ctatttttat cactaaagaa atggttggtt ggtggttttt tttagtttgt atgccaagtg 1140
agaagatggt atatttggta ctgtatttcc ctctcatttt gacctactct catgctgcag 1200
agggtacttt aagacatact ccttccatca aatagaacca ctatgaagct acctcaaact 1260
tccagtcagg tagttgcaat tgaattaaat taggaataaa taaaaatgga tactggtgca 1320
gtcattatga gaggcaatga ttgttaattt acagctttca tgattagcaa gttacagtga 1380
tgctgtgcta tgaattttca agtaattgtg aaaaagttaa acattgaagt aatgaatttt 1440
tatgatattc cccccactta agactgtgta ttctagtttt gtcaaactgt agaaatgatg 1500
atgtggaaga acttaggcat ctgtgggcat ggtcaaaggc tcaaaccttt attttagaat 1560
tgatatacac ggatgactta actgcatttt tagaccattt atctgggatt atggttttgt 1620
gatgtttgtc ctgaacactt ttgttgtaaa aaaataataa taatgtttaa tattgagaaa 1680
gaaactaata ttttatgtga gagaaagtgt gagcaaacta acttgacttt taaggctaaa 1740
acttaacatt catagagggg tggagtttta actgtaaggt gctacaatgc ccctggatct 1800
accagcataa atatcttctg atttgtccct atgcatatca gttgagcttc atataccagc 1860
aatatatctg aagagctatt atataaaaac cccaaactgt tgattattag ccaggtaatg 1920
tgaataaatt ctataggaac atatgaaaat acaacttaaa taataaacag tggaatataa 1980
ggaaagcaat aaatgaatgg gctgagctgc ctgtaacttg agagtagatg gtttgagcct 2040
gagcagagac atgactcagc ctgttccatg aaggcagagc catggaccac gcaggaaggg 2100
cctacagccc atttctccat acgcactggt atgtgtggat gatgctgcca gggcgccatc 2160
gccaagtaag aaagtgaagc aaatcagaaa cttgtgaagt ggaaatgttc taaaggtggt 2220
gaggcaataa aaatcatagt actctttgta gcaaaattct taagtatgtt attttctgtt 2280
gaagtttaca atcaaaggaa aatagtaatg ttttatactg tttactgaaa gaaaaagacc 2340
tatgagcaca taggactcta gacggcatcc agccggaggc cagagctgag ccctcagccc 2400
gggaggcagg ctccaggcct cagcaggtgc ggagccgtca ctgcaccaag tctcactggc 2460

tgtcagtatg acatttcacg ggagatttct tgttgctcaa aaaatgagct cgcatttgtc 2520

aatgacagtt tctttttct tactagacct gtaacttttg taaatacaca tagcatgtaa 2580

tggtatctta aagtgtgttt ctatgtgaca attttgtaca aatttgttat tttccatttt 2640

tatttcaaaa tatacattca aacttaaaat taaaaaaaaa aaaaaaaa 2688

<210> 55
<211> 2522
<212> DNA
<213> Homo sapiens

<400> 55

acatctcccg gcggcgggcc gcggaagcag tgcagacgcg gctcctagcg gatgggtgct 60
attgtgaggc ggttgtagaa gttaataaag gtatccatgg agaacactga aaactcagtg 120
gattcaaaat ccattaaaaa tttggaacca aagatcatac atggaagcga atcaatggac 180
tctggaatat ccctggacaa cagttataaa atggattatc ctgagatggg tttatgtata 240
ataattaata ataagaattt tcataaaagc actggaatga catctcggtc tggtacagat 300
gtcgatgcag caaacctcag ggaaacattc agaaacttga aatatgaagt caggaataaa 360
aatgatctta cacgtgaaga aattgtggaa ttgatgcgtg atgtttctaa agaagatcac 420
agcaaaagga gcagttttgt ttgtgtgctt ctgagccatg gtgaagaagg aataattttt 480
ggaacaaatg gacctgttga cctgaaaaaa ataacaaact ttttcagagg ggatcgttgt 540
agaagtctaa ctggaaaacc caaacttttc attattcagg cctgccgtgg tacagaactg 600
gactgtggca ttgagacaga cagtggtgtt gatgatgaca tggcgtgtca taaaatacca 660
gtggaggccg acttcttgta tgcatactcc acagcacctg gttattattc ttggcgaaat 720
tcaaaggatg gctcctggtt catccagtcg ctttgtgcca tgctgaaaca gtatgccgac 780
aagcttgaat ttatgcacat tcttacccgg gttaaccgaa aggtggcaac agaatttgag 840
tccttttcct ttgacgctac ttttcatgca aagaaacaga ttccatgtat tgtttccatg 900
ctcacaaaag aactctattt ttatcactaa agaaatggtt ggttggtggt ttttttagt 960
ttgtatgcca agtgagaaga tggtatattt ggtactgtat ttccctctca ttttgaccta 1020
ctctcatgct gcagagggta ctttaagaca tactccttcc atcaaataga accactatga 1080
agctacctca aacttccagt caggtagttg caattgaatt aaattaggaa taaataaaaa 1140
tggatactgg tgcagtcatt atgagaggca atgattgtta atttacagct ttcatgatta 1200
gcaagttaca gtgatgctgt gctatgaatt ttcaagtaat tgtgaaaaag ttaaacattg 1260
aagtaatgaa tttttatgat attcccccca cttaagactg tgtattctag ttttgtcaaa 1320

```
ctgtagaaat gatgatgtgg aagaacttag gcatctgtgg gcatggtcaa aggctcaaac   1380
ctttatttta gaattgatat acacggatga cttaactgca tttttagacc atttatctgg   1440
gattatggtt ttgtgatgtt tgtcctgaac acttttgttg taaaaaaata ataataatgt   1500
ttaatattga gaaagaaact aatattttat gtgagagaaa gtgtgagcaa actaacttga   1560
cttttaaggc taaaacttaa cattcataga ggggtggagt tttaactgta aggtgctaca   1620
atgcccctgg atctaccagc ataaatatct tctgatttgt ccctatgcat atcagttgag   1680
cttcatatac cagcaatata tctgaagagc tattatataa aaaccccaaa ctgttgatta   1740
ttagccaggt aatgtgaata aattctatag gaacatatga aaatacaact taaataataa   1800
acagtggaat ataaggaaag caataaatga atgggctgag ctgcctgtaa cttgagagta   1860
gatggtttga gcctgagcag agacatgact cagcctgttc catgaaggca gagccatgga   1920
ccacgcagga agggcctaca gcccatttct ccatacgcac tggtatgtgt ggatgatgct   1980
gccagggcgc catcgccaag taagaaagtg aagcaaatca gaaacttgtg aagtggaaat   2040
gttctaaagg tggtgaggca ataaaaatca tagtactctt tgtagcaaaa ttcttaagta   2100
tgttattttc tgttgaagtt tacaatcaaa ggaaaatagt aatgttttat actgtttact   2160
gaaagaaaaa gacctatgag cacataggac tctagacggc atccagccgg aggccagagc   2220
tgagccctca gcccgggagg caggctccag gcctcagcag gtgcggagcc gtcactgcac   2280
caagtctcac tggctgtcag tatgacattt cacgggagat ttcttgttgc tcaaaaaatg   2340
agctcgcatt tgtcaatgac agtttctttt ttcttactag acctgtaact tttgtaaata   2400
cacatagcat gtaatggtat cttaaagtgt gtttctatgt gacaattttg tacaaatttg   2460
ttattttcca tttttatttc aaaatataca ttcaaactta aaattaaaaa aaaaaaaaaa   2520
aa                                                                  2522
```

<210> 56
<211> 6246
<212> DNA
<213> Homo sapiens

<400> 56

```
gacttaaact ctggggcccg ggaggccgcc ggttttctcc ccgcttgccg gggtggtcct      60
cttccctttg tcggaccaaa gaagtaaaca ctgtgtggag agggactgac gtgtttggag     120
ggaaatggga atgtacacga taattaaaga gtaaagttgt gctcaacgct gtttacttct     180
aattatttcc tgattcacaa attaattttt gcaattcaca acttcaagaa tacttggact     240
attggattca atccatagta atctgcaaaa gaagagaagc agaattatac ctagaagtct     300
```

actaatggac agttagtcac ccacctacat gttgagattc ctgacattca gacaactgac 360
ttgtaactga cttataactg acttgtaata cactgctact atatcaaacc gacaatgaat 420
tggaatgaaa aaccaaagag tgctacatta ccaccactgt atcctaaaag ccagccacct 480
tttttgcacc agtctttaat aaaccaaatt accacaacat ctcagagttc tttcagctat 540
cctggaagta accaagaagc atgcatgtat cccggtaatt caaatccaat ttcacagcca 600
ctgctgaata tccaaaatta tcctcaacaa atttctgttt ctgatatgca taatgggaca 660
gttgtggcct cacacacttc agtagaaaga ataacatatg caaatgttaa tggacccaaa 720
caactaactc acaatttgca gatgtcttca ggagttaccc aaaacgtatg gttgaactca 780
ccaatgagga atcctgtgca ttctcatata ggggcaactg tatctcatca aactgatttt 840
ggagctaacg tacccaatat gccggcacta cagagtcaac tgataacatc agatacctat 900
tctatgcaaa tgcagatgat cccttctaat tctacacgac ttcctgtagc ttaccaagga 960
aatcagggac ttaaccagtc tttttcagag caacaggttg attggacaca acagtgtata 1020
tctaagggac tgacttaccc agattacaga ccacctccaa agctataccg ttactcacca 1080
caaagctttt taccagattc taccattcaa aaacaaaact ttataccaca tacatcattg 1140
caagttaaaa atagtcagct tctaaattct gtattaactt taccatcaag gcagacctca 1200
gctgtaccat cacagcagta tgccacgcaa actgacaaaa gacctcctcc tcctccttac 1260
aactgtagat atggaagcca gcctttgcaa agtactcagc atattactaa acacttgtct 1320
atggaagttc ctcagagtcg agaaatgctg tcatctgaaa taaggaccag ctttcaacag 1380
cagtggcaaa accctaatga aaatgtcagc acaattggaa atttcactaa cttgaaagta 1440
aataccaaca gcaaacagcc ttttaacagt cccattagat cttctgtgga tggtgttcag 1500
actcttgctc aaactaatga agagaaaata atggattctt gcaatccaac ttcaaatcaa 1560
gtactggaca caagtgttgc aaaagaaaag ctagtaaggg atattaaaac attagtagag 1620
ataaaacaga agttttcaga acttgcaagg aaaattaaaa tcaataaaga tcttttgatg 1680
gcagcaggtt gtattaaaat gactaatact tcttatagtg aaccagctca gaattctaaa 1740
ttgtctctaa aacaaactgc caaaatccag tctggacccc agataactcc agtaatgcca 1800
gagaatgcag agagacaaac accaacagta gtggaatctg cagaaacaaa taagactcaa 1860
tgtatgttga attctgacat tcaggaagtc aattgcagaa ggtttaacca agttgattct 1920
gttttaccaa atcctgtcta ttctgaaaag cggccaatgc cagacccatc tcatgatgtg 1980
aaagttctca cttcaaagac atcagctgtt gagatgaccc aggcagtatt gaatactcag 2040

ctttcatcag aaaatgttac caaagttgag caaaattcac cagcagtttg tgaaacaatt 2100
tctgttccca agtccatgtc cactgaggaa tataaatcaa aaattcaaaa tgaaaatatg 2160
ctacttctcg ctttgctttc acaggcacgt aagactcaga agacagtatt aaaagatgct 2220
aatcaaacta ttcaggattc taaaccagac agttgtgaaa tgaatccaaa tacccaaatg 2280
actggtaacc aactgaattt gaagaacatg gaaactccaa gtacttctaa tgtaagtggc 2340
agggttttgg acaactcctt ttgcagtgga caagaatcct caacaaaagg aatgcctgct 2400
aaaagtgaca gtagctgttc catggaagtg ctagcaacct gtctttccct gtggaaaaag 2460
caaccttcag atactgcaaa agaaaaggag tgtgataaac tcagaacaaa cacaacagca 2520
gttggaattt caaagcctgc taacatccac gttaagagtc cttgttcagt tgtgggaaat 2580
tcaaattctc agaataaaat aagtaatccc tcacagcaga cagctttgtc gatggtaatg 2640
cacaattatg agtcttcagg tataaatata acaaagggaa cagaacttca gatagctgta 2700
gtgtcaccgt tagttctgtc agaggtcaaa acattgtctg tcaaaggaat aacacctgca 2760
gtgttacctg aaacagtgta tcccgttatt aaagaaggca gtgtttgtag tttacaaaat 2820
caattggcag aaaatgcaaa ggcaactgct gctttgaaag ttgatgttag tggaccagta 2880
gcaagtacag caacatcaac caagattttt ccactaactc agaaggaaaa gcagaatgag 2940
tcaactaatg gtaattcaga agtcacacct aatgtcaatc aaggaaagca taacaaatta 3000
gagtcagcta tccattctcc tatgaacgat cagcaaatct cacaggagtc aaggaatagt 3060
actgttgtga gtagtgatac attacagatt gacaatattt gttctctggt tgaaggtgat 3120
acctcttaca attcccaaat agcaaagata ttcagctctc ttcccttgaa aatggttgag 3180
ccacagaaac cttctctacc caatcagcaa gggattggca gcagagaacc agaaaaacaa 3240
ttagataata ccactgaaaa taaagacttt ggttttcaaa aagataaacc tgtacagtgc 3300
acagatgttt cacataaaat atgtgatcag tcaaagtcag agccaccctt agagtcatct 3360
tttaacaatc ttgaaacaaa cagagttatt ctagagaaaa gtagtttgga gcatgccact 3420
gaaaaaagca cagctaacga tacgtgctcg tcagctgcta ttcaggagga tatttaccct 3480
caggaaatag atgcatccag caactatact ccccaagatc ctgcaagaaa tgaaatccac 3540
agtgataagg cacctgtctt atacctacat gaccagctgt cagaacttct aaaagagttt 3600
ccttatggca ttgaggctgt gaatacacgt gaaggttctg tgggccagca aactacatac 3660
cagacctcag aagatcaaac tgctgataaa accagttctg actccaaaga cccagcagat 3720
caaatacaaa ttacaatatt aagctcagag caaatgaaag aaatatttcc tgaacaggat 3780
gatcaaccct atgtagtaga caagttggca gaacctcaga aagaagagcc catcacagaa 3840

gtagttagcc agtgtgacct gcaggcacct gcagctggac aaagtcgtga ttctgtgata 3900
ctggactctg agaaagatga tatccactgc tgtgcattgg gctggctctc catggtttac 3960
gaaggagtac cccagtgtca gtgtaattcc atcaagaact catcttcaga ggaagagaaa 4020
caaaaagagc agtgttctcc tttggatacc aacagttgta aacaaggaga gagaacttct 4080
gatagagatg tcactgttgt tcaatttaag agccttgtaa ataatccaaa gactcctcca 4140
gatgggaaaa gtcattttcc tgaactacaa gacgacagta gaaaagatac acccaaaaca 4200
aaacataaaa gcttaccaag gacagaacaa gaattagttg ctggtcagtt ttcatctaaa 4260
tgtgataaac taaatccctt gcaaaatcac aaaagaaaaa aattgaggtt tcacgaggta 4320
acctttcact ccagtaataa aatgacagca tcttatgaac aagcttctca ggaaacccga 4380
cagaagaaac atgtaacaca gaactcacgt ccactaaaaa caaaaacagc tttttttgcca 4440
aataaagatg tgtataagaa gcatagttct ttgggacagt cattatcacc agaaaagata 4500
aaattgaaac tcaaatcagt tagcttcaaa caaaaacgaa agttagacca agggaacgta 4560
ttagatatgg aagtaaagaa aaagaaacat gataaacaag aacagaaagg aagtgtggga 4620
gctacattca aattaggtga ctctttgtca aacccaaacg aaagagccat tgttaaagaa 4680
aagatggtat caaatactaa gtctgtagac acgaaagcga gttcatctaa atttagtaga 4740
attctaactc ctaaggagta tttacaaagg cagaagcata aagaagctct gagtaataaa 4800
gcatcgaaga aaatctgtgt gaaaaacgtg ccatgtgatt ctgaacatat gagaccaagt 4860
aaacttgccg tgcaggttga aagttgtggg aaatcaaatg agaaacacag cagcggcgtg 4920
cagacctcta aagaatcatt aaatggcttg acaagccatg gtaaaaacct caaaatccac 4980
cattctcagg agtctaaaac atacaacatt ctaaggaatg ttaaagaaaa agttggtggg 5040
aagcagcctg ataaaatatg gattgataag actaaattag acaaattaac caatataagc 5100
aacgaagctc aattcagcca aatgcctccc caagtaaagg atcaaaagaa attatatctg 5160
aatagagttg ggtttaaatg cactgaacgt gaaagcattt ctctcaccaa attagaaagt 5220
tcacccagga agcttcataa agataagaga caggaaaata aacataagac ctttttaccg 5280
gtgaaaggta acacagaaaa atcaaacatg ctggagttta aattatgtcc agatatctta 5340
ctaaagaata caaactctgt ggaagaacgg aaggatgtaa agcctcatcc taggaaggag 5400
caagcccctc tgcaagtttc aggaataaaa agtacaaaag aagactggtt aaaatttgtt 5460
gctacaaaga aaaggacaca gaaagacagc caagagagag ataatgttaa ttcaagactc 5520
tcgaagagaa gcttcagtgc agatggattt gagatgctac aaaacccagt aaaagattca 5580

aaagaaatgt ttcaaaccta caaacagatg tacctggaga agagaagcag aagccttggt 5640
agcagtcctg taaaataatt acaagatgtg gttttgtaat tgccactggg aaatttcttt 5700
ccttttctgt tcaaaatatt tcgctgaaac taatgagaaa tgccatgata aagatttctc 5760
agagtttggt tcccactttc attgtatttc attgaaagtg cttaattaaa atggcttgag 5820
aactttgggt agccatgtgt aagaaatgga tggtattcac cggggaaaca aggtatttga 5880
atttctactt tattgaacca gatttaccat tattttaaaa ggaatgctta tacaaatcaa 5940
tttgaaattc tacccatctt gagggaggac cgttcctcag ttaaggactt gtttatttaa 6000
atgggactgt aaatatgttt tggtttctaa gctatattag caaaatttat ttttcaaaaa 6060
tgcccactgt gatgtgaatg tcaaaatata ttcttaagtg ttttataact aattgtaaac 6120
tttttttcag aagtcttatt ttatacttgt gaaactgaac acaattttgg gacaacgttt 6180
aaacattact tttcatactt gaaataaaca tttatttttt aaaaaactaa aaaaaaaaaa 6240
aaaaaa 6246

<210> 57
<211> 807
<212> DNA
<213> Homo sapiens

<400> 57

```
gcgctcgggc tgccgctggc tcttcgcacg cggccatggc cgactccgag ctgcagctgg      60
ttgagcagcg gatccgcagc ttccccgact tccccacccc aggcgtggta ttcagggaca     120
tctcgcccgt cctgaaggac cccgcctcct tccgcgccgc catcggcctc ctggcgcgac     180
acctgaaggc gacccacggg ggccgcatcg actacatcgc aggcctagac tcccgaggct     240
tcctctttgg cccctccctg gcccaggagc ttggactggg ctgcgtgctc atccgaaagc     300
gggggaagct gccaggcccc actctgtggg cctcctattc cctggagtac gggaaggctg     360
agctggagat tcagaaagac gccctggagc caggacagag ggtggtcgtc gtggatgatc     420
tgctggccac tggtggaacc atgaacgctg cctgtgagct gctgggccgc ctgcaggctg     480
aggtcctgga gtgcgtgagc ctggtggagc tgacctcgct taagggcagg gagaagctgg     540
cacctgtacc cttcttctct ctcctgcagt atgagtgacc acagggcctc ccagcccaac     600
atctccagct ggatcccagg gaaatatcag ccttgggcaa ctgcagtgac caggggcacc     660
ggctgcccac agggaacaca ttcctttgct ggggttcagc gcctctcctg gggctggaag     720
tgccaaagcc tggggcaaag ctgtgtttca gccacactga acccaattac acacagcggg     780
agaacgcagt aaacagcttt cccacaa                                         807
```

<210> 58
<211> 673
<212> DNA
<213> Homo sapiens

<400> 58

gcgctcgggc tgccgctggc tcttcgcacg cggccatggc cgactccgag ctgcagctgg 60
ttgagcagcg gatccgcagc ttccccgact tccccacccc aggcgtggta ttcagggaca 120
tctcgcccgt cctgaaggac cccgcctcct tccgcgccgc catcggcctc ctggcgcgac 180
acctgaaggc gacccacggg ggccgcatcg actacatcgc aggcctagac tcccgaggct 240
tcctctttgg cccctccctg gcccaggagc ttggactggg ctgcgtgctc atccgaaagc 300
gggggaagct gccaggcccc actctgtggg cctcctattc cctggagtac gggaaggctg 360
agctggagat tcagaaagac gccctggagc caggacagag ggtggtcgtc gtggatgatc 420
tgctggccac tggtgtatga gtgaccacag ggcctcccag cccaacatct ccagctggat 480
cccagggaaa tatcagcctt gggcaactgc agtgaccagg ggcaccggct gcccacaggg 540
aacacattcc tttgctgggg ttcagcgcct ctcctggggc tggaagtgcc aaagcctggg 600
gcaaagctgt gtttcagcca cactgaaccc aattacacac agcgggagaa cgcagtaaac 660
agctttccca caa 673

<210> 59
<211> 2840
<212> DNA
<213> Homo sapiens

<400> 59

gtttttgtgc aggaacagcc cctcccgtct ttgtcctggc ggtgagcacc cagggctaag 60
cttttgaaca ctttctttgt gtttggattc agcccaggca atgcatattt gctttcattt 120
cttcttgagc ttgaggagct cctgggtgca aatcttggaa aatgaggatc tctgagcctt 180
tccaggccag ctctttgttt tgtagcagac aattgaggct ttgaaaagga aagtgggtgg 240
gggcacccca caggtggccc tcatcaccca attgccagtg cctgcaggct gcttcagcag 300
aggcccagag tcaaagagga cttaaaacca gctgtcgttt ctcccttagc ttctgtgtat 360
gagagaaacg acttctgttt ttcaaagtaa gaacaaggag gaatttgttt ctaaaagaac 420
attaaaacac aggctcgtgg tctaaaagca aatggttcag caggatgttc agggccttaa 480
agcacagtca gcaggactca gcatctccca gcacctgctc tccggttgtc atggtaacat 540
catccccaac ccaaccacct tgtccagccg agagacagca atcataagga gggacctcgg 600
tttcccccga ggatcctggg cttcctttct gaaacgcttg cttctgagct cagcaaccag 660

gaacaccagg ccagcccatc cccagcacct ctgtggagat gagggacaaa gtcctacagt 720
ccctcttcct gttctgatga gaaagggagg gaagaaaaca taccccgagc gcctgcaata 780
tggtcatgac actttcaaaa agcctgtgct atggagtcat gatcagaaac cagagtgtgg 840
agagggtcag cagcctgcct cagagcagcc agctaggcgg ggagtggtaa atttgggact 900
tgtacccagg catgactggc tccgagccca gtgctccact ctatggaatg ttccctgggc 960
ctcagttgct ttcctttcct ttgcaggccg cgggctgctg ccactctggc agctggtgag 1020
ttagctggag ggcaacattc caaagcaggg gcagcatgct gctttcctcc tgtgcccact 1080
cctgcgggga agtccgttga ctcccaccgc tgaagggagc tggcaacacc aggatgaggt 1140
cccaggggac gggagcaggt acccactgtc tgtctacctt cccactggaa aagcacggac 1200
aggccagccc ttgcgggggc aggcagagga cagagttggc tttgcgcggt ctctgcctgc 1260
tgagcagttc caattcctct catgggagaa acaaggaggc agtcgcttgt gcatgttcca 1320
gaagttttac tggggaggag gaagcggaca gaggaagctg tgtgtgcatg tgaaggggtg 1380
ggcagggtgg gagggatgca cgcgtatgtg agcatagcat gtgtgagtac tacacacatc 1440
tccatgcaga agcacaactg ggcagccctg gcttccagct ctgggcttca gcacaacaga 1500
caccagcctg tggtctctca gaagccaggg agaccacatc gggctcagga cgttttaccc 1560
aaagtccaga gtttttatgc ctctccctgg cattctccat aaagaaggga aggtcagatg 1620
accccttaga tctgtgtcat ctgggaattt ccttgggctg gtttagacac gatgccctct 1680
ttttctcagg atagcagata acctgctttg aaagagggct taattctgtg ggtcctaaat 1740
tttctccttt ctctctctct ttctgtgtgt gtgtgttggg aaaatggcaa gtttccaata 1800
ccagctttgg aggaacgatt acgttttccc tccaatttca agtccgaaag accagagccc 1860
tcattccaaa gccccccacc cagatggatt ttttcgtttc atttgtcatc cgtcccatgg 1920
gagggcccca tgtctcctca gaacccatcc tggaggcagc aggtcgggta gagtgagttt 1980
ggcctgctca tgacctccac ccctgagatt gtgaacaagg atgtctgggg cgatgctgag 2040
aatgtttttg aagctgctcc cagatgacgc tgatgatcac accagattga gtgctgcgat 2100
cgccttgagt ccaacctctg cataaacgag gttctcataa acaagttcac tctaccctaa 2160
gctaagtcta tgtgagcaaa cccacttcat cctttgtacc tggagacctg gttacactaa 2220
cctgatactg acctgttcat gtagctggaa tggtgtgttt catgcagtgt ggaccaagca 2280
atggcatggg gtgtgtgtgt gtgtgtgtgt gtgtctgtgt gtgtgtgttt gtgtatgcgt 2340
tcacacttgt gtgtgtatat gtgcatgtag atgctgcata aatgattttt gatgtcaaag 2400

```
acaaacacat tccattgttt taaatattct attatgtaaa caatacgcag agggaccata   2460
tctactcttg tcatattatt tgtgatggta aaacatgcat ttgcaataaa ttaagctttc   2520
tgggaaggca agcagtattg gagccaaacg actgtctcgg aacatgtgtg tgttatctcg   2580
gttcatatca agtccaaagc taatggagcc ttccccgcca tccagggagg aacaccagga   2640
ccccggagtt tcttcttagt gctatatttt aaagttgcat tgacgttttc ctccccttcc   2700
ttttgtgcaa gttggaagta gcagtgttct aaaagatggt ttgacgtttt tgctgttgtt   2760
ttatgttttt aaaaatgtat ctgctttgtg tttggaaata aaaatctcta ttttggtcta   2820
tgaaaaaaaa aaaaaaaaaa                                               2840
```

<210> 60
<211> 2872
<212> DNA
<213> Homo sapiens

<400> 60

```
tggctacatg ctctccccgt gatggggccg gagctgggct ggggacaccc ccgcggcggc     60
gacgtctgct cctctgattc cttcaacgag gacaacactg ccttcgccaa gcaggttcgc    120
tctgagaggc ccttcttctc ctccaaccca gaactggaca atctgatgat ccaggcaatc    180
caagtgctgc ggttccacct gctagagctg gagaagggaa agatgcccat cgacctggtc    240
atcgaggatc gggatggcgg ctgcagggag gacttcgagg actacccagc ctcctgcccc    300
agcctcccag accagaataa tatatggatt cgagaccatg aggatagcgg gtctgtacat    360
ttggggaccc caggtccatc cagcgggggg ctggcctccc agagtgggga caactccagt    420
gaccaaggag tcgggctgga caccagcgtg gcctctccca gttctggtgg agaagatgag    480
gacttggacc aggagccacg gcgaaacaag aagaggggga tcttccccaa ggtggccacc    540
aacatcatgc gagcctggtt gttccagcac ctctcacacc cgtacccctc ggaggagcag    600
aagaaacagc tggcgcagga cacggggctt accatcctgc aagtcaacaa ctggttcatt    660
aatgcccgga gacgcatcgt gcaacctatg atcgatcaat ccaaccgcac agggcagggt    720
gcagccttca gcccagaggg ccagcccatc gggggctata ccgagacaga gccacacgtg    780
gccttccggg ctccagcatc agtggggatg agtttgaact cggaaggaga atggcattat    840
ctatagaggc tgatgcggga gagacccagc ctccggctgt gacccccagc ctcacacctg    900
gctctggttc ccgcctggtc ctctggcttc aggatcccac ctccaaaggc ctctccgctc    960
aacgcctacc tccctagggc cctgctggga catgggggcc tgagtgccca cccaaggggc   1020
tctcaaggac accggcaagg cctccaggct ctgagccccg cttctgcctt cacctctgcc   1080
```

```
tgggacccga gctgggctcc tgggccttgg tccccagaag atggcagcta gggcctcgcc   1140
gcgaggacag agaagggaca gggtggctgg gcagtcaggg aaggagggtc gcccggatcc   1200
gacattttgg agagattctt tcaccctcct gtcccccca cctcccttct ctaatttctt   1260
cttttttttaa tgataaagtc ttaaaacaca gaaaaaaaa agacttcaga gaattaggta   1320
gatgaagcac tttatacagt atatatcttc agcttaaatt tgttttgagt attttttta   1380
tttttaagta ggcaaagatt taaaattttt tatttttagt aaatgtttga agcaaactaa   1440
aaagacgtgg gcaatattta ccaaaacaaa acagaaccca aaaaattgta catcttgatc   1500
ttagcaaata tccttattgt agagacactt aataaagaga tggtatttta atatctgcag   1560
ttctgaggta gggtgtaact tagttctaca ttgtgattta ggaattttta aacctttctt   1620
cttcagggga gaagtgaccc aggcctcgag tttagtgcta aagccactag tgtacttatg   1680
ctgtcaccta accaccacat gcgacatgga ggcagaggct aaatataggg gctttcttaa   1740
gaaagtgaga ggaaattagc aagcattatt agtgtttgac tactgctatc aagtgaattc   1800
aaaggaaaca ggtttttatg ccatatttaa gttacagaaa ccaggcatgc ttagaatagt   1860
ttctaaggtg acagagtgag actccatctc aaaaaaaaaa aaaaaaaaaa gaatagtttc   1920
tagaggttat tggagaatag aaagctaaga aaacttggta tacatttaca attgaaatat   1980
aattacactt tttactctca gaatattatt cagattagac ttcctgttta tcttttatat   2040
tcttgcattg atataatgcc tgatccttca aagttctttc acatattata tgatcttctt   2100
tatgaaaaaa agtagatgct ttattctgat atattcagtt tcccacttta ggcaaaagtg   2160
gattaataga atgatgaatt caaagtagat gaggaaaatc aggcacagag aagtaaaggt   2220
aggtacagac ccaaattcac acaagataat gacatcacca gcgtttaagt tgatcatcaa   2280
aggctgggct ggatttgtct tgctgtatgt gtcaggaaat ttatacctat tacattttcc   2340
attttctcaa aataagtcac atgattgtaa tgtttagctg caacttttct cctaataaat   2400
agtgtcatga agaatgttat agtgcgaagt ttgtacattt cagggtcagt tatacaatgt   2460
gaactcttta tctacgggaa tgagaatgga ggatcattga aggccatgat ataaacaaat   2520
ttgcatgttg aagcctgtat aaaacatggt acagtgagtg aatgccccca tccccaagaa   2580
cactttatac atattaaatg gatatatgat tactgtgcaa aaattcattc tggaaatgaa   2640
catatatttg agcactaata tgtaatgtac atcttctgcc ctaaggagaa aataaatcat   2700
aaaacttgtt ttacattcaa aattactttc ccgagcatgt cttagagtaa tctacgtgtt   2760
gatgtatgta aattgtattt taggtaggca aagaaacaag tctggttatt tatgtaaaaa   2820
ctagtctaat aaagtgagtg gctttatcac tttgaaaaaa aaaaaaaaaa aa           2872
```

<210> 61
<211> 2106
<212> DNA
<213> Homo sapiens

<400> 61

```
accacggccc taaggagggc ggaagtcgtc gctccgtgga gcctcctcct ctcgcgagag      60
gcgcaaggcg tggagtcgac ggctggagag aagccgggag cgagcccagg cggcagtctt     120
gattcccttt tggccagcag tttttaggtc tgtcagtact gcactgcaag aatggcagat     180
tttgggatct ctgctggcca gtttgtggca gtggtctggg ataagtcatc cccagtggag     240
gctctgaaag gtctggtgga taagcttcaa gcgttaaccg gcaatgaggg ccgcgtgtct     300
gtggaaaaca tcaagcagct gttgcaatct gcccacaaag aatccagctt tgacattatt     360
ttgtcaggtt tagtcccagg aagcaccact ctgcacagtg ctgagatttt ggctgaaatc     420
gcccggatcc ttcggcctgg tggatgtctt tttctgaagg agccagtaga gacagctgta     480
gataacaata gcaaagtgaa gacagcatct aagctgtgtt cagccctgac tctttctggt     540
cttgtggaag tgaaagagct gcagcgggag cccctaaccc ctgaggaagt acagtctgtt     600
cgagaacacc ttggtcatga aagtgacaac ctgctgtttg ttcagatcac aggcaaaaaa     660
ccaaactttg aagtgggttc ttctaggcag cttaagcttt ccatcaccaa gaagtcttct     720
ccttcagtga aacctgctgt ggaccctgct gctgccaagc tgtggaccct ctcagccaac     780
gatatggagg acgacagcat ggatctcatt gactcagatg agctgctgga tccagaagat     840
ttgaagaagc cagatccagc ttccctgcgg gctgcttctt gtggggaagg gaaaaagagg     900
aaggcctgta agaactgcac ctgtggcctt gccgaagaac tggaaaaaga gaagtcaagg     960
gaacagatga gctcccaacc caagtcagct tgtggaaact gctacctggg cgatgccttc    1020
cgctgtgcca gctgccccta ccttgggatg ccagccttca aacctgggga aaaggtgctt    1080
ctgagtgata gcaatcttca tgatgcctag gaggttcctg acatgggacc catctgctcc    1140
tccagccaac tcctgtccct cacatcccac catggtggct cctcccacct cctctggatt    1200
tgttcactct gagatctgtt tgcagagtgg gtgcttagca gacagagtga agctggctgg    1260
ggggcacagt ggtgtgtagt gctgctgtgt atcaaaagac caaggtatta tggacctgg     1320
tttcagaatg ggatgggttt cttcacctca tgttaagaga agggagtgtg tcctgaagaa    1380
gcccttcttc tgatgttaaa atgctgacca gaacgctctt gagcccaggc atcgttgagc    1440
attaacactc tgtgacagag ctgcagaccc ctgccttgag tctcatctca gcaatgctgc    1500
caccctcttg tctttcagag ttgttagttt actccattct ttgtgacacg agtcaagtgg    1560
```

```
ctcacaacct cctcagggca ccagaggact cactcactgg ttgctgtgat gatatccagt   1620
gtccctctgc ccccttccat ccccaaccac atttgactgt agcattgcat ctgtgtcctg   1680
ttgtcattta tgttaacctt caggtattaa acttgctgca tatcttgaca tatcttgaga   1740
ttctgcatgt cttgtaaaga gaggggatgt gcatttgtgt gtgatgttgg atagtcatcc   1800
acgctcagtt tggaccattg gaggaactta gtgtcacgca caaatggggc tattcctacg   1860
cttagaatag ggcttgtctg cccactttag aagagtccag gttggtgagc atttagaggg   1920
aagcagggca gaactctgaa cgacaatacg tctctctgag cagagacccc tttgttcttg   1980
ttatccaccc atatggactt ggaatcaatc ttgccaaata tttggagaga ttgtgtggat   2040
ttaagagacc tggattttta tattttacca gtaaataaaa gttttcattg atatctgtcc   2100
ttgaaa                                                              2106
```